# EUROPEAN PATENT APPLICATION

(11) **EP 3 572 078 A1**
(43) Date of publication of application: **27.11.2019**
(21) Application number: 18174145.5
(22) Date of filing: 24.05.2018
(51) Int. Cl.: A61K 31/122, A61K 31/421, A61K 31/4418, A61K 31/4439, A61K 31/454, A61K 36/062, A61K 36/07, A61K 36/09, A61K 36/74, A61P 25/00, A61P 25/28

(54) **TOMM6-INTERACTING EXTRACTS AND COMPOUNDS FOR USE IN THE TREATMENT AND PROPHYLAXIS OF NERVOUS SYSTEM DISEASES**

(71) Applicant: ETH Zurich, 8092 Zurich (CH)
(72) Inventor: QUITTERER, Ursula, 8057 Zürich (CH); ABDALLA, Said, 55122 Mainz (DE)

(57) **Abstract**

The present invention is directed to a TOMM6 (Translocase of Outer Membrane 6kDa subunit homologue; Mitochondrial import receptor subunit TOM6 homolog)-interacting plant or fungal extract comprising an anthraquinone or anthraquinone derivative, a TOMM6-interacting anthraquinone or anthraquinone derivative, and TOMM6-interacting compounds for use in the treatment or prophylaxis of a nervous system disease. Furthermore, the present invention relates to a corresponding method of therapeutic or prophylactic treatment of a nervous system disease or disorder, as well as to a method for the identification of a TOMM6-interacting compound or composition. Also, the present invention provides a non-human transgenic animal expressing a transgenic, preferably human TOMM6 or a TOMM6 homolog.

SEQUENCE LISTING

<110>  ETH Zurich

<120>  TOMM6-interacting extracts and compounds for use in the treatment
        and prophylaxis of nervous system diseases

<130>  2018-059

<160>  18

<170>  PatentIn version 3.5

<210>  1
<211>  74
<212>  PRT
<213>  Homo sapiens

<400>  1

Met Ala Ser Ser Thr Val Pro Val Ser Ala Ala Gly Ser Ala Asn Glu
1               5                   10                  15

Thr Pro Glu Ile Pro Asp Asn Val Gly Asp Trp Leu Arg Gly Val Tyr
            20                  25                  30

Arg Phe Ala Thr Asp Arg Asn Asp Phe Arg Arg Asn Leu Ile Leu Asn
        35                  40                  45

Leu Gly Leu Phe Ala Ala Gly Val Trp Leu Ala Arg Asn Leu Ser Asp
    50                  55                  60

Ile Asp Leu Met Ala Pro Gln Pro Gly Val
65                  70

<210>  2
<211>  74
<212>  PRT
<213>  Mus musculus

<400>  2

Met Ala Ser Ser Gly Val Thr Val Ser Ala Ala Gly Ser Ala Ser Glu
1               5                   10                  15

Ala Ser Glu Val Pro Asp Asn Val Gly Asp Trp Leu Arg Gly Val Phe
            20                  25                  30

Arg Phe Ala Thr Asp Arg Asn Asp Phe Arg Arg Asn Leu Ile Leu Asn
        35                  40                  45

Leu Gly Leu Phe Ala Ala Gly Val Trp Leu Ala Arg Asn Leu Ser Asp
    50                  55                  60

Ile Asp Leu Met Ala Pro Gln Pro Gly Val
65                  70

<210>  3
<211>  63
<212>  PRT
<213>  Danio rerio

<400>  3

Met Ser Gly Lys Lys Ser Glu Gly Lys Ser Gly Gly Val Thr Gly Trp
1               5                   10                  15

Ile Ser Ser Ala Cys Arg Phe Ala Ala Asp Arg Asn Asp Phe Arg Arg
            20                  25                  30

Asn Leu Leu Val Asn Leu Gly Leu Phe Ala Ala Gly Val Trp Val Ala
        35                  40                  45

Arg Asn Leu Ser Asp Phe Asp Leu Met Ser Pro Gln Pro Val Thr
    50                  55                  60

<210>  4
<211>  74
<212>  PRT
<213>  Mus musculus

<400>  4

Met Ala Ser Ser Gly Val Thr Val Ser Ala Ala Gly Ser Ala Ser Glu
1               5                   10                  15

Ala Ser Glu Val Pro Asp Asn Val Gly Asp Trp Leu Arg Gly Val Phe
            20                  25                  30

Arg Phe Ala Thr Asp Arg Asn Asp Phe Arg Arg Asn Leu Ile Leu Asn
        35                  40                  45

Leu Gly Leu Phe Ala Ala Gly Val Trp Leu Ala Arg Asn Leu Ser Asp
    50                  55                  60

Ile Asp Leu Met Ala Pro Gln Pro Gly Val
65                  70

<210>  5
<211>  14
<212>  PRT
<213>  Mus musculus

<400>  5

Asn Leu Ser Asp Ile Asp Leu Met Ala Pro Gln Pro Gly Val
1               5                   10

<210>  6
<211>  14
<212>  PRT
<213>  Artificial Sequence

<220>
<223>  Partial peptide sequence of mouse TOMM6 with oxidized M


<220>
<221>  MISC_FEATURE
<222>  (8)..(8)
<223>  oxidized

<400>  6

Asn Leu Ser Asp Ile Asp Leu Met Ala Pro Gln Pro Gly Val
1               5                   10


<210>  7
<211>  15
<212>  PRT
<213>  Artificial Sequence

<220>
<223>  Partial peptide sequence of mouse TOMM6 with oxidized M


<220>
<221>  MISC_FEATURE
<222>  (9)..(9)
<223>  oxidized

<400>  7

Arg Asn Leu Ser Asp Ile Asp Leu Met Ala Pro Gln Pro Gly Val
1               5                   10                  15


<210>  8
<211>  348
<212>  DNA
<213>  Artificial Sequence

<220>
<223>  Partial sequence of vector pcDNA3.1 carrying the human TOMM6 gene

<400>  8
ccactagtaa cggccgccag tgtgctggaa ttcatggctt ccagcactgt cccggtgagc      60

gctgctggct cggctaatga aactcccgaa ataccggaca acgtgggaga ttggcttcgg     120

ggcgtctacc gctttgccac tgataggaat gacttccgga ggaacttgat actaaatttg     180

ggactctttg ctgcgggagt ttggctggcc aggaacttga gtgacattga cctcatggca     240

cctcagccag gggtgtagct cgagcatgca tctagagggc cctattctat agtgtcacct     300

aaatgctaga gctcgctgat cagcctcgac tgtgccttct agttgcca                  348

<210>  9
<211>  34
<212>  DNA
<213>  Artificial Sequence

<220>
<223>  primer TOMM6 forward

<400>  9
tatttagaat tcatggcttc cagcactgtc ccgg                                    34


<210>  10
<211>  34
<212>  DNA
<213>  Artificial Sequence

<220>
<223>  primer TOMM6 reverse

<400>  10
tatttactcg agctacaccc ctggctgagg tgcc                                    34


<210>  11
<211>  30
<212>  DNA
<213>  Artificial Sequence

<220>
<223>  primer TOMM6-internal forward

<400>  11
ggtgagcgct gctggctcgg ctaatgaaac                                         30


<210>  12
<211>  30
<212>  DNA
<213>  Artificial Sequence

<220>
<223>  primer pcDNA3-reverse

<400>  12
tagaaggcac agtcgaggct gatcagcgag                                         30


<210>  13
<211>  429
<212>  DNA
<213>  Artificial Sequence

<220>
<223>  Partial sequence of vector CosSHa.Tet carrying the human TOMM6
      gene

<400>  13
gtcacacatt tgttctgcaa cagatgtcaa gtgcttcagc ctgcgtgctg gacaatgacg      60

tgttgctgga gtacaatgat gccttgttct tcattttgca gatcagtcga caccatggct     120

tccagcactg tcccggtgag cgctgctggc tcggctaatg aaactcccga aataccggac 180

aacgtgggag attggcttcg gggcgtctac cgctttgcca ctgataggaa tgacttccgg 240

aggaacttga tactaaattt gggactcttt gctgcgggag tttggctggc caggaacttg 300

agtgacattg acctcatggc acctcagcca ggggtgtagg tcgacctccc tgcttgtact 360

tcctcgttct tgtggtctag gctgggggag gggttatcca ccgtagctct tttaattgag 420

gtggtggct 429

<210> 14
<211> 30
<212> DNA
<213> Artificial Sequence

<220>
<223> primer CosSHa.Tet-forward

<400> 14
gcttcagcct gcgtgctgga caatgacgtg 30

<210> 15
<211> 31
<212> DNA
<213> Artificial Sequence

<220>
<223> primer TOMM6-reverse

<400> 15
cctggctgag gtgccatgag gtcaatgtca c 31

<210> 16
<211> 28
<212> DNA
<213> Artificial Sequence

<220>
<223> primer Prp-forward

<400> 16
aagcggccaa agcctggagg gtggaaca 28

<210> 17
<211> 25
<212> DNA
<213> Artificial Sequence

<220>
<223> primer APP-forward

<400> 17
ctgaccactc gaccaggttc tgggt 25

<210> 18
<211> 29

<212>  DNA
<213>  Artificial Sequence

<220>
<223>  primer Prp-reverse

<400>  18
gtggataacc cctcccccag cctagacca                                          29

## Description

The present invention is directed to a TOMM6 (Translocase of Outer Membrane 6kDa subunit homologue; Mitochondrial import receptor subunit TOM6 homolog)-interacting plant or fungal extract comprising an anthraquinone or anthraquinone derivative, a TOMM6-interacting anthraquinone or anthraquinone derivative, and TOMM6-interacting compounds for use in the treatment or prophylaxis of a nervous system disease. Furthermore, the present invention relates to a corresponding method of therapeutic or prophylactic treatment of a nervous system disease or disorder, as well as to a method for the identification of a TOMM6-interacting compound or composition. Also, the present invention provides a non-human transgenic animal expressing a transgenic, preferably human TOMM6 or a TOMM6 homolog.

Nervous system diseases or disorders, also known as nervous system or neurological diseases or disorders, refer to medical conditions affecting the nervous system. This category encompasses central nervous and peripheral nervous diseases or disorders, including genetic disorders, seizure disorders (such as epilepsy), conditions of cardiovascular origin (such as stroke), congenital and developmental disorders (such as spina bifida), degenerative disorders such as multiple sclerosis (MS), Parkinson's disease (PD), and amyotrophic lateral sclerosis (ALS) and forms of dementia such as vascular dementia, Lewy Body dementia, frontotemporal dementia, and Alzheimer's disease (AD). Currently, there is a wide range of treatments for central and peripheral nervous system diseases and disorders. These can range from surgery to neural rehabilitation or prescribed medications.

AD is the most frequent form of dementia and on the rise worldwide. Major neuropathological features related to AD and other nervous system diseases are characterized by protein aggregation, neurotoxic tau filaments and mitochondrial dysfunction.

Age is the best-established non-genetic risk factor for dementia and AD, and with increasing life expectancy, the incidence of dementia is on the rise world-wide. Currently, there is no cure for dementia, including AD. There are only four different drugs approved for the treatment of AD: three different acetylcholinesterase inhibitors, which enhance the availability of the cognition-enhancing acetylcholine, and the NMDA receptor antagonist memantine (Kulshreshtha and Piplani, Neurol. Sci. 37, 1403-1435 (2016)). All of these drugs have major side effects. Also, the currently used drugs can only retard the disease progression by several months and cannot modify disease progression and/or relief AD symptoms only for a short time period. Therefore, there is an urgent need for disease-modifying treatment approaches for AD. A possible target is the aberrant protein aggregation process leading finally to Abeta plaque formation. However, approaches that only interfere with Abeta plaque formation have not demonstrated efficacy in retarding AD progression, and even showed major side effects (Kulshreshtha and Piplani, Neurol. Sci. 37, 1403-1435 (2016)). The underlying reason could be the fact that the sole increase in Abeta aggregates does not necessarily cause substantial neuronal loss (Calderon-Garciduenas & Duyckaerts, Handb. Clin. Neurol. 145, 325-337, 2017).

TOMM6 (Translocase of Outer Membrane 6kDa subunit homolog; Mitochondrial import receptor subunit TOM6 homolog) is an essential component of the TOMM machinery, i.e. the multi-subunit translocases in the outer mitochondrial membrane, which are required for the import of nucleus-encoded precursor proteins. The TOMM machinery contains import receptors for the binding of cytosolically synthesized preproteins and the general import pore (GIP). Tomm20, Tomm22 and Tomm70 are the import receptors for preproteins. These receptors are attached to the other components of the Tom machinery, which form the core complex. Tomm6 is a subunit of the core complex, which together with the other subunits (Tomm40, Tomm22, Tomm7, Tomm5) is embedded in the outer membrane and forms the translocation pore (Dembowski et al., J. Biol. Chem. 276, 17679-17685, 2001). Tomm6 stabilizes the interaction between the receptors, specifically Tomm22, and the general insertion pore (Dekker et al., Mol. Cell. Biol. 18, 6515-6525, 1998). However, the *in vivo* role of TOMM6 was not investigated so far. Moreover, a pathophysiological role of TOMM6 has not been established.

A pathophysiological relevance between the Tomm machinery and late-onset Alzheimer's was proposed for TOMM40 with the S/VL and VL/VL (VL: very long) genotype of TOMM40 being reported to result in a higher expression of TOMM40 and apparently in a better protection against the APOE e3/3 background (Zeitlow et al., Biochim. Biophys. Acta 1863, 2973-2986, 2017). However, several studies did not replicate this observation, and the possible association between the TOMM40 genotype and AD is still under discussion (Roses et al., Alzheimers Dement 12, 687-694, 2016). The complexity of gene polymorphism-disease association could be the reason for difficulties to reproduce the initial data. These problems are also reflected by the fact that translation of genetic data into identification of novel targets for drug treatment of AD usually has not occurred (Lutz et al., Curr. Neurol. Neurosci. Rep 16, 48 (2016)). Thus, there have been no reports of successful treatment approaches for dementia or AD that involve TOMM40 and/or the TOMM machinery. In view of ongoing discussions about the relationship between TOMM40 genotypes and late-onset AD, researchers ask for more basic science data (Roses et al., Alzheimers Dement 12, 687-694, 2016). In this respect, Tomm40 knockout mice could help to elucidate the function of Tomm40 *in vivo.* Knockout of Tomm40 in mice is lethal. Unpublished data are available from heterozygous Tomm40 knockout mice, which have a reduced performance, a 30 % higher mortality than wild-type mice and motor defects (unpublished data by R. Zeh were cited by Zeitlow et al., Biochim. Biophys. Acta 1863, 2973-2986, 2017). However, aged heterozygous Tomm40 knockout mice did not develop defects of behaviour, learning, memory and/or other typical symptoms of AD (R. Zeh., unpublished). These observations further demonstrate that to date there is no established causality between TOMM40 or any other member of the TOMM machinery with neurodegeneration and/or AD.

Anthraquinones (also called anthracenediones) and specifically 9,10-anthraquinones are used as digester additive in the production of paper pulp by alkaline processes, like the Kraft-, the alkaline sulfite- or the Soda-AQ-processes. Anthraquinones also have utility as drugs, in particular as laxatives (e.g. dantron, emodin, aloe emodin, and some of the senna glycosides), anti-malarials (e.g. rufigallol), antineoplastics (treatment of cancer, mitoxantrone, pixantrone, and the anthracyclines), and DNA dyes / nuclear counterstains (e.g. DRAQ5, DRAQ7 and CyTRAK Orange for use in flow cytometry and fluorescence microscopy). However, anthraquinones such as, e.g. rhein, emodin, aloe emodin, Physcion, and Chrysophanol can also be toxic, e.g. by causing hepatomyoencephalopathy in children.

Some natural pigments are anthraquinones and derivatives of anthraquinone, which are found, e.g., in aloe latex, senna, rhubarb, cascara buckthorn, fungi, lichens, and some insects. Generally, anthraquinone-comprising plant families include but are not limited to *Rubiaceae, Verbenaceae, Bignoniaceae, Rhamnaceae, Polygonaceae, Leguminosae, Scropulariaceae, Fabaceae,* and *Liliaceae.*

The *Rubiaceae* are a family of flowering plants, commonly known as the coffee, madder, or bedstraw family. This family is mostly known as a source of a variety of alkaloids, the most familiar of which is quinine, one of the first agents effective in treating malaria. Woodruff *(Galium odoratum*) is a small herbaceous perennial that contains coumarin, a natural precursor of warfarin, and the South American plant *Carapichea ipecacuanha* is the source of the emetic ipecac. The leaves of the Kratom plant (*Mitragyna speciosa*) contain a variety of alkaloids, including several psychoactive alkaloids and are traditionally prepared and consumed in Southeast Asia for both painkilling and stimulant qualities. It functions as a µ-opioid receptor agonist, and is often used in traditional Thai medicine similar to opioids, and often as a replacement for opioid pain-killers like morphine.

The *Galium* and *Rubia* genera of the *Rubiaceae* family are known to comprise glycosides of colored anthraquinone derivatives and related hydroxyanthraquinone compounds (Hill and Richter, J. Chem. Soc. 1714-1719, 1936). There are several studies, which show antioxidant effects of *Rubia cordifolia* L. root extracts, which contain a wide variety of non-anthraquinone antioxidants, e.g. mollugin, terpenes, glycosides etc. (Priya and Siril, Int. J. Pharm. Sci. Rev. Res. 25, 154-164, 2014). Antioxidant effects are comparable to vitamin E and C (Priya and Siril, Int. J. Pharm. Sci. Rev. Res. 25, 154-164, 2014). However, a panoply of data from controlled clinical trials shows that the sole treatment with antioxidants has no significant effect in patients with nervous system diseases such as, e.g., dementia, AD and cardiovascular diseases (Farina et al., Cochrane Database Syst. Rev. 4:CD002854, 2017; Gugliandolo et al., Int. J. Mol. Sci. 18, E2594, 2017; Reddy AP & Reddy PH, Prog. Mol. Biol. Transl. Sci. 146, 173-201, 2017; Baradaran et al., J. Res. Med. Sci. 19, 358-367, 2014).

Chitra and Kumar (Int. J. Pharm Tech Res. 1, 1000-1009, 2009) investigated the effect of *Rubia cordifolia L.* root extract in mice, which received intracerebroventricular injection of a synthetic peptide, Abeta25-35, to cause neuronal damage. It is true that the synthetic peptide Abeta25-35 causes neurotoxicity and neuronal cell death. But for the following reasons, the acute brain damage induced in this mouse model has nothing to do with the human Alzheimer's disease in patients. (1) Chitra and Kumar used a synthetic peptide, the Abeta25-35 peptide. (2) This synthetic Abeta25-35 peptide does **not** occur in human brain under physiological conditions (Kubo et al., J. Neurosci. Res. 70, 474-483 (2002)). (3). Moreover, this synthetic Abeta25-35 peptide does **not** occur in brains of AD patients (Kubo et al., J. Neurosci. Res. 70, 474-483, 2002). (4) In contrast to the synthetic Abeta25-35 peptide used by Chitra and Kumar, only the protease-resistant [D-Ser26]-Abeta25-35/40 fragment accumulates over years in low quantities in brains of AD patients (Kubo et al., J. Neurosci. Res. 70, 474-483, 2002). (5) This naturally occurring beta-amyloid [D-Ser26]-Abeta25-35/40 fragment, which is racemized at Serine26 residue to yield D-Serine26, is highly resistant to degradation by proteinases and therefore can accumulate during the pathogenesis of AD, which takes decades to develop (Kaneko et al., Neuroscience 104, 1003-1011, 2001). (6) Because Chitra and Kumar tested the acute neurotoxicity of a single injection of a synthetic Abeta25-35 peptide, which is not detectable in healthy or diseased human brain, neither without nor with Alzheimer's disease, this mouse model is irrelevant for the pathogenesis of AD in humans, which requires decades to develop. For the same reasons, data obtained with this model are also irrelevant for the treatment of patients with AD. Hence, this study has no relevance for the human Alzheimer disease and AD patients.

It is the objective of the present invention to provide new means for use in the treatment and/or prophylaxis of a nervous system disease or disorder, preferably a human nervous system disease or disorder, preferably a central nervous system (CNS) or peripheral nervous system (PNS) disease or disorder.

In a first aspect, the present invention is directed to a TOMM6 (Translocase of Outer Membrane 6kDa subunit homologue)-interacting compound or composition selected from the group consisting of
(i) a plant or fungal extract comprising an anthraquinone or anthraquinone derivative;
(ii) an anthraquinone or anthraquinone derivative; and
(iii) a compound selected from the group consisting of compounds of Formula (I) and (II), 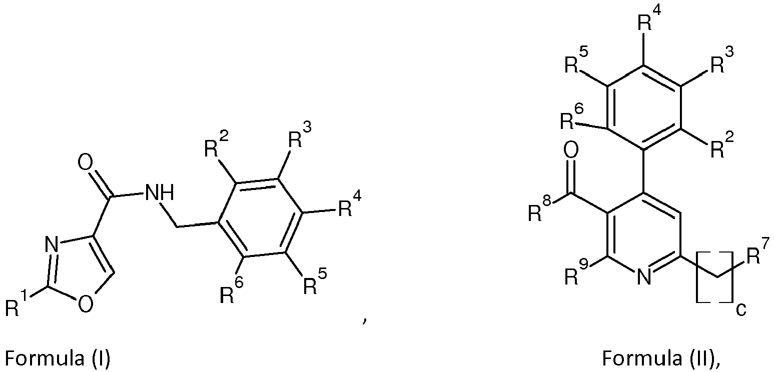 wherein
   **R¹** is selected from the group consisting of
      (i) H, F, Cl, Br, or -OH;
      (ii) -O(C₁₋₁₀)alkyl, preferably -OMe and -OEt, -O(C₃₋₁₀)cycloalkyl, -O(C=O)(C₁₋₁₀)alkyl, preferably-O(C=O)Me, or -O(C=O)(C₃₋₁₀)cycloalkyl;
      (iii) -COOH, -COO(C₁₋₁₀)alkyl, -COO(C₃₋₆)cycloalkyl, -COONH₂, -COON((C₁₋₁₀)alkyl)₂, -COONH(C₁₋₁₀)alkyl, preferably -COOH, -COOMe or COOEt;
      (iv) linear or branched, substituted or non-substituted (C₂₋₁₀)alkyl ether, (C₃₋₁₀)alkenyl ether, (C₃₋₁₀)alkynyl ether or (C₄₋₁₀)carbocyclic ether, wherein the ether is bonded to formula (I) via its carbon atom;
      (v) linear or branched, substituted or non-substituted (C₁₋₁₀)alkyl, (C₂₋₁₀)alkenyl, (C₂₋₁₀)alkynyl, preferably (C₁₋₁₀)alkyl, more preferably substituted or non-substituted -CHO, butyl or pentyl;
      (vi) substituted or non-substituted carbocycle selected from the group consisting of (C₃₋₁₀)carbocycle, preferably (C₃)carbocycle and (C₅₋₆)carbocycle, preferably aromatic (C₆)carbocycle, more preferably a non-substituted phenyl or a phenyl that is substituted by a substituent selected from the group consisting of Cl, F, Br, substituted or non-substituted methyl, preferably -(CF₃), ethyl, propyl and cyclopropyl;
   (vii) substituted or non-substituted (C₃₋₆)heterocycle or (C₇₋₁₀)carbo- or hetero-bicycle having 1 to 3 heteroatoms each independently selected from N, O and S, preferably substituted or non-substituted heterocycle having 1 nitrogen atom, preferably substituted or non-substituted piperidine, most preferably piperidine-3-yl,
   preferably R¹ is selected from the group consisting of 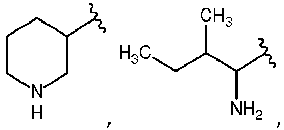 and 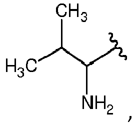 more preferably 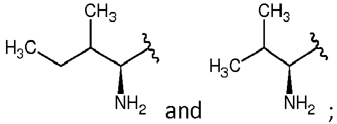
   **R¹** to **R⁶** are each independently selected from the group consisting of H, Cl, Br, F, -OMe, -OEt,-O(C=O)Me, or -OH,
   preferably for Formula (I), R⁵ is Cl and R² to R⁴ and R⁶ are H, more preferably R³ and R⁴ are Cl and R², R⁵ and R⁶ are H,
   preferably for Formula (II), R⁴ is Cl and R², R³, R⁵ and R⁶ are H, more preferably R² and R⁴ are Cl and R³, R⁵ and R⁶ are H;
   **R⁷** is selected from the group consisting of
      (i) NH₂, NH(C₁₋₁₀)alkyl, -OH, or -O(C₁₋₁₀)alkyl, preferably -NH₂ or -OH;
      (ii) substituted or non-substituted carbocycle selected from the group consisting of (C₃₋₁₀)carbocycle, preferably (C₃)carbocycle and (C₅₋₆)carbocycle, preferably aromatic (C₆)carbocycle, more preferably a non-substituted phenyl or a phenyl that is substituted by a substituent selected from the group consisting of Cl, F, Br, substituted or non-substituted methyl, preferably -(CF₃), ethyl, propyl and cyclopropyl; and
      (iii) substituted or non-substituted (C₃₋₆)heterocycle or (C₇₋₁₀)carbo- or hetero-bicycle having 1 to 3 heteroatoms each independently selected from N, O and S, preferably substituted or non-substituted heterobicycle having 1 nitrogen atom, more preferably isoindoline-1,3-dione, most preferably 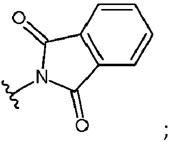
   **R⁸** is selected from the group consisting of -NH₂, NH(C₁₋₁₀)alkyl, -OH, or -O(C₁₋₁₀)alkyl, preferably -NH₂;
   **R⁹** is selected from the group consisting of H, Me and Et; and/or
   c is an integer between 1 and 4
   and pharmaceutically acceptable salts and solvates thereof for use in the treatment or prophylaxis of a nervous system disease or disorder, preferably a human nervous system disease or disorder, preferably a central nervous system (CNS) or peripheral nervous system (PNS) disease or disorder.

It was surprisingly found that interaction, e.g. binding, induction, stabilization and/or activation, of TOMM6 with a compound or composition for use in the present invention, a plant or fungal extract comprising an anthraquinone or anthraquinone derivative and/or an anthraquinone or anthraquinone derivative for use in the present invention is sufficient to prevent mitochondrial dysfunction, toxic protein aggregation, Abeta plaque formation, PHF-tau formation and neuronal loss.

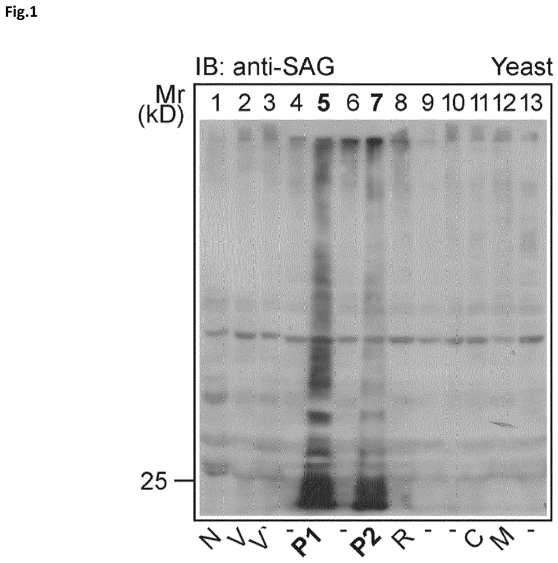

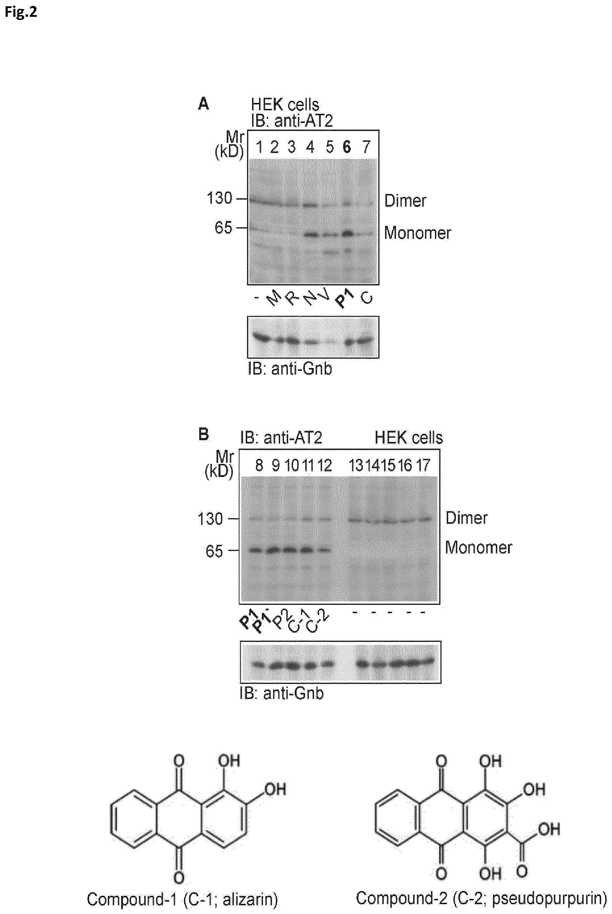

For example, among different plant extracts, the extract P1 from *Galium aparine* and P2 from the related *Galium verum,* inhibited HBsAg (surface antigen of the hepatitis B virus, HBV) aggregation and promoted the appearance of monomeric HBsAg in a non-Abeta-based protein aggregation assay system, which was demonstrated by immunoblot detection of HBsAg (see Example 2, Figure 1 below). Also, representative extracts for use in the present invention reduced the formation of stable AT2 receptor (angiotensin II receptor type 2; AGTR2; AT2R) dimers in human cells (see Figure 2A below) and AT2R dimerization/aggregation was prevented with two different extract preparations from *Galium aparine,* and with an extract from the related *Galium verum* (see Figure 2B below).

It was also surprisingly found that anthraquinones and anthraquinone derivatives signifycantly retarded the accumulation of SDS-insoluble Abeta1-40 and Abeta1-42 and significantly decreased the content of hyperphosphorylated PHF-Tau in the hippocampus of AD model mice (see Examples 4 and 5 below). Hence, it was found that anthraquinone-containing extracts, anthraquinones and anthraquinone derivatives are neuroprotective and prevent neuronal loss in the Tg-2576 genetic model of familial AD.

Furthermore, it was surprisingly found that the extracts, anthraquinones and anthraquinone derivatives of the present invention interact with and preferably stabilize TOMM6, which is sufficient to promote neuroprotection against major neuropathological features in the hippocampus of Tg2576 AD mice, i.e. accumulation of insoluble Abeta peptides, formation of hyperphosphorylated PHF-Tau and overt neuronal loss (see Examples 9 to 12 below). Hence, the interaction with TOMM6 is the common principle of action of the extracts and compounds of the present invention which results in their utility in the treatment or prophylaxis of a nervous system disease or disorder, preferably a human nervous system disease or disorder, preferably a central nervous system (CNS) or peripheral nervous system (PNS) disease or disorder.

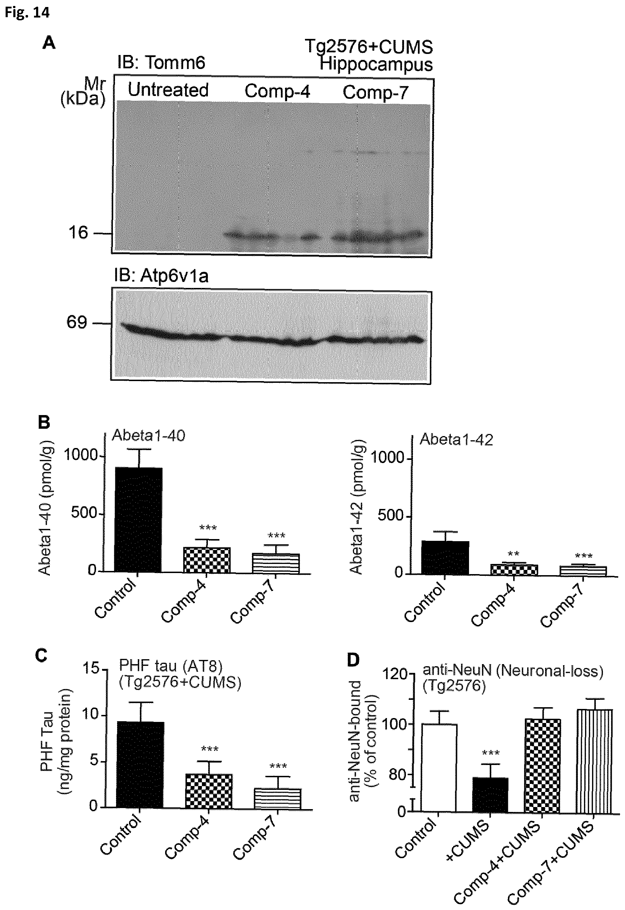

Last but not least, compounds of formula (I) and (II) were identified as TOMM6 -interacting compounds (Examples 13 and 14 below) which induce TOMM6 and, thus, decrease hippocampal PHF tau hyperphosphorylation (Figure 14C) and retard overt neuronal loss in AD model mice, i.e. retard major symptoms of AD and neurodegeneration *in vivo.*

In the context of the present invention, the term "TOMM6" refers to the Translocase of Outer Membrane 6kDa subunit homolog and the Mitochondrial import receptor subunit TOMM6 homolog, which are synonyms. Preferably, TOMM6 is a protein comprising, preferably consisting of an amino acid sequence selected from the group of SEQ ID NO: 1, 2 and 3. The term "TOM-M6" also preferably encompasses homologs of TOMM6, termed "TOMM6 homolog(s)", with TOMM6 activity that comprise, preferably consist of an amino acid sequence having a sequence identity of at least 85%, preferably 90%, more preferably 95%, most preferably 98% sequence identity with the amino acid sequence of TOMM6, preferably with an amino acid sequence selected from the group consisting of SEQ ID NO: 1, 2 and 3.

The percentage identity of related amino acid molecules can be determined with the assistance of known methods. In general, special computer programs are employed that use algorithms adapted to accommodate the specific needs of this task. Preferred methods for determining identity begin with the generation of the largest degree of identity among the sequences to be compared. Preferred computer programs for determining the identity among two amino acid sequences comprise, but are not limited to, TBLASTN, BLASTP, BLASTX, TBLASTX (Altschul et al., J. Mol. Biol., 215, 403-410, 1990), or ClustalW (Larkin MA et al., Bioinformatics, 23, 2947-2948, 2007). The BLAST programs can be obtained from the National Center for Biotechnology Information (NCBI) and from other sources (BLAST handbook, Altschul et al., NCB NLM NIH Bethesda, MD 20894). The ClustalW program can be obtained from http://www.clustal.org.

In the context of the present invention, the term "TOMM6-interacting compound or composition" is meant to encompass any compound or composition that (i) directly or indirectly binds to TOMM6 and thus stabilizes and/or enhances its physiological activity, preferably vs. an untreated control (as can be determined, e.g., in the assay of Example 9 below), and/or (ii) induces the expression (increases the protein content) of TOMM6 in a cell, preferably vs. an untreated control (as can be determined, e.g. in an immunoblot assay using anti-TOMM6 antibodies, e.g. as described in Example 13 below). The terms "induction, stabilization and/or activation of TOMM6", as used herein, also refer to any stabilization and/or enhancement of TOMM6's physiological activity and/or induction of expression of TOMM6 as defined above.

A "plant or fungal extract comprising an anthraquinone or anthraquinone derivative" for use in the present invention can be any extract from any part of a plant or fungus that comprises an anthraquinone or anthraquinone derivative, preferably in a detectable amount, more preferably in a physiologically effective amount, e.g. by detection with gas chromatography, HPLC, TLC, NMR, mass spectrometry or any known method suitable for detecting anthraquinones or anthraquinone derivatives. Preferably, the extract is a plant extract from fresh or dried plant material, preferably powdered plant material, more preferably from fresh or dried roots. In a preferred embodiment, the extract is selected from the group consisting of a hydrophilic extract, a hydrophobic extract, preferably an alcoholic extract, preferably ethanolic extract, methanolic extract, diethyl ether extract, chloroform extract, and hot water extract. Mixtures of different solvents (e.g ethanol with water, methanol with water) can also be used for extraction. Also preferred are extracts prepared by carbon dioxide based, preferably critical or supercritical carbon dioxide based extraction. The extract can be dried, e.g. by freeze-drying, evaporation, and/or formulated as a powder, compressed as pellets, and/or formulated as instant tea. The powdered plant extract can also be encapsulated, or compressed as tablets. The extract can also be formulated and used as a dietary supplement. Inactive ingredients can be added, which are required for (drug) formulation and delivery.

In a preferred embodiment, the extract for use in the present invention comprises at least 0.01 weight-%, preferably 0.1-1.0 weight-%, more preferably at least 5-10 weight-%, anthraquinones or anthraquinone derivatives.

The term "anthraquinone or anthraquinone derivative", as used herein, refers to (i) 9,10-anthracenedione, (ii) any compound that comprises the structural ring scaffold of 9,10-anthracenedione, either aromatic or (partially) saturated, and has one or more or all hydrogen atoms replaced by other atoms or functional groups, (iii) anthracenediones, and/or (iv) any compound falling under the general formulae (III), (IV) and/or (V) as described below.

In the below Examples, it is demonstrated that the representative plant extracts P1 and P3 from anthraquinone-containing *Galium* and *Rubia* genera, and alizarin (Compound-1) and pseudopurpurin (Compound-2) as representative anthraquinones/anthraquinone derivatives derived from the *Galium* and *Rubia* genera of the Rubiaceae family are potent TOMM6-interacting compounds and compositions, e.g. by stabilizing and/or inducing TOMM6 in Tg2576 mice. Thus, the neuroprotective effects of *Galium* and *Rubia*-derived anthraquinones are related to the newly identified neuroprotective and mitochondrial-protective activity of TOMM6.

It is noted that the compounds selected from the group consisting of Formula (I) and (II) are TOMM6-inducing compounds, which are generally devoid of any antioxidant activity. Hence, an antioxidant activity of TOMM6-interacting compounds is not a prerequisite for the technical effect of the compounds and extracts for use in the present invention. Moreover, the compounds selected from the group consisting of Formulae (I) and (II) without antioxidant activity also show neuroprotective activity and prevent neuronal loss in AD models. Thus, cell-protective TOMM6-stabilizing activity of small molecule compounds does not require antioxidant activity.

In summary, the Examples herein demonstrate that TOMM6-interaction (e.g. induction, stabilization and/or activation) by the anthraquinones or anthraquinone derivatives (e.g. Compounds 1,2, and the below-listed anthraquinone compounds), or by compounds selected from the group consisting of Formulae (I) and (II) (e.g. Compounds 3-10) is sufficient to prevent mitochondrial dysfunction, toxic protein aggregation, Abeta plaque formation, PHF-tau formation and neuronal loss. As noted above, the antioxidant activity of anthraquinones is not a necessary condition for the technical effect of the present invention, and the sole treatment with antioxidants, e.g. vitamin E, has no substantial effect in patients with dementia and AD (Farina et al., Cochrane Database Syst. Rev. 4:CD002854, 2017; Gugliandolo et al., Int. J. Mol. Sci. 18, E2594, 2017; Reddy AP and Reddy PH, Prog. Mol. Biol. Transl. Sci. 146, 173-201, 2017).

In a preferred embodiment, the extract for use in the present invention is prepared from a plant selected from the group of plant families consisting of *Rubiaceae, Verbenaceae, Bignoniaceae, Rhamnaceae, Polygonaceae, Leguminosae, Scropulariaceae, Fabaceae,* and *Liliaceae.*

In a further preferred embodiment, the extract for use in the present invention is prepared from a plant selected from the group of plant genera consisting of *Rubia*; *Galium; Rhamnus; Ventilago*; *Rheum; Rumex; Polygonum; Cassia*; *Senna*; *Andira,* and *Aloe.*

In another preferred embodiment, the extract for use in the present invention is prepared from a plant selected from the group consisting of *Galium abaujense* Borbás; *Galium abruptorum* Pomel; *Galium absurdum* Krendl; *Galium achurense* Grossh.; *Galium acrophyum* Hochst. ex Chiov.; *Galium acuminatum* Ball; *Galium acutum* Edgew.; *Galium adhaerens* Boiss. & Balansa; *Galium advenum* Krendl; *Galium aegeum* (Stoj. & Kitam.) Ancev; *Galium aetnicum* Biv.; *Galium afropusillum* Ehrend.; *Galium agrophilum* Krendl; *Galium aladaghense* Parolly; *Galium* ×*albertii* Rouy (hybrid from *Galium boreale* × *Galium verum*; *Galium albescens* Hook. f.; *Galium album* Mill.; *Galium album* Mill. subsp. *album*; *Galium album* subsp. *amani* Ehrend. & Schönb.-Tem.; *Galium album* subsp. *prusense* (K.Koch) Ehrend. & Krendl; *Galium album* subsp. *pycnotrichum* (Haw. ex Schult. & Schult. f.) Krendl.; *Galium album* subsp. *suberectum* (Klokov) Michalk.; *Galium amatymbicum* Eckl. & Zeyh.; *Galium amblyophyllum* Schrenk; *Galium amorginum* Halácsy; *Galium andrewsii* A.Gray; *Galium andringitrense* Homolle ex Puff; *Galium anfractum* Sommier & Levier; *Galium anguineum* Ehrend. & Schönb.-Tem.; *Galium angulosum* A.Gray; *Galium angustifolium* Nutt.; *Galium angustissimum* (Hausskn. ex Bornm.) Ehrend.; *Galium anisophyllon* Vill.; *Galium ankaratrense* Homolle ex Puff; *Galium antarcticum* Hook. f.; *Galium antitauricum* Ehrend.; *Galium antuneziae* Dempster; *Galium aparine* L.; *Galium aparinoides* Forssk.; *Galium aragonesii* Sennen; *Galium araucanum* Phil.; *Galium arenarium* Loisel.; *Galium arequipicum* Dempster; *Galium aretioides* Boiss.; *Galium argense* Dempster & Ehrend.; *Galium aristatum* L.; *Galium arkansanum* A.Gray; *Galium armenum* Schanzer; *Galium ascendens* Willd. ex Spreng.; *Galium aschenbornii* Schauer; *Galium asparagifolium* Boiss. & Heldr.; *Galium asperifolium* Wall.; *Galium asperuloides* Edgew.; *Galium asprellum* Michx.; *Galium atherodes* Spreng.; *Galium atlanticum* Pomel; *Galium aucheri* Boiss.; *Galium auratum* Klokov; *Galium australe* DC.; *Galium austriacum* Jacq.; *Galium avascense* Krendl; *Galium azerbayjanicum* Ehrend. & Schönb.-Tem.; *Galium azuayicum* Dempster; *Galium babadaghense* Yild.; *Galium baeticum* (Rouy) Ehrend. & Krendl; *Galium baghlanense* Ehrend. & Schönb.-Tem.; *Galium baillonii* Brandza; *Galium baldense* Spreng.; *Galium baldensiforme* Hand.-Mazz.; *Galium balearicum* Briq.; *Galium* ×*borcinonense* Sennen *(Galium lucidum* × *Galium maritimum*); *Galium basalticum* Ehrend. & Schönb.-Tem.; *Galium baytopianum* Ehrend. & Schönb.-Tem.; *Galium beckhausianum* G.H.Loos; *Galium belizianum* Ortega Oliv., Devesa & Rodr.Riaño; *Galium bellatulum* Klokov; *Galium bermudense* L.; *Galium bifolium* S.Watson; *Galium bigeminum* Griseb.; *Galium binifolium* N.A.Wakef.; *Galium blinii* H.Lév.; *Galium boissierianum* (Steud.) Ehrend. & Krendl; *Galium bolanderi* A.Gray; *Galium boreale* L.; *Galium boreoaethiopicum* Puff; *Galium bornmuelleri* Hausskn. ex Bornm.; *Galium bourgaeanum* Coss. ex Batt.; *Galium boyacanum* Dempster; *Galium brachyphyllum* Schult.; *Galium bracteatum* Boiss.; *Galium bredasdorpense* Puff; *Galium brenanii* Ehrend. & Verdc.; *Galium brevifolium* Sm.; *Galium breviramosum* Krendl; *Galium brockmannii* Briq.; *Galium broterianum* Boiss. & Reut.; *Galium brunneum* Munby; *Galium bryoides* Merr. & L.M.Perry; *Galium buchtienii* Dempster; *Galium* ×*buekkense* Hulják (*Galium abaujense* × *Galium vernum*); *Galium bullatum* Lipsky; *Galium bulliforme* I.Thomps.; *Galium bungei* Steud.; *Galium bungoniense* I.Thomps.; *Galium buschiorum* Mikheev; *Galium bussei* K.Schum. & K.Krause; *Galium buxifolium* Greene; *Galium cajamarcense* Dempster; *Galium californicum* Hook. & Arn.; *Galium caminianum* Schult.; *Galium campanelliferum* Ehrend. & Schönb.-Tem.; *Galium campylotrichum* Nazim. & Ehrend.; *Galium canescens* Kunth; *Galium cankiriense* Yιld.; *Galium canum* Req. ex DC.; *Galium canum* subsp. *antalyense* Ehrend.; *Galium canum* Req. ex DC. subsp. *canum; Galium canum* subsp. *ovatum* Ehrend.; *Galium canum* subsp. *ulukislaense* Yιld.; *Galium capense* Thunb.; *Galium capitatum* Bory & Chaub.; *Galium cappadocicum* Boiss.; *Galium caprarium* Natali; *Galium capreum* Krendl; *Galium carmenicola* Dempster; *Galium* ×*carmineum* Beauverd (*Galium anisophyllon* × *Galium pumilum* × *Galium rubrum*).; *Galium carterae* Dempster; *Galium caspicum* Steven; *Galium cassium* Boiss.; *Galium catalinense* A.Gray; *Galium* ×*centroniae* Cariot *(Galium pumilum* × *Galium rubrum*); *Galium ceratoamanianum* Ehrend.; *Galium ceratocarpon* Boiss.; *Galium ceratophylloides* Hook. f.; *Galium ceratopodum* Boiss.; *Galium cespitosum* Lam.; *Galium chaetopodum* Rech. f.; *Galium chekiangense* Ehrend.; *Galium chloroionanthum* K.Schum.; *Galium chloroleucum* Fisch. & C.A.Mey.; *Galium ciliare* Hook. f.; *Galium cilicicum* Boiss.; *Galium cinereum* All.; *Galium circae* Krendl; *Galium circaezans* Michx.; *Galium clausonis* Pomel; *Galium clementis* Eastw.; *Galium cliftonsmithii* (Dempster) Dempster & Stebbins; *Galium collomiae* J.T.Howell; *Galium coloradoense* W.Wight; *Galium comberi* Dempster; *Galium cometerhizon* Lapeyr.; *Galium compactum* Ehrend. & McGill.; *Galium concatenatum* Coss.; *Galium concinnum* Torr. & A.Gray; *Galium confertum* Royle ex Hook. f.; *Galium conforme* Krendl; *Galium consanguineum* Boiss.; *Galium coriaceum* Bunge; *Galium cornigerum* Boiss. & Hausskn.; *Galium coronadoense* Dempster; *Galium correllii* Dempster; *Galium corsicum* Spreng.; *Galium corymbosum* Ruiz & Pav.; *Galium cossonianum* Jafri; *Galium cotinoides* Cham. & Schltdl.; *Galium cracoviense* Ehrend.; *Galium crassifolium* W.C.Chen; *Galium craticulatum* R.R.Mill; *Galium crespianum* Rodr.; *Galium cryptanthum* Hemsl.; *Galium curvihirtum* Ehrend. & McGill.; *Galium cuspidulatum* Miq.; *Galium cyllenium* Boiss. & Heldr.; *Galium czerepanovii* Pobed.; *Galium dahuricum* Turcz. ex Ledeb.; *Galium davisii* Ehrend.; *Galium debile* Desv.; *Galium decorum* Krendl; *Galium decumbens* (Ehrend.) Ehrend. & Schönb.-Tem.; *Galium degenii* Bald. ex Degen; *Galium deistelii* K.Krause; *Galium delicatulum* Boiss. & Hohen.; *Galium demissum* Boiss.; *Galium dempsterae* B.L.Turner; *Galium densum* Hook. f.; *Galium denticulatum* Bartl. ex DC.; *Galium desereticum* Dempster & Ehrend.; *Galium diabolense* Dempster; *Galium dieckii* Bornm.; *Galium diffusoramosum* Dempster & Ehrend.; *Galium* ×*digeneum* A.Kern. (*Galium sylvaticum* × *Galium verum*); *Galium diphyllum* (K.Schum.) Dempster; *Galium diploprion* Boiss. & Hohen.; *Galium divaricatum* Pourr. ex Lam.; *Galium domingense* Iltis; *Galium dumosum* Boiss.; *Galium duthiei* R.Bhattacharjee; *Galium echinocarpum* Hayata; *Galium ecuadoricum* Dempster; *Galium* ×*effulgens* Beck (*Galium lucidum* × *Galium verum*); *Galium ehrenbergii* Boiss.; *Galium elbursense* Bornm. & Gauba; *Galium elegans* Wall. ex Roxb.; *Galium elongatum* C.Presl; *Galium emeryense* Dempster & Ehrend.; *Galium ephedroides* Willk.; *Galium equisetoides* (Cham. & Schltdl.) Standl.; *Galium ericoides* Lam.; *Galium eriocarpum* Bartl. ex DC.; *Galium eruptivum* Krendl; *Galium erythrorrhizon* Boiss. & Reut.; *Galium espiniacicum* Dempster; *Galium estebanii* Sennen; *Galium exaltatum* Krendl; *Galium exile* Hook. f.; *Galium exstipulatum* P.H.Davis; *Galium exsurgens* Ehrend. & Schönb.-Tem.; *Galium extensum* Krendl; *Galium falconeri* R.Bhattacharjee; *Galium fendleri* A.Gray; *Galium ferrugineum* K.Krause; *Galium festivum* Krendl; *Galium* ×*fictum* E.G.Camus (= *Galium glaucum* × *Galium mollugo*); *Galium filipes* Rydb.; *Galium firmum* Tausch; *Galium fissurense* Ehrend. & Schönb.-Tem.; *Galium fistulosum* Sommier & Levier; *Galium flavescens* Borbás ex Simonk.; *Galium flaviflorum* (Trautv.) Mikheev; *Galium floribundum* Sm.; *Galium foliosum* Munby ex Burnat & Barbey; *Galium fontanesianum* Pomel; *Galium formosense* Ohwi; *Galium forrestii* Diels; *Galium fosbergii* Dempster; *Galium friedrichii* N.Torres & al.; *Galium fruticosum* Willd.; *Galium fuegianum* Hook. f.; *Galium fuscum* M.Martens & Galeotti; *Galium galapagoense* Wiggins; *Galium galiopsis* (Hand.-Mazz.) Ehrend.; *Galium gaudichaudii* DC.; *Galium geminiflorum* Lowe; *Galium ghilanicum* Stapf; *Galium gilliesii* Hook. & Arn.; *Galium glaberrimum* Hemsl.; *Galium glabrescens* (Ehrend.) Dempster & Ehrend.; *Galium glabriusculum* Ehrend.; *Galium glaciale* K.Krause; *Galium glandulosum* Hand.-Mazz.; *Galium glaucophyllum* Em.Schmid; *Galium glaucum* L.; *Galium globuliferum* Hub.-Mor. & Reese; *Galium gracilicaule* Bacigalupo & Ehrend.; *Galium graecum* L.; *Galium grande* McClatchie; *Galium grayanum* Ehrend.; *Galium gymnopetalum* Ehrend. & Schönb.-Tem.; *Galium hainesii* Schönb.-Tem.; *Galium hallii* Munz & I.M.Johnst.; *Galium hardhamae* Dempster; *Galium hatschbachii* Dempster; *Galium haussknechtii* Ehrend.; *Galium heldreichii* Halácsy; *Galium hellenicum* Krendl; *Galium hexanarium* Knjaz.; *Galium hierochuntinum* Bornm.; *Galium hierosolymitanum* L.; *Galium hilendiae* Dempster & Ehrend.; *Galium* ×*himmelbaurianum* (Ronniger) Soó (*Galium humifusum* × *Galium verum*); *Galium hintoniorum* B.L.Turner; *Galium hirtiflorum* Req. ex DC.; *Galium hirtum* Lam.; *Galium hoffmeisteri* (Klotzsch) Ehrend. & Schönb.-Tem. ex R.R.Mill; *Galium homblei* De Wild.; *Galium huancavelicum* Dempster; *Galium huber-morathii* Ehrend. & Schönb.-Tem.; *Galium humifusum* M.Bieb.; *Galium humile* Cham. & Schltdl.; *Galium* ×*hungaricum* A.Kern. (*Galium mollugo* × *Galium schultesii*); *Galium hupehense* Pamp.; *Galium* ×*huteri* A.Kern. (*Galium laevigatum* × *Galium lucidum*); *Galium hypocarpium* (L.) Endl. ex Griseb.; *Galium hypotrichium* A.Gray; *Galium hypoxylon* Ehrend. & Schönb.-Tem.; *Galium hyrcanicum* C.A.Mey; *Galium hystricocarpum* Greenm.; *Galium idubedae* (Pau & Debeaux) Pau ex Ehrend.; *Galium iltisii* Dempster; *Galium incanum* Sm.; *Galium incanum* subsp. *centrale* Ehrend.; *Galium incanum* subsp. *creticum* Ehrend.; *Galium incanum* subsp. *elatius* (Boiss.) Ehrend.; *Galium incanum* Sm. subsp. *Incanum; Galium incanum* subsp. *libanoticum* Ehrend.; *Galium incanum* subsp. *pseudocornigerum* Ehrend.; *Galium inconspicuum* Phil.; *Galium incrassatum* Halácsy; *Galium incurvum* Sibth. & Sm.; *Galium innocuum* Miq.; *Galium insulare* Krendl; *Galium intermedium* Schult., Syn.; *Galium schultesii* Vest; *Galium aristatum* subsp. *schultesii* (Vest) Nyman); *Galium intricatum* Margot & Reut.; *Galium ionicum* Krendl; *Galium iranicum* Hausskn. ex Bornm.; *Galium irinae* Pachom.; *Galium isauricum* Ehrend. & Schönb.-Tem.; *Galium* ×*jansenii* Kloos (*Galium sylvaticum* × *Galium mollugo*); *Galium japonicum* Makino; *Galium* ×*jarynae* Wol. (*Galium aristatum* × *Galium mollugo*); *Galium javalambrense* López Udias, Mateo & M.B.Crespo; *Galium javanicum* Blume; *Galium jemense* Kotschy; *Galium jepsonii* Hilend & J.T.Howell; *Galium johnstonii* Dempster & Stebbins; *Galium jolyi* Batt.; *Galium judaicum* Boiss.; *Galium jungermannioides* Boiss.; *Galium junghuhnianum* Miq.; *Galium juniperinum* Standl.; *Galium kaganense* R.Bhattacharjee; *Galium kahelianum* Deflers; *Galium kamtschaticum* Steller ex Schult.; *Galium karakulense* Pobed.; *Galium karataviense* (Pavlov) Pobed.; *Galium kasachstanicum* Pachom.; *Galium kenyanum* Verdc.; *Galium kerneri* Degen & Dörfl.; *Galium khorasanense* Griff.; *Galium kikumuyura* Ohwi; *Galium killipii* Dempster & Ehrend.; *Galium kinuta* Nakai & H.Hara.; *Galium kitaibelianum* Schult.; *Galium xkondratjukii* Ostapko (*Galium cincinnatum* × *Galium tomentosum*); *Galium kuetzingii* Boiss. & Buhse; *Galium kunmingense* Ehrend.; *Galium kurdicum* Boiss. & Hohen.; *Galium labradoricum* (Wiegand) Wiegand; *Galium laconicum* Boiss. & Heldr.; *Galium lacrimiforme* Dempster; *Galium laevigatum* L.; *Galium lahulense* Ehrend. & Schönb.-Tem.; *Galium lanceolatum* (Torr. & A.Gray) Torr.; *Galium lanuginosum* Lam.; *Galium* ×*lanulosum* Ostapko (*Galium humifusum* × *Galium tomentosum*); *Galium lasiocarpum* Boiss.; *Galium latifolium* Michx.; *Galium latoramosum* Clos; *Galium leiocarpum* I.Thomps.; *Galium leptogonium* I.Thomps.; *Galium leptum* Phil.; *Galium libanoticum* Ehrend.; *Galium lilloi* Hicken; *Galium xlindbergii* Giraudias; *Galium linearifolium* Turcz.; *Galium liratum* N.A.Wakef.; *Galium litorale* Guss.; *Galium lovcense* Urum.; *Galium lucidum* All.; *Galium macedonicum* Krendl; *Galium magellanicum* Hook. f.; *Galium magellense* Ten.; *Galium magnifolium* (Dempster) Dempster; *Galium mahadivense* G.Singh; *Galium malickyi* Krendl.; *Galium mandonii* Britton; *Galium maneauense* P.Royen; *Galium marchandii* Roem. & Schult.; *Galium margaceum* Ehrend. & Schönb.-Tem.; *Galium margaritaceum* A.Kern.; *Galium maritimum* L.; *Galium martirense* Dempster & Stebbins; *Galium masafueranum* Skottsb.; *Galium matthewsii* A.Gray; *Galium maximowiczii* (Kom.) Pobed.; *Galium mechudoense* Dempster; *Galium megacyttarion* R.R.Mill; *Galium megalanthum* Boiss.; Schweizer Labkraut (*Galium megalospermum* All.); *Galium megapotamicum* Spreng.; *Galium melanantherum* Boiss.; *Galium meliodorum* (Beck) Fritsch; *Galium membranaceum* Ehrend.; *Galium mexicanum* Kunth; *Galium microchiasma* Gilli; *Galium microlobum* I.Thomps.; *Galium microphyllum* A.Gray; *Galium migrans* Ehrend. & McGill.; *Galium minutissimum* T.Shimizu; *Galium minutulum* Jord.; *Galium mirum* Rech. f.; *Galium mite* Boiss. & Hohen.; *Galium moldavicum* (Dobrocz.) Franco; *Galium mollugo* L.; *Galium monachinii* Boiss. & Heldr.; *Galium monasterium* Krendl; *Galium monticolum* Sond.; *Galium montis-arerae* Merxm. & Ehrend.; *Galium moralesianum* Ortega Oliv. & Devesa; *Galium moranii* Dempster; *Galium morii* Hayata; *Galium mucroniferum* Sond.; *Galium muelleri* (K.Schum.) Dempster; *Galium multiflorum* Kellogg; *Galium munzii* Hilend & J.T.Howell; *Galium murale* (L.) All.; *Galium murbeckii* Maire; *Galium muricatum* W.Wight; *Galium xmutabile* Besser (*Galium mollugo* × *Galium verum*); *Galium nabelekii* Ehrend. & Schönb.-Tem.; *Galium nakaii* Kudô; *Galium nankotaizanum* Ohwi; *Galium xneglectum* Le Gall ex Gren. & Godr. (*Galium album* × *Galium arenarium*); *Galium nepalense* Ehrend. & Schönb.-Tem.; *Galium nevadense* Boiss. & Reut.; *Galium nigdeense* Yild.; *Galium nigricans* Boiss.; *Galium nigroramosum* (Ehrend.) Dempster; *Galium nolitangere* Ball; *Galium noricum* Ehrend.; *Galium normanii* Dahl; *Galium novoguineense* Diels; *Galium noxium* (A.St.-Hil.) Dempster; *Galium numidicum* Pomel; *Galium nupercreatum* Popov; *Galium nuttallii* A.Gray; *Galium obliquum* Vill.; *Galium obovatum* Kunth; *Galium obtusum* Bigelow; *Galium octonarium* (Klokov) Pobed.; *Galium odoratum* (L.) Scop.; *Galium oelandicum* Ehrend.; *Galium olgae* Klokov; *Galium olivetorum* Le Houér; *Galium olympicum* Boiss.; *Galium ophiolithicum* Krendl; *Galium oreganum* Britton; *Galium oreophilum* Krendl; *Galium oresbium* Greenm.; *Galium orizabense* Hemsl.; *Galium oshtenicum* Ehrend. & Schanzer ex Mikheev; *Galium ossirwaense* K.Krause; *Galium ostenianum* (Standl.) Dempster; *Galium ovalleanum* Phil.; *Galium pabulosum* Sommier & Levier; *Galium palaeoitalicum* Ehrend.; *Galium palustre* L.; *Galium pamiroalaicum* Pobed.; *Galium pamphylicum* Boiss. & Heldr.; *Galium paniculatum* (Bunge) Pobed.; *Galium papilliferum* Ehrend. & Schönb.-Tem.; *Galium papillosum* Lapeyr.; *Galium papuanum* Wernham; *Galium paradoxum* Maxim.; *Galium parishii* Hilend & J.T.Howell; *Galium parisiense* L.; *Galium parvulum* Hub.-Mor. ex Ehrend. & Schönb.-Tem.; *Galium paschale* Forssk.; *Galium pastorale* Krendl; *Galium patzkeanum* G.H.-Loos; *Galium peloponnesiacum* Ehrend. & Krendl; *Galium penduliflorum* Boiss.; *Galium pendulum* Greenm.; *Galium penicillatum* Boiss.; *Galium pennellii* Dempster; *Galium peplidifolium* Boiss.; *Galium perralderi* Coss.; *Galium peruvianum* Dempster & Ehrend.; *Galium pestalozzae* Boiss.; *Galium petrae* Oliv. ex Hart; *Galium philippianum* Dempster; *Galium philippinense* Merr.; *Galium philistaeum* Boiss.; *Galium pilosum* Aiton; *Galium pisiferum* Boiss.; *Galium pisoderium* Krendl; *Galium platygalium* (Maxim.) Pobed.; *Galium plumosum* Rusby; *Galium poiretianum* Ball; *Galium pojarkovae* Pobed.; *Galium polyacanthum* (Baker) Puff; *Galium polyanthum* I.Thomps.; *Galium xpomeranicum* Retz. (*Galium album* × *Galium verum*); *Galium porrigens* Dempster; *Galium praemontanum* Mardal.; *Galium praetermissum* Greenm.; *Galium* × *pralognense* Beauverd *(Galium pumilum* × *Galium verum); Galium prattii* Cufod.; *Galium pringlei* Greenm.; *Galium problematicum* (Ehrend.) Ehrend. & Schönb.-Tem.; *Galium procurrens* Ehrend.; *Galium productum* Lowe; *Galium* ×*prolazense* Nyár. (*Galium album* × *Galium flavescens*); *Galium proliferum* A.Gray; *Galium propinquum* A.Cunn.; *Galium pruinosum* Boiss.; *Galium pseudoaristatum* Schur; *Galium* ×*pseudo-boreale* Klokov (*Galium boreale* × *Galium rubioides*); *Galium pseudocapitatum* Hub.-Mor. ex Ehrend. & Schönb.-Tem.; *Galium pseudohelveticum* Ehrend.; *Galium pseudokurdicum* (Ehrend.) Schönb.-Tem.; *Galium pseudorivale* Tzvelev; *Galium pseudotriflorum* Dempster & Ehrend.; *Galium psilocladum* Ehrend.; *Galium pterocarpum* Ehrend.; *Galium pulvinatum* Boiss.; *Galium pumilio* Standl.; *Galium pumilum* Murray; *Galium pusillosetosum* H.Hara; *Galium pusillum* L.; *Galium pyrenaicum* Gouan; *Galium qaradaghense* Schönb.-Tem.; *Galium* ×*querceticola* Wol. (*Galium abaujense* subsp. *polonicum* × *Galium intermedium*); *Galium quichense* Dempster; *Galium radulifolium* Ehrend. & Schönb.-Tem.; *Galium ramboi* Dempster; *Galium rebae* R.R.Mill; *Galium reiseri* Halácsy; *Galium* ×*retzii* Bouchard (*Galium papillosum* × *Galium verum*); *Galium rhodopeum* Velen.; *Galium richardianum* (Gillies ex Hook. & Arn.) Endl. ex Walp.; *Galium rigidifolium* Krendl; *Galium rivale* (Sibth. & Sm.) Griseb.; *Galium roddii* Ehrend. & McGill.; *Galium rosellum* (Boiss.) Boiss. & Reut.; *Galium rotundifolium* L.; *Galium rourkei* Puff; *Galium rubidiflorum* Dempster; *Galium rubioides* L.; *Galium rubrum* L.; *Galium runcinatum* Ehrend. & Schönb.-Tem.; *Galium rupifragum* Ehrend.; *Galium ruwenzoriense* (Cortesi) Ehrend.; *Galium rzedowskii* Dempster; *Galium sacrorum* Krendl; *Galium saipalense* Ehrend. & Schönb.-Tem.; *Galium salsugineum* Krylov & Serg.; *Galium salwinense* Hand.-Mazz.; *Galium samium* Krendl; *Galium samuelssonii* Ehrend.; *Galium saturejifolium* Trevir.; *Galium saurense* Litv.; *Galium saxatile* L., Syn.: *Galium harcynicum* Weigel, *Galium pawlowskii* Kucowa, *Galium pumilum* subsp. *saxatile* (L.) Dostál; *Galium saxosum* (Chaix) Breistr.; *Galium scabrelloides* Puff; *Galium scabrellum* K.Schum.; *Galium scabrifolium* (Boiss.) Hausskn.; *Galium scabrum* L.; *Galium schlumbergeri* Boiss.; *Galium* ×*schmidelyi* Chenevard & W.Wolf (*Galium mollugo* × *Galium rubrum*); *Galium schmidii* Arrigoni; *Galium* ×*schneebergense* Ronniger (*Galium anisophyllon* × *Galium meliodorum*); *Galium schoenbecktemesyae* Ehrend.; *Galium scioanum* Chiov.; *Galium scopulorum* Schönb.-Tem.; *Galium seatonii* Greenm.; *Galium sellowianum* (Cham.) Walp.; *Galium semiamictum* Klokov; *Galium serpenticum* Dempster; *Galium serpylloides* Royle ex Hook. f.; *Galium setaceum* Lam.; *Galium setuliferum* Ehrend. & Schönb.-Tem.; *Galium shanense* R.Bhattacharjee; *Galium shepardii* Post; *Galium shepherdii* Jung-Mend.; *Galium sichuanense* Ehrend.; *Galium sidamense* Chiov. ex Chiarugi; *Galium sieheanum* Ehrend.; *Galium simense* Fresen.; *Galium similii* Pavlov; *Galium sinaicum* (Delile ex Decne.) Boiss.; *Galium smithreitzii* Dempster; *Galium sojakii* Ehrend. & Schönb.-Tem.; *Galium songaricum* Schrenk; *Galium sorgerae* Ehrend. & Schönb.-Tem.; *Galium sparsiflorum* W.Wight; *Galium spathulatum* I.Thomps.; *Galium speciosum* Krendl; *Galium sphagnophilum* (Greenm.) Dempster; *Galium spurium* L.; *Galium stellatum* Kellogg; *Galium stenophyllum* Baker; *Galium stepparum* Ehrend. & Schönb.-Tem.; *Galium sterneri* Ehrend.; *Galium subfalcatum* Nazim. & Ehrend.; *Galium subnemorale* Klokov & Zaver.; *Galium subtrifidum* Reinw. ex Blume; *Galium subtrinervium* Ehrend. & Schönb.-Tem.; *Galium subuliferum* Sommier & Levier; *Galium subvelutinum* (DC.) K.Koch; *Galium subvillosum* Sond.; *Galium sudeticum* Tausch; *Galium suecicum* (Sterner) Ehrend.; *Galium suffruticosum* Hook. & Arn. (*Rubia margaritifera* Reiche); *Galium sungpanense* Cufod.; *Galium surinamense* Dempster; *Galium sylvaticum* L.; *Galium taiwanense* Masam.; *Galium takasagomontanum* Masam.; *Galium talaveranum* Ortega Oliv. & Devesa; *Galium tanganyikense* Ehrend. & Verdc.; *Galium tarokoense* Hayata; *Galium taygeteum* Krendl; *Galium tendae* Rchb. f.; *Galium tenuissimum* M.Bieb.; *Galium terrae-reginae* Ehrend. & McGill.; *Galium tetraphyllum* Nazim. & Ehrend.; *Galium texense* A.Gray; *Galium thasium* Stoj. & Kitanov; *Galium thiebautii* Ehrend.; *Galium thracicum* Krendl; *Galium thunbergianum* Eckl. & Zeyh.; *Galium thymifolium* Boiss. & Heldr.; *Galium tianschanicum* Popov; *Galium timeroyi* Jord.; *Galium tinctorium* L.; *Galium tmoleum* Boiss.; *Galium tokyoense* Makino; *Galium tolosianum* Boiss. & Kotschy; *Galium tomentosum* Thunb.; *Galium tortumense* Ehrend. & Schönb.-Tem.; *Galium transcarpaticum* Stojko & Tasenk.; *Galium trichocarpum* DC.; *Galium tricornutum* Dandy; *Galium trifidum* L.; *Galium trifloriforme* Kom.; *Galium triflorum* Michx.; *Galium trilobum* Colenso; *Galium trinioides* Pomel; *Galium trojanum* Ehrend.; *Galium truniacum* (Ronniger); *Galium tubiflorum* Ehrend.; *Galium tuncelianum* Yild.; *Galium tunetanum* Lam.; *Galium turgaicum* Knjaz.; *Galium turkestanicum* Pobed.; *Galium tyraicum* Klokov; *Galium uliginosum* L.; *Galium uncinulatum* DC.; *Galium undulatum* Puff; *Galium uniflorum* Michx.; *Galium uruguayense* Bacigalupo; *Galium valantioides* M.Bieb.; *Galium valdepilosum* Heinr.Braun; *Galium valentinum* Lange; *Galium vartanii* Grossh.; *Galium vassilczenkoi* Pobed.; *Galium velenovskyi* Ancev; *Galium verrucosum* Huds.; *Galium verticillatum* Danthoine ex Lam.; *Galium verum* L.; *Galium verum* subsp. *asiaticum* (Nakai) T.Yamaz.; *Galium verum* subsp. *glabrescens* Ehrend.; *Galium verum* L. subsp. *verum; Galium verum* subsp. *wirtgenii* (F.W.Schultz) Oborny (Syn. *Galium wirtgenii* F.W.Schultz); *Galium* ×*viciosorum* Sennen & Pau (*Galium maritimum* × *Galium verum*); *Galium vile* (Cham. & Schltdl.) Dempster; *Galium violaceum* Krendl; *Galium virgatum* Nutt.; *Galium viridiflorum* Boiss. & Reut.; *Galium viscosum* Vahl; *Galium volcanense* Dempster; *Galium volhynicum* Pobed.; *Galium watsonii* (A.Gray) A.Heller; *Galium weberbaueri* K.Krause; *Galium wendelboi* Ehrend. & Schönb.-Tem.; *Galium werdermannii* Standl.; *Galium wigginsii* Dempster; *Galium wrightii* A.Gray; *Galium xeroticum* (Klokov) Pobed.; *Galium xylorrhizum* Boiss. & A.Huet; *Galium yunnanense* H.Hara & C.Y.Wu; *Galium zabense* Ehrend.; *Rubia agostinhoi* Dans. & P.Silva; *Rubia aitchisonii* Deb & Malick; *Rubia alaica* Pachom.; *Rubia alata* Wall.; *Rubia albicaulis* Boiss.; *Rubia angustisissima* Wall. ex G.Don; *Rubia argyi* (H.Lév. & Vaniot) Hara ex Lauener, Syn.; *Rubia akane* Nakai, *Rubia chekiangensis* Deb, *Rubia nankotaizana* (Masam); *Rubia atropurpurea* Decne.; *Rubia austrozhejiangensis* Z.P.Lei, Y.Y.Zhou & R.W.Wang; *Rubia balearica* (Willk.) Porta; *Rubia balearica* (Willk.) Porta subsp. *Balearica; Rubia balearica* subsp. *caespitosa* (Font Quer & Marcos) Rosselló, L.Sáez & Mus (*Rubia angustifolia* var. *caespitosa* Font Quer & Marcos, *Rubia angustifolia* subsp. *caespitosa* (Font Quer & Marcos) Rosselló, *Rubia caespitosa* (Font Quer & Marcos) Rosselló); *Rubia caramanica* Bornm.; *Rubia charifolia* Wall. ex G.Don.; *Rubia chinensis* Regel & Maack; *Rubia chinensis* f. *chinensis* (*Rubia chinensis* f. *mitis* (Miq.) Kitag.); *Rubia chinensis* f. *glabrescens* (Nakai) Kitag.; *Rubia chitralensis* Ehrend.; *Rubia clematidifolia* Blume ex Decne.; *Rubia cordifolia* L.; *Rubia cordifolia* subsp. *conotricha* (Gand.) Verdc.; *Rubia cordifolia* subsp. *Cordifolia; Rubia crassipes* Collett & Hemsl.; *Rubia cretacea* Pojark.; *Rubia danaensis* Danin; *Rubia davisiana* Ehrend.; *Rubia deserticola* Pojark.; *Rubia discolor* Turcz.; *Rubia dolichophylla* Schrenk; *Rubia edgeworthii* Hook. f.; *Rubia falciformis* H.S.Lo; *Rubia filiformis* F.C.How ex H.S.Lo; *Rubia florida* Boiss; *Rubia garrettii* Craib; *Rubia gedrosiaca* Bornm.; *Rubia haematantha* Airy Shaw; *Rubia hexaphylla* (Makino) Makino; *Rubia hispidicaulis* D.G.Long; *Rubia infundibularis* Hemsl. & Lace; *Rubia jesoensis* (Miq.) Miyabe & Kudo; *Rubia komarovii* Pojark; *Rubia krascheninnikovii* Pojark; *Rubia laevissima* Tsckern.; *Rubia latipetala* H.S.Lo; *Rubia laurae* (Holmboe) Airy Shaw; *Rubia laxiflora* Gontsch.; *Rubia linii* J.M.Chao; *Rubia magna* P.G.Xiao; *Rubia mandersii* Collett & Hemsl.; *Rubia manjith* Roxb. ex Fleming; *Rubia maymanensis* Ehrend. & Schönb.-Tem; *Rubia membranacea* Diels; *Rubia oncotricha* Hand.-Mazz.; *Rubia oppositifolia* Griff.; *Rubia ovatifolia* Z.Ying Zhang ex Q.Lin; *Rubia pallida* Diels; *Rubia pauciflora* Boiss.; *Rubia pavlovii* Bajtenov & Myrz.; *Rubia peregrina* L.; *Rubia petiolaris* DC.; *Rubia philippinensis* Elmer; *Rubia pianmaensis* R.Li & H.Li; *Rubia podantha* Diels; *Rubia polyphlebia* H.S.Lo; *Rubia pseudogalium* Ehrend.; *Rubia pterygocaulis* H.S.Lo; *Rubia rechingeri* Ehrend.; *Rubia rezniczenkoana* Litv.; *Rubia rigidifolia* Pojark.; *Rubia rotundifolia* Banks & Sol.; *Rubia salicifolia* H.S.Lo; *Rubia schugnanica* B.Fedtsch. ex Pojark.; *Rubia schumanniana* E.Pritz.; *Rubia siamensis* Craib; *Rubia sikkimensis* Kurz; *Rubia sylvatica* (Maxim.) Nakai; *Rubia tatarica* (Trevir.) F.Schmidt; *Rubia tenuifolia* d'Urv.; *Rubia tenuifolia* subsp. *brachypoda* (Boiss.) Ehrend. & Schönb.-Tem.; *Rubia tenuifolia* subsp. *doniettii* (Griseb.) Ehrend. & Schönb.-Tem.; *Rubia tenuifolia* subsp. *Tenuifolia; Rubia tenuissima* ined. (Syn.: *Rubia tenuis* H.S.Lonom. illeg.); *Rubia thunbergii* DC.; *Rubia tibetica* Hook. f.; *Rubia tinctorum* L.; *Rubia transcaucasica* Grossh.; *Rubia trichocarpa* H.S.Lo; *Rubia truppeliana* Loes.; *Rubia wallichiana* Decne.; and *Rubia yunnanensis* Diels; preferably a plant selected from *Galium aparine, Galium verum* and *Rubia cardifolia L.*

In a further preferred embodiment, the extract for use in the present invention is prepared (i) from a fungus of a fungal genus selected from the group consisting of: *Alternaria genus; Aspergillus genus,* preferably *Aspergillus chevalieri, Aspergillus glaucus* and *Aspergillus fumigatus;* Boletus genus, preferably comprising Boletol and Carminic acid; *Cortinarius genus; Curvularia genus; Eurotium genus; Fusarium genus; Haloresellinia genus; Helminthosporium genus,* preferably *H. cynodontis, H. catenarium* and *H. tritici-vulgaris; Microshaeropsis genus; Monodictys genus; Nigrospora genus; Paecilomyces genus; Penicillium genus,* preferably *P*. *cyclopium, P. citreo-roseum, P. carmine-violaceum, P. roseo-purpureum* and *P. cyclopium; Phomopsis; Preussia genus,* preferably *Pressuia multispora (Saito et Minoura) Cain; Valsaria genus,* preferably *Valsaria rubricosa,* preferably comprising Papulosin, valsarin; *Talaromyces genus,* preferably *Talaromyces avellaneus (Thom et Turreson) C.R. Benjamin;* or (ii) from a fungus of a *Lichen* genus, preferably a *Lichen* genus selected from the group consisting of *Xanthoria genus,* preferably *Xanthoria parietina; Lecidella genus,* preferably *Lecidella asema; Lasallia genus,* preferably *Lasallia papulose var. rubiginosa; Trypethelium genus,* preferably *Trypethelium cruentum* (*Pyrenula cruenta).*

In another preferred embodiment, the anthraquinone or anthraquinone derivative for use in the present invention is selected from the group consisting of compounds of Formula (III), (IV) and (V) or a pharmaceutically acceptable salt or solvate thereof, 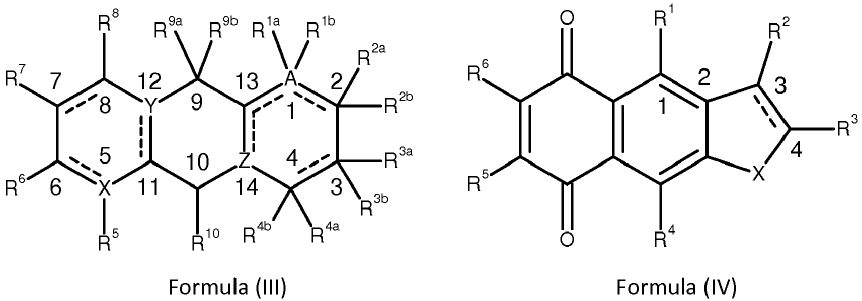 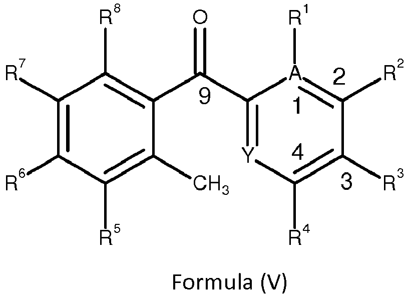 wherein
the bonds between positions 1 and 2, 1 and 13, 3 and 4, 5 and 6, 7 and 8, 11 and 12, and 13 and 14 are independently selected from single bonds or double bonds, preferably double bonds;
**A, X, Y** and **Z** are each independently selected from the group consisting of C, N and O, preferably X is C or O, and A, Y and Z are C or N, more preferably A, X, Y and Z are C, preferably for Formula (IV) X is O and/or the bond between position 3 and 4 is a double bond;
**R¹, R², R³, R⁴, R⁵, R⁶, R⁷, R⁸, R^{1a}** and **R^{1b}, R^{2a}** and **R^{2b}, R^{3a}** and **R^{3b},** and **R^{4a}** and **R^{4b},** are each independently selected from the group consisting of:
   (i) H, F, Cl, Br, -OH, -OSO₃H and -SO₃H;
   (ii) -O(C₁₋₁₀)alkyl, preferably -OMe and -OEt, -O(C₃₋₁₀)cycloalkyl, -O(C=O)(C₁₋₁₀)alkyl, preferably-O(C=O)Me, or -O(C=O)(C₃₋₁₆)cycloalkyl;
   (iii) -COOH, -COO(C₁₋₁₀)alkyl, -COO(C₃₋₆)cycloalkyl, -COONH₂, -COON((C₁₋₁₀)alkyl)₂, -COONH(C₁₋₁₀)alkyl, preferably -COOH, -COOMe or COOEt;
   (iv) linear or branched, substituted or non-substituted (C₂₋₁₀)alkyl ether, (C₃₋₁₀)alkenyl ether, (C₃₋₁₀)alkynyl ether or (C₄₋₁₀)carbocyclic ether, wherein the ether is bonded to formula (III), (IV) or (V) via its carbon atom;
   (v) linear or branched, substituted or non-substituted (C₁₋₁₀)alkyl, (C₂₋₁₀)alkenyl, (C₂₋₁₀)alkynyl, preferably (C₁₋₁₀)alkyl, more preferably substituted or non-substituted -CHO, methyl, ethyl or propyl, most preferably substituted or non-substituted methyl;
   (vi) substituted or non-substituted carbocycle selected from the group consisting of (C₃₋₁₀)carbocycle, preferably (C₃)carbocycle and (C₅₋₆)carbocycle, preferably aromatic (C₆)carbocycle, more preferably a non-substituted phenyl or a phenyl that is substituted by a substituent selected from the group consisting of Cl, F, Br, substituted or non-substituted methyl, preferably -(CF₃), ethyl, propyl and cyclopropyl;
   (vii) substituted or non-substituted (C₃₋₆)heterocycle or (C₇₋₁₀)carbo- or hetero-bicycle having 1 to 3 heteroatoms each independently selected from N, O and S, preferably substituted or non-substituted heterobicycle having 2 oxygen atoms, preferably substituted or non-substituted benzodioxolyl connected via position (5) or (6) of the benzodioxolyl;
   (viii) a mono- or polysaccharide, preferably a mono-, di- or trisaccharide, more preferably a mono- or disaccharide, wherein the mono- or polysaccharide ring carbon is directly attached or attached via an oxygen atom; preferably for Formula (V) **R²** is 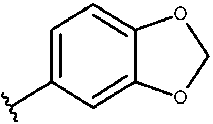 and/or **A** and/or **Y** are C or N;
**R²**, **R^{2a}** or **R^{2b}** and **R³, R^{3a}** or **R^{3b}** optionally together form a ring selected from the group consisting of
   (i) substituted or non-substituted carbocycle selected from the group consisting of (C₃₋₁₀)carbocycle, preferably (C₃)carbocycle, and (C₅₋₆)carbocycle, preferably aromatic (C₆)carbocycle, more preferably a non-substituted phenyl or a phenyl that is substituted by a substituent selected from the group consisting of Cl, F, Br, substituted or non-substituted methyl, preferably -(CF₃), ethyl, propyl and cyclopropyl; and
   (ii) substituted or non-substituted (C₃₋₆)heterocycle or (C₇₋₁₀)carbo- or hetero-bicycle having 1 to 3 heteroatoms each independently selected from N, O and S, preferably substituted or non-substituted heterobicycle having 2 oxygen atoms;
**R^{9a}** and **R^{9b}** are independently selected from the group consisting of
   (i) H, Me, OH, OMe, and =O;
   (ii) a mono- or polysaccharide, preferably a mono-, di- or trisaccharide, more preferably a mono- or disaccharide, wherein the mono- or polysaccharide ring carbon is directly attached or attached via an oxygen atom;
preferably R^{9a} is H and R^{9b} is OH, more preferably R^{9a} is =O and R^{9b} is absent; and/or
**R¹⁰** is H, -OH, =O, or a mono- or polysaccharide, preferably a mono-, di- or trisaccharide, more preferably a mono- or disaccharide, wherein the mono- or polysaccharide ring carbon is directly attached or attached via an oxygen atom.

The compounds described herein are generally named by using the nomenclature that was computed based on the structural drawings by the software ACD/Chemsketch 2015 provided by Advanced Chemistry Development, Inc., Canada and BIOVIA Draw 2016 provided by BIOVIA, USA. The compounds can be obtained by chemical synthesis and/or by extraction from plants or other organisms, preferably fungal organisms.

For compounds having asymmetric centers, it is understood that, unless otherwise specified, all of the optical isomers and mixtures thereof are encompassed. Each stereogenic carbon may be in the *(R)-* or *(S)-* configuration or a combination of configurations if not indicated differently. Also, compounds with two or more asymmetric elements can be present as mixtures of diastereomers. Furthermore, the compounds of the present invention preferably have a diastereomeric purity of at least 50%, preferably at least 60%, 70%, 80%, 85%, more preferably at least 90%, 95%, 96%, 97%, most preferably at least 98%, 99% or 100%. In addition, compounds with carbon-carbon double bonds may occur in *Z*- and *E*- forms, with all isomeric forms of the compounds being included in the present invention unless otherwise specified. Where a compound exists in various tautomeric forms, a recited compound is not limited to any one specific tautomer, but rather is intended to encompass all tautomeric forms.

Recited compounds are further intended to encompass compounds in which one or more atoms are replaced with an isotope, *i.e.,* an atom having the same atomic number but a different mass number. By way of general example, and without limitation, isotopes of hydrogen include tritium and deuterium and isotopes of carbon include ¹¹C, ¹³C, and ¹⁴C.

Compounds according to the formulas provided herein, which have one or more stereogenic center(s), have an enantiomeric excess of at least 50%. For example, such compounds may have an enantiomeric excess of at least 60%, 70%, 80%, 85%, preferably at least 90%, 95%, or 98%. Some embodiments of the compounds have an enantiomeric excess of at least 99%. It will be apparent that single enantiomers (optically active forms) can be obtained by asymmetric synthesis, synthesis from optically pure precursors, biosynthesis, extraction from plants or organisms, or by resolution of the racemates, e.g. enzymatic resolution or resolution by conventional methods such as crystallization in the presence of a resolving agent, or chromatography, using, for example, a chiral HPLC column.

As used herein, a "substituent" or "residue" or **"R",** refers to a molecular moiety that is covalently bound to an atom within a molecule of interest. For example, a "substituent", **"R"** or "residue" may be a moiety such as a halogen, alkyl group, haloalkyl group or any other substituent described herein that is covalently bonded to an atom, preferably a carbon or nitrogen atom, that forms part of a molecule of interest. The term "substituted" as used herein, means that any one or more hydrogens on the designated atom is replaced with a selection from the indicated substituents, provided that the designated atom's normal valence is not exceeded, and that the substitution results in a stable compound, *i.e.,* a compound that can be isolated and characterized using conventional means. For example, substitution can be in the form of an oxygen bound to any other chemical atom than carbon, e.g. hydroxyl group, or an oxygen anion. When a substituent is oxo, *i.e.,* =O, then 2 hydrogens on the atom are replaced. An oxo group that is a substituent of an aromatic carbon atom results in a conversion of -CH- to -C(=O)- and a loss of aromaticity. For example, a pyridyl group substituted by oxo is a pyridone.

The term "heteroatom" as used herein shall be understood to mean atoms other than carbon and hydrogen such as and preferably O, N, S and P.

If a first compound, a substituent or a residue ends, e.g., in the name "-yl", this ending indicates that the first compound, substituent or residue is covalently bound to a second compound, substituent or residue.

In the context of the present invention it is understood that antecedent terms such as "linear or branched", "substituted or non-substituted" indicate that each one of the subsequent terms is to be interpreted as being modified by said antecedent term. For example, the scope of the term "linear or branched, substituted or non-substituted alkyl, alkenyl, alkynyl, carbocycle" encompasses linear or branched, substituted or non-substituted alkyl; linear or branched, substituted or non-substituted alkenyl; linear or branched, substituted or non-substituted alkynyl; linear or branched, substituted or non-substituted alkylidene; and linear or branched, substituted or non-substituted carbocycle. For example, the term "(C₂₋₁₀) alkenyl, alkynyl or alkylidene" indicates the group of compounds having 2 to 10 carbons and alkenyl, alkynyl or alkylidene functionality.

The expression "alkyl" refers to a saturated, straight-chain or branched hydrocarbon group that contains the number of carbon items indicated, e.g. "(C₁₋₁₀)alkyl" denotes a hydrocarbon residue containing from 1 to 10 carbon atoms, e.g. a methyl, ethyl, propyl, *is*o-propyl, *n*-butyl, *iso*-butyl, *sec*-butyl, *tert*-butyl, *n*-pentyl, *iso*-pentyl, *n*-hexyl, 2,2-dimethylbutyl, etc.

The expression "alkenyl" refers to an at least partially unsaturated, substituted or non-substituted straight-chain or branched hydrocarbon group that contains the number of carbon atoms indicated, e.g. "(C₂₋₁₀)alkenyl" denotes a hydrocarbon residue containing from 2 to 10 carbon atoms, for example an ethenyl (vinyl), propenyl (allyl), *iso*-propenyl, butenyl, isoprenyl or hex-2-enyl group, or, for example, a hydrocarbon group comprising a methylene chain interrupted by one double bond as, for example, found in monounsaturated fatty acids or a hydrocarbon group comprising methylene-interrupted polyenes, e.g. hydrocarbon groups comprising two or more of the following structural unit -[CH=CH-CH₂]-, as, for example, found in polyunsaturated fatty acids. Alkenyl groups have one or more, preferably 1, 2, 3, 4, 5, or 6 double bond(s).

The expression "alkynyl" refers to at least partially unsaturated, substituted or non-substituted straight-chain or branched hydrocarbon groups that contain the number of carbon items indicated, e.g. "(C₂₋₁₀)alkynyl" denotes a hydrocarbon residue containing from 2 to 10 carbon atoms, for example an ethinyl, propinyl, butinyl, acetylenyl, or propargyl group. Preferably, alkynyl groups have one or two (especially preferably one) triple bond(s).

Furthermore, the terms "alkyl", "alkenyl" and "alkynyl" refer to groups in which one or more hydrogen atom(s) have been replaced, e.g. by a halogen atom, preferably F or Cl, such as, for example, a 2,2,2-trichloroethyl or a trifluoromethyl group.

The term "carbocycle" shall be understood to mean a substituted or non-substituted hydrocarbon cycle containing the number of carbon items indicated, e.g. "(C₃₋₁₀)carbocycle" or from 3 to 20, preferably from 3 to 12 carbon atoms, more preferably 5 or 6 carbon atoms. These carbocycles may be either aromatic or non-aromatic systems. The non-aromatic ring systems may be mono- or polyunsaturated.

The term "carbobicycle" refers to a carbocycle as defined above comprising more than 1 ring, preferably two rings. Preferred carbocycles and carbobicycles include but are not limited to cyclopropyl, cyclobutyl, cyclopentyl, cyclopentenyl, cyclohexyl, cyclohexenyl, cycloheptanyl, cycloheptenyl, phenyl, indanyl, indenyl, benzocyclobutanyl, dihydronaphthyl, tetrahydronaphthyl, naphthyl, decahydronaphthyl, benzocycloheptanyl, benzocycloheptenyl, spiro[4,5]decanyl, norbornyl, decalinyl, bicyclo[4.3.0]nonyl, tetraline, or cyclopentylcyclohexyl. The carbo- and/or carbobicyclic residue may be bound to the remaining structure of the complete molecule by any atom of the cycle, which results in a stable structure

The term "carbocycle" shall also include "cycloalkyl" which is to be understood to mean aliphatic hydrocarbon-containing rings preferably having from 3 to 12 carbon atoms. These non-aromatic ring systems may be mono- or polyunsaturated, i.e. the term encompasses cycloalkenyl and cycloalkynyl.

The term "heterocycle" refers to a stable substituted or non-substituted, aromatic or non-aromatic, preferably 3 to 20 membered, more preferably 3-12 membered, most preferably 5 or 6 membered, monocyclic, heteroatom-containing cycle. Each heterocycle consists of carbon atoms and one or more, preferably 1 to 4, more preferably 1 to 3 heteroatoms preferably chosen from nitrogen, oxygen and sulphur. A heterocycle may contain the number of carbon atoms in addition to the non-carbon atoms as indicated: a "(C₃₋₆)heterocycle" is meant to have 3 to 6 carbon atoms in addition to a given number of heteroatoms.

The term "heterobicycle" refers to a heterocycle as defined above comprising more than 1 ring, preferably two rings.

The hetero- and/or heterobicyclic residue may be bound to the remaining structure of the complete molecule by any atom of the cycle, which results in a stable structure. Exemplary heterocycles and heterobicycles include, but are not limited to pyrrolidinyl, pyrrolinyl, morpholinyl, thiomorpholinyl, thiomorpholinyl sulfoxide, thiomorpholinyl sulfone, dioxalanyl, piperidinyl, piperazinyl, tetrahydrofuranyl, 1-oxo-λ4-thiomorpholinyl, 13-oxa-11-aza-tricyclo[7.3.1.0-2,7]-tridecy-2,4,6-triene, tetrahydropyranyl, 2-oxo-2H-pyranyl, tetrahydrofuranyl, 1,3-dioxolanone, 1,3-dioxanone, 1,4-dioxanyl, 8-oxa-3-aza-bicyclo[3.2.1]octanyl, 2-oxa-5-aza-bicyclo[2.2.1]hept-anyl, 2-thia-5-aza-bicyclo[2.2.1]heptanyl, piperidinonyl, tetrahydro-pyrimidonyl, pentamethylene sulphide, pentamethylene sulfoxide, pentamethylene sulfone, tetramethylene sulphide, tetramethylene sulfoxide and tetramethylene sulfone, indazolyl, benzimidazolyl, benzodioxolyl, imidazolyl, 1,3-benzodioxolyl and pyrazolyl.

The expressions "alkyl/alkenyl/alkynyl ether" refer to a saturated or non-saturated, straight-chain or branched hydrocarbon group that contains the number of carbon items indicated. For example, "(C₁₋₁₀)alkyl ether" denotes a hydrocarbon residue containing from 1 to 10 carbon atoms, and any suitable number of oxygen atoms that will result in an ether structure. Alkyl/alkenyl/alkynyl ether groups as used herein shall be understood to mean any linear or branched, substituted or non-substituted alkyl/alkenyl/alkynyl chain comprising an oxygen atom either as an ether motif, i.e. an oxygen bound by two carbons. The ether residue can be attached to the Formulas provided in the present invention either via the carbon atom or via the oxygen atom of the ether residue.

The "substituent" or "residue" or **"R"** as used herein, preferably **R^{1a}, R^{1b}, R^{2a}, R^{2b}, R^{3a}, R^{3b}, R^{4a}, R^{4b}, R⁵, R⁶, R⁷, R⁸, R^{9a}, R^{9b},** and/or **R¹⁰** can be attached directly to the Formulae provided in the present invention or by means of a linker. Said linker may be a polyethyleneglycol. The term polyethyleneglycol as used herein refers to a chain of substituted or non-substituted ethylene oxide monomers. If the forumulae and description note the residues **R¹, R², R³, R⁴,** and/or **R⁹** (i.e. without the letters "a" or "b"), then these residues can be residues as defined for either **R^{1a}/R^{1b}, R^{2a}/R^{2b}, R^{3a}/R^{3b}, R^{4a}/R^{4b},** and **R^{9a}/R^{9b}.**

As used herein, the terms "nitrogen" or "N" and "sulphur" or "S" include any oxidized form of nitrogen and sulphur and the quaternized form of any basic nitrogen as long as the resulting compound is chemically stable. For example, for an -S-C₁₋₆ alkyl radical shall be understood to include -S(O)-C₁₋₆alkyl and -S(O)₂-C₁₋₆ alkyl.

As used herein, a wording defining the limits of a range of length such as, e. g., "from 1 to 5" or "(C₁₋₅)" means any integer from 1 to 5, i. e. 1, 2, 3, 4 and 5. In other words, any range defined by two integers explicitly mentioned is meant to comprise and disclose any integer defining said limits and any integer comprised in said range.

By way of example, the term "substituted in *meta* position or substituted in *para* position", as used herein, means that a compound is either substituted by at least one given substituent in *para* position to the position where the compound is attached to another compound or residue, or substituted in two of its *meta* positions by at least one substituent. As denoted above for the *para* position, the *meta* position denotes the position *meta* to the position where the compound is attached to another compound or residue.

The scope of the present invention includes those analogs of the compounds as described above and in the claims that, e.g. for reasons of metabolic stability, feature the exchange of one or more carbon-bonded hydrogens, preferably one or more aromatic carbon-bonded hydrogens, with halogen atoms such as F, Cl, or Br, preferably F. For example, Compounds 1 to 10 can feature one or more halogen atoms, preferably F, instead of the aromatic carbon-bonded hydrogens in the phenyl ring.

Also, the scope of the present invention includes analogs of the compounds as described above that are in glycosylated form, e.g. anthraquinone glycosides, preferably beta-D-glucopyranosyl-, alpha-L-mannopyranosyl-beta-D-glucopyranosyl-, beta-D-mannopyranosyl-beta-D-gluco-pyranosyl-, beta-D-xylopyranosyl-beta-D-glucopyranosyl-, and/or beta-D-Glucopyranosyl-beta-D-glucopyranosyl-glycosides, or have a mono- or di-saccharide linked via an oxygen atom, preferably beta-D-glucopyranosyloxy-, alpha-L-mannopyranosyl-beta-D-glucopyranosyloxy-, beta-D-mannopyranosyl-beta-D-glucopyranosyloxy-, beta-D-xylopyranosyl-beta-D-glucopyranosyloxy-, and/or beta-D-Glucopyranosyl-beta-D-glucopyranosyloxy-glycosides.

The invention includes pharmaceutically acceptable salts or solvates of the compounds of the present invention. A "pharmaceutically acceptable salt or solvate" refers to any pharmaceutically acceptable salt, solvate or ester or any other compound which, upon administration to a patient, is capable of providing (directly or indirectly) a compound of the invention, or a pharmacologically active metabolite or pharmacologically active residue thereof. A pharmacologically active metabolite shall be understood to mean any compound for use in the invention capable of being metabolized enzymatically or chemically. This includes, for example, hydroxylated or oxidized derivative compounds of the present invention.

Pharmaceutically acceptable salts include those derived from pharmaceutically acceptable inorganic and organic acids and bases. Examples of suitable acids include hydrochloric, hydrobromic, sulphuric, nitric, perchloric, fumaric, maleic, phosphoric, glycolic, lactic, salicylic, succinic, toluene-p-sulfuric, tartaric, acetic, citric, methanesulfonic, formic, benzoic, malonic, naphthalene-2-sulfuric and benzenesulfonic acids. Other acids, such as oxalic acid, while not themselves pharmaceutically acceptable, may be employed in the preparation of salts useful as intermediates in obtaining the compounds and their pharmaceutically acceptable acid addition salts. Salts derived from appropriate bases include alkali metal (e.g., sodium), alkaline earth metal (e.g. magnesium), ammonium and N-(C₁-C₄ alkyl)₄⁺ salts.

In addition, the scope of the invention also encompasses prodrugs of compounds of the present invention. Prodrugs include those compounds that, upon simple chemical transformation, are modified to produce compounds of the invention. Simple chemical transformations include hydrolysis, oxidation and reduction. Specifically, when a prodrug is administered to a patient, the prodrug may be transformed into a compound disclosed hereinabove, thereby imparting the desired pharmacological effect.

In another preferred embodiment, the anthraquinone or anthraquinone derivative for use in the present invention is an anthraquinone or anthraquinone derivative according to Formula (IIIa), 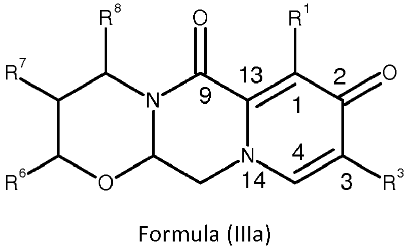 and R¹, R³ and R⁶ to R⁸ are as defined above for Formulas (III), (IV) and (V), preferably:
R⁶ and R⁷ are hydrogen;
R⁸ is a linear or branched, substituted or non-substituted (C₁₋₁₀)alkyl, (C₂₋₁₀)alkenyl, (C₂₋₁₀)alkynyl, preferably (C₁₋₁₀)alkyl, more preferably substituted or non-substituted methyl, ethyl or propyl, most preferably substituted or non-substituted methyl; and/or R¹ is OH or -O(C₁₋₁₀)alkyl, preferably -OMe, -OEt, or -O(C=O)(C₁₋₁₀)alkyl, more preferably-O(C=O)Me or OH; and/or
R³ is OH, a linear or branched, substituted or non-substituted (C₁₋₁₀)alkyl, (C₂₋₁₀)alkenyl, (C₂₋₁₀)alkynyl, preferably (C₁₋₁₀)alkyl, more preferably substituted or non-substituted methyl, ethyl or propyl, most preferably substituted or non-substituted methyl, -O(C₁₋₁₀)alkyl, preferably -OMe or -OEt, -O(C₃₋₁₀)cycloalkyl, preferably -O-cyclopropyl, -O(C=O)(C₁₋₁₀)alkyl, preferably -O(C=O)Me, -O(C=O)(C₃₋₁₀)cycloalkyl, preferably -O(C=O)-cyclopropyl, COOH, COOMe, COOEt, or C(C=O)NH₂.

In another preferred embodiment, the anthraquinone or anthraquinone derivative for use in the present invention is an anthraquinone or anthraquinone derivative according to Formula (IIIb), 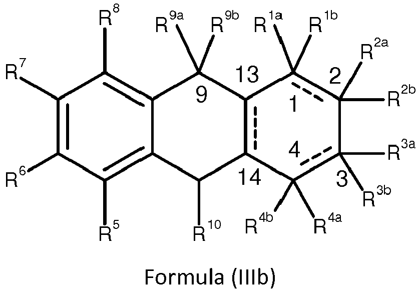 wherein R¹ to R⁹ are as defined above for Formulas (III), (IV) and (V), and R¹⁰ is -OH or =O.

In a further preferred embodiment, the anthraquinone or anthraquinone derivative for use in the present invention is an anthraquinone or anthraquinone derivative according to Formula (IIIc), 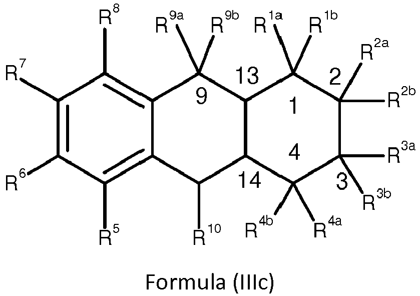 wherein
**R^{1a}** and **R^{1b}, R^{2a}** and **R^{2b}, R^{3a}** and **R^{3b}, R^{4a}** and **R^{4b},** and **R⁵** to **R⁸** are each independently selected from the group consisting of:
   (i) H, or-OH;
   (ii) -O(C₁₋₆)alkyl, preferably -OMe or -OEt, -O(C₃₋₆)cycloalkyl, preferably -O-cyclopropyl,-O(C=O)(C₁₋₃)alkyl, preferably -O(C=O)Me, or -O(C=O)(C₃₋₆)cycloalkyl, preferably-O(C=O)cyclopropyl;
   (iii) linear or branched, substituted or non-substituted (C₂₋₁₀)alkyl ether, (C₃₋₁₀)alkenyl ether, (C₃₋₁₀)alkynyl ether or (C₄₋₁₀)carbocyclic ether, wherein the ether is bonded to formula (IIIc) via its carbon atom;
   (iv) linear or branched, substituted or non-substituted (C₁₋₆)alkyl, (C₂₋₆)alkenyl, (C₂₋₆)alkynyl, preferably (C₁₋₆)alkyl, more preferably substituted or non-substituted methyl, ethyl or propyl, most preferably substituted or non-substituted methyl;
   (v) substituted or non-substituted carbocycle selected from the group consisting of (C₃₋₁₀)carbocycle, preferably (C₃)carbocycle and (C₅₋₆)carbocycle, preferably aromatic (C₆)carbocycle, more preferably a non-substituted phenyl or a phenyl that is substituted by a substituent selected from the group consisting of Cl, F, Br, substituted or non-substituted methyl, preferably-(CF₃), ethyl, propyl and cyclopropyl;
   (vi) substituted or non-substituted (C₃₋₆)heterocycle or (C₇₋₁₀)carbo- or hetero-bicycle having 1 to 3 heteroatoms each independently selected from N, O and S, preferably substituted or non-substituted heterobicycle having 2 oxygen atoms, preferably substituted or non-substituted benzodioxolyl connected via position (5) or (6) of the benzodioxolyl;
**R^{9a}** and **R^{9b}** are independently selected from the group consisting of H, Me, -OH, -OMe, and =O, preferably R^{9a} is H and R^{9b} is -OH, more preferably R^{9a} is OH and R^{9b} is Me; and/or **R¹⁰** is -OH or =O.

In a further preferred embodiment, the anthraquinone or anthraquinone derivative for use in the present invention is an anthraquinone or anthraquinone derivative according to Formula (IIId), 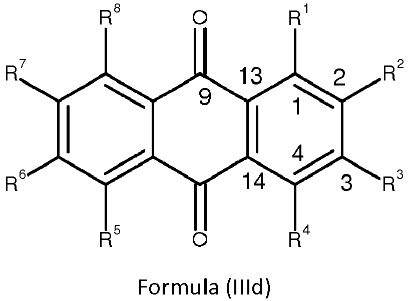 wherein
**R¹** to **R⁸** are each independently selected from the group consisting of:
   (i) H, F, Cl, Br, -OH, -OSO₃H and -SO₃H;
   (ii) -O(C₁₋₆)alkyl, preferably -OMe or -OEt, -O(C₃₋₆)cycloalkyl, preferably -O-cyclopropyl,-O(C=O)(C₃₋₆)cycloalkyl, preferably -O(C=O)cyclopropyl or -O(C=O)(C₁₋₃)alkyl, preferably-O(C=O)Me;
   (iii) -COOH, -COO(C₁₋₁₀)alkyl, -COO(C₃₋₆)cycloalkyl, -COONH₂, -COON((C₁₋₁₀)alkyl)₂, or-COONH(C₁₋₁₀)alkyl, preferably -COOH, -COOMe or COOEt;
   (iv) linear or branched, substituted or non-substituted (C₂₋₁₀)alkyl ether, (C₃₋₁₀)alkenyl ether, (C₃₋₁₀)alkynyl ether or (C₄₋₁₀)carbocyclic ether, wherein the ether is bonded to formula (IIId) via its carbon atom;
   (v) linear or branched, substituted or non-substituted (C₁₋₁₀)alkyl, (C₂₋₁₀)alkenyl, or (C₂₋₁₀)-alkynyl, preferably (C₁₋₁₀)alkyl, more preferably substituted or non-substituted -CHO, methyl, ethyl or propyl, most preferably substituted or non-substituted methyl;
   (vi) substituted or non-substituted carbocycle selected from the group consisting of (C₃₋₁₀)-carbocycle, preferably (C₃)carbocycle and (C₅₋₆)carbocycle, preferably aromatic (C₆)carbocycle, more preferably a non-substituted phenyl or a phenyl that is substituted by a substituent selected from the group consisting of Cl, F, Br, substituted or non-substituted methyl, preferably-(CF₃), ethyl, propyl and cyclopropyl;
   (vii) substituted or non-substituted (C₃₋₆)heterocycle or (C₇₋₁₀)carbo- or hetero-bicycle having 1 to 3 heteroatoms each independently selected from N, O and S, preferably substituted or non-substituted heterobicycle having 2 oxygen atoms, preferably substituted or non-substituted benzodioxolyl connected via position (5) or (6) of the benzodioxolyl; and/or
**R²** and **R³** together form a ring selected from the group consisting of
   (i) substituted or non-substituted (C₃₋₁₀)carbocycle, preferably (C₃)carbocycle or (C₅₋₆)-carbocycle, preferably aromatic (C₆)carbocycle, more preferably a non-substituted phenyl or a phenyl that is substituted by a substituent selected from the group consisting of Cl, F, Br, substituted or non-substituted methyl, preferably -(CF₃), ethyl, propyl and cyclopropyl; and
   (ii) substituted or non-substituted (C₃₋₆)heterocycle or (C₇₋₁₀)carbo- or hetero-bicycle having 1 to 3 heteroatoms each independently selected from N, O and S, preferably substituted or non-substituted heterobicycle having 2 oxygen atoms.

In another preferred embodiment, the anthraquinone or anthraquinone derivative for use according to the present invention is an anthraquinone or anthraquinone derivative according to Formula (IIId), 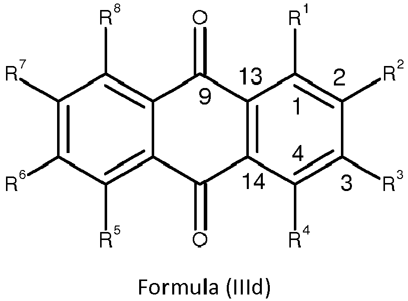 wherein
**R¹** to **R⁸** are each independently selected from the group consisting of:
   (i) H, F, Cl, -OH, and -OSO₃H;
   (ii) -OMe, -OEt, -O-cyclopropyl, -O(C=O)cyclopropyl, -O(C=O)Me or -O(C=O)Et;
   (iii) -COOH, -COONH₂, -COOMe or -COOEt;
   (iv) linear or branched, substituted or non-substituted (C₂₋₁₀)alkyl ether wherein the ether is bonded to formula (IIId) via its carbon atom, preferably an ether selected from the group consisting of 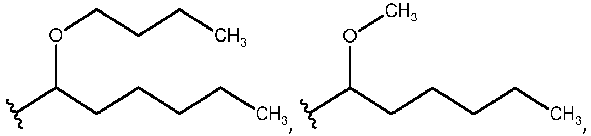 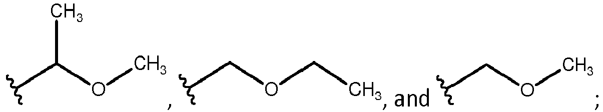
   (v) linear or branched, substituted or non-substituted (C₁₋₁₀)alkyl or (C₂₋₁₀)alkenyl, preferably substituted or non-substituted methyl, ethyl and propyl, most preferably selected from the group consisting of methyl, ethyl, -MeOH, -EtOH, -CHO, 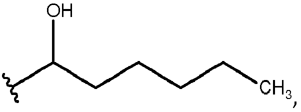 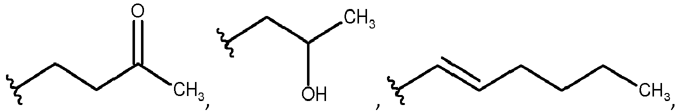 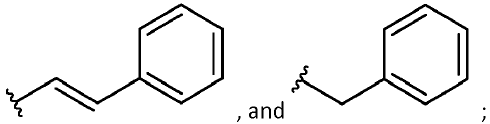
   (vi) substituted or non-substituted carbocycle (C₃₋₆)carbocycle, preferably (C₃)carbocycle or (C₅₋₆)carbocycle, preferably aromatic (C₆)carbocycle, more preferably a substituted or non-substituted carbocycle selected from the group consisting of phenyl, benzenediol, preferably *p,m*-benzenediol, cyclohexyl, cyclopentyl, and cyclopenta-1,3-dienyl;
   (vii) substituted or non-substituted (C₃₋₆)heterocycle or (C₇₋₁₀)carbo- or hetero-bicycle having 1 to 3 heteroatoms each independently selected from N, O and S, preferably substituted or non-substituted benzodioxolyl connected via position (5) or (6) of the benzodioxolyl; and/or
**R²** and **R³** together form a ring selected from the group consisting of
   (i) substituted or non-substituted (C₃₋₁₀)carbocycle; and
   (ii) substituted or non-substituted (C₃₋₆)heterocycle or (C₇₋₁₀)carbo- or hetero-bicycle having 1 to 3 heteroatoms each independently selected from N, O and S, preferably substituted or non-substituted heterobicycle having 2 oxygen atoms,
preferably R² and R³ together form a ring selected from the group consisting of

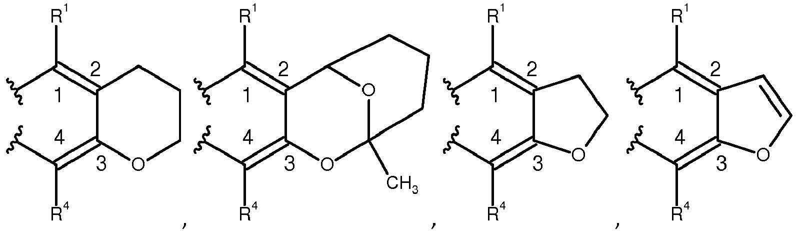

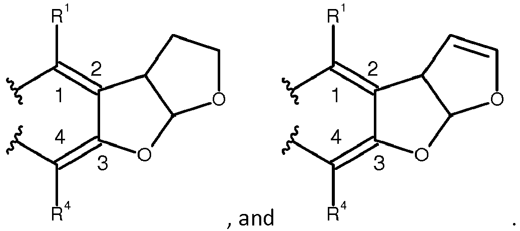

In a further preferred embodiment, the anthraquinone or anthraquinone derivative for use in the present invention is selected from the group consisting of:
1,2-dihydroxyanthracene-9,10-dione (Alizarin); 1,3,4-trihydroxy-9,10-dioxo-anthracene-2-carboxylic acid (Pseudopurpurin); 1,2,4-trihydroxyanthracene-9,10-dione (Purpurin); 1,3-dihydroxy-2-methyl-anthracene-9,10-dione (Rubiadin); 1,3,-dihydroxy-2(hydroxymethyl)-anthracene-9,10-dione (Lucidin); 2-methylanthracene-9,10-dione (Tectochinone); 2-(hydroxyl-methyl)anthracene-9,10-dione; 2-methoxyanthracene-9,10-dione; 2-hydroxyanthracene-9,10-dione; 1-hydroxyanthracene-9,10-dione; 1-hydroxy-2-methyl-anthracene-9,10-dione; 1-hydroxy-2-(hydroxymethyl)anthracene-9,10-dione; ethyl 1-hydroxy-9,10-dioxo-anthracene-2-carboxylate; 1-methoxy-2-methyl-anthracene-9,10-dione; 1-hydroxy-2-methoxy-anthracene-9,10-dione; 2-hydroxy-1-methoxy-anthracene-9,10-dione; 1,2,-dimethoxyanthracene-9,10-dione; 1,3-dihydroxy-9,10-dioxo-anthracene-2-carbaldehyde; 1,3-dihydroxy-9,10-dioxo-anthracene-2-carboxylic acid; methyl-1,3-dihydroxy-9,10-dioxo-anthracene-2-carboxylate; 2-(ethoxymethyl)-1,3,-dihydroxy-anthracene-9,10-dione; 1,3-dihydroxy-2-(methoxymethyl)anthracene-9,10-dione; 2-(hydroxymethyl)-1,3-dimethoxy-anthracene-9,10-dione; 1,3-dihydroxy-9,10-dioxo-anthracene-2-carboxylic acid; 1,3-dimethoxy-9,10-dioxo-anthracene-2-carboxylic acid; 1,2,3-trihydroxyan-thracene-9,10-dione; 1,3-dihydroxyanthracene-9,10-dione; 1,3-dihydroxy-2-phenyl-anthracene-9,10-dione; 2-benzyl-1,3-dihydroxy-anthracene-9,10-dione; 1,3-dihydroxy-2-methoxy-anthracene-9,10-dione; 1,2-dihydroxy-3-methoxy-anthracene-9,10-dione; 1-hydroxy-2,3-dimethoxy-anthracene-9,10-dione; 1-hydroxy-3-methoxy-9,10-dioxo-anthracene-2-carboxylic acid; 3-hydroxy-1-methoxy-2-(methoxymethyl)anthracene-9,10-dione; 1,4-dihydroxyanthracene-9,10-dione (Quinizarin); 1,4-dihydroxy-2-(hydroxymethyl)anthracene-9,1-dione; ethyl-1,4-dihydroxy-9,10-dioxo-anthracene-2-carboxylate; 2-(ethoxymethyl)-1,4-dihydroxy-anthracene-9,10-dione (Christofin); 1,4-dihydroxy-2-methyl-anthracene-9,10-dione; 1-hydroxy-3-methoxy-anthracene-9,10-dione; 1,3-dimethoxyanthracene-9,10-dione; methyl-4-hydroxy-9,10-dioxo-anthracene-2-carboxylate; 1,4-dihydroxy-5-methoxy-2-methyl-anthracene-9,10-dione; 1,4-dihydroxy-8-methoxy-2-methyl-anthracene-9,10-dione; 1,4-diyhdroxy-6-methyl-anthracene-9,10-dione; 1,5-dihydroxy-2-methyl-anthracene-9,10-dione; 1,8-dihydroxy-3-methoxy-6-methyl-anthracene-9,10-dione (Physcion, Parietin); 1,3,6-trihydroxy-2-methyl-anthracene-9,10-dione; 1,8-dihydroxy-3-methoxy-6-methyl-anthracene-9,10-dione; 3,6-dihydroxy-1-methoxy-2-methyl-anthracene-9,10-dione; 2-hydroxy-7-methyl-anthracene-9,10-dione; 4-hydroxy-9,10-dioxo-anthracene-9,10-dione; 1,6-dihydroxy-2-methyl-anthracene-9,10-dione; 1,3,8-trihydroxy-6-(hydroxymethyl)-anthracene-9,10-dione (Citreorosein); 1,8-dihydroxy-3-(hydroxymethyl)-6-methoxy-anthracene-9,10-dione (Telochistin); 3,4,6-trihydroxy-2,7-dimethoxy-benzo[f]benzofuran-5,8-dione (Rhodocladonic acid); 1,8-dihydroxy-3,6-dimethoxy-anthracene-9,10-dione (Fallacinol); 1,3,6,8-tetrahydroxy-2-[E-styryl]anthracene-9,10-dione (Averythrin); 1,6,8-trihydroxy-3-methyl-9,10-dioxo-anthracene-2-carboxylic acid (Endocrocin); 1,6,8-trihydroxy-3-methyl-9-oxo-10H-anthracene-2-carboxylic acid; 1,4,5-trihydroxy-7-methoxy-2-methyl-anthracene-9,10-dione; 1,4,5,7-tetrahydroxy-2-(hydroxymethyl)anthracene-9,10-dione (Tritisporin); 1,5,8-trihydroxy-3-methylanthracene-9,10-dione (Helminthosporin); 1,4,5,8-tetrahydroxy-2-methyl-anthracene-9,10-dione (Cynodontin); 7-alpha-D-Glucopyranosyl-9,10-dihydro-3,5,6,8-tetrahydroxy-1-methyl-9,10-dioxo-anthracenecarboxylic acid (Carminic acid); 1,5,-dihydroxy-8-methoxy-3-methyl-anthracene-9,10-dione (Questin); 4,5,7-trihydroxy-9,10-dioxo-anthracene-2-carboxylic acid (Emodic acid); 1,6-dihydroxy-3-(hydroxymethyl)-8-methoxy-anthracene-9,10-dione (Questinol); 1,3-dihydroxy-6-(hydroxymethyl)-8-methoxy-anthracene-9,10-dione (Carviolin); 1,3,8-trihydroxy-6-(hydroxyl-methyl)anthracene-9,10-dione (Citreorosein); 2,8-dihydroxy-1-methoxy-3-methyl-anthracene-9,10-dione; 3-acetyl-1,2,4,5,7-pentahydroxy-anthracene-9,10-dione; 1,3,8-trihydroxy-6-methylanthracene-9,10-dione (Emodin); Emodin-6-O-rhamnoside (Cascarin); 4,5-dihydroxy-9,10-dioxo-anthracene-2-carboxylic acid (Rhein); 1,4,5,7-tetrahydroxy-2-methyl-anthracene-9,10-dione (Catenarin); 1,8-dihydroxy-3-(hydroxymethyl)anthracene-9,10-dione (Aloe-emodin); 1,8-dihydroxy-3-methyl-anthracene-9,10-dione (Chrysophanol); Rhein-8-glucoside; 1,3,8-trihydroxy-6-methyl-anthracene-9,10-dione (Alatinone); 4,5-diacetoxy-7-hydroxy-9,10-dioxo-anthracene-2-carboxylic acid (Diacerhein); 1,3,5,8-tetrahydroxy-6-methyl-anthracene-9,10-dione; 1,4-dihydroxy-6,7-dimethoxy-3-methyl-9,10-dioxo-anthracene-2-carboxylic acid; 1,3-dihydroxy-6,8-dimethoxy-anthracene-9,10-dione; 1,3,5,8-tetrahydroxy-2-methyl-anthracene-9,10-dione; 1,3,8-trihydroxy-2-methyl-1,2-dihydroanthracene-9,10-dione; 1-methoxy-anthracene-9,10-dione; 1,8-dihydroxy-6-methoxy-2-methyl-anthracene-9,10-d ione; 1,8-dihydroxy-3-methoxy-6-methyl-anthracene-9,10-dione; 2,3,4,8-tetrahydroxy-6-methoxy-3-methyl-2,4-dihydro-1H-anthracene-9,10-dione; 1,3,6,8-tetrahydroxyanthracene-9,10-dione; 2,3,5-trihydroxy-7-methyl-1,2,3,4-tetrahydroanthracene-9,10-dione; 1,3-dihydroxy-8-methoxy-6-methyl-anthracene-9,10-dione; 2,3,4,8,10-pentahydroxy-6-methoxy-3-methyl-1,2,4,10-tetrahydroanthracen-9-one; 1,3,7-trihydroxy-6-(2-hydroxypropyl)anthracene-9,10-dione; 1,3,6,8-tetrahydroxy-2-(1-hydroxyethyl)-anthracene-9,10-dione; 5,7,9-trihydroxy-3,4-dihydro-2H-naptho[2,3-g]chromene-6,11-dione; 2-chloro-1,6-dihydroxy-8-methoxy-3-methyl-anthracene-9,10-dione; 1,3,6,8-tetrahydroxy-2-(1-methoxyethyl)anthracene-9,10-dione; 1,3,8-trihydroxy-9,10-dioxo-anthracene-2-carbaldehyde; 2,3,5,8,10-pentahydroxy-6-methoxy-3-methyl-1,2,4a,9a,10-hexahydroanthracen-9-one; (3,5,8,10-tetrahydroxy-6-methoxy-3-methyl-9-oxo-1,2,4,4a,9a,10-hexahydroanthracen-2-yl)acetate; 2,16,18-trihydroxy-7,9-dioxapentacyclo[10.8.0.03,10.04,8.014,19]icosa-1(12),2,10,14(19),-15,17-hexaene-13,20-dione; 2,16,18-trihydroxy-7,9-dioxapentacyclo[10.8.0.03,10.04,-8.014,19]icosa-1(12),2,5,10,14(19),15,17-heptaene-13,20-dione; 4-hydroxy-5-methoxy-9,10-dioxo-anthracene-2-carbaldehyde; 6,8-dimethoxy-1-methyl-2-(3-oxobutyl)anthracene-9,10-dione; 2-[(E)-hex-1-enyl]-1,3,6,8-tetrahydroxy-anthracene-9,10-dione; 3,7-dihydroxy-9-methoxy-17-methyl-16,21-dioxapentacyclo[15.3.1.02,15.04,13.06,11]henicosa-2,4(13),6(11),7,9,14-hexaene-5,12-dione; 1,3,6,8-tetrahydroxy-2-(1-methoxyhexyl)anthracene-9,10-dione; 1,3,6,8-tetrahydroxy-2-(1-hydroxyhexyl)anthracene-9,10-dione; 2-chloro-1,3,6,8-tetrahydroxy-7-(1-hydroxyhexyl)anthracene-9,10-dione; 2-(1-butoxyhexyl)-7-chloro-1,3,6,8-tetrahydroxy-anthracene-9,10-dione; (3,5,8,10-tetrahydroxy-6-methoxy-3-methyl-9-oxo-1,2,4,4a,9a,10-hexa-hydroanthracen-2-yl) acetate; 11-hydroxy-7-methyl-9,12-dioxo-4-oxa-1,8-diazatricyclo[8.4.0.-03,8]tetradeca-10,13-diene-13-carboxylic acid; 11-hydroxy-7,13-dimethyl-4-oxa-1,8-diaza-tricyclo[8.4.0.03,8]tetradeca-10,13-diene-9,12-dione; 11-hydroxy-7-methyl-9,12-dioxo-4-oxa-1,8-diazatricyclo[8.4.0.03,8]tetradeca-10,13-diene-13-carboxylic acid; 1,3-dihydroxy-2-phenyl-anthracene-9,10-dione; 2-(3,4-dihydroxyphenyl)-1,3-dihydroxy-anthracene-9,10-dione; [3-(1,3-benzodioxol-5-yl)phenyl]-(o-tolyl)methanone; [4-(1,3-benzodioxol-5-yl)pyrimidin-2-yl]-(o-tolyl)-methanone; 2-(1,3-benzodioxol-5-yl)-3-hydroxy-benzo[g]quinoline-5,10-dione; 2-(1,3-benzodi-oxol-5-yl)-1,3-dihydroxy-anthracene-9,10-dione; 1-hydroxy-3-methylanthraquinone; 1,3,6,8-Tetrahydroxyanthraquinone; Coniothranthraquinone 1((2*S*,3*R*)-2,3,5-trihydroxy-7-methyl-1,2,3,4-tetrahydroanthraquinone); Macrosporin (1,7-dihydroxy-3-methoxy-6-methylanthraquinone); Austrocortinin (1,4-dihydroxy-2-methoxy-7-methylanthraquinone); 1-Methylemodin (1,3-dihydroxy-8-methoxy-6-methylanthraquinone); Macrospin (1,6-dihydroxy-3-methoxy-7-methyl-anthraquinone); Monodictyquinone (1,8-dihydroxy-2-methoxy-6-methylanthraquinone); 2,3,5,8-Tetrahydroxy-7-methoxy-2-methyl-1,2,3,4-tetrahydroanthraquinone; 10-Deoxybostrycin; Dihydroaltersolanol C((1*R*,2*R*,3*R*)-1,2,3,5-tetra-hydroxy-7-methoxy-2-methyl-1,2,3,4,4a,9a-hexa-hydroanthraquinone); Lunatin(1,3,8-trihydroxy-6-methoxyanthraquinone); Trichodermaquinone ((2*S*,3*R*)-2,3,5-trihydroxy-7-(hydroxylmethyl)-1,2,3,4-tetrahydroanthraquinone); 2,8-dihydroxy-1,3-dimethoxy-6-methyl anthraquinone; Erythroglaucin (1,4,5-trihydroxy-7-methoxy-2-methyl-anthraquinone); Fusaquinon C((2*S*,3*R*,4a*R*,9a*R*,10*S*)-2,3,5,8,10-pentahydroxy-6-methoxy-3-methyl-1,3,4,4a,9a,10-hexahydroanthracen-9(2*H*)-one); Rubocristin(1,4,7-trihydroxy-5-methoxy-2-methylanthraquinone); 6-*O*-methylalaternin(1,2,8-trihydroxy-6-methoxy-3-methylanthraquinone); 1,4,6-Trihydroxy-2-methoxy-7-methyl-anthraquinone; 7-Chloroemodin; Altersolanol B ((2*S*,3*R*)-2,3,5-trihydroxy-7-methoxy-2-methyl-1,2,3,4-tetrahydroanthraquinone); Fusaquinon A ((2*R*,3*S*,4a*R*,9*S*,9a*S*)-3,5,8-trihydroxy-7-methoxy-2-methyl-2,3,4,4a,9,9a-hexahydro-2,9-epoxy-anthracen-10(1*H*)-one); Dihydroaltersolanol B((2*S*,3*R*)-2,3,5-trihydroxy-7-methoxy-2-methyl-1,2,3,4,4a,9a-hexahydroanthraquinone); Xylanthraquinone; Isorhodoptilometrin((*R*)-1,3,8-trihydroxy-6-(2-hydroxypropyl)anthraquinone); 1,3,6,8-Tetrahydroxy-2-(1-hydroxyethyl) anthraquinone; 1-*O*-Methyl-7-chloroemodin(2-chloro-1,6-dihydroxy-8-methoxy-3-methylanthraquinone); 7-Chlorocitreorosein; Austrocortirubin(1,4-dihydroxy-2-methoxy-7-methylanthraquinone); 1-Deoxytetrahydrobostrycin((2*R*,3*S*)-2,3,5,8,10-pentahydroxy-6-methoxy-3-methyl-1,3,4,4a,9a,10-hexahydroanthracen-9(2*H*)-one); Fragilin(2-chloro-1,8-dihydroxy-3-methoxy-6-methylanthraquinone); 5-Acetyl-2-methoxy-1,4,6-trihydroxyanthraquinone; Isorhodoptilo-metrin-1-methylether(1,3-dihydroxy-6-2-hydroxypropyl-8-methoxyanthraquinone); 1,3,6,8-Tetrahydroxy-2-(1-methoxyethyl)anthraquinone; Phomopsanthraquinone((2*R*,3*S*)-7-ethyl-1,2,3,4-tetrahydro-2,3,8-trihydroxy-6-methoxy-3-methylanthraquinone); Altersolanol A ((1*R*,2*S*,3*R*,4*S*)-1,2,3,4,5-pentahydroxy-7-methoxy-2-methyl-1,2,3,4-tetrahydroanthraquinone); Bostrycin((5*S*,6*R*,7*S*)-5,6,7,9,10-pentahydroxy-2-methoxy-7-methyl-5,6,7,8-tetrahydroanthracene-1,4-dione); SZ-685C ((1,2,3,5,8-pentahydroxy-6-methoxy-3-methyl-1,2,3,4-tetrahydro-anthraquinone); Fusaquinon B ((1*R*,2*S*,3*R*,4a*R*,9a*S*,10*S*)-1,2,3,5,8,10-hexahydroxy-6-methoxy-3-methyl-1,3,4,4a,9a,10-hexahydroanthracen-9(2*H*)-one); Tetrahydroxybostrycin((1,2,3,5,8,10-hexahydroxy-6-methoxy-3-methyl-1,3,4,4a,9a,10-hexahydroanthracen-9(2*H*)-one); Versicolorin C((4,6,8-trihydroxy-3,3a-dihydroanthra[2,3-b]furo[3,2-d]furan-5,10(2*H*,12a*H*)-dione); 2-*O*-Acetylaltersolanol B((2*R*,3*S*)-3,8-dihydroxy-6-methoxy-3-methyl-9,10-dioxo-1,2,3,4,9,10-hexahydro-anthracen-2-yl acetate); 1,2,3,6,8-Pentahydroxy-7-(1-methoxyethyl)anthraquinone; Emodin-3-*O* Sulfate((4,5-dihydroxy-7-methyl-9,10-dioxo-9,10-dihydroanthracen-2-yl hydrogen sulfate); Auxarthrol C((1*S*,2*R*,3*R*,4*R*,4a*R*,9a*S*)-1,2,3,4,5-pentahydroxy-7-methoxy-2-methyl-1,2,3,4-tetrahydro-4a,9a-epoxyanthraquinone); 8-*O*-MethylversicolorinB((4,8-dihydroxy-6-methoxy-3,3a-dihydroanthra[2,3-b]furo[3,2-d]furan-5,10(2*H*,12a*H*)-dione); 6,8-Dimethoxy-1-methyl-2-(3-oxobutyl)anthraquinone; 8-*O*-Methylversicolorin A (4,8-dihydroxy-6-methoxyanthra[2,3-b]furo-[3,2-d]furan-5,10(3a*H*,12a*H*)-dione); Averythrin((*E*)-2-(hex-1-en-1-yl)-1,3,6,8-tetrahydroxy-anthraquinone); Skyrin (2,2',4,4',5,5'-hexahydroxy-7,7'-dimethyl-[1,1'-bianthracene]-9,9',10,10'-tetraone); Macrosporin-7-*O*-sulfate(Sodium 8-hydroxy-6-methoxy-3-methyl-9,10-dioxo-9,10-dihydroanthracen-2-yl sulfate); Citreorosein-3-*O*-sulfate(4,5-dihydroxy-7-(hydroxymethyl)-9,10-dioxo-9,10-dihydroanthracen-2-yl hydrogen sulfate); 6,8-di-*O*-methylversicolorinA (4-hydroxy-6,8-dimethoxyanthra[2,3-b]furo[3,2-d]furan-5,10(3a*H*,12a*H*)-dione); 8-*O*-Methylaverythrin((*E*)-2-(hex-1-en-1-yl)-1,3,6-trihydroxy-8-methoxyanthraquinone); Aversin (4-hydroxy-6,8-dimethoxy-3,3a-dihydroanthra[2,3-b]furo[3,2-d]furan-5,10(2*H*,12a*H*)-dione); Aversin((-)-isomer of (4-hydroxy-6,8-dimethoxy-3,3a-dihydroanthra[2,3-b]furo[3,2-d]furan-5,10(2*H*,12a*H*)-dione); Averantin((*S*)-1,3,6,8-tetrahydroxy-2-(1-hydroxyhexyl)anthraquinone); 6-*O*-Methylaverufin(7,9-dihydroxy-11-methoxy-2-methyl-3,4,5,6-tetrahydro-2*H*-2,6-epoxyanthra[2,3-b]oxocine-8,13-dione); Nidurufin(5,7,9,11-tetrahydroxy-2-methyl-3,4,5,6-tetrahydro-2*H*-2,6-epoxyanthra[2,3-b]oxocine-8,13-dione); Averufin(7,9,11-trihydroxy-2-methyl-3,4,5,6-tetrahydro-2*H*-2,6-epoxy-anthra[2,3-b]oxocine-8,13-dione); 1'-*O*-Methylaverantin(1,3,6,8-tetrahydroxy-2-(1-methoxy-hexyl)anthraquinone); (2*S*)-2,3-Dihydroxy-propyl-1,6,8-trihydroxy-3-methyl-9,10-d ioxoanthracene-2-carboxylate; 7-Chloroaverythrin((*E*)-2-chloro-7-(hex-1-en-1-yl)-1,3,6,8-tetrahydroxy-anthraquinone); 6,8-Di-*O*-methylversiconol(2-(1,4-dihydroxybutan-2-yl)-1,3-dihydroxy-6,8-dimethoxyanthraquinone); 6,8-Di-*O*-methylaverufin(7-hydroxy-9,11-dimetho-xy-2-methyl-3,4,5,6-tetra-hydro-2*H*-2,6-epoxyanthra[2,3-b]oxocine-8,13-dione); 6,8-Di-*O*-methylnidurufin (5,7-dihydroxy-9,11-dime-thoxy-2-methyl-3,4,5,6-tetrahydro-2*H*-2,6-epoxyanthra[2,3-b]oxocine-8,13-dione); 1,3-dihydroxy-6,8-dimethoxy-2-(6-methyltetra-hydro-2*H*-pyran-2-yl)anthracene-9,10-dione; 6,1'-*O*,*O*-Dimethylaverantin((*S*)-1,3,8-trihydroxy-6-methoxy-2-(1-methoxyhexyl)-anthraquinone); Variecolorquinone A ((*S*)-2,3-dihydroxypropyl 1,6-dihydroxy-8-methoxy-3-methyl-9,10-dioxo-9,10-dihydroanthracene-2-carboxylate); 6,8-Di-*O-*methylaverantin((*S*)-1,3-dihydroxy-2-(1-hydroxyhexyl)-6,8-dimetho-xyanthraquinone); 6-*O*-Methyl-7-chloroaverythrin ((*E*)-2-chloro-7-(hex-1-en-1-yl)-1,6,8-trihydroxy-3-methoxyanthraquinone); (1'S)-7-Chloroaverantin((*S*)-2-chloro-1,3,6,8-tetrahydroxy-7-(1-hydroxyhexyl)anthraquinone); 6,1'-*O,O*-Dimethyl-7-chloroaverantin((*S*)-2-chloro-1,6,8-trihydroxy-3-methoxy-7-(1-methoxyhexyl)anthraquinone); 6,8,1'-Tri-*O*-methyl-averantin(1,3-dihydroxy-6,8-dimethoxy-2-(1-methoxyhexyl)anthraquinone); 6-3-*O*-(Ribofuranosyl)questin(1,6-dihydroxy-6-*O*-(ribofuranosyl)-8-methoxy-3-methylanthraquinone); 1'-*O*-methyl-7-chloro averantin((*S*)-2-chloro-1,3,6,8-tetrahydroxy-7-(1-methoxyhexyl)anthraquinone); 6-*O*-methyl-7-chloro-averantin((*S*)-2-chloro-1,6,8-trihydroxy-7-(1-hydroxyhexyl)-3-methoxyanthraquinone); Averantin-1'-butyl ether((*S*)-2-(1-butoxyhexyl)-1,3,6,8-tetrahydroxy-anthraquinone); 7-Chloroaverantin-1'-butyl ether((*S*)-2-(1-butoxyhexyl)-7-chloro-1,3,6,8-tetrahy-droxyanthraquinone); 6-*O*-Methyl-7-bromoaverantin((*S*)-2-bromo-1,6,8-trihydroxy-7-(1-hydroxyhexyl)-3-methoxyanthraquinone); 6,1'-*O*,*O*-Dimethyl-7-bromoaverantin ((*S*)-2-bromo-1,6,8-trihydroxy-3-methoxy-7-(1-methoxyhexyl)anthraquinone); Macrosporin2-*O-*(6'-acetyl)-a-d-glucopyranoside((2*R*,3*S*,4*S*,5*R*,6*R*)-3,4,5-trihydroxy-6-((8-hydroxy-6-methoxy-3-methyl-9,10-d ioxo-9,10-dihydroanthracen-2-yl)oxy)tetrahydro-2H-pyran-2-yl)methyl acetate); Penicillanthranin A ((1*S*,3*R*,4*S*)-1-(6-ethyl-1,3,8-trihydroxy-9,10-dioxo-9,10-dihydroanthracen-2-yl)-6,8-dihy-droxy-3,4,5-trimethyliso-chroman-7-carboxylic acid); Penicillanthranin B ((1*S*,3*R*,4*S*)-6,8-dihydroxy-3,4,5-trimethyl-1-(1,3,8-trihydroxy-6-(hydroxy-methyl)-9,10-dioxo-9,10-dihydro-anthracen-2-yl)isochroman-7-carboxylic acid); (*trans*)-*R* (*cis*)-Emodin-Physcion bianthrone (2,4,4',5,5'-pentahydroxy-2'-methoxy-7,7'-dimethyl-[9,9'-bianthracene]-10,10'(9*H*,9'*H*)-dione); Alterporriol Q (1',2,7',8-tetrahydroxy-3',6-dimethoxy-3,6'-dimethyl-[1,2'-bianthracene]-9,9',10,-10'-tetraone); Alterporriol R (2,4',6',8-tetrahydroxy-2',6-dimethoxy-3,7'-dimethyl-[1,1'-bianthracene]-9,9',10,10'-tetraone); Alterporriol V (2,2',8,8'-tetrahydroxy-6,6'-dimethoxy-3,3'-dimethyl-[1,1'-bianthracene]-9,9',10,10'-tetraone); Cytoskyrin A ((6*R*,14*R*,17*S*,18*R*,19*R*,20*S*)-1,7,9,15,17,-20-hexahydroxy-3,11-dimethoxy-6,13a,5a,14-(epibutane[1,2,3,4]tetrayl)cycloocta[1,2-b:5,6-b']dinaphtha-lene-5,8,13,16(6*H*,14*H*)-tetraone); Alterporriol K ((5*S,*8*R*)-4,4',5,7',8-penta-hydroxy-1,1'-dimethoxy-6',8-dimethyl-5,6,7,8-tetrahydro-[2,2'-bianthracene]-9,9',10,10'-tetraone); Alterporriol T ((6*R*,6'*S*,7*R*,7'*R*,8*R*)-1',4,6,6',7,7',8-heptahydroxy-2,3'-dimethoxy-6',7-dime-thyl-5,5',6,6',7,7',8,8'-octahydro-[1,2'-bianthracene]-9,9',10,10'-tetraone); Alterporriol L ((6*S*,7*R*,8*R*)-4,4',6,7,7',8-hexahydroxy-1,1'-dimethoxy-6',7-dimethyl-5,6,7,8-tetrahydro-[2,2'-bianthracene]-9,9',10,10'-tetraone); Alterporriol M ((6*S*,7*S*,8*R*)-4,4',6,7,7',8-hexahydroxy-1,1'-dimethoxy-6',7-dimethyl-5,6,7,8-tetrahydro-[2,2'-bianthracene]-9,9',10,10'-tetraone); Alterporriol P ((5'*R*,6'*R*,-7'*R*)-1',2,5',6',7',8-hexahydroxy-3',6-dimethoxy-3,6'-dimethyl-5',6',7',8',8'a,10'a-hexahydro-[1,2'-bianthracene]-9,9',10,10'-tetraone); Alterporriol W ((1'*R*,6*R*,7*R*,8*R*)-2',4,6,7,8,8'-hexahydroxy-2,6'-dimethoxy-3',7-dimethyl-5,6,7,8-tetrahydro-[1,1'-bianthracene]-9,9',10,10'-tetraone); Alterporriol U ((6*R*,6'*S*,7*S*,7'*R*)-1',4,6,6',7,7'-hexahydroxy-2,3'-di-methoxy-6',7-dimethyl-5,5',6,6',-7,7',8,8'-octahy-dro-[1,2'-bianthracene]-9,9',10,10'-tetraone); Alterporriol S ((2'*S*,3'*R*,4'*S*,6*R*,-75,9'*R*)-1,2',3',4,5',6, 7,8'-octahy-droxy-7'-methoxy-2',4',7-trimethyl-2',3',4',4'a,5,6,7,8,9',9'a-decahydro-[2,9'-bianthracene]-9,10,10'(1'*H*)-trione);(+)-aS-alterporriol C ((1*S*,5'*S*,6'*R*,7'*S*,8'*R*)-1',2,5',6',7',8,8'-heptahydroxy-3',6-dimethoxy-3,6'-dime-thyl-5',6',7',8',8'a,10'a-hexahydro-[1,2'-bianthracene]-9,9',10,10'-tetraone); Alterporriol C ((1*S*,5'*R*,6'*S*,7'*R*,8'*S*)-1',2,5',6',7',8,8'-hepta-hydroxy-3',6-dimethoxy-3,6'-dime-thyl-5',6',7',8',8'a,10'a-hexahydro-[1,2'-bianthracene]-9,9',10,-10'-tetraone); Alterporriol N((6*R*,6'*R*,7*R*,7'*R*,8*R*,8'*R*)-4,4',6,6',7,7',8,8'-octahy-droxy-2,2'-dime-thoxy-7,7'-dimethyl-5,5',6,6',7,7',8,8'-octahydro-[1,1'-bianthracene]-9,9',10,10'-tetraone); Alterporriol O ((2*R*,2'*R*,3*S*,3'*S*)-2,2',3,3',8,8'-hexahydroxy-6,6'-dimethoxy-3,3'-dimethyl-1,1',2,2',-3,3',4,4'-octahy-dro-[1,1'-bianthracene]-9,9',10,10'-tetraone); Acetylalterporriol D ((1'*S*,5*S*,5'*S*,-6*R*,6'*R*,7S,7'*S*,8*R*,8'*R*)-4,4',5,5',6',7,7',8,8'-nonahydroxy-2,2'-dimethoxy-7,7'-dimethyl-9,9',10,10'-tetraoxo-5,5',6,6',7,7',8,8',9,9',10,10'-dodecahydro-[1,1'-bianthracen]-6-ylacetate); Acetylalterporriol E((1'*R*,5*S*,5'*S*,6*R*,6'*R*,75,7'*S*,8*R*,8'*R*)-4,4',5,5',6',7,7',8,8'-nonahydroxy-2,2'-dimethoxy-7,7'-dimethyl-9,9',10,10'-tetraoxo-5,5',6,6',7,7',8,8',9,9',10,10'-dodecahydro-[1,1'-bianthracen]-6-yl acetate); Alterporriol D ((1*S*,5*S*,5'*S*,6*R*,6'*R*,7*S*,7'*S*,8*R*,8'*R*)-4,4',5,5',6,6',7,7',8,8'-decahydroxy-2,2'-dimethoxy-7,7'-dimethyl-5,5',6,6',7,7',8,8'-octahy-dro-[1,1'-bianthracene]-9,9',10,10'-tetraone); Alterporriol E ((1*R*,5*S*,5'*S*,6*R*,6'*R*,7*S*,7'*S*,8*R*,8'*R*)-4,4',5,5',6,6',7,7',8,8'-decahydroxy-2,2'-dime-thoxy-7,7'-dimethyl-5,5',6,6',7,7',8,8'-octahy-dro-[1,1'-bianthracene]-9,9',10,10'-tetraone); Stemphylanthranol A ((5*S*,5"*S*,6*R*,6"*R*,7*S*,7"*S*,8*R*,8"*R*)-2',4,4",5,5",6,6",7,7",8,8',8"-dodecahy-droxy-2,2",6'-trimethoxy-3',7,7"-trimethyl-5,5",6,6",7,7",8,8"-octahydro-[1,1':5',1"-teranthra-cene]-9,9',9",10,10',10"-hexaone); Stemphylanthranol B ((5*S*,5"*R*,6*R*,6"*R*,7*S*,7"*R*,8*R*,8"*R*)-2',4,4",5,5",6,7,7",8,8',8"-undecahydroxy-2,2",6'-trimethoxy-3',6",7,7"-tetramethyl-5,5",6,6",-7,7",8,8"-octahydro-[1,1':7',1"-tetranthracene]-9,9',9",10,10',10"-hexaone);7-Chloroemodic acid; 7-Chloroemodinal; 7-Chloro-1,6,8-trihydroxy-3-methyl-10-anthrone; Damnacanthal (3-hydroxy-1-methoxy-anthraquinone-2-carboxaldehyde); 2,6-dihydroxy-anthraquinone; Austrocortinin(2-methoxy-7-methyl-1,4-dihydroxy-anthraquinone); Danthron (1,8-dihydroxy-anthraquinone); Dermolutein (8-methoxy-3-methyl-1,6-dihydroxy-anthraquinone-2-carboxylic acid); Fallacinal 3-formyl-6-methoxy-1,8-dihydroxy-anthraquinone); Phomarin (3-methyl-1,6-dihydroxy- anthraquinone); Anthragallol (alizarin brown; 1,2,3-trihydroxy-anthraquinone); Citreorosein (6-hydroxymethyl-1,3,8-trihydroxy-anthraquinone); Dermoglaucin (3-methyl-1,7,8-trihydroxy-6-methoxy-anthraquinone); Dermorubin (3-methyl-1,4,6-trihydroxy-8-methoxy-anthraquinone-2-carboxylic acid); Endocrocin (3-methyl-1,6,8-trihydroxy-anthraquinone-2-carboxylic acid); Erythroglaucin (3-methyl-1,4,8-trihydroxy-6-methoxy-anthraquinone); Flavokermesic acid (Laccaic acid D; 1-methyl-3,6,8-trihydroxy-anthraquinone-2-carboxylic acid); Flavopurpurin (alizarin Y; 1,2,6-trihydroxy-anthraquinone); Islandicin (2-methyl-1,4,5-trihydroxy-anthraquinone); Morindone (6-methyl-1,2,5-trihydroxy-anthraquinone); Rubrocristin (2-methyl-1,4,7-trihydroxy- 5-methoxy-anthraquinone); Dermocybin (3-methyl-1,5,7,8-tetrahydroxy-6-methoxy-anthraquinone); and Kermesic acid (8-methyl-1,3,4,6-tetrahydroxy-anthraquinone-7-carboxylic acid); 3-(beta-D-Glucopyranosyloxy)-1,6-dihydroxy-2-methyl-anthraquinone; 3-(6-O-Acetyl-beta-D-glucopyranosyloxy)-1,6-dihydroxy-2-methyl-anthraquinone; 3-[(2-O-6-Deoxy-alpha-L-mannopyranosyl-beta-D-glucopyranosyl)oxy]-1,6-dihydroxy-2-methyl-anthraquinone; 3-[(3-O-Acetyl-2-O-6-deoxy-beta-D-mannopyranosyl-beta-D-glucopyranosyl)oxy]-1,6-dihydroxy-2-methyl-anthraquinone; 3-[(6-O-Acetyl-2-O-6-deoxy-beta-D-mannopyranosyl-beta-D-glucopy-ranosyl)oxy]-1,6-dihydroxy-2-methyl-anthraquinone; 3-[(3,6-O-Diacetyl-2-O-6-deoxy-beta-D-mannopyranosyl-beta-D-glucopyranosyl)oxy]-1,6-dihydroxy-2-methyl-anthraquinone; 3-[(4,6-O-Diacetyl-2-O-6-deoxy-beta-D-mannopyranosyl-beta-D-glucopyranosyl)oxy]-1,6-dihydroxy-2-methyl-anthraquinone; 3-[(4-O-Acetyl-2-O-6-deoxy-beta-D-mannopyranosyl-beta-D-glucopy-ranosyl)oxy]-1,6-dihydroxy-2-methyl-anthraquinone; 3-[(6-O-Acetyl-2-O-beta-D-xylopyranosyl-beta-D-glucopyranosyl)oxy]-1,6-dihydroxy-2-methyl-anthraquinone; Ruberythric acid (1-Hydroxy-2-[(6-O-beta-D-xylopyranosyl-beta-D-glucopyranosyl)oxy]-anthraquinone); Lucidin prime-veroside (1-Hydroxy-2-(hydroxymethyl)-3-[(6-O-beta-D-xylopyranosyl-beta-D-glucopyranosyl)-oxy]-anthraquinone); 1-Acetyl-3-[(4-O-6-deoxy-beta-D-mannopyranosyl-beta-D-glucopyranosyl)oxy]-6-hydroxy-2-methyl-anthraquinone; 2-{[(6-O-beta-D-Glucopyranosyl-beta-D-glucopyranosyl)oxy]methyl}-11-hydroxy-anthraquinone; 3-[(2-O-6-Deoxy-beta-D-mannopyranosyl-beta-D-glucopyranosyl)oxy]-1-hydroxy-2-(methoxycarbonyl)-anthraquinone; 3-(beta-D-Glucopyranosyloxy)-2-(hydroxymethyl) anthraquinone; 3-(beta-D-Glucopyranosyloxy)-8-hydroxy-2-(hydroxymethyl)-anthraquinone; 2-(beta-D-Glucopyranosyloxy)-1,3-dihydroxy-anthraquinone; and 3-(beta-D-Glucopyranosyloxy)-1-hydroxy-2-(hydroxymethyl)-anthraquinone.

In another preferred embodiment, the compound selected from Formula (I) or (II) for use in the present invention is one, wherein
R¹ is selected from the group consisting of
   (i) linear or branched, substituted or non-substituted (C₂₋₆)alkyl, preferably substituted or non-substituted butyl or pentyl, more preferably, 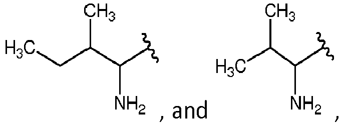 most preferably 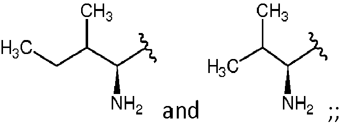
   (ii) substituted or non-substituted (C₃₋₆)heterocycle having 1 to 3 heteroatoms each independently selected from N, O and S, preferably substituted or non-substituted heterobicycle having 1 nitrogen atom, preferably substituted or non-substituted piperidine, most preferably 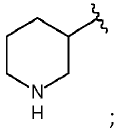
**R¹** to **R⁶** are each independently selected from the group consisting of H, Cl, Br, or F,
preferably for Formula (I), R⁵ is Cl and R² to R⁴ and R⁶ are H, more preferably R³ and R⁴ are Cl and R² , R⁵ and R⁶ are H,
preferably for Formula (II), R⁴ is Cl and R², R³, R⁵ and R⁶ are H, more preferably R² and R⁴ are Cl and R³, R⁵ and R⁶ are H;
**R⁷** is selected from the group consisting of
   (i) -NH₂, or -OH;
   (ii) substituted or non-substituted (C₃₋₆)heterocycle or (C₇₋₁₀)carbo- or hetero-bicycle having 1 to 3 heteroatoms each independently selected from N, O and S, preferably substituted or non-substituted heterobicycle having 1 nitrogen atom, more preferably isoindoline-1,3-dione, most preferably 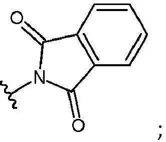
**R⁸** is -NH₂;
**R⁹** is selected from the group consisting of H and Me; and/or **c** is 1.

In a further preferred embodiment, the compound selected from Formula (I) or (II) for use in the present invention is selected from the group consisting of 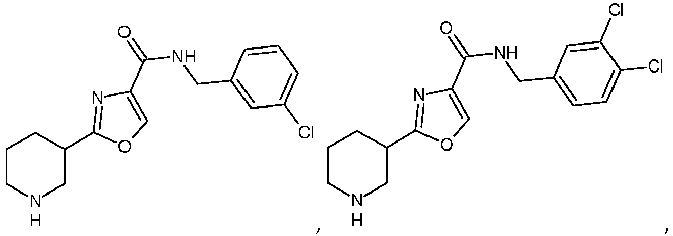 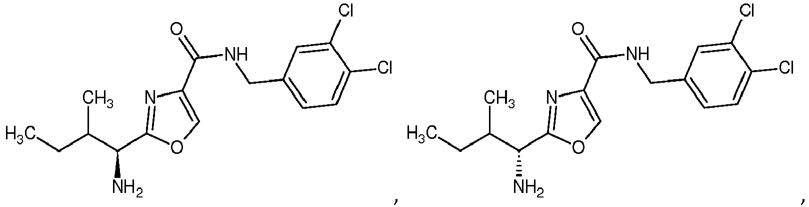 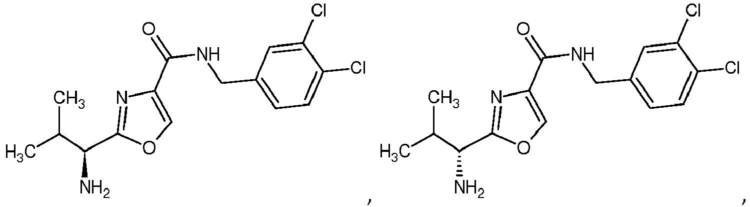 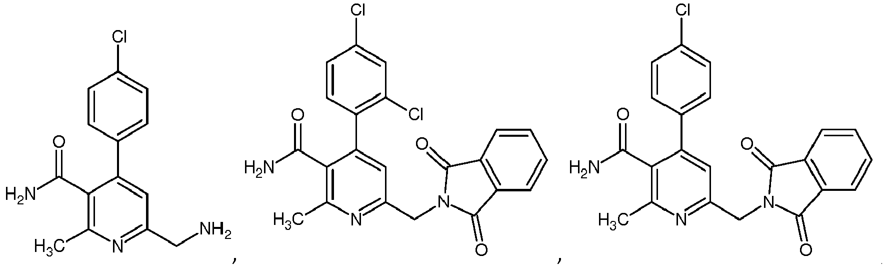 and

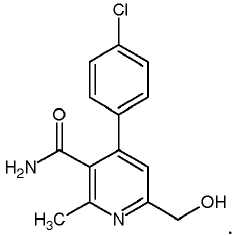

In a preferred embodiment, the TOMM6 (Translocase of Outer Membrane 6kDa subunit homologue)-interacting compound or composition for use in the present invention is one, wherein the nervous system disease or disorder is selected from the group consisting of:
brain injuries; cerebrovascular diseases; consequences of cerebrovascular diseases; motor neuron disease; dementias; ALS; multiple sclerosis; traumatic brain injury; small-vessel cerebrovascular disease; familial forms of Alzheimer's Disease; sporadic forms of Alzheimer's Disease; vascular dementia; M. Parkinson; frontotemporal dementia; Parkinson linked to chromosome-17; diabetic neuropathy; symptoms of depression and depression-related symptoms, preferably anhedonia and anorexia; schizophrenia with dementia; Korsakoff's psychosis; Lewy Body diseases; progressive supranuclear palsy; corticobasal degeneration; Pick's disease; Huntington's disease; thalamic degeneration; Creutzfeld-Jacob disease; HIV Dementia; disorders with mitochondrial dysfunction, preferably neurodegenerative diseases of ageing; cognitive-related disorder; mild cognitive impairment; age-associated memory impairment; age-associated cognitive decline; vascular cognitive impairment; central and peripheral symptoms of atherosclerosis and ischemia; perivascular disease; renal dysfunction and renal failure; stress-related disorders; attention deficit disorders; attention deficit hyperactivity disorders; and memory disturbances in children.

For therapeutic use, the compounds of the invention may be administered in any conventional dosage form in any conventional manner. Routes of administration include, but are not limited to oral, parenteral, intravenous, intramuscular and subcutaneous administration, preferably injections. The preferred modes of administration are oral, intravenous or subcutaneous.

The present invention is also directed to a pharmaceutical composition comprising a TOMM6 (Translocase of Outer Membrane 6kDa subunit homologue)-interacting compound or composition for use in the present invention and/or to the use of a TOMM6 (Translocase of Outer Membrane 6kDa subunit homologue)-interacting compound or composition in the manufacture of a medicament for the treatment or prophylaxis of a nervous system disease or disorder, preferably a human nervous system disease or disorder, preferably a central nervous system (CNS) or peripheral nervous system (PNS) disease or disorder.

The compounds, compositions or pharmaceutical compositions for use in the invention may be administered alone or in combination with adjuvants that enhance stability of the compounds, facilitate administration of pharmaceutical compositions containing them in certain embodiments, provide increased dissolution or dispersion, increase activity, provide adjunct therapy (e.g. an acetylcholinesterase inhibitor, memantine, a renin-angiotensin system-inhibiting agent such as an ACE inhibitor and/or an AT1 antagonist, and/or an L-type calcium channel inhibitor such as amlodipine), and the like, including other active ingredients. Advantageously such combination therapies utilize lower dosages of the conventional therapeutics, thus avoiding possible toxicity and adverse side effects incurred when those agents are used as monotherapies. The above-described compounds and compositions may be physically combined with conventional therapeutics or other adjuvants into a single pharmaceutical composition. Reference in this regard may be made to Cappola et al.: U.S. patent application no. 09/902,822, PCT/US 01/21860 und US provisional application no. 60/313,527, each incorporated by reference herein in their entirety. Advantageously, the compounds or compositions may then be administered together in a single dosage form. In some embodiments, the (pharmaceutical) compositions comprising such combinations of compounds contain at least about 5 %, but more preferably at least about 20 %, of a compound for use in the present invention (w/w). The optimum percentage (w/w) of a compound of the invention may vary and is within the purview of those skilled in the art. Alternatively, the compounds may be administered separately (either serially or in parallel). Separate dosing allows for greater flexibility in the dosing regime.

As mentioned above, dosage forms of the compounds described herein include pharmaceutically acceptable carriers and adjuvants known to those of ordinary skill in the art. Methods for preparing such dosage forms are known (see, for example, H. C. Ansel and N. G. Popovish, Pharmaceutical Dosage Forms and Drug Delivery Systems, 5th ed., Lea and Febiger (1990)). Dosage levels and requirements are well-recognized in the art and may be selected by those of ordinary skill in the art from available methods and techniques suitable for a particular patient. In some embodiments, dosage levels range from 1-300 mg/dose for a 70 kg patient. Although one dose per day may be sufficient, up to 5 doses per day may be given. For oral doses, up to 2000 mg/day may be required. Reference in this regard may also be made to US provisional application no. 60/339,249. As the skilled artisan will appreciate, lower or higher doses may be required depending on particular factors. For instance, specific doses and treatment regimens will depend on factors such as the patient's general health profile, the severity and course of the patient's disorder or disposition thereto, and the judgment of the treating physician.

In a further aspect, the present invention is directed to a method for the therapeutic or prophylactic treatment of a nervous system disease or disorder, preferably a central nervous system disorder or a peripheral nervous system disorder in a patient, preferably a mammal, preferably a rodent, mouse, primate, or human patient, the method comprising the step of administering a therapeutically and/or prophylactically effective amount of a TOMM6 (Translocase of Outer Membrane 6kDa subunit homologue)-interacting compound or composition for use in the present invention to a patient in need of such treatment.

In a preferred embodiment, the method of therapeutic or prophylactic treatment of the present invention is one, wherein the nervous system disease or disorder is selected from the group consisting of: brain injuries; cerebrovascular diseases; consequences of cerebrovascular diseases; motor neuron disease; dementias; ALS; multiple sclerosis; traumatic brain injury; small-vessel cerebrovascular disease; familial forms of Alzheimer's Disease; sporadic forms of Alzheimer's Disease; vascular dementia; M. Parkinson; frontotemporal dementia; Parkinson linked to chromosome-17; diabetic neuropathy; symptoms of depression and depression-related symptoms, preferably anhedonia and anorexia; schizophrenia with dementia; Korsakoff's psychosis; Lewy Body diseases; progressive supranuclear palsy; corticobasal degeneration; Pick's disease; Huntington's disease; thalamic degeneration; Creutzfeld-Jacob disease; HIV Dementia; disorders with mitochondrial dysfunction, preferably neurodegenerative diseases of ageing; cognitive-related disorder; mild cognitive impairment; age-associated memory impairment; age-associated cognitive decline; vascular cognitive impairment; central and peripheral symptoms of atherosclerosis and ischemia; perivascular disease; stress-related disorders; attention deficit disorders; attention deficit hyperactivity disorders; and memory disturbances in children, preferably a neurodegenerative disease, Alzheimer's disease, a severe form of dementia and/or a disease with mitochondrial dysfunction.

In another aspect, the present invention is directed to a method for the identification of a TOMM6-interacting compound or composition, preferably a compound or composition for use in the treatment or prophylaxis of a nervous system disease or disorder, which modifies TOMM6 function and/or activity, the method comprising the steps of:
(i) providing a TOMM6 or TOMM6 homologue protein, preferably in the form of a homogenized cell solubilisate, preferably prepared from the frontal cortex or hippocampus of a mammalian animal, more preferably prepared from AD (Alzheimer's disease) model Tg2576 mice;
(ii) addition of a compound or composition of interest to the TOMM6 or TOMM6 homologue under physiological conditions, preferably in a physiological buffer with a physiological salt composition mimicking the ionic composition of blood, that allow for the interaction of the compound of interest with the TOMM6 or TOMM6 homologue; and
(iii) identifying the absence or presence of an interaction of the compound of interest with the TOMM6 or TOMM6 homologue.

The compound or composition of interest for use in the methods described herein can be any chemical synthetic or biological compound that is to be investigated with regard to its interaction with TOMM6. The term "identifying the absence or presence of an interaction" encompasses the determination whether the compound or composition (i) directly or indirectly binds to TOMM6 and thus stabilizes and/or enhances its physiological activity, preferably vs. an untreated control (as can be determined, e.g., in the assay of Example 9 below), and/or (ii) induces the expression (increases the protein content) of TOMM6 in a cell, preferably vs. an untreated control (as can be preferably determined in an immunoblot assay, e.g. using TOMM6 antibodies, e.g. as described in Example 13 below).

The term "TOMM6 homolog", as used herein, describes homologs of TOMM6 with TOM-M6 activity that comprise, preferably consist of an amino acid sequence having a sequence identity of at least 85%, preferably 90%, more preferably 95% most preferably 98% sequence identity with the amino acid sequence of, preferably human or rodent TOMM6, preferably sequence identity with the amino acid sequence selected from the group consisting of SEQ ID NOs: 1, 2 and 3.

The term "physiological conditions" as used herein means any assay conditions that will allow for the interaction, e.g. direct or indirect binding and/or induction of the expression of TOMM6, of the compound or composition of interest with TOMM6 or a TOMM6 homolog. Exemplary and non-limiting physiological conditions can be found below and in the Examples, e.g. in Example 9, 10 and 13.

Preferably, and as an example for step (i) of the above method for the identification of a TOMM6-interacting compound or composition, the TOMM6 or TOMM6 homologue protein can be isolated from an organ specimen, cells or organoids with TOMM6 or TOMM6 homologue expression (both referred to as TOMM6). Preferably a brain specimen from a mammalian organism, preferably a rodent organism, preferably a rat or mouse, preferably a mouse, preferably a transgenic mouse, without or with (transgenic) expression of TOMM6, preferably expression in neurons is used as source of TOMM6. For neuronal expression, TOMM6 expression can placed under control of a neuron-specific promoter including but not limited to the tyrosine hydroxyl-lase promoter (Thy1.2 regulatory element) or a prion protein promoter, preferably a prion protein promoter, more preferably the hamster prion protein promoter. Neuronal and/or cellular expression of TOMM6 can also preferably be achieved by expression of TOMM6 under control of the CMV promoter (cytomegalovirus immediate-early promoter/enhancer). Transgenic expression of TOMM6 can be achieved by generation of a transgenic organism by established techniques, and/or by systemic viral delivery, which, for example, includes but is not limited to a lentivirus. The method can also be performed with endogenously expressed TOMM6. The method can also apply an AD model without or with additional expression of TOMM6. One AD model expresses an APP (amyloid precursor protein) mutant isolated from a familial AD (FAD) case, preferably the APPSwe mutation (APP 695 isoform, KM670/671NL) under control of a neuron-specific promoter including but not limited to the tyrosine hydroxylase promoter (Thy1.2 regulatory element) or a mouse or hamster prion protein promoter, preferably a prion protein promoter, more preferably the hamster prion protein promoter. An AD model can express additional dementia-associated proteins to enhance disease progression, which include but are not limited to PS1M146V, and/or Tau P301L. The preferred AD model is Tg2576 mice. The method preferably uses specimens prepared from frontal cortex or more preferably hippocampus. Specimens are isolated from AD model mice without or with stable (transgenic) TOMM6 expression, aged 0-24 months, preferably 16-20 months, more preferably 18 months. Depending on experiment size, hippocampi can be isolated from several mice (up to several hundreds). A small-scale experiment preferably uses 5-10 aged Tg2576 mice. After PBS perfusion of anesthetized mice, brains can be isolated, and hippocampi can be rapidly dissected on ice and frozen in liquid nitrogen. In addition to mice, the method can also apply organoids, cells, including but not limited to standard cell lines available from ATCC, such as COS1, COS7, NIH-3T3, BHK, CHO, HEK cells, preferably HEK cells with low endogenous or exogenous TOMM6 overexpression. Transient or stable cellular overexpression of TOMM6 can be performed by a standard transfection protocol with a TOMM6 expression plasmid, preferably a plasmid, which directs TOMM6 expression under control of a ubiquitous promoter, preferably the CMV promoter. For example, dissected hippocampi, organoids, organs, brain specimens and cells comprising TOMM6 (hereafter grouped and together referred to as hippocampi) can be homogenized. Frozen hippocampi can be homogenized mechanically or manually at a temperature ranging from, e.g., -210 °C to +30 °C, preferably under liquid nitrogen (temperature -210 °C -196 °C). Fresh or frozen cell pellets can be solubilized directly. For example, solubilization of homogenized hippocampi, fresh or frozen cell pellets can be accomplished as follows. Proteins from frozen powdered hippocampi or cells can be extracted for 15 min-120 min, preferably 30 min at 4°C - 24 °C preferably at 4 °C with any standard solubilization buffer. Such a standard solubilisation buffer can be (but is not restricted to) RIPA (radioimmunoprecipitation assay) buffer, which, for example, can feature but is not restricted to the following composition: sodium deoxycholate at a concentration of 0.1 %-2 %, preferably 1 %, SDS at a concentration of 0.05 % - 2 %, preferably 0.1 %, NP40 (IGEPAL) ranging from 0.01 %-0.5 %, preferably 0.1 %, EDTA, EGTA or other divalent cation chelator ranging from 0 mM to 20 mM, preferably 5 mM, Tris ranging from 5 mM - 500 mM, preferably 50 mM with a pH ranging from pH6- pH10, preferably pH 8.0) supplemented without or with additional salts (e.g. NaCl ranging from 0-500 mM) to modify ionic strength. Other exemplary buffers suitable for extraction include but are not restricted to PBS, PIPES, HEPES, or bicine, with a pH varying from pH 5-pH 10, preferably pH 6-9, supplemented with any state of the art detergent (anionic, cationic, non-ionic, zwitterionic). For example, suitable detergents or mixtures thereof include but are not limited to CHAPS, CHAPSO, C7BzO, ASB-14, n-Dodecyl beta-D-maltoside, Octyl beta-D-glucopyranoside, Octyl beta-Dl-thioglucopyranoside, Polyoyethylene 10 tridecyl ether, Brij® 56, Triton X-100, 3-(Decyldimethylammonio)-propanesulfonate inner salt. For example, for protein extraction any commercially available protein extraction buffer or kit can be used such as but not restricted to T-PER Tissue Protein Extraction Reagent (ThermoFisher Scientific), M-PER Mammalian Protein Extraction Reagent (ThermoFisher Scientific), Pierce IP Lysis buffer, any protein extraction kit from SigmaAldrich (PROTMEM, PROTTWO, PROTOT) supplemented with any state of the art cocktail of protease/phosphatase inhibitors (e.g. Cat. No. P8349, and/or PPC1010, Sigma-Aldrich, St. Louis, MO, USA). Solubilization can be enhanced by sonification. It is preferred to remove particulate material after solubilisation. Methods for the removal of insoluble material include but are not limited to filtration or centrifugation at 5 000 x g - 100 000 x g, preferably 50 000 x g for 1-120 min, preferably 20 min at 4 °C - 30 °C, preferably 4 °C. It may be further desirable to dilute the solubilisate, for example, diluting the supernatant solubilisate by 1:1 - 1:20, preferably 1:5, in a suitable buffer as described above (preferably supplemented with protease inhibitors).

Preferably, and as an example for step (ii) of the above method for the identification of a TOMM6-interacting compound or composition, the compound or composition of interest suspected of possibly interacting with TOMM6 can be added to TOMM6, preferably in the form of a solubilisate, under physiological conditions that allow for the interaction of the compound of interest with TOMM6. For example, to a diluted solubilisate as described above the compound of interest, e.g. a compound or composition for use in the present invention, e.g. Compound-1, as described in the Examples, can be added at a concentration of 1 - 1000 microM, preferably of 10-20 microM.

Preferably, and as an example for step (iii) of the above method for the identification of a TOMM6-interacting compound or composition, the absence or presence of an interaction of the compound or composition of interest with the TOMM6 or TOMM6 homologue, can be identified by any suitable method. For example, if Tris buffer was not used as incubation buffer, the buffer can be exchanged into Tris buffer for subsequent SDS-PAGE. For example, the concentration of Tris may range between 10 to 100 mM Tris, preferably 20 mM Tris, between pH 6-10, preferably pH 7.4. And the exchange method may be any suitable method, for example, dialysis, gel filtration or centrifugation, preferably over Centrifugal Filter Units, preferably Amicon Ultra Centrifugal Filter Units (with a MWCO 3kDa; EMD Millipore Merck KGaA, Darmstadt, Germany). For protein separation by, e.g. SDS-PAGE, SDS-PAGE, Laemmli sample buffer or sample buffer for native gel electrophoresis can be added. According to the original protocol, SDS-PAGE Laemmli sample buffer contains 2 % SDS, 5 % mercaptoethanol. If SDS-PAGE is performed under non-reducing conditions, mercaptoethanol can be omitted. To improve disaggregation of aggregated proteins, the buffer can be supplemented with urea ranging from 1 M-8 M, preferably about 6 M urea. Solubilized proteins in SDS-PAGE Laemmli sample buffer can be incubated for 10 min-24 h, preferably 90 min at room temperature. For native electrophoresis a suitable buffer can be used without SDS. For separation of proteins one- or two-dimensional SDS-PAGE can be applied. For example, solubilized proteins can be subjected to 7-15 %, preferably 8 % SDS-PAGE under reducing conditions supplemented without or with 1-8 M urea, preferably 6-8 M. As alternative, two-dimensional gel electrophoresis or native gel electrophoresis is also suitable. After separation of proteins, electrophoretic protein transfer can be performed to a suitable membrane, which can be but is not restricted to a PVDF membrane or a nitrocellulose membrane, preferably a PVDF membrane in a transfer cell, preferably a tank transfer cell (e.g. Mini Trans-Blot cell, Bio-Rad GmbH, München, Germany). In the case that the compounds of interest are colored compounds, e.g. the compounds or compositions for use in the present invention, e.g. Compound 1 in the Examples, or other coloured anthraquinone-derivatives, they can be directly identified as TOM-M6-interacting by co-migration with TOMM6. Alternatively, a compound or composition of interest can be rendered easily identifiable by labelling the compound, for example, with a radiolabel (e.g. ³H, ¹²⁵I, ³⁵S, ³³S, ¹⁴C), a dye, e.g. a fluorescent dye (e.g. the BODIPY series fluorescent dyes). For example, subsequent detection may involve autoradiography, or UV-light detection, as applicable. If the compound or composition of interest forms part of a composition that comprises further compounds and/or proteins, e.g. forms part of a partially purified originally cell-based composition, preferably a homogenized and solubilized originally cell-based composition, the further TOMM6-non-binding proteins in the composition may be identified as additional binding partners for the compound of interest. For example, further TOMM6-non-binding proteins can be identified by nano-LC-ES-MS/MS. Protein bands with co-migration of the compound of interest can be cut out and be subjected to nano-LC-ES-MS/MS (performed e.g. by Proteome Factory AG, Berlin, Germany). Proteins can be identified using MS/MS ion search of the Mascot search engine (e.g. Matrix Science, London, England) and nr protein database (National Center for Biotechnology Information, Bethesda, MD). Ion charge in search parameters for ions from ESI-MS/MS data acquisition can be set to '1+, 2+, or 3+ 'according to the common charge state distribution for the instrument and the method.

The preferred general method of TOMM6 detection is by a standard immunological method, preferably immunoblotting after electrophoretic transfer of proteins to a membrane by Western blotting. Alternative preferred immunological methods for TOMM6 detection are by ELISA, immunohistology, immunofluorescence, fluorescence microscopy, Vertico-SMI, STED-microscopy, 3D-SIM microscopy, photoactivated-localization microscopy, fluorescence-activated cell sorting, flow cytometry, and electron microscopy. For immunological detection, an antibody (e.g. polyclonal, monoclonal, from mouse, rabbit, any species including single-domain antibodies from cameloids, sharks) against TOMM6 is preferably used. Preferably, the antibody is raised in rabbit, or mouse against full-length recombinant TOMM6 protein. Alternatively, an antibody against TOMM6 can be raised against a peptide sequence of TOMM6 (10-20 amino acids, up to 30-40 amino acids) or a recombinant fusion protein, or the recombinant full-length TOMM6 protein. An antibody recognition epitope is typically 5-7 amino acids in length. Antibodies against TOMM6 can also be isolated from a phage display antibody library by panning with purified recombinant TOMM6 protein.

A further preferred method of TOMM6 detection is by Western blotting. A protein lysate is prepared from any cell, organ, organoids, whole organisms, preferably from brain, more preferbly from the hippocampus. A preferred disease model for AD is the Tg2576 AD mouse model. Another preferred system for TOMM6 protein detection are cells. TOMM6 protein detection can be performed with any cell, preferably a human cell or biopsy specimen isolated from healthy or diseased individuals. Preferred human specimens are HEK cells, human peripheral blood and/or total circulating blood cells and/or a cell fraction isolated thereof, which can be platelets, leucocytes, erythrocytes and/or polymorphonuclear cells. For immunoblot detection of proteins, preferably the Tomm6/TOMM6 protein, tissue, biopsy specimen, cultured cells, blood cells, or hippocampal tissue (fresh or frozen tissue or cells, preferably frozen) is/are homogenized (mechanically, manually) at a temperature ranging from -210 °C to +30 °C, preferably under liquid nitrogen (temperature -210 °C - -196 °C), and extracted for 15 min-120 min, preferably for 30 min at 4°C - 24 °C preferably at 4 °C with any standard solubilization buffer. Such a standard solubilisation buffer can be but is not restricted to RIPA (radioimmunoprecipitation assay) buffer, which can be but is not restricted to the following composition: sodium deoxycholate at a concentration of 0.1 %-2 %, preferably 1 %, SDS at a concentration ranging between 0.05 % to 2 %, preferably 0.1 %, NP40 (IGEPAL) ranging from 0.01 % to 0.5 %, preferably 0.1 %, EDTA, EGTA or another divalent cation chelator ranging from 0 mM to 20 mM, preferably 5 mM, Tris ranging from 5 mM to 500 mM, preferably 50 mM with a pH ranging from pH6 to pH10, preferably pH 8.0, supplemented without or with additional salts (e.g. NaCl ranging from 0-500 mM) to modify ionic strength. Any other buffer (PBS, PIPES, HEPES, bicine, but not restricted to these), with a pH varying from pH 5 - pH 10, preferably pH 6-9, supplemented with any state of the art detergent (anionic, cationic, non-ionic, zwitterionic) is also suitable for extraction. Other suitable detergents or mixtures thereof include but are not limited to CHAPS, CHAPSO, C7BzO, ASB-14, n-Dodecyl beta-D-maltoside, Octyl beta-D-glucopyranoside, Octyl beta-Dl-thioglucopyranoside, Polyoyethylene 10 tridecyl ether, Brij® 56, Triton X-100, 3-(Decyldimethylammonio)propanesulfonate inner salt. As an alternative to the buffers described above, any commercially available protein extraction buffer (non-denaturing or denaturing) or kit can be used for protein extraction, which includes (but is not restricted to) the following examples: T-PER Tissue Protein Extraction Reagent (ThermoFisher Scientific), M-PER Mammalian Protein Extraction Reagent (ThermoFisher Scientific), Pierce IP Lysis buffer, a protein extraction kit from SigmaAldrich (PROTMEM, PROTTWO, PROTOT). The used protein extraction buffer is routinely-supplemented with any state of the art cocktail of protease/phosphatase inhibitors (e.g. Cat. No. P8349, and/or PPC1010, Sigma-Aldrich, St. Louis, MO, USA). In case of cells, direct solubilisation of fresh or frozen cell pellets is also possible without prior homogenization. In addition, prior enrichment of mitochondria from tissue specimens or cells by a standard method, which can include (but is not restricted to) density-gradient centrifugation (generated by density media, e.g. Percoll, Nycodenz, lodixanol or sucrose) can be performed prior to protein solubilisation. Protein solubilisation can be enhanced by sonification. Particulate material is removed. Methods for the removal of insoluble materials include but are not limited to filtration or centrifugation at 5 000 x g - 100 000 x g, preferably 50 000 x g for 1-120 min, preferably 20 min at 4 °C - 30 °C, preferably 4 °C. Solubilized proteins can be used directly for Tomm6/TOMM6 protein detection, or proteins are further concentrated. Concentration of proteins can be performed by precipitation with a suitable solvent, which can be but is not limited to TCA, ethanol, isopropanol, acetone/methanol. Preferably the method applies a mixture of ice-cold acetone/methanol, preferably 12:2, added to a final concentration of 60-95 %, preferably 83 % for at least >5 min up to an indefinite time preferably 90 min at a temperature ranging between -210 °C to 4 °C, preferably 4 °C. Any other method of protein concentration is also suitable. For example, protein concentration can also be achieved by centrifugation over a protein concentration cartridge, which can be but is not restricted to Amicon Ultracentrifugal filter units, MWCO 3 kDa, (Millipore). For protein separation by SDS-PAGE (Laemmli system), the protein pellet is dissolved in SDS-PAGE sample buffer supplemented with SDS. The standard SDS-PAGE Laemmli sample buffer contains 2 % SDS, 0.1 M DTT (or 5 % mercaptoethanol). To improve disaggregation of aggregated proteins, the buffer is supplemented without or with urea ranging from 0M-8 M, preferably 6 M urea and incubated for 10 min to 24 h, preferably 90 min at room temperature. Proteins can be stored frozen (-210 °C -20 °C) at a concentration ranging from 0.01 - 100 mg/ml, preferably 0.5 mg - 1 mg/ml, for further use. Immunoblot detection of proteins can be performed with antibodies, derivatives, fragments or analogs thereof, preferably with affinity-purified antibodies or F(ab)₂ fragments of the respective antibodies or antibody analogs after separation of proteins by denaturing SDS-PAGE (7.5 % - 20 %, preferably 10-15 %) supplemented without or with urea, preferably with urea (6-8 M, preferably 8M) under reducing conditions and electrophoretic protein transfer to a suitable membrane, which can be but is not restricted to a PVDF membrane or a nitrocellulose membrane, preferably PVDF membrane in a transfer cell, preferably a tank transfer cell (e.g. Mini Trans-Blot cell, Bio-Rad GmbH, München, Germany). Bound antibodies are visualized with seconddary enzyme-coupled antibodies, more preferably F(ab)₂ fragments of enzyme-coupled (preferably peroxidase-coupled) secondary antibodies (e.g. Dianova GmbH, Hamburg, Germany), which are pre-absorbed to mouse and/or human serum proteins, and followed by enhanced chemiluminescent detection (e.g. with ECL Plus, and/or ECL Prime, Amersham, GE Healthcare Life Sciences, Glattbrugg, Switzerland). An alternative is the detection by enzyme-coupled protein A or G (e.g. EMD Millipore, Merck KGaA, Darmstadt, Germany), which is also followed by enhanced chemiluminescent detection. Another alternative is the direct labelling of the primary or secondary antibody or the protein A or G with a radiolabel (preferably ¹²⁵I).

To control for equal protein loading, a control protein can be detected. A standard loading control involves detection of a house-keeping protein, e.g. actin, tubulin, Gapdh. Also, detection of Gnb (i.e. the Gbeta subunit of heterotrimeric G-proteins) can be an alternative loading control. For quantitation of mitochondrial proteins, detection of a mitochondrial protein is performed, preferably Atp6vla. Due to signal amplification, visualization of bound primary antibody by a secondary antibody is the preferred method of detection.

Alternative detection methods for TOMM6 are based on any TOMM6 -interacting compound or composition, e.g. a TOMM6-interacting compound or composition for use in the present invention, a synthetic antibody, an antibody fragment (synthetic or native), a peptide, a protein, an enzyme, which is labelled for further detection. If the TOMM6-interacting compound or composition is not labelled, detection can be performed by a secondary detection reaction (see above). Labelling of the TOMM6-interacting compound or composition can be performed by a radiolabel (e.g. ³H, ¹²⁵I, ³⁵S, ³³S, ¹⁴C), or a non-radioactive method, e.g. an enzyme (e.g. peroxidase, alkaline phosphatase), biotin, fluorescent label (e.g. FITC, TRITC, ALEXA Fluor Dyes), colloidal gold particles, any other chemical dye, a protein or a fluorescent protein, which is attached by chemical crosslinking or fusion of the DNA. Examples for fluorescent proteins include but are not limited to the green fluorescent protein and variants thereof (e.g. EYFP, EGFP, Cerulean, ECFP, mCherry fluorescent protein; HyPer; RoGFP; rxYFPM PROPS, VSFP, zoanFP). By similar methods a short peptide Tag (e.g. HA, FLAG) can be attached to allow visualization and/or quantification of TOMM6. Other possible labelling methods for a TOMM6-interacting compound or composition also include the SNAP-Tag® or the CLIP-Tag® technology (New England Biolabs, Biotechnology, USA).

A preferred method also includes quantitation of TOMM6 by secondary detection of the TOMM6-interacting compound or composition with a secondary entity, which interacts with the primary TOMM6-interacting compound or composition. The secondary interacting entity (e.g. protein/compound) can be similarly modified as detailed above for the primary TOMM6-interacting compound or composition

Another alternative preferred detection method for TOMM6 is based on direct labelling of TOMM6 by a radiolabel (e.g. ³H, ¹²⁵I, ³⁵S, ³³S, ¹⁴C), or a non-radioactive method, e.g. an enzyme (e.g. peroxidase, alkaline phosphatase), biotin, fluorescent label (e.g. FITC, TRITC, ALEXA Fluor Dyes), colloidal gold particles, any other chemical dye, a protein or a fluorescent protein, which is attached by chemical crosslinking or fusion of the DNA. Examples for fluorescent proteins include but are not limited to the green fluorescent protein and variants thereof (e.g. EYFP, Cerulean, ECFP, mCherry fluorescent protein; HyPer; RoGFP; rxYFPM PROPS, VSFP, zoanFP). By similar methods, a short peptide Tag (e.g. HA, FLAG) can be attached, to allow visualization and/or quantification of TOMM6. Other possible direct labelling methods for TOMM6 include but are not limited to the SNAP-Tag® or the CLIP-Tag® technology (New England Biolabs, Biotechnology, USA). A preferred method also includes quantitation of TOMM6 by determination of gene expression level by state of the art methods: e.g. Northern blotting, microarray gene expression analysis, transcriptome sequencing.

In another aspect, the present invention is directed to a method for the identification of a TOMM6-interacting compound or composition, preferably a compound or composition for use in the treatment or prophylaxis of a nervous system disease or disorder, which modifies TOMM6 and TOMM6 homolog protein amounts, the method comprising the following steps:
(i) providing isolated cells or isolated tissue, preferably isolated cells or tissue derived from the group of cells and tissues consisting of mammalian frontal cortex or hippocampus, mammalian brain, peripheral or circulating mammalian blood cells, human HEK cells, and AD (Alzheimer's disease) model Tg2576 mice, a non-human mammal, or a transgenic disease animal model, preferably Tg2576 mice;
(ii) optionally providing a control group of step (i) not to be contacted with a TOMM6- and/or TOMM6 homolog-interacting compound or composition;
(iii) contacting the isolated cells, isolated tissue, non-human mammal, or a transgenic disease animal model of step (i) with a TOMM6- and/or TOMM6 homolog-interacting compound or composition, preferably a TOMM6- and/or TOMM6 homolog-interacting compound or composition for use in the present invention in a physiologically effective concentration under physiological conditions suitable for the interaction of the interacting compound or composition;
(iv) contacting the mixture of isolated cells, isolated tissue, non-human mammal, or a transgenic disease animal model and the TOMM6- and/or TOMM6 homolog-interacting compound or composition of step (iii) with a quantifying agent specifically binding to TOMM6 protein and TOMM6 homologue protein under physiological conditions suitable for the specific binding of TOMM6 protein and TOMM6 homologue protein to the quantifying agent, preferably a quantifying agent selected from the group consisting of an antibody, an antibody fragment, antibody derivative or analogue, and a TOMM6- or TOMM6 homolog-binding compound for use in the present invention, more preferably a labelled compound;
(v) optionally also contacting the control group of step (ii) with the quantifying agent of step (iv) under the same physiological conditions; and
(vi) determining the amount of quantifying agent bound specifically to the TOMM6 protein and TOMM6 homologue protein, optionally versus the control.

The term "physiologically effective concentration" as used herein refers to any concentration of a given compound, e.g. a TOMM6- and/or TOMM6 homolog-interacting compound or composition, which brings about the physiological effects of this compound or composition, e.g. as determined in the Examples. The term" physiological conditions suitable" was defined above, and examples for physiologically suitable conditions are given throughout the description in the context of any method, wherein the interaction of an interacting compound or composition with TOMM6 is investigated.

The terms "an antibody fragment, antibody derivative or analogue" refer to any antibody-based molecule that is capable of specifically binding to a given target, preferably refer to any antibody-based molecule that is a functional antibody fragment, functional derivative or functional analogue of an antibody. A very convenient antibody fragment for targeting applications is the single-chain Fv fragment, in which a variable heavy and a variable light domain are joined together by a polypeptide linker. Other antibody fragments for targeting applications include Fab fragments, Fab₂ fragments, miniantibodies (also called small immune proteins), tandem scFv-scFv fusions, as well as scFv fusions with suitable domains (e.g. with the Fc portion of an immunoglobulin). For a review on certain antibody formats, please see Holliger P, Hudson PJ.; Engineered antibody fragments and the rise of single domains. Nat Biotechnol. 2005 Sep., 23(9):1126-36. Review.

The term "antibody derivative or analog" of an antibody for use in the present invention is meant to include any functional antibody or fragment thereof that has been chemically modified in its amino acid sequence, e.g. by addition, substitution and/or deletion of amino acid residue(s) and/or has been chemically modified in at least one of its atoms and/or functional chemical groups, e.g. by additions, deletions, rearrangement, oxidation, reduction, etc. as long as the derivative has substantially the same binding affinity to the corresponding antigen and, preferably, has a dissociation constant in the micro-, nano- or picomolar range.

In a preferred embodiment, the antibody, fragment, derivative or analogue thereof according to the invention is one, that is selected from the group consisting of polyclonal antibodies, monoclonal antibodies, chimeric antibodies, humanized antibodies, CDR-grafted antibodies, FV-fragments, Fab-fragments and Fab₂-fragments and antibody-like binding proteins.

Preferably for step (i) of the above method for the identification of a TOMM6-interacting compound or composition which modifies TOMM6 and TOMM6 homolog protein amounts, mammalian cells may be a rodent or human cell, or a biopsy specimen isolated from healthy or diseased individuals. Preferred specimen are peripheral blood cells and/or (total) circulating blood cells and/or a cell fraction isolated thereof, e.g. platelets, leucocytes, erythrocytes and/or polymorphonuclear cells. The method can also be applied to cultured cells, preferably cultured human cells, more preferably HEK cells. Another preferred specimen for practicing the above method is a brain specimen, preferably (pre) frontal cortex and/or hippocampus, more preferably hippocampus. The TOMM6 for use in the methods of the present invention can be partially or fully purified, e.g. be present in the form of a homogenized, solubilized, concentrated, partially or fully purified cell composition as described above. It is also within the scope of the above method that TOMM6 is partially or even fully isolated from the isolated cells or isolated tissue in step (i) of the above method in order to avoid interaction(s) of non-TOMM6 materials, e.g. further proteins in the composition. For example, an SDS-PAGE may be performed as described above to isolate TOMM6. As alternatives, other methods of gel electrophoresis are also suitable for purification, e.g. two-dimensional gel electrophoresis or native gel electrophoresis. After separation of proteins, an electrophoretic protein transfer may be performed to a suitable membrane, which can be but is not restricted to a PVDF membrane or a nitrocellulose membrane, preferably a PVDF membrane in a transfer cell, preferably a tank transfer cell (e.g. Mini Trans-Blot cell, Bio-Rad GmbH, München, Germany).

Preferred examples for practicing step (iii) of the above method for the identification of a TOMM6-interacting compound or composition which modifies TOMM6 and TOMM6 homolog protein amounts are given above in the context of step (ii) of the method for the identification of a TOMM6-interacting compound or composition.

Preferably for steps (iv) to (vi) of the above method for the identification of a TOMM6-interacting compound or composition which modifies TOMM6 and TOMM6 homolog protein amounts, immunoblot detection of TOMM6 proteins can be performed with any TOMM6-interacting quantifying agent, which can be, e.g. a TOMM6-interacting protein, a monobody, an antibody, an antibody fragment, an antibody derivative or antibody analogue, preferably a polyclonal or monoclonal antibody contained in serum, an affinity-purified antibody, an F(ab)₂ fragment and/or by quantification with a TOMM6- or TOMM6 homolog-binding compound for use in the present invention, preferably a labelled TOMM6- or TOMM6 homolog-binding compound for use in the present invention. It is noted that if a TOMM6- or TOMM6 homolog-binding compound for use in the present invention is used for quantification, the TOMM6- or TOMM6 homolog-binding compound used in the previous step (iii) should preferably be removed before any quantification is performed. For immunological detection, preferably an antibody, fragment, or derivative thereof, preferably from mouse, rat, rabbit, sheep, goat, donkey, horse, or any other species including cameloids and sharks can be used. Preferably, the antibody may be raised against full-length recombinant TOMM6 protein (see SEQ ID NOs: 1 to 3) or against an immunogenic peptide sequence of TOMM6, e.g. 10-20, up to 30-40 amino acids of the TOMM6 amino acid sequence. An antibody recognition epitope is typically 5-7 amino acids in length. Antibodies against TOMM6 can also be isolated from a phage display antibody (fragment) library by panning with purified recombinant TOMM6 protein or a fragment/peptide thereof. For example, for immunoblotting TOMM6 the blot membrane can be incubated with the primary antibody dilution, which can range from 1:500 to 1:50,000, preferably 1:2,000 - 1:10,000. Bound antibodies may be visualized with enzyme-coupled secondary antibodies or preferably F(ab)₂ fragments of enzyme-coupled secondary antibodies (e.g. Dianova GmbH, Hamburg, Germany; dilution 1: 40,000), or by enzyme-coupled protein A or G (e.g. EMD Millipore, Merck KGaA, Darmstadt, Germany), and followed by enhanced chemiluminescent detection (e.g. ECL Plus and/or ECL Prime, Amersham, GE Healthcare Life Sciences, Glattbrugg, Switzerland). Another alternative may be the direct labelling of the primary antibody with an enzyme followed by a chemiluminescent detection method. A further alternative is the direct labelling of the primary or seconddary antibody or the protein A or G with a radiolabel, e.g. ¹²⁵I. As suitable loading controls for immunoblotting in order to control for equal mitochondrial protein loading, e.g. a control protein can be detected, preferably a mitochondrial protein, more preferably Atp6v1a/ATP6V1A. As a standard loading control, detection of a house-keeping protein, e.g. actin, tubulin, GAPDH may be performed. Detection of the total amount of Gnb (i.e. the Gbeta subunit of heterotrimeric G-proteins) can be another protein loading control. Due to signal amplification, visualization of a bound primary antibody by a secondary antibody is the preferred method of detection. Labelling of the TOMM6-quantifying agent can be performed by a radiolabel, e.g. ³H, ¹²⁵I, ³⁵S, ³³S, ¹⁴C, or a non-radioactive labl, e.g. an enzyme, preferably peroxidase, alkaline phosphatase, biotin, fluorescent label (e.g. FITC, TRITC, ALEXA Fluor Dyes), colloidal gold particles, any other chemical dye, a protein or a fluorescent protein, which is attached by chemical crosslinking or DNA fusion. Examples for fluorescent proteins include but are not limited to the green fluorescent protein and variants thereof (e.g. EYFP, EGFP, Cerulean, ECFP, mCherry fluorescent protein; HyPer; RoGFP; rxYFPM PROPS, VSFP, zoanFP). By similar methods, a short peptide Tag (e.g. HA, FLAG) can be attached, to allow visualization and/or quantitation of TOMM6. Alternatively to the labelling of the quantifying agent TOMM6 can be labelled for easier detection of TOMM6-bound quantifying agent, e.g. labelled by a radiolabel (e.g. ³H, ¹²⁵I, ³⁵S, ³³S, ¹⁴C), or a non-radioactive method, e.g. an enzyme (e.g. peroxidase, alkaline phosphatase), biotin, fluorescent label (e.g. FITC, TRITC, ALEXA Fluor Dyes), colloidal gold particles, any other chemical dye, a protein or a fluorescent protein, which is attached by chemical crosslinking or fusion of the DNA. Examples for fluorescent proteins include but are not limited to the green fluorescent protein and variants thereof (e.g. EYFP, Cerulean, ECFP, mCherry fluorescent protein; HyPer; RoGFP; rxYFPM PROPS, VSFP, zoanFP). By similar methods, a short peptide Tag (e.g. HA, FLAG) can be attached, to allow visualization and/or quantitation of TOMM6. Other possible direct labelling methods for TOMM6 include (but are not limited to) the SNAP-Tag® or the CLIP-Tag® technology (New England Biolabs, Biotechnology, USA).

The above-described method for identification of TOMM6 can also be applied for identification of TOMM6 and/or TOMM6-interacting compounds or compositions, e.g. inducers/- stabilizers/activators such as small molecule compounds, antibodies, peptides, genes, in whole organisms, organs, organoids, tissues, multiple cells or a single cell (e.g. eukaryotic cells, mammalian cells, human cells, HEK cells or any other cell, insect cells, yeast cells or bacterial cells) with transient or stable transfection with an expression plasmid encoding TOMM6. An example for a preferred plasmid used for TOMM6 expression in mammalian cells is pcDNA3.1 (e.g. Invitrogen), which directs ubiquitous expression under control of the strong cytomegalovirus (CMV) immediate-early promoter/enhancer. There are other preferred plasmids, which direct expression under control of the CMV promoter. Any other promoter suitable for expression in eukaryotic cells can also be used. High level expression of a protein in bacteria (e.g. E. coli BL21 (DE3), or BL21 (DE3) pLysS) is preferably obtained by the pET expression system (e.g. Novagen®, EMD Millipore, Merck KGaA, Darmstadt, Germany), which is driven by the strong bacteriophage T7 promoter and translation signals.

TOMM6-interacting compounds or compositions can also be identified in whole organisms, organs, organoids, tissues, multiple cells or a single cell (e.g. eukaryotic cells, mammalian cells, human cells, HEK cells or any other cell, insect cells, yeast cells or bacterial cells) after viral transduction of TOMM6 by a virus-based expression system, which can be but is not restricted to an adenovirus, lentivirus, retrovirus, AAV (adeno-associated virus), HSV-1, HSV-2, or baculovirus. The above-described method for TOMM6 identification can also be used to detect TOMM6-interacting compounds or compositions, such as, e.g. inducers, stabilizers and/or activators, after viral transduction of TOMM6.

TOMM6 inducers, stabilizers and/or activators can also be identified in whole organisms, organs, organoids, tissues, multiple cells or a single cell (e.g. eukaryotic cells, mammalian cells, human cells, HEK cells or any other cell, insect cells) with endogenous TOMM6 expression levels without exogenously modified expression of TOMM6.

In a further aspect, the present invention is directed to a method for the identification of a physiological effect of a TOMM6- and TOMM6 homologue-interacting compound or composition comprising the steps of:
(i) contacting isolated cells, isolated tissue, a non-human mammal, a human or a transgenic disease animal model with a TOMM6- and/or TOMM6 homolog-interacting compound or composition, preferably a TOMM6- and/or TOMM6 homolog-interacting compound or composition for use in the present invention in a physiologically effective concentration under physiological conditions suitable for the interaction with the interacting compound or composition;
(ii) optionally providing a control group not to be contacted with a TOMM6- and/or TOMM6 homolog-interacting compound or composition; and
(iii) quantifying dysfunctional AGTR2 protein aggregates, preferably without or with functional AGTR2 proteins, in the isolated cells, isolated tissue, non-human mammal, human or transgenic disease animal model of step (i), and optionally in the control group of step (ii), preferably in the form of a homogenized cell solubilisate, preferably prepared from human HEK cells, peripheral blood cells, the frontal cortex or hippocampus of a mammalian species, more preferably prepared from AD (Alzheimer's disease) model Tg2576 mice, stresssed rats with symptoms of sporadic AD, or human patients with AD, preferably quantifying dysfunctional AGTR2 protein aggregates by an antibody, an antibody fragment, antibody derivative or analogue.

The method for identification of a physiological effect of a TOMM6-interacting and TOMM6 homologue-interacting compound or composition can also be used to monitor/quantify the therapy effect of TOMM6/Tomm6-interacting compounds or compositions such as inducers, stabilizers and/or activators (e.g. small molecule compounds, large molecule compounds, antibodies, proteins, peptides, genes, viruses) in a whole organism (e.g. eukaryotes, mammals, rodents, primates, humans).

The method of TOMM6 identification can also be used to detect TOMM6 in body fluids, which include but are not restricted to blood, serum, blood plasma, blood cells, urine. Thus, the method of TOMM6 identification and/or quantification, e.g. as described above, preferably in the blood, serum or urine, more preferably in the blood cells of blood of a subject, can generally be used to determine a subject's risk of developing a nervous system disease or disorder, preferably a human nervous system disease or disorder, preferably a central nervous system (CNS) or peripheral nervous system (PNS) disease or disorder, or to determine a subject's state of the nervous system disease or disorder, preferably human nervous system disease or disorder, preferably a central nervous system (CNS) or peripheral nervous system (PNS) disease or disorder based on the detection and/or quantification of TOMM6.

In a typical example, treatment can be performed with a compound for use in the present invention, e.g. Compound-1 - Compound-10 (e.g. 10 mg/kg/day) for a specified period of time (hours, days, months), e.g. by administration in drinking water (alternatives are, e.g. injection, i.p. or i.v., etc.) and optionally compared to untreated controls. After treatment, organs, e.g. brain, cortex, hippocampus, heart, skin, blood, circulating blood cells, can be isolated and endogenous TOMM6 protein levels can be quantified as detailed above.

In a preferred embodiment, the method for the identification of TOMM6-interacting compounds or compositions, such as activators, stabilizers and/or inducers, can be performed by inhibition of HbsAg protein aggregation (e.g. dimerization, oligomerization, heteromerization), preferably in yeast cells.

Because TOMM6-interacting compounds or compositions inhibit mitochondrial dysfunction, any aggregation-prone protein, which is sensitive to mitochondrial dysfunction can be studied. A preferred example is the protein aggregation of HbsAg protein over-expressed in yeast. Preferably, the recombinant HBsAg cDNA (e.g. synthesized by GeneScript, Piscataway, NJ, USA) is inserted into a yeast expression vector, preferably p42K-TEF (D3011001-1 Dualsystems Biotech AG, Zurich). Any other plasmid, which allows protein expression in yeast is also suitable. p42K-TEF has the advantage that it allows selection of yeast transformants by the presence of G418 in a yeast growth medium (e.g. YPD; Bacto-yeast extract 10 g/L, Bacto-peptone 20 g/L and D-Glucose 20 %). Any yeast strain can be used for protein expression. In a preferred example the yeast (*Saccharomyces cerevisae*) strain, INVSc1, was used (e.g. Invitrogen GmbH, Nr. C810-00). Any other yeast strain is also suitable. After cultivation for 1-5 days, preferably 3-4 days, to allow for protein expression, yeast cells are harvested by a suitable method, which includes but is not restricted to freeze-drying, spray drying, ultrafiltration, sedimentation, flocculation or centrifugation, followed by removal of cultivation medium by a suitable method, which can apply (but is not restricted to) washing with an isotonic buffer, which can be (but is not restricted to) TBS or PBS at a temperature ranging from 3 °C to 38 °C, preferably 4 °C. The yeast cell pellet is frozen at -12°C to -210°C, preferably at >70°C until further use. The frozen cell pellet is extracted for 15 min to 120 min, preferably 30 min at 4°C - 24 °C preferably at 4 °C with any standard solubilization buffer. Such a standard solubilisation buffer can be (but is not restricted to) RIPA (radioimmunoprecipitation assay) buffer, which can be of (but is not restricted to) the following composition: sodium deoxycholate at a concentration of 0.1 %-2 %, preferably 1 %, SDS at a concentration ranging from 0.05 % to 2 %, preferably 0.1 %, NP40 (IGEPAL) ranging from 0.01 % to 0.5 %, preferably 0.1 %, EDTA, EGTA or any other divalent cation chelator ranging from 0 mM to 20 mM, preferably 5 mM, Tris ranging from 5 mM to 500 mM, preferably 50 mM with a pH ranging from pH6 to pH10, preferably pH8.0. The buffer can be supplemented without or with additional salts (e.g. NaCl ranging from 0 to 500 mM) to modify ionic strength. Any other suitable buffer, which includes (but is not restricted to) PBS, PIPES, HEPES, bicine, can be used with a pH varying from pH 5 to pH 10, preferably pH 6-9. To enhance protein extraction, the used buffer is supplemented with any state of the art detergent (anionic, cationic, non-ionic, Zwitterionic). Suitable detergents or mixtures thereof include (but are not limited to) CHAPS, CHAPSO, C7BzO, ASB-14, n-Dodecyl beta-D-maltoside, Octyl beta-D-glucopyranoside, Octyl beta-Dl-thioglucopyranoside, Polyoyethylene 10 tridecyl ether, Brij® 56, Triton X-100, 3-(Decyldimethylammonio)propanesulfonate inner salt. Similarly, for protein extraction any commercially available protein extraction buffer or kit can be used, which includes (but is not restricted to) T-PER Tissue Protein Extraction Reagent (ThermoFisher Scientific), M-PER Mammalian Protein Extraction Reagent (ThermoFisher Scientific), Pierce IP Lysis buffer, a protein extraction kit from SigmaAldrich (PROTMEM, PROTTWO, PROTOT) supplemented with any state of the art cocktail of protease/phosphatase inhibitors (e.g. Cat. No. P8349, and/or PPC1010, Sigma-Aldrich, St. Louis, MO, USA). Protein solubilization can be enhanced by sonification. Particulate material is removed. Methods for removal of insoluble materials include (but are not limited to) filtration, or centrifugation at 5 000 x g - 100 000 x g, preferably 50 000 x g for 1-120 min, preferably for 20 min at 4 °C - 30 °C, more preferably at 4 °C. Solubilized proteins can be separated e.g. by denaturing SDS-PAGE, non-denaturing gel electrophoresis, gel filtration without further concentration, or proteins are further concentrated. Concentration of proteins can be performed by precipitation with a suitable solvent, which can be (but is not limited to) TCA, ethanol, isopropanol, acetone/methanol, preferably a mixture of ice-cold acetone/methanol, preferably 12:2, added to a final concentration of 60-95 %, preferably 83 % for at least >5 min up to an indefinite time preferably for 90 min at a temperature ranging between -210 °C to 4 °C, more preferably at 4 °C. Any other method of protein concentration is also suitable and can apply centrifugation over a protein concentration cartridge, which can be (but is not restricted to) an Amicon Ultracentrifugal filter unit, MWCO 3 kDa, (Millipore). For protein separation by SDS-PAGE (Laemmli system), the protein pellet is dissolved in SDS-PAGE sample buffer supplemented with SDS. The standard SDS-PAGE Laemmli sample buffer contains 2 % SDS, 0.1 M DTT (or 5 % mercaptoethanol). To improve disaggregation of high molecular mass protein aggregates, the buffer is supplemented with urea ranging from 1M to 8M, preferably 6 M urea and incubated for 10 min to 24 h, preferably for 90 min at room temperature. Proteins can be stored frozen (at -210 °C to -15 °C) at a concentration ranging from 0.01mg to 100 mg/ml, preferably 0.5 mg - 1 mg/ml, for further use.

Proteins can be separated by e.g. by denaturing SDS-PAGE, non-denaturing gel electrophoresis, gel filtration, preferably denaturing SDS-PAGE ranging from 7.5 % to 20 %, preferably 7.5 %, and supplemented without or with urea, preferably with urea (6-8 M, preferably 8M urea) under reducing conditions. Protein separation is followed by electrophoretic protein transfer to a suitable membrane, which can be (but is not restricted to) a PVDF membrane or a nitrocellulose membrane, preferably a PVDF membrane in a transfer cell, preferably a tank transfer cell (e.g. Mini Trans-Blot cell, Bio-Rad GmbH, München, Germany). Transferred HBsAg proteins are detected in immunoblot with HBsAg-specific antibodies raised in a suitable organism, which includes but is not restricted to rabbit, rat, mouse, goat, cameloid species, sheep, horse, donkey, shark. Polyclonal or monoclonal antibodies are raised against an immunogenic epitope of HBsAg. Preferably, the antibody/antibodies is/are raised in rabbit or mouse against full-length recombinant HBsAg protein. Alternatively, an antibody against HbsAg can be raised against an immunogenic peptide sequence of HBsAg (10-20 amino acids, up to 30-40 amino acids) or against a recombinant fusion protein. An antibody recognition epitope is typically 5-7 amino acids in length. Antibodies against HBsAg can also be isolated from a phage display antibody library by panning with purified recombinant HBsAg protein or a fragment/peptide thereof. In a typical example the antibody can be raised against (but is not restricted to) an N-terminal epitope of HBsAg. In another example, a monoclonal HBsAg antibody produced in mouse (e.g. SAB4700767, Sigma-Aldrich, St. Louis, MO, USA) was used. Suitable antibodies for detection of HBsAg expression and aggregation can also be isolated from human blood serum from individuals undergoing vaccination against Hepatitis B.

Primary antibody dilution can range from 1:500 to 1:50,000, preferably 1:2,000 - 1:10,000. Bound antibodies are visualized with F(ab)₂ fragments of enzyme-coupled secondary antibodies (e.g. Dianova GmbH, Hamburg, Germany; dilution 1: 40,000), or by enzyme-coupled protein A or G (e.g. EMD Millipore, Merck KGaA, Darmstadt, Germany), and followed by enhanced chemiluminescent detection (e.g. ECL Plus and/or ECL Prime, Amersham, GE Healthcare Life Sciences, Glattbrugg, Switzerland). Another alternative is the direct labelling of the primary antibody with an enzyme followed by a chemiluminescent detection method. Another alternative is the direct labelling of the primary or secondary antibody or the protein A or G with a radiolabel (preferably ¹²⁵I).

The above method is also suitable to monitor the treatment outcome of a subject treated with a TOMM6-interacting compound or composition, or any other compound, which improves mitochondrial function because dysfunctional AGTR2 protein aggregation is disease-relevant and contributes to neurodegeneration in brains of AD patients and mice (AbdAlla et al., J. Biol. Chem. 284, 6554-6565 (2009); AbdAlla et al., J. Biol. Chem. 284, 6566-6574 (2009). Therefore, detection of dysfunctional AGTR2 protein aggregation directly monitors the treatment effect of a TOMM6-interacting compound or composition. A preferred method to monitor the treatment outcome of a patient/individual treated with a TOMM6-interacting compound or composition comprises the following steps:
(i) Treatment of a subject suffering from a neurodegenerative disease, preferably AD, or a subject at risk for development of a neurodegenerative disease, preferably AD, with a TOMM6-interacting compound or composition. To monitor treatment outcome in a subject suffering from a neurodegenerative disease, preferably AD, or a subject at risk for development of a neurodegenerative disease, preferably AD, the method can be performed with any human biopsy specimen isolated from a diseased subject and compared with specimens isolated from healthy untreated subjects. The preferred human specimen are human peripheral blood cells and/or a cell fraction isolated thereof, which can be platelets, leucocytes, erythrocytes and/or polymorphonuclear cells. For monitoring of treatment outcome, the method preferably uses mononuclear cells/lymphocytes isolated from peripheral blood. For post-mortem studies, the method preferably uses brain biopsy specimens from AD patients and non-AD patients. Preferred specimens from rodent AD models are rat brain specimens from stressed rats with symptoms of sporadic AD, or stresssed Tg2576 mouse brain specimens with severe symptoms of FAD. Similarly, the method is applicable to study treatment outcome of other AD models, which include but are not restricted to aged primates or aged dogs. The subject suffering from a neurodegenerative disease, preferably AD, or the subject at risk for development of a neurodegenerative disease, preferably AD, are treated with a TOMM6-interacting compound or composition in a therapeutic dose ranging from days to several years depending on the lifetime of the subject. Preferably duration of treatment lasts more than 1 week. The method is also suitable to study the treatment effect in cultured cells with mitochondrial dysfunction and with endogenously expressed AGTR2 without or with exogenous AGTR2 expression. Under experimental conditions, this method uses cells, preferably human cells, more preferably HEK cells with endogenously expressed and/or transfected/transduced (e.g. transiently, or stably) AGTR2. Mitochondrial dysfunction is induced by (but not restricted to) cell culture stress and/or the expression of NOX3 (NADPH oxidase 3). HEK cells are cultivated in the absence and presence of compounds or compositions for use in the present inventtion, e.g. plant extracts (e.g. Compound-1 (alizarin), Compound-2 (pseudopurpurin), Compound3-10, an extract from *Galium aparine* or an extract from *Rubia tinctorum*)*.* Compounds or compositions can be applied at concentrations ranging between 1 microM to 1mM for the initial screening, preferably 10-20 microM. Plant extracts can be added to culture medium (e.g. 20 microL/10 ml), preferably a 10-fold-concentrated plant extract prepared from 200g pulverized plant material/1L of solvent is used. Compounds and compositions (e.g. plant extracts) can be dissolved in an inert solvent (e.g. DMSO, ethanol, medium) and incubated, e.g. for 24-96 h, preferably for 48-72h, more preferably for 60-72h. After the incubation period, cells may be washed, harvested, and collected by a suitable method, which can apply but is not restricted to centrifugation. The cell pellet can be frozen at -12°C - -210°C, preferably at >70°C until further use. As an alternative, the harvested cell pellet can be directly used for detection of AGTR2 protein aggregation without homogenization.
(ii) Homogenization of cells, organoids, tissue, biopsy specimens, blood cells or hippocampal tissue (fresh or frozen tissue or cells, preferably frozen) from step (i). The frozen biopsy specimen or cells is/are preferably homogenized (mechanically, manually) at a temperature ranging from -210 °C to +30 °C, preferably under liquid nitrogen (temperature -210 °C - -196 °C).
(iii) Solubilization/extraction of proteins. Proteins in the frozen or fresh cell pellet, or pulverized tissue specimen from step (ii) can be extracted for 15 min to 120 min, preferably for 30 min at 4°C - 24 °C, more preferably at 4 °C with any standard solubilization buffer. Such a standard solubilisation buffer can be (but is not restricted to) RIPA (radioimmunoprecipitation assay) buffer, which can have but is not restricted to the following composition: sodium deoxycholate at a concentration of 0.1 %-2 %, preferably 1 %, SDS at a concentration ranging from 0.05 % to 2 %, preferably 0.1 %, NP40 (IGEPAL) ranging from 0.01 % to 0.5 %, preferably 0.1 %, EDTA, EGTA or any other divalent cation chelator ranging from 0 mM to 20 mM, preferably 5 mM, Tris ranging from 5 mM to 500 mM, preferably 50 mM and with a pH ranging from pH6 to pH10, preferably pH8.0) supplemented without or with additional salts (e.g. NaCl ranging from 0 to 500 mM) to modify ionic strength. Any other suitable buffer, which includes but is not restricted to PBS, PIPES, HEPES, bicine, can be used with a pH varying from pH 5 to pH 10, preferably pH 6-9. To enhance protein extraction, the buffer can be supplemented with any state of the art detergent (anionic, cationic, non-ionic, Zwitterionic). Suitable detergents or mixtures thereof include but are not limited to CHAPS, CHAPSO, C7BzO, ASB-14, n-Dodecyl beta-D-maltoside, Octyl beta-D-glucopyranoside, Octyl beta-Dl-thioglucopyranoside, Polyoyethylene 10 tridecyl ether, Brij® 56, Triton X-100, 3-(Decyldimethylammonio)propanesulfonate inner salt. Similarly, for protein extraction any commercially available protein extraction buffer or kit can be used, which includes (but is not restricted to) T-PER Tissue Protein Extraction Reagent (ThermoFisher Scientific), M-PER Mammalian Protein Extraction Reagent (ThermoFisher Scientific), Pierce IP Lysis buffer, a protein extraction kit from SigmaAldrich (PROTMEM, PROTTWO, PROTOT) supplemented with any state of the art cocktail of protease/phosphatase inhibitors (e.g. Cat. No. P8349, and/or PPC-1010, Sigma-Aldrich, St. Louis, MO, USA). Solubilization can be enhanced by sonification. Particulate material can be removed. Methods for removal of insoluble materials include but are not limited to filtration or centrifugation at 5 000 x g - 100 000 x g, preferably 50 000 x g for 1-120 min, preferably 20 min at 4 °C - 30 °C, preferably 4 °C. Solubilized proteins can be separated by denaturing SDS-PAGE without further concentration, or proteins are further concentrated. Concentration of proteins can be performed by precipitation with a suitable solvent, which can be but is not limited to TCA, ethanol, isopropanol, acetone/methanol, preferably a mixture of ice-cold acetone/methanol, preferably 12:2, added to a final concentration of 60-95 %, preferably 83 % for at least >5 min up to an indefinite time preferably 90 min at a temperature ranging between -210 °C to 4 °C, preferably at 4 °C. Any other method of protein concentration is also suitable and can apply centrifugation over a protein concentration cartridge, which can be (but is not restricted to) Amicon Ultracentrifugal filter units, MWCO 3 kDa, (Millipore). For protein separation by SDS-PAGE (Laemmli system), the protein pellet is dissolved in SDS-PAGE sample buffer. The standard SDS-PAGE Laemmli sample buffer contains 2 % SDS, 0.1 M DTT (or 5 % mercaptoethanol). To improve disaggregation of high molecular mass protein aggregates, the buffer is supplemented with urea ranging from 1 M to 8 M, preferably 6 M urea and incubated for 10 min to 24 h, preferably for 90 min at room temperature. Solubilized proteins can be stored frozen (at -210 °C to -15 °C) at a concentration ranging from 0.01mg to 100 mg/ml, preferably 0.5 mg to 1 mg/ml, for further use.
(iv) Separation of proteins by denaturing SDS-PAGE. Solubilized proteins may be separated by denaturing SDS-PAGE (7.5 % - 20 %, preferably 7.5 %) supplemented without or with urea, preferably with urea (6-8 M, preferably 8M) under reducing conditions.
(v) Electrophoretic protein transfer. After separation, proteins preferably can be transferred by electrophoretic protein transfer to a suitable membrane, which can be but is not restricted to a PVDF membrane or a nitrocellulose membrane, preferably a PVDF membrane in a transfer cell, preferably a tank transfer cell (e.g. Mini Trans-Blot cell, Bio-Rad GmbH, München, Germany).
(vi) Generation of AGTR2-protein aggregate-specific antibodies. Transferred AGTR2 proteins can be detected in immunoblot with AGTR2-specific antibodies, which cross-react with AGTR2 monomers and/or AGTR2 protein aggregates. Antibodies may be raised in a suitable organism, which includes but is not restricted to rabbit, rat, mouse, goat, a cameloid species, sheep, horse, donkey, shark. Polyclonal or monoclonal antibodies may be raised against an immunogenic epitope of AGTR2. Preferably, the antibody/antibodies is/are raised in an animal against full-length recombinant AGTR2 protein. Alternatively, an antibody against AGTR2 can be raised against an immunogenic peptide sequence of AGTR2 (10-20 amino acids, up to 30-40 amino acids) or against a recombinant fusion protein where a fragment of AGTR2 is fused to GST, HIS6-Tag or any other state of the art protein tag. An antibody recognition epitope is typically 5-7 amino acids in length. Antibodies against AGTR2 can also be isolated from a phage display antibody library by panning with purified recombinant AGTR2 protein, protein fragment, fusion protein, or peptide. In a typical example the antibody can be raised against but not restricted to the C-terminus of AGTR2, against an antigen encompassing amino acids 320-349 of the human AT2R (AGTR2) sequence (AbdAlla et al., J. Biol. Chem. 276, 39721-39726, 2001); J. Biol. Chem. 284, 6566-6574, 2009). For specific detection of AGTR2 aggregates, immunization can be performed with an aggregated AGTR2 protein and/or antigen, preferably the aggregated C-terminal AGTR2 antigen, which was covalently and stably aggregated by crosslinking with a bivalent crosslinker, which includes but is not restricted to DST, DSP, DSS, DSG, DFDNB, EDC, and BS3, preferbly DFDNB. A list of frequently used crosslinkers is available (Crosslinking Technical Handbook, ThermoScientific). The crosslinking reaction can be performed by the addition of 0.1 - 10 mM, preferably 1mM of crosslinking agent, which may be freshly added to the antigen (protein, peptide) dissolved at a concentration of 0.01 mg/ml - 10 mg/ml, more preferably 0.1 mg-0.5 mg/ml in a suitable buffer according to the recommendations of the manufacturer (Crosslinking Technical Handbook, ThermoScientific), and incubated for 30 min - 4 h, preferably for 60 min at room temperature or 4 °C, preferably at room temperature. The reaction can be stopped as indicated by the manufacturer by the addition of 1 M Tris or glycine, pH 8.0. High molecular weight antigen aggregates can be enriched, e.g. by gel filtration or centrifugation over a protein concentration cartridge, which can be (but is not restricted to) an Amicon Ultracentrifugal filter unit, MWCO 10 kDa, (Millipore). Immunization of the native, or cross-linked antigen can be performed by standard state of the art immunization protocol. Immunization may involve injection of the antigen (0.001mg - 1mg, preferably 0.1 mg of antigen), preferably emulsified with an adjuvant, which can be but is not restricted to Freund's Complete adjuvant (FCA) followed by booster injections of the antigen (every week, or bi-weekly) with Incomplete Freund's adjuvant (IFA).
(vii) Immunoblot detection of AGTR2 aggregates. Immunoblot detection of AGTR2 is preferbly performed with affinity-purified antibodies against AGTR2 or F(ab)₂ fragments of the respective antibodies (from step (vi).). For detection of AGTR2 protein aggregation and AGTR2 monomers in immunoblot, the primary antibody dilution can be 1:500-1:50,000, preferably 1:2,000 - 1:-10,000, more preferably 1:2,000. Bound antibodies may be visualized with F(ab)₂ fragments of enzyme-coupled secondary antibodies (e.g. Dianova GmbH, Hamburg, Germany; preferred dilution 1: 40,000), which can be pre-absorbed to human or mouse/rat/primate/dog serum proteins, and followed by enhanced chemiluminescent detection (e.g. with ECL Plus, and/or ECL Prime, Amersham, GE Healthcare Life Sciences, Glattbrugg, Switzerland). An alternative is the detection of bound primary antibodies by enzyme-coupled protein A or G (e.g. EMD Millipore, Merck KGaA, Darmstadt, Germany), which can also befollowed by enhanced chemiluminescent detection. Another alternative is the direct labelling of the primary antibody with an enzyme followed by a chemiluminescent detection method. Another alternative is the direct labelling of the primary or secondary antibody or the protein A or G with a radiolabel (preferably ¹²⁵I).

In a preferred embodiment, the method for the identification of TOMM6-interacting compounds or compositions, such as activators, stabilizers and/or inducers is performed by reciprocal protein folding of AGTR2/AGTR1.

This method detects TOMM6-interacting compounds or compositions by the reciprocal effect of TOMM6 on AGTR2 and AGTR1 protein folding. ROS (reactive oxygen species) inhibits AGTR2 and induces AGTR1. Consequently, inhibition of mitochondrial dysfunction/ROS by TOMM6 (and TOMM6-interacting compounds or compositions, such as activators, stabilizers and/or inducers) enhances AGTR2 protein folding whereas AGTR1 protein folding is decreased.

This method can apply any detection method for AGTR2 and AGTR1, for endogenously expressed AGTR2/AGTR1 or exogenously expressed AGTR2/AGTR1 (or any variant, homologue, mutant, fusion protein referred herein as AGTR2 and AGTR1) in a cell (organism, organ, organoid) and/or a cellular expression system (any cell, bacteria, yeast, eukaryotic cell, mammalian cell, rodent cell, primate cell, human cell; preferably HEK cell). AGTR2/AGTR1 can be detected by any state-of-the-art-method, which is based on but not restricted to immunological, non-immunological methods, radiolabelling, fluorescent labelling, chemical dye labelling, by direct, or indirect detection methods, which involves a primary interacting molecule, which is either labelled for direct detection or not labelled and followed by a secondary detection reaction as e.g. specified above.

In a preferred example, AGTR2 and AGTR1 are labelled, e.g. at the C-terminus with a fluorescent protein, preferably by gene fusion with a 5-Gly-spacer. The fluorescent protein can be but is not limited to the green fluorescent protein and variants thereof (e.g. EYFP, Cerulean, ECFP, mCherry fluorescent protein; HyPer; RoGFP; rxYFPM PROPS, VSFP, zoanFP). Preferably the fluorescent protein is a variant of the enhanced green fluorescent protein, preferably Cerulean.

The AGTR2-Cerulean (and AGTR1-Cerulean) fusion protein can be expressed in a cell (eukaryotic cell, preferably a mammalian, rodent, mouse, rat, primate cell, more a preferably human cell). HEK cells are preferred because they can be easily transfected by any standard transfection method, e.g. but not limited to the liposome transfection method (e.g. with Lipofectamine, Lipofectamine 2000, Lipofectamine 3000; e.g. Invitrogen by Thermo Fisher Scientific), calcium phosphate transfection method, DEAE-dextran method, electroporation or any other suitable method. Viral transduction is a preferred method for bringing the cDNA of AGTR2-Cerulean (and AGTR1-Cerulean) into any cell, also primary cells or neurons. Other cells, which can be easily transfected can also be used. Examples include but are not restricted to COS7, COS1, NIH-3T3, CHO cells. TOMM6 protein/expression levels are modified during protein synthesis of AGTR2-Cerulean and AGTR1-Cerulean. TOMM6 expression level can be modified by co-transfection of a TOMM6-expression plasmid, e.g. TOMM6-pcDNA3.1. Depending on the desired effect, increasing DNA amounts of TOMM6-pcDNA3.1 plasmid and AGTR2-Cerulean (and TOMM6-pcDNA3.1 plasmid and AGTR1-Cerulean) can be used (preferably 1-20 µg/10⁶ cells).

For testing of a TOMM6-interacting compound or composition, the compound or composition of interest may be dissolved in a non-toxic solvent (e.g. DMSO, ethanol, medium) and applied to the AGTR2-Cerulean-transfected cell (and AGTR1-Cerulean-transfected cell) at a concentration ranging preferably between 1 nM - 1 mM, more preferably 1 microM-20 microM for initial screening purposes. In the absence and presence of TOMM6 protein/TOMM6 activity modification, AGTR2-Cerulean levels (and AGTR1-Cerulean levels) can be quantified by fluorescence spectrometry, fluorescence microscopy, fluorescence-activated cell sorting analysis or another direct or indirect quantitation method of cellular fluorescence.

Preferably, the AGTR1 fluorescence (i.e. AGTR1-Cerulean fluorescence) and AGTR2 fluorescence (i.e. AGTR2-Cerulean fluorescence) are quantified by fluorescence spectrometry in a photometer or plate reader, at an excitation wavelength of preferably 420 nm, and emission preferably between 440-600 nm. Peak fluorescence intensity, e.g. at 495 nm, can be determined in the absence and presence of TOMM6 co-expression or a TOMM6-interacting compound or composition.

This method of TOMM6 activity detection by reciprocal effects on AGTR2/AGTR1 folding can similarly apply immune-techniques (e.g. immunoblot, immmuno-histology, ELISA), direct radiolabelling (e.g. ³H, ¹²⁵I, ³⁵S, ³³S, ¹⁴C), indirect methods by a binding assay involving a competetion/binding assay with an AGTR2-specific (and AGTR1-specific) ligand/radioligand (e.g. with ¹²⁵I-labelled angiotensin II or [³H]-labelled angiotensin II, or derivatives thereof) and an unlabelled AT2-specific competitive ligand (e.g. PD123319, and CGP-42112A; unlabelled AT1-specific competitors include losartan, candesartan and any AT1-specific antagonist)). As an alternative, a binding assay with a radiolabelled AGTR2-/AGTR1-specific antibody can be applied. In addition, a direct or indirect detection method is suitable, which detects AGTR2/AGTR1, which is modified, e.g. with a protein Tag, peptide Tag or chemical Tag (e.g. protein, fluorescent protein, enhanced fluorescent protein and variants; enzyme; SNAP-tag®, CLIP-tag®, peptide-tag, e.g., HA, FLAG; fluorescent label, radioactive isotope label, chemical label, biotin, small molecule, metal, ions) for visualization and/or quantitation.

Hence, this method includes identification of endogenously expressed AGTR2/AGTR1 (and variants) in native tissue, organs, organoids, cells, eukaryotic cells mammalian cells, rodent cells, primate cells, human with and without (or before and after) the manipulation of TOMM6 (Tomm6 homologues, variants, mutants) protein/activity/expression levels.

Thus, the method of AGTR2 aggregate identification and/or quantification, e.g. as described above, e.g. by immunoblot detection, preferably in the blood, serum or urine, more preferably in the blood cells of blood of a subject, can generally be used to determine a subject's risk of developing a nervous system disease or disorder, preferably a human nervous system disease or disorder, preferably a central nervous system (CNS) or peripheral nervous system (PNS) disease or disorder, or to determine a subject's state of the nervous system disease or disorder, preferably human nervous system disease or disorder, preferably a central nervous system (CNS) or peripheral nervous system (PNS) disease or disorder based on the detection and/or quantification of ATGR2 aggregates, preferably vs. a suitable (healthy) control.

In a further preferred embodiment, the method for the identification of TOMM6-interacting compounds or compositions is performed by co-migration under reducing urea-containing SDS-PAGE. This method identifies TOMM6-interacting compounds or compositions in a protein lysate from an organism, organ, organoid, and/or a cellular expression system (any cell, bacteria, yeast, eukaryotic cell, mammalian cell, rodent cell, primate cell, human cell; preferably HEK cell). Preferably the method applies brain, brain cortex or hippocampus, preferably hippocampus from mammals, primates, rodents, rat or mice, preferably Tg2576 AD mice.

In a typical preferred example, TOMM6-interacting compound or composition-interacting proteins, preferably Compound-1-interacting hippocampal proteins are isolated. Hippocampi can be isolated from 5-10 aged Tg2576 mice without or with transgenic TOMM6 expression, homogenized mechanically, manually) at a temperature ranging from -210 °C to +30 °C, preferably under liquid nitrogen (temperature -210 °C - -196 °C), and extracted for 15 min-120 min, preferably 30 min at 4°C - 24 °C preferably at 4 °C with any standard solubilization buffer. Such a standard solubilisation buffer can be but is not restricted to RIPA (radioimmunoprecipitation assay) buffer, which can have (but is not restricted to) the following composition: sodium deoxycholate at a concentration of 0.1 %-2 %, preferably 1 %, SDS at a concentration of 0.05 % - 2 %, preferably 0.1 %, NP40 (IGEPAL) ranging from 0.01 %-0.5 %, preferably 0.1 %, EDTA, EGTA or other divalent cation chelator ranging from 0 mM to 20 mM, preferably 5 mM, Tris ranging from 5 mM - 500 mM, preferably 50 mM with a pH ranging from pH6- pH10, preferably pH8.0) supplemented without or with additional salts (e.g. NaCl ranging from 0-500 mM) to modify ionic strength. Any other buffer, which can be but is not restricted to PBS, PIPES, HEPES, or bicine, with a pH varying from pH 5-pH 10, preferably pH 6-9, supplemented with any state of the art detergent (anionic, cationic, non-ionic, Zwitterionic) is also suitable for extraction. Other suitable detergents or mixtures thereof include but are not limited to CHAPS, CHAPSO, C7BzO, ASB-14, n-Dodecyl beta-D-maltoside, Octyl beta-D-glucopyranoside, Octyl beta-Dl-thioglucopyranoside, Polyoyethylene 10 tridecyl ether, Brij® 56, Triton X-100, 3-(Decyldimethylammonio)propanesulfonate inner salt. For protein extraction any commercially available protein extraction buffer or kit can be used such as but not restricted to T-PER Tissue Protein Extraction Reagent (ThermoFisher Scientific), M-PER Mammalian Protein Extraction Reagent (ThermoFisher Scientific), Pierce IP Lysis buffer, any protein extraction kit from SigmaAldrich (PROTMEM, PROTTWO, PROTOT) supplemented with any state of the art cocktail of protease/phosphatase inhibitors (e.g. Cat. No. P8349, and/or PPC-1010, Sigma-Aldrich, St. Louis, MO, USA). Solubilization can be enhanced by sonification. Particulate material can be removed. Methods for removal of insoluble materials include but are not limited to filtration or centrifugation at 5 000 x g - 100 000 x g, preferably 50 000 x g for 1-120 min, preferably 20 min at 4 °C - 30 °C, preferably 4 °C. The supernatant is diluted 1:1 - 1:20, preferably 1:5, in a suitable buffer as used above (supplemented with protease inhibitors). To this solubilisate, the test compound or composition of interest, e.g. Compound-1 is added at a concentration of 1 - 1000 microM, preferably 10 microM. Buffer exchange for subsequent SDS-PAGE can be performed into Tris buffer ranging between 10-100 mM Tris, preferably 20 mM Tris between pH 6-10, preferably pH7.4 by a suitable method which includes but is not restricted to dialysis, gel filtration or centrifugation over Centrifugal Filter Units, preferably Amicon Ultra Centrifugal Filter Units (with a MWCO 3kDa; EMD Millipore Merck KGaA, Darmstadt, Germany). The concentrated proteins are dissolved in SDS-PAGE Laemmli sample buffer, which contains 2 % SDS, 5 % mercaptoethanol. To improve disaggregation of aggregated proteins, the buffer is supplemented without or with urea ranging from 0M-8 M, preferably 6 M urea and incubated for 10 min-24 h, preferably 90 min at room temperature. Solubilized proteins are subjected to 7-15 %, preferably 8 % SDS-PAGE under reducing conditions supplemented with urea. Compound or composition-interacting (e.g. Compound 1-interacting) proteins are identified by co-migration, protein bands are cut and subjected to nano-LC-ES-MS/MS (performed e.g. by Proteome Factory AG, Berlin, Germany). Proteins are identified using MS/MS ion search of the Mascot search engine (e.g. Matrix Science, London, England) and nr protein database (National Center for Biotechnology Information, Bethesda, MD). Ion charge in search parameters for ions from ESI-MS/MS data acquisition can be set to '1+, 2+, or 3+ 'according to the common charge state distribution for the instrument and the method.

This method can be applied to any compound or composition of interest by labelling the compound with a radiolabel (e.g. ³H, ¹²⁵I, ³⁵S, ³³S, ¹⁴C) or a dye, e.g. a fluorescent dye (e.g. the BODIPY series fluorescent dyes). Subsequent detection involves, e.g. autoradiography, or UV-light detection.

A preferred method for the identification of a physiological effect or the treatment effect of a TOMM6- and TOMM6 homologue-interacting compound or composition also includes quantitation of TOMM6/Tomm6 levels by proteome analysis and sequencing, and determination of gene expression level by state of the art methods, which include but are not restricted to Northern blotting, microarray gene expression analysis, quantitative real-time RT-PCR, transcriptome sequencing, SAGE.

In another aspect, the present invention is directed to a non-human transgenic animal expressing a transgene for TOMM6 or TOMM6 homolog, preferably from human, mouse, rat, bovine, zebrafish, chimpanzee, canis familiaris, or horse, preferably expressing a transgene for TOMM6 or a TOMM6 homolog having an amino acid sequence with at least 85 % sequence identity to one of SEQ ID NOs: 1 to 3, preferably under control of a neuron-specific promoter, preferably selected from the group consisting of the synapsin I (SYN) promoter, the calcium/calmodulin-dependent protein kinase II promoter, the tubulin alpha I promoter, the neuron-specific enolase promoter, the platelet-derived growth factor beta chain promoter, the tyrosine hydroxylase promoter (Thy1.2 regulatory element), a prion protein promoter, the cytomegalovirus immediate-early promoter/enhancer (CMV promoter) and hybrid promoters comprising the cytomegalovirus promoter fused to a neuron-specific promoter, more preferably the SYN promoter, the Thy1.1 promoter, the mouse prion protein promoter and the hamster prion protein promoter.

In another aspect, the present invention is directed to a use of a transgenic animal of the present invention for identifying TOMM6-interacting compounds or compositions for use in the treatment or prophylaxis of a disease or disorder selected from the group consisting of a nervous system disease or disorder from the group consisting of: brain injuries; cerebrovascular diseases; consequences of cerebrovascular diseases; motor neuron disease; dementias; ALS; multiple sclerosis; traumatic brain injury; small-vessel cerebrovascular disease; familial forms of Alzheimer's Disease; sporadic forms of Alzheimer's Disease; vascular dementia; M. Parkinson; frontotemporal dementia; Parkinson linked to chromosome-17; diabetic neuropathy; symptoms of depression and depression-related symptoms, preferably anhedonia and anorexia; schizophrenia with dementia; Korsakoff's psychosis; Lewy Body diseases; progressive supranuclear palsy; corticobasal degeneration; Pick's disease; Huntington's disease; thalamic degeneration; Creutzfeld-Jacob disease; HIV Dementia; disorders with mitochondrial dysfunction, preferably neurodegenerative diseases of ageing; cognitive-related disorder; mild cognitive impairment; age-associated memory impairment; age-associated cognitive decline; vascular cognitive impairment; central and peripheral symptoms of atherosclerosis and ischemia; perivascular disease; renal dysfunction and renal failure; stress-related disorders; attention deficit disorders; attention deficit hyperactivity disorders; and memory disturbances in children.

In another aspect, the present invention is directed to a method for identifying TOMM6-interacting compounds or compositions for use in the treatment of a nervous system disease or disorder comprising the steps of:
(i) administering a compound of interest to a transgenic animal according to the present invention in a physiologically effective amount;
(ii) identifying a change in at least one TOMM6 and TOMM6 homolog-related physiological parameter in the transgenic animal; and
(iii) correlating the change in TOMM6 and TOMM6 homolog-related physiological parameter(s) in the transgenic animal with TOMM6- and TOMM6 homolog-interacting activity of the compound of interest.

The following Figures and Examples serve to illustrate the invention and are not intended to limit the scope of the invention as described in the appended claims.
**Fig. 1** shows the identification of a plant extract, which inhibits insoluble HBsAg aggregation in yeast. Immunoblot detection of HBsAg protein in yeast (IB: anti-SAG) showed that P1 extract from *Galium aparine* and P2 extract from *Galium verum* prevented insoluble HBsAg aggregation, which was induced by overexpression in yeast. Extracts from *Nardostachys jatamansi* (N), *Valeriana officinalis* radix (V), *Ruta graveolens* (R), *Cyperus esculentus* tuber (C), and *Myrrha commiphora* (M) had no effect.
**Fig. 2** shows that plant extracts from *Galium aparine* and *Galium verum* and two active ingredients, alizarin and pseudopurpurin, prevent AT2R aggregation in HEK cells. **(A):** The plant extract from *Galium aparine* (P1) promoted AT2R monomer formation in HEK cells. Extracts from *Myrrha commiphora* (M), *Ruta graveolens* (R), *Nardostachys jatamansi* (N), *Valeriana officinalis* radix (V), and *Cyperus esculentus* tuber (C) had no/less effect compared to untreated control HEK cells (-). HEK cells were incubated for 72 h with different plant extracts as indicated, and stable AT2R aggregates were detected in immunoblot. The lower blot is a loading control detecting Gnb. **(B):** Two different preparations of the P1 extract from *Galium aparine* (P1 and P1'), an extract from the related plant *Galium verum* (P2), and two major ingredients thereof, i.e. alizarin (C-1, Compound-1) and pseudopurpurin (C-2, Compound-2) prevented AT2R aggregation in HEK cells. AT2R aggregation was detected in immunoblot after incubation of HEK cells for 72 h.

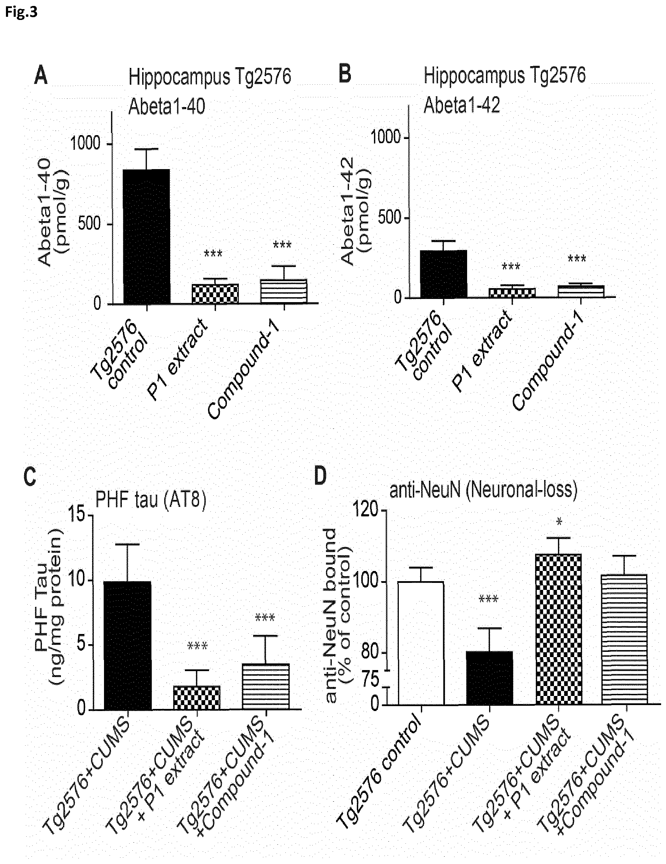

**Fig. 3****:** shows that the P1 extract from *Galium aparine* and alizarin (Compound-1) retard Abeta plaque formation in Tg2576 mice, and PHF tau hyperphosphorylation and neuronal loss in Tg2576 AD mice subjected to CUMS. **(A,B):** The P1 extract and Compound-1 retard the accumulation of SDS-insoluble Abetal-40 **(A)** and Abeta1-42 **(B)** in the hippocampus of 18 months-old Tg2576 AD mice. Mice were treated for 6 months starting at an age of 12 months. Untreated Tg2576 mice were used as controls {± s.d., n=4; ***, p<0.001 vs. untreated control; Tukey's test). **(C)** Quantification of hyperphosphorylated PHF-Tau with [¹²⁵I]-labeled AT8 antibody in hippocampi isolated from 15 months-old Tg2576 mice subjected to the CUMS protocol for three months and treated with P1 extract from *Galium aparine* and Compound-1 compared to untreated stressed (+CUMS) Tg2576 mice (±s.d., n=8; ***, p<0.001 vs. stressed (+CUMS) Tg2576 mice; Tukey's test). **(D)** Neuronal loss was determined by quantitative assessment of neuronal cell bodies by direct binding assay with [¹²⁵I]-labeled anti-NeuN antibody in hippocampi isolated from 15 months-old Tg2576 mice subjected to the CUMS protocol for three months and treated with P1 extract from *Galium aparine* and Compound-1 compared to untreated stressed (+CUMS) Tg2576 mice. Neuronal cell bodies are presented as percentage of 15 month-old untreated non-stressed Tg2576 controls (±s.d., n=8; ***, p<0.001 and *, p<0.05; vs. non-stressed Tg2576 controls; ***, p<0.001 vs. Tg2576+CUMS+P1 extract and Tg2576+CUMS+Compound-1; Tukey's test).

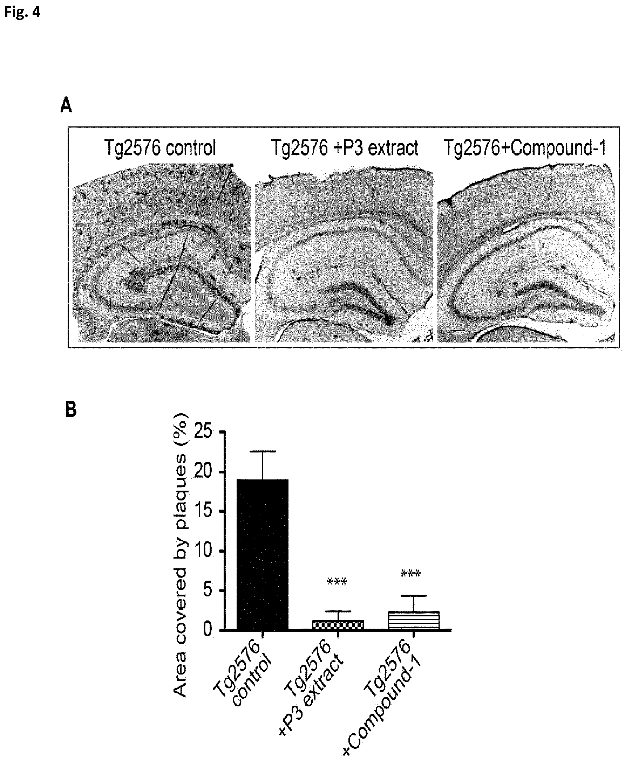

**Fig. 4** shows that the P3 extract from *Rubia cordifolia* and alizarin (Compound-1) retard Abeta plaque formation in hippocampus and brain cortical areas of Tg2576 mice. **(A)** Immunohistological assessment of Abeta plaque load with Abeta-specific BAM-10 antibody detects decreased Abeta plaque load in hippocampal and frontal cortex areas from 18 months-old Tg2576 mice treated with P3 extract and Compound-1 for 6 months compared to untreated Tg2576 AD mice, bar: 200 micro-m. **(B)** Quantitative analysis of treatment effects of P3 extract from *Rubia cordifolia L.* and alizarin (Compound-1) by quantitation of hippocampal area covered by plaques (±s.d., n=8 mice/group; ***,p<0.001 vs. Tg2576 control; Tukey's test).

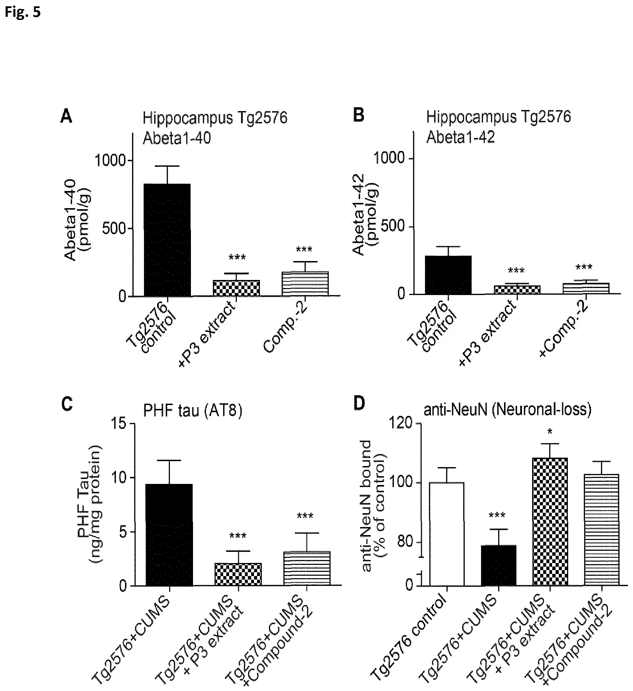

**Fig. 5** shows that The P3 extract from *Rubia cordifolia* and pseudopurpurin (Compound-2) retard hippocampal Abeta peptide accumulation in Tg2576 mice, and PHF tau hyperphosphorylation and neuronal loss in Tg2576 AD mice subjected to CUMS. **(A,B)** The P3 extract and Compound-2 retard the accumulation of SDS-insoluble Abetal-40 **(A)** and Abeta1-42 **(A)** in the hippocampus of 18 months-old Tg2576 AD mice. Mice were treated for 6 months starting at an age of 12 months. Untreated Tg2576 mice were used as controls {± s.d., n=4; **,p<0.01; *,p<0.05 vs. untreated control; Tukey's test). **(B)** Quantification of hyperphosphorylated PHF-Tau with [¹²⁵I]-labeled AT8 antibody in hippocampi isolated from 15 months-old Tg2576 mice subjected to the CUMS protocol for three months and treated with P3 extract from *Rubia cordifolia L.* and Compound-2 compared to untreated stressed (+CUMS) Tg2576 mice (±s.d., n=8; ***,p<0.001 vs. stressed (+CUMS) Tg2576 mice; Tukey's test). **(D)** Neuronal loss was determined by quantitative assessment of neuronal cell bodies by direct binding assay with [¹²¹I]-labeled anti-NeuN antibody in hippocampi isolated from 15 months-old Tg2576 mice subjected to the CUMS protocol for three months and treated with P3 extract from *Rubia cordifolia L.* and Compound-2 compared to untreated stressed (+CUMS) Tg2576 mice. Neuronal cell bodies are presented as percentage of 15 month-old untreated non-stressed Tg2576 controls (±s.d., n=8; ***,p<0.001 and *,p<0.05; vs. non-stressed Tg2576 controls; ***,p<0.001 vs. Tg2576+CUMS+P1 extract and Tg2576+CUMS+Compound-1; Tukey's test).

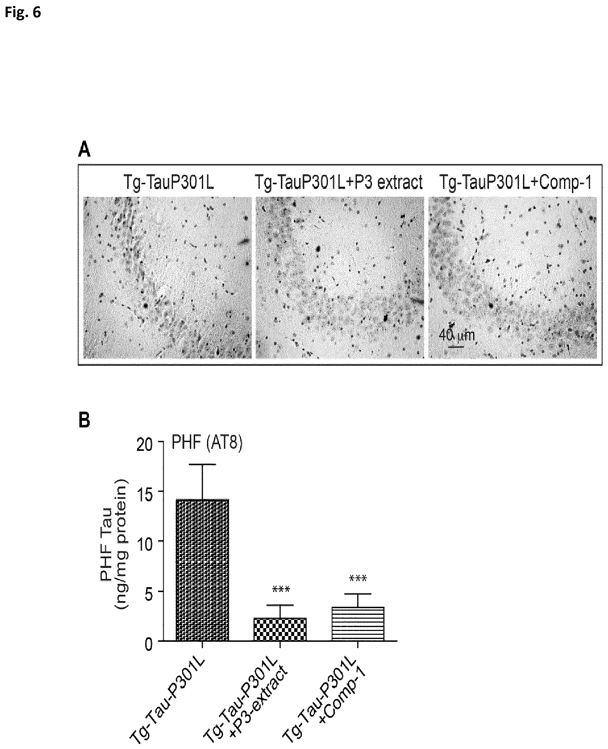

**Fig. 6** shows that the P3 extract from *Rubia cordifolia L.* and alizarin (Compound-1) retard PHF-Tau hyperphosphorylation in the Tg-TauP301L model of tauopathy. **(A)** Immunohistological detection of hippocampal PHF-Tau hyperphosphorylation was performed with anti-PHF antibody (AT8) on hippocampal sections of 12 months-old Tg-TauP301L mice after treatment for 6 months with P3 extract from *Rubia cordifolia L.* and Compound-1 compared to untreated 12-months-old Tg-TauP301L controls; bar: 40 micro-m. **(B)** Quantitative determination of hippocampal Tau hyperphosphorylation in 12 months-old Tg-TauP301L mice after treatment for 6 months with P3 extract from *Rubia cordifolia L.* and Compound-1 compared to untreated Tg-TauP301L controls was performed by direct binding assay with [¹²⁵I]-labeled AT8 antibody (±s.d., n=8, ***,p<0.001; vs. Tg-Tau-P301L, Tukey's Test).

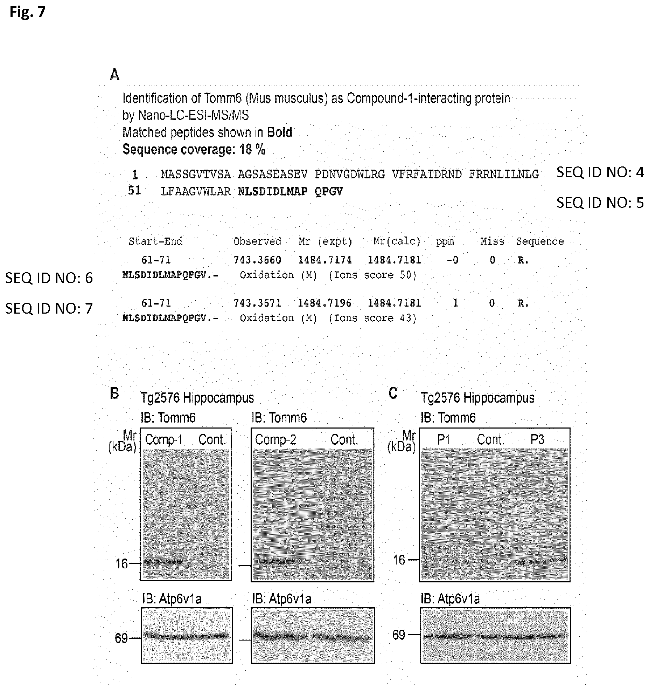

**Fig. 7** shows the identification of Tomm6 as an interacting/stabilizing protein of Compound-1, Compound-2, and extract P1 and P3 from *Galium aparine* and *Rubia cordifolia L.* (A) Solubilized hippocampal proteins from Tg2576 mice were incubated with Compound-1 at a concentration of 10 microM, proteins were concentrated by centrifugation over Amicon Ultra Centrifugal Filter Units (MWCO 3kDa), and separated by urea-containing SDS-PAGE under reducing conditions. Compound-1-interacting proteins were identified by co-migration, the protein band was cut and subjected to protein identification. Nano-LC-ESI-MS/MS analysis identified Tomm6 as a hippocampal protein, which interacts with Compound-1. **(B, C)** Immunoblot detection of hippocampal Tomm6 in aged 18-month-old Tg2576 mice without (Cont.) or with 6 months of treatment with Compound-1 (Comp-1) and Compound-2 (Comp-2) is shown in panels **(B)** and treatment with P1 extract from *Galium aparine* (P1) and P3 from *Rubia cordifolia L.* (P3) is shown in panel **(C).** The lower panels show control immunoblots detecting Atp6vla (V-type protein ATPase catalytic subunit A); (**B,** left panel n=4 mice/group; **B,** right panel n=5 mice/group; **C,** n=5 treated mice/group and n=4 controls).

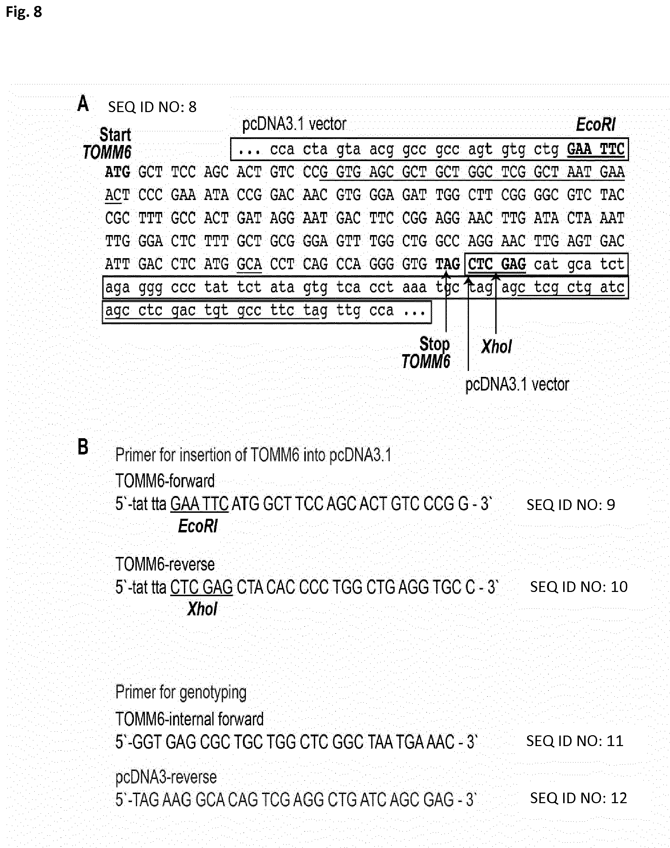

**Fig. 8** shows the construction of TOMM6 expression plasmid. **(A)** Insertion of TOMM6 cDNA into EcoRI and Xhol sites of pcDNA3.1 expression plasmid. **(B)** Sequences of PCR-primers used for construction of TOMM6-pcDNA3.1 plasmid, and for genotyping PCR are given.

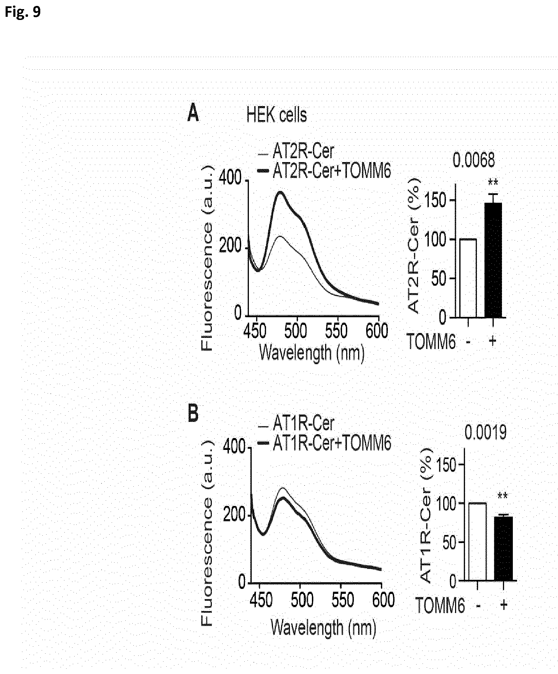

**Fig. 9** shows that TOMM6 enhances AT2R (AGTR2) protein folding in cells. **(A)** Expression of TOMM6 with AT2R-Cer in HEK cells significantly increased cell surface AT2R-Cer protein levels as determined by fluorescence spectrophotometry of AT2R-Cer (±s.d., n=8). **(B)** Expression of TOMM6 with AT1R-Cer in HEK cells significantly decreased cell surface AT1R-Cer (AGTR1-Cerulean) protein levels (±s.d., n=8).

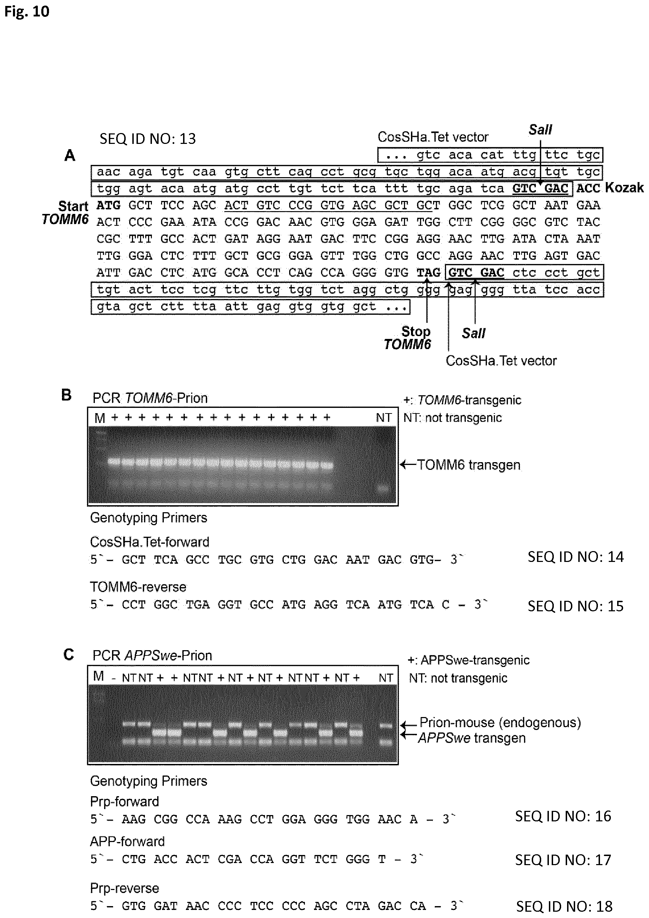

**Fig. 10** shows the generation of Tg-TOMM6 mice with neuron-specific expression of TOMM6. **(A)** Construction of the plasmid used for generation of Tg-TOMM6 mice. The cDNA encoding TOMM6 was inserted into the *Sail site* of the CosS.Ha.Tet vector. **(B, C)** Identification of double-transgenic Tg-2576-TOMM6 mice by genotyping PCR. Primer sequences for detection of TOMM6-transgen **(B),** and APPSwe- transgen are given **(C).** As an internal control, the endogenous *Prp* was co- amplified **(C).**

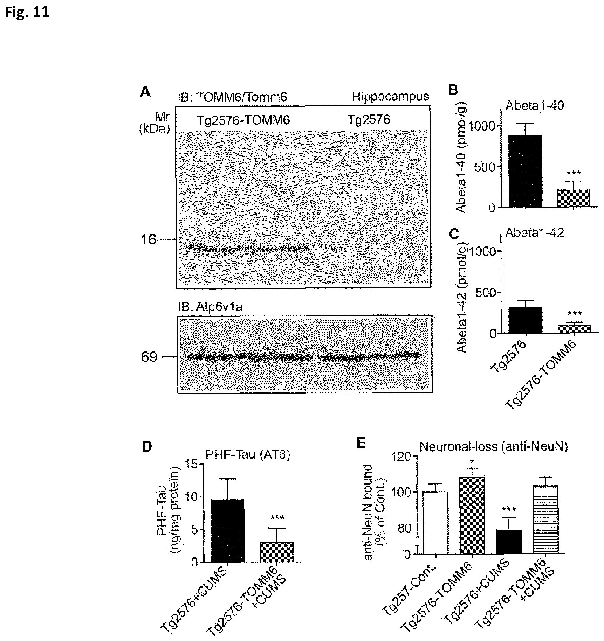

**Fig. 11** shows that transgenic TOMM6 expression retards major neuropathological features in the hippocampus of Tg2576 AD mice. **(A):** Immunoblot detection of hippocampal content of TOMM6/Tomm6 protein in 18 months-old double-transgenic Tg2576-TOMM6 mice (n=10) relative to single-transgenic Tg2576 controls (n=8). The lower panel shows a control immunoblot detecting mitochondrial Atp6vla (V-type protein ATPase catalytic subunit A). **(B, C):** Transgenic expression of *TOMM6* retarded the accumulation of SDS-insoluble Abetal-40 **(B)** and Abeta1-42 **(C)** in the hippocampus of 18 months-old double-transgenic Tg2576-TOMM6 mice compared to single-transgenic Tg2576 control mice (±s.d., n=8 (Tg2576) and n=10 (Tg2576-TOMM6); ***, p<0.0001. **(D):** Transgenic *TOMM6* expression led to a decreased hippocampal content of hyperphosphorylated PHF-Tau (detected with AT8 antibody) in 15 months-old Tg2576-TOMM6 mice with 3 months of CUMS (±s.d., n=8; ***,p=0.0003). **(E)** Transgenic *TOMM6* expression prevented the hippocampal neuronal loss (detected with anti-NeuN antibody) in 15 months-old Tg2576-TOMM6 mice with 3 months of CUMS compared to single-transgenic Tg2576 mice subjected to CUMS (±s.d., n=8; ***, p<0.001 vs. Tg2576-Cont, Tg2576-TOMM6, and Tg2576-TOMM6+CUMS; *p<0.05 vs. Tg2576-Cont.; Tukey's test).

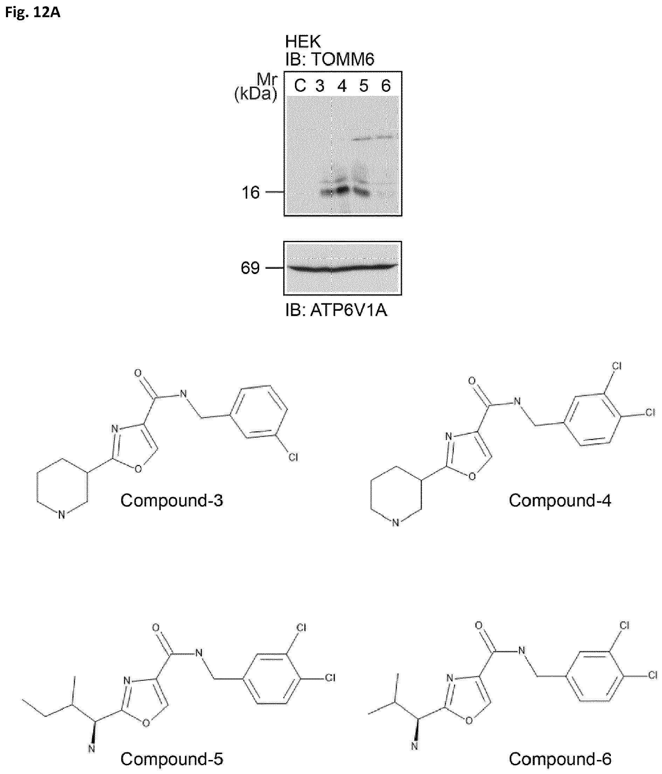

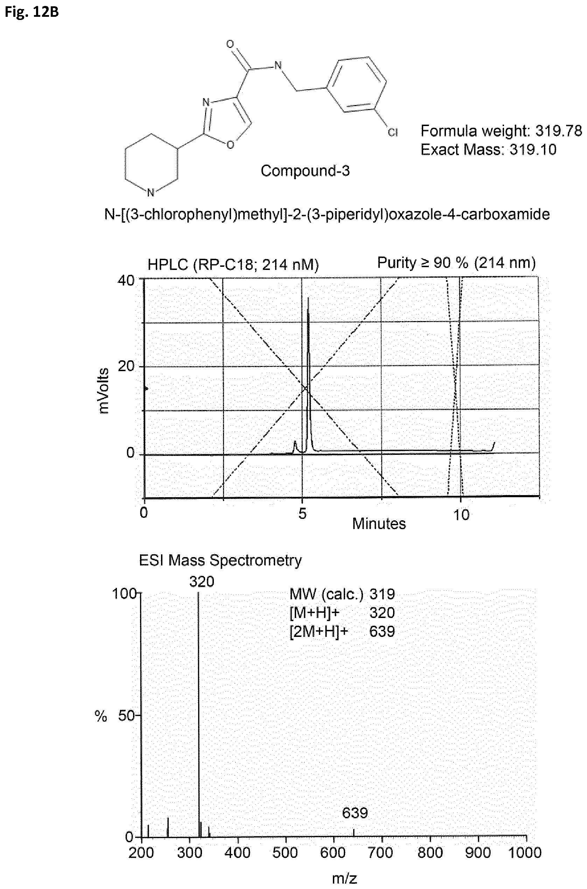

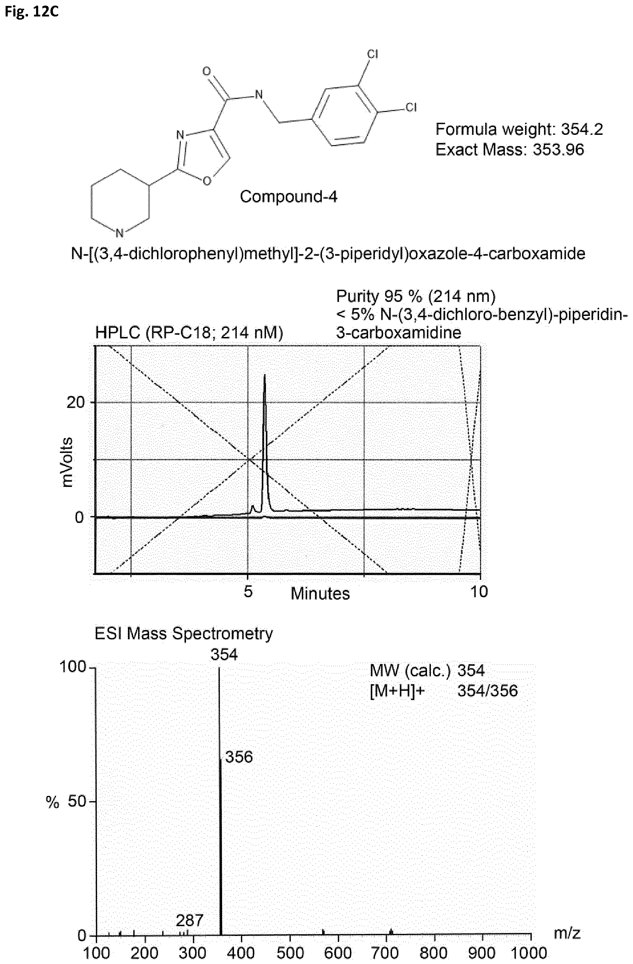

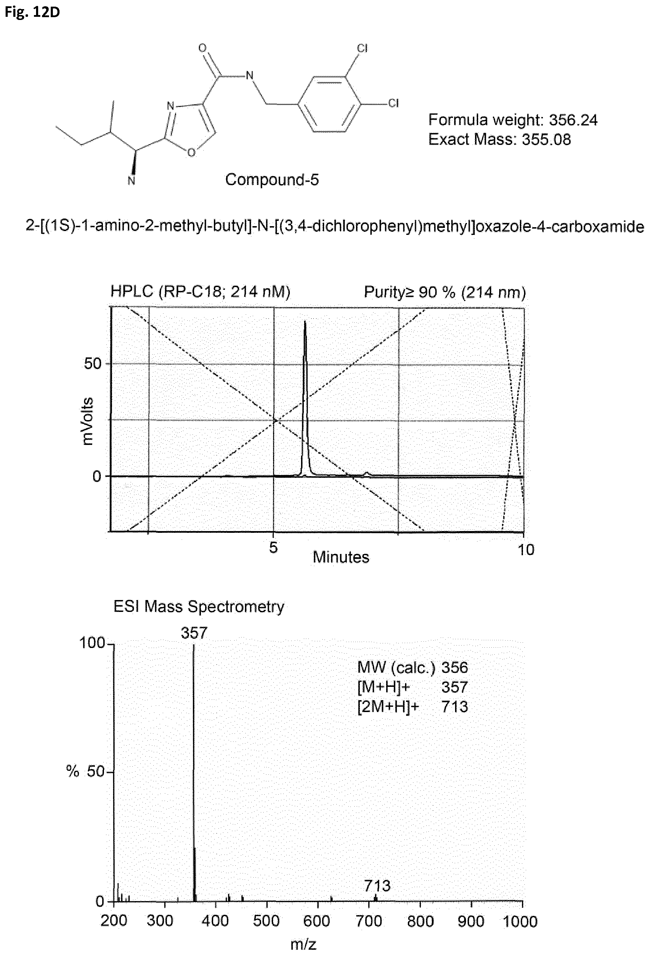

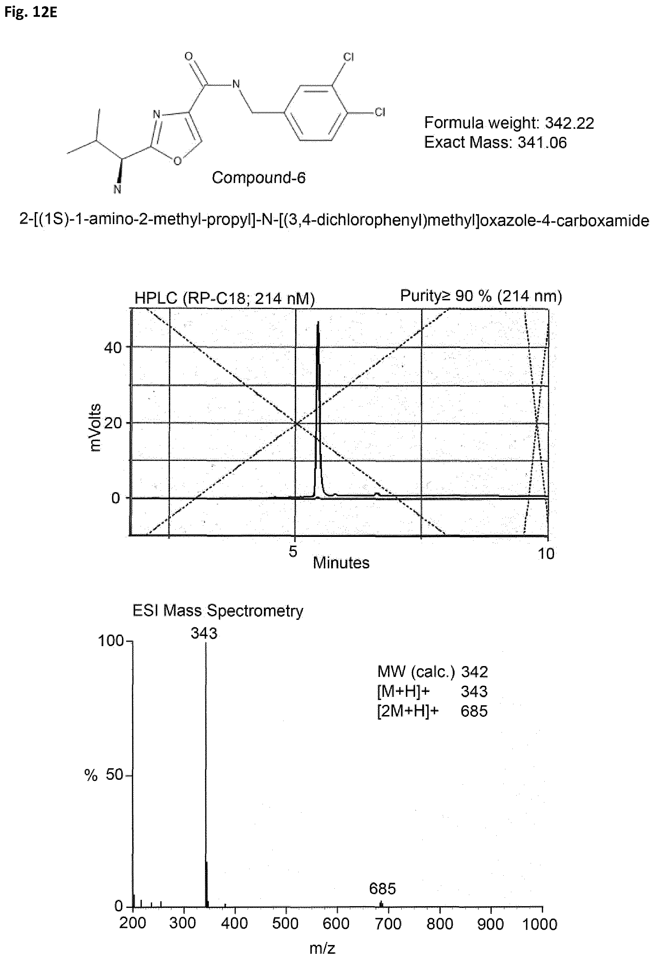

**Fig. 12** **shows that compounds 3-6, which are not antioxidants increase neuroprotective TOMM6 in HEK cells. (A):** Immunoblot detection of TOMM6 protein in HEK cells cultured for 60 h without (control) or with compound-3, compound-4, compound-5, and compound-6 (final concentration 20 microM). The lower panel shows chemical formulas of compounds. **(B-E):** Analytical HPLC and ESI-MS data of compound-3 **(B),** compound-4 **(C),** compound-5 **(D),** and compound-6 **(E).**

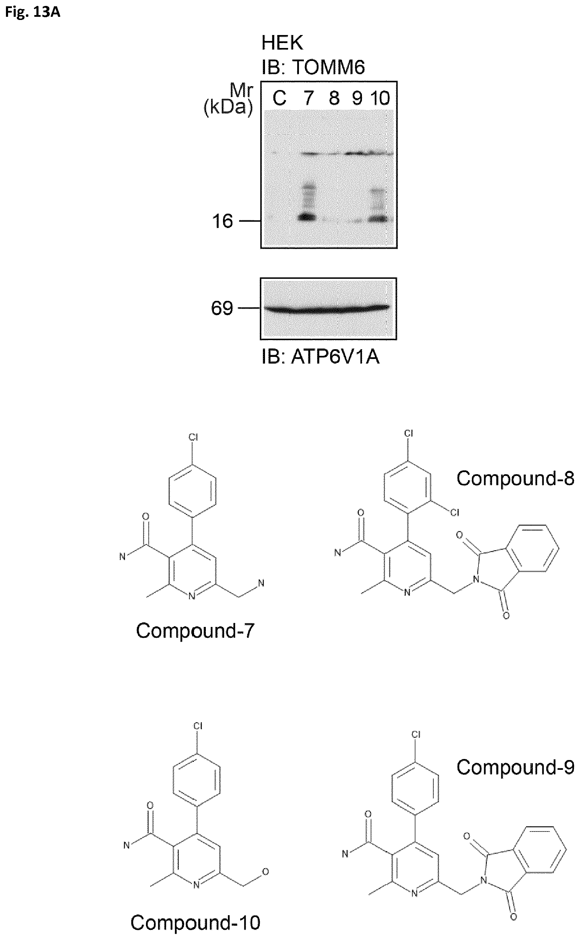

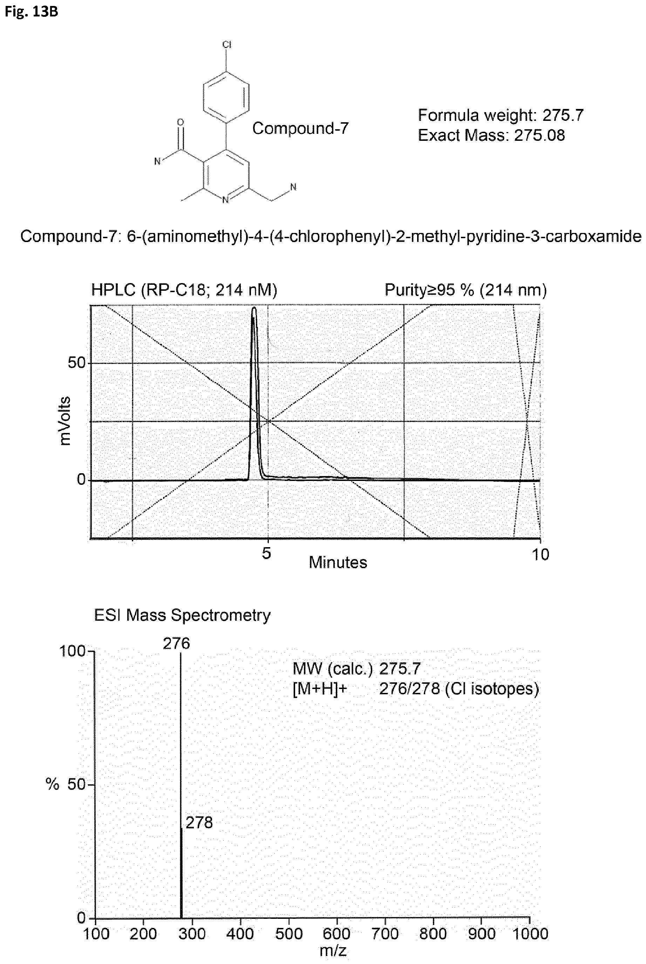

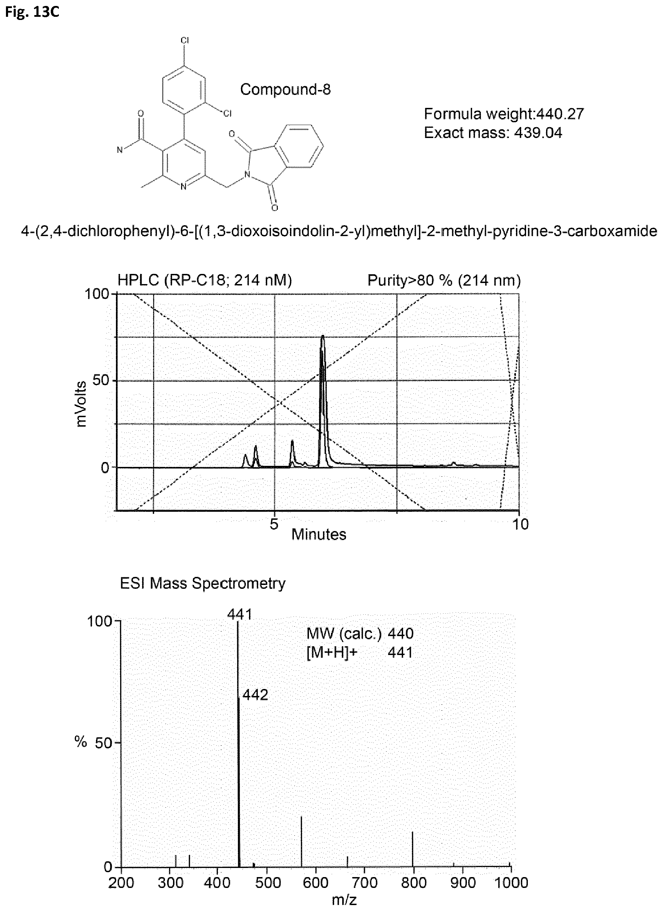

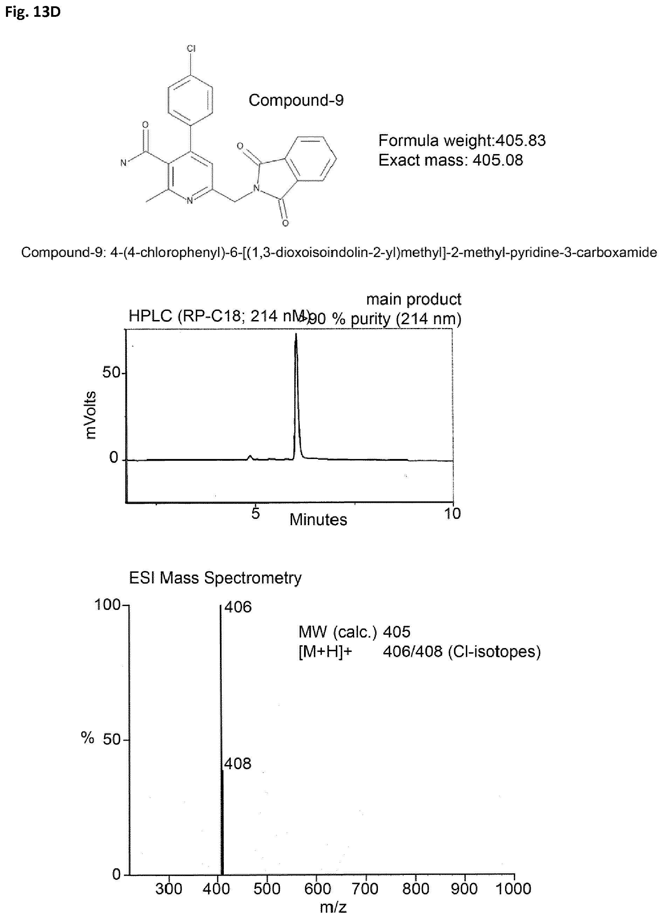

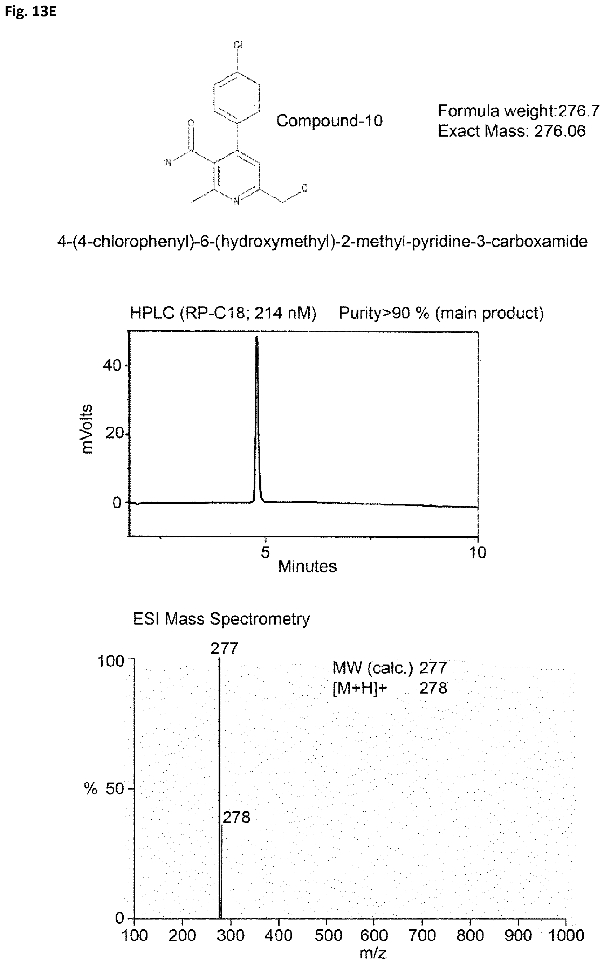

**Fig. 13** **shows that compounds 7-10, which are not antioxidants increase neuroprotective TOMM6 in HEK cells. (A):** Immunoblot detection of TOMM6 protein in HEK cells cultured for 60 h without (control) or with compound-7, compound-8, compound-9, and compound-10 (final concentration 20 microM). The lower panel shows chemical formulas of compounds. **(B-E):** Analytical HPLC and ESI-MS data of compound-7 **(B),** compound-8 **(C),** compound-9 **(D),** and compound-10 **(E).**
**Fig. 14** **shows that TOMM6-interacting compound-4 and compound-7 inhibit symptoms of AD and neurodegeneration in AD model mice. (A):** Treatment with compound-4 and compound-7 of Tg2576 AD mice imposed to chronic mild stress by the CUMS protocol led to an increased hippocampal Tomm6 protein content (n=5-6 mice/group). **(B):** Treatment for 6 months with Tomm6-interacting compound-4 and compound-7 inhibited hippocampal Abetal-40 and Abeta1-42 aggregation in 18-month-old Tg2576 AD mice (±s.d., n=4, **, p<0.01; ***, p<0.001; Tukey's test). **(C,D):** Compound-4 and compound-7 inhibited hippocampal PHF tau hyperphosphorylation **(C)** and hippocampal neuronal loss **(D)** in 15 month-old Tg2576 AD mice subjected to the CUMS protocol (±s.d., n=8, ***,p<0.001; Tukey's test).

### Example 1: Materials and Methods

### Animal models and generation of Tg-TOMM6 mice

(a) Tg2576 mice (Taconic Biosciences, Rensselaer, NY, USA) with overexpression of human APP695 with the double mutation K670N/M671L were used as a genetic model of familial AD (FAD) (see Hsiao et al., Science 274, 99-103 (1996)).
(b) Tg-TauP301L mice (Model 2508, Taconic Biosciences, Rensselaer, NY, USA) with neuron-specific expression of the most common FTDP-17 (frontotemporal dementia and parkinsonism linked to chromosome 17) mutation (Lewis et al., Nature Genetics 25, 402-405 (2000)).
(c) To generate Tg-TOMM6 mice, the cDNA of TOMM6 was inserted into the *SalI* site of the CosSHa.Tet vector (InPro Biotechnology Inc. San Francisco, CA, USA), which directs neuron-specific expression under control of the Syrian hamster prion protein (HaPrP) promoter (Hsiao et al., Science 274, 99-103 (1996), Scott et al., Protein Sci. 1, 986-997 (1992)). Plasmid sequences were removed by *NotI* digestion. The purified linear DNA (2ng/microL) was injected into fertilized oocytes dissected out from super-ovulated mice (FVB or B6 (C57BL/6J) mice). For DNA injection, oocytes were kept in M2 medium. After DNA injection, injected embryos were transferred into M16 medium and incubated overnight in M16 medium in the presence of 10 % CO₂ at 37°C in a humidified incubator. Thereafter, double-cell stage embryos were selected and transferred into the oviduct of pseudo-pregnant CD-1 foster mice. Pseudopregnancy was induced by overnight breeding with an infertile (vasectomized) male and detected by the presence of a vaginal plug. After birth, genomic DNA from F0 mice was isolated from ear punch biopsies taken at 3-4 weeks of age. Mice with stable genomic integration of the *TOMM6* transgene were identified by genotyping PCR and used for further breeding.
   Tg-CMV-TOMM6 mice with transgenic TOMM6 expression under control of the ubiquitous CMV immediate-early promoter/enhancer were also generated, which directs ubiquitous expression of a transgene (Fu et al., J. Biol. Chem. 288, 7738-7766 (2013)). For ubiquitous expression, the cDNA coding for TOMM6 was inserted into *EcoRI* and *XhoI* sites of pcDNA3.1 plasmid; Thermo Fisher Scientific, Waltham, MA, USA). Transgenic mice were generated as detailed above.
(d) The CUMS protocol was performed with 12 months-old male Tg2576 mice for 3 months essentially as described (AbdAlla et al., J. Biol. Chem. 284, 6554-6565 (2009)). The following stimuli were administered each week in a random order: 3 h of 45 ° cage tilt, two times a week; soaked cage for 12 h, once a week; water deprivation for 14h, once a week; lights on for 9 h during the dark phase, once a week; noise in the room for 3h, three times a week; flashing light for 30 min, three times a week (AbdAlla et al., J. Biol. Chem. 284, 6554-6565 (2009)). After the CUMS protocol, more than 90 % of untreated stressed mice showed signs of anhedonia in the sucrose preference test (≤50% of sucrose consumption compared to non-stressed controls).
(e) Extracts and compounds: As indicated, extract P1 from *Galium aparine,* extract P3 from *Rubia cordifolia L,* and small-molecule compounds (Compound-1, Compound-2, Compound-4, and Compound-7: 10 mg/kg body weight/d) were added to drinking water. Compound-1 (alizarin; 1,2-dihydroxyanthraquinone) was purchased from Sigma (Sigma-Aldrich, St. Louis, MO, USA), and Compound-2 (pseudopurpurin, purpurin-3-carboxylic acid) was isolated from *Rubia tinctorum* according to established protocols (Richter D., Biochem. J. 31, 591-595 (1937); Derksen et al., Phytochem. Anal. 15, 397-406 (2004)). Synthesis of Compound-4 and Compound-7 is described below. Plant extracts were made by decoction of dried plant material for 1 h in boiling water (200 g pulverized dried plant material/1L water). *For in vivo* treatment, the plant extract was filtered, and the alizarin/anthraquinone content was adjusted to an amount of 0.05-0.1 mg/ml of alizarin by thin layer chromatography (TLC silica gel precoated plates 60F254 Merck, Darmstadt, Germany; mobile phase toluene/ethyl acetate-/formic acid (70:25:5) or dichloromethane:methanol (9:1); visualization by visible light, UV 254 nm, 366 nm and/or by Bornträger reaction with KOH reagent (5 -10% KOH in methanol)). For incubation with HEK cells, the plant extract was further concentrated by rotary evaporation to a final volume of 10 ml, filtrated and added to the cell culture medium (20 micro-l/10 ml medium).
(f) Treatments: Treatment of the Tg2576 model was performed for six months without CUMS, or for three months (during the CUMS protocol), starting at an age of 12 months. Treatment of the Tg-TauP301L model was started at an age of 6 months and continued until 12 months. As a model, which reproduces major features of sporadic AD, aged 15 months-old male rats were subjected to the CUMS protocol for 4 weeks similarly as described (AbdAlla et al., Biomed. Res. Int. 2015:917156 (2015)). All mice/rats were kept on a light/dark cycle of 12 h light/12h dark, had free access to food and water (unless the CUMS protocol required a restriction) and were fed a standard rodent chow. At the end of the observation period, mice or rats were anesthetized (i.p.) with tribromoethanol (250 mg/kg) or urethane (1.2 g/kg), perfused intracardially with sterile PBS, and brains were isolated, and processed for histology or biochemical analyses. For protein extraction, hippocampi were dissected and immediately frozen in liquid nitrogen. All animal experiments were performed in accordance with NIH guidelines and approved by the local committees on animal experiments (University of Zurich Switzerland and MRC Cairo).

### Compound synthesis

Compounds 3-10 were synthesized by EMC microcollections GmbH, Tübingen according to established published protocols. Synthesis was performed by solid phase chemical synthesis methods, which were adapted from established protocols (for compound-3, compound-4, compound-5, compound-6: Singh et al., Organic Letters 14, 1198-1201 (2012); for compound-7, compound-8, compound-9, compound-10: Kaczanowska et al., J. Heterocyclic Chem. 48, 792 (2011), Kaczanowska et al., ACS Med. Chem. Lett. 1, 530-535 (2010)).

### Antibodies

The following antibodies were used for immunoblotting and immunohistology: Abeta plaques were stained with monoclonal mouse antibody BAM-10 (crossreactive with residues 1-12 of the Abeta peptide, Sigma-Aldrich, St. Louis, MO, USA); antibodies against AT2R (AGTR2) were raised in rabbit against an antigen encompassing amino acids 320-349 of the human AT2R (AGTR2) sequence (AbdAlla et al., J. Biol. Chem. 276, 39721-39726 (2001); J. Biol. Chem. 284, 6566-6574 (2009). HBsAg was detected with polyclonal antibodies raised against an antigen encompassing amino acids 1-20 of surface antigen HBsAg, and by a monoclonal HBsAg antibody produced in mouse (SAB4700767, Sigma-Aldrich, St. Louis, MO, USA). Suitable antibodies for detection of HBsAg expression, aggregation and monomerization were also isolated from human blood from individuals undergoing vaccination against Hepatitis B. Antibodies against TOMM6 were raised in rabbit against a recombinant human TOMM6 protein (HPA004801, Sigma-Aldrich, St. Louis, MO, USA); polyclonal antibodies were also raised in rabbit against a synthetic peptide encompassing amino acids 24-74 of human TOMM6 (ab205396; Abcam Cambridge, MA, USA). PHF-Tau was detected with monoclonal AT8 antibody (MN1020; Thermo Fisher Scientific, Waltham, MA, USA). Mouse monoclonal anti-NeuN antibody was raised against the neuron-specific protein NeuN (MAB377, clone A60, EMD Millipore, Merck KGaA, Darmstadt, Germany). Antibody against ATP6V1A was raised in rabbit against a recombinant fragment corresponding to amino acids 60-344 of human ATP6V1A (ab137574; Abcam, Cambridge, MA, USA).

### Immunohistochemistry

For Abeta plaque detection by immunohistology, paraffin-embedded brain sections (8 microm, 10-15 sections/brain taken at 30-50 microm intervals) from sham-treated Tg2576 mice (controls), and Tg2576 mice with 6 months of treatment with P1 extract or P2 extract and Compound-1 were used as indicated. After antigen retrieval, sections were stained with monoclonal BAM-10 antibody (Sigma Aldrich, St. Louis, MO, USA), which cross-reacts with residues 1-12 of the Abeta peptide. Plaque burden was analyzed by computerized quantitative image analysis (AbdAlla et al., Int. J. Mol. Sci. 14, 16917-16942 (2013)). Hyperphosphorylated Tau was detected with AT8 antibody on paraffin-embedded brain sections from 12 months-old Tg-TauP301L mice (Model 2508, Taconic Biosciences, Rensselaer, NY, USA) without and with treatment with P1 or P2 extract, and Compound-1 for 6 months.

### Expression of HBsAg in yeast

Recombinant HBsAg cDNA (synthesized by GeneScript, Piscataway, NJ, USA) was inserted into the expression vector p42K-TEF (D3011001-1 Dualsystems Biotech AG, Zurich). Yeast transformants were selected in the presence of G418 in the yeast growth medium (YPD; Bacto-yeast extract 10 g/L, Bacto-peptone 20 g/L and D-Glucose 20 %). Yeast (Saccharomyces cerevisae) strain INVSc1 was used (Invitrogen GmbH, Nr. C810-00). After 3-4 days of cultivation to allow for protein expression, yeast cells were harvested by centrifugation, followed by washing with ice-cold PBS. The yeast cell pellet was immediately frozen at -70°C. Proteins were extracted for 30 min. at 4°C with solubilization buffer (1 % sodium deoxycholate, 0.1 % SDS, 0.1% NP40, 5 mM EDTA, 50 mM Tris, pH 8.0) supplemented with protease/phosphatase inhibitors (Cat. No. P8349, and/or PPC1010, Sigma-Aldrich, St. Louis, MO, USA). Particulate material was removed by centrifugation at 50 000 x g for 20 min at 4 °C. Solubilized proteins were concentrated and delipidated by precipitation with ice-cold acetone/methanol (12:2, final concentration 83 %) for 90 min at 4 °C. The pellet was dissolved in SDS-sample buffer supplemented with 2 % SDS, 0.1 M DTT (or 5 % mercaptoethanol), and 6 M urea for 90 min at room temperature. Proteins were stored at a concentration of 0.5 mg - 1 mg/ml at -70 °C for further use. Protein were separated by urea-containing SDS-PAGE (7.5-10 %) under reducing conditions, transferred to PVDF membranes by electrophoretic protein transfer in a tank transfer cell (Mini Trans-Blot cell, Bio-Rad GmbH, München, Germany), and transferred HBsAg proteins were detected in immunoblot with HBsAg-specific antibodies raised in rabbit against an N-terminal epitope of HBsAg, or by a monoclonal HBsAg antibody produced in mouse (SAB4700767, Sigma-Aldrich, St. Louis, MO, USA). Suitable antibodies for detection of HBsAg expression and aggregation were also isolated from human blood from individuals undergoing vaccination against Hepatitis B. Bound antibodies were visualized with F(ab)₂ fragments of enzyme-coupled secondary antibodies (Dianova GmbH, Hamburg, Germany), or by enzyme-coupled protein A (EMD Millipore, Merck KGaA, Darmstadt, Germany), and followed by enhanced chemiluminescent detection (ECL Prime, Amersham, GE Healthcare Life Sciences, Glattbrugg, Switzerland).

### Nano-LC-ESI-MS/MS

To isolate Compound-1-interacting hippocampal proteins, hippocampi were isolated from 5-10 aged Tg2576 mice, pulverized under liquid nitrogen and solubilized for 30 min at 4°C in solubilization buffer (1 % sodium deoxycholate, 0.05 % SDS, 0.05 % Tween 20 in PBS, pH 7.4, supplemented with protease inhibitors). Insoluble material was removed by centrifugation. The supernatant was diluted 1:5 in PBS (supplemented with protease inhibitors). To this solubilisate, Compound-1 was added at a concentration of 10 µM. Buffer exchange into 20 mM Tris pH 7.4 was performed by centrifugation over Amicon Ultra Centrifugal Filter Units (MWCO 3kDa; EMD Millipore Merck KGaA, Darmstadt, Germany). The concentrated proteins were dissolved in Laemmli sample buffer (Bio-Rad GmbH, München, Germany) supplemented with mercaptoethanol (355 mM) and subjected to 8% urea-containing SDS-PAGE under reducing conditions. Compound-1-interacting proteins were identified by co-migration, protein bands were cut and subjected to nano-LC-ES-MS/MS (performed by Proteome Factory AG, Berlin, Germany). Proteins were identified using MS/MS ion search of the Mascot search engine (Matrix Science, London, England) and nr protein database (National Center for Biotechnology Information, Bethesda, MD). Ion charge in search parameters for ions from ESI-MS/MS data acquisition were set to '1+, 2+, or 3+'according to the common charge state distribution for the instrument and the method.

### Immunoblot detection of proteins and biochemical analyses

Immunoblot detection of PHF-Tau in the hippocampus of stressed rats subjected to the CUMS protocol was performed with monoclonal PHF (AT8) antibody and the guanidine-hydrochloride-extracted hippocampal protein fraction (6.25 M guanidine hydrochloride in 50 mM Tris, pH 8.0). Proteins were separated by 8% urea-containing SDS-PAGE under reducing conditions. SDS-insoluble hippocampal contents of Abetal-40 and Abeta1-42 were determined by ELISA after serial tissue extraction in the presence of protease inhibitors according to the protocol of the manufacturer (Invitrogen KHB3481 and KHB3441).

For immunoblot detection of (hippocampal or HEK cell) proteins, e.g. Tomm6/TOMM6 and Atp6v1a/ATP6V1A, (hippocampal) tissue was pulverized under liquid nitrogen, and HEK cell pellets were directly subjected to protein extraction. Proteins were extracted for 30 min at 4°C with solubilization buffer (1 % sodium deoxycholate, 0.1 % SDS, 0.1% NP40, 5 mM EDTA, 50 mM Tris, pH 8.0) supplemented with protease/phosphatase inhibitors (Cat. No. P8349, and/or PPC1010, Sigma-Aldrich, St. Louis, MO, USA). Particulate material was removed by centrifugation at 50 000 x g for 20 min at 4 °C. Solubilized proteins were concentrated and delipidated by precipitation with ice-cold acetone/methanol (12:2, final concentration 83 %) for 90 min at 4 °C. The pellet was dissolved in SDS-sample buffer supplemented with 2 % SDS, 0.1 M DTT (or 5 % mercaptoethanol), and 6 M urea for 90 min at room temperature. Proteins were stored at a concentration of 0.5mg - 1 mg/ml at -70 °C for further use. Immunoblot detection of proteins was performed with affinity-purified antibodies or F(ab)₂ fragments of the respective antibodies after separation of proteins by urea-containing SDS-PAGE (7.5 % - 10 %) under reducing conditions and electrophoretic protein transfer to PVDF membranes in a tank transfer cell (Mini Trans-Blot cell, Bio-Rad GmbH, München, Germany). Bound antibodies were visualized with F(ab)₂ fragments of enzyme-coupled secondary antibodies (Dianova GmbH, Hamburg, Germany) pre-absorbed to mouse serum proteins, or by enzyme-coupled protein A (EMD Millipore, Merck KGaA, Darmstadt, Germany) as applicable, and followed by enhanced chemiluminescent detection (ECL Plus, and/or ECL Prime, Amersham, GE Healthcare Life Sciences, Glattbrugg, Switzerland).

Proteins were also extracted from yeast cells and HEK cells expressing the indicated protein (i.e. HBsAg or AGTR2) as described above. Neuronal cell loss and hyperphosphorylated Tau in hippocampi of 15 months-old Tg2576 mice subjected to the CUMS protocol for 3 months was determined with crude homogenates of dissected hippocampi by direct binding assay with neuron-specific [¹²⁵I]-labeled anti-NeuN antibody (MAB377, clone A60, EMD Millipore, Merck KGaA, Darmstadt, Germany) and [¹²⁵I]-labeled AT8 antibody similarly as described (AbdAlla et al., J. Biol. Chem. 284, 6554-6565 (2009)).

### Fluorescence spectrophotometry of AT2R-Cer and AT1R-Cer expressed in HEK cells

AT2R-Cer (AGTR2-Cerulean) is a fusion protein, in which full-length AGTR2 is fused C-terminally with a 5-Gly linker to the Cerulean variant (Cer) of the enhanced green fluorescent protein. The analogous construct of AGTR1-Cer, in which full-length AGTR1 is fused C-terminally with a 5-Gly linker to the Cerulean variant (Cer) of the enhanced green fluorescent protein, was generated in frame of a previous study (Quitterer et al., Biochem. Biophys. Res. Commun. 409, 544-549 (2011)). AGTR2-Cer and AGTR1-Cer were expressed in HEK cells with and without *TOMM6* (cDNA coding for TOMM6 was inserted into *EcoRI* and *XhoI* sites of pcDNA3.1 plasmid; Figure 8). Thirty-six to forty-eight hours after transfection, cells were detached by 0.05 % Trypsin-EDTA solution, trypsin-EDTA was rapidly removed by washing with PBS, cells were collected by centrifugation and suspended in incubation buffer (335 mg KCI, 294 mg CaCl2, 407 mg MgCl2, 8.2 g NaCl, 2.4 g HEPES-Na+ ad 1000 ml H2O, pH 7.4). Cells were suspended at a concentration of 0.5 - 1 x 10 6 cells /ml. The AT2R-Cer and AT1R-Cer fluorescence were recorded in a fluorescence spectrometer (Perkin Elmer LS50B: Perkin Elmer, Waltham, MA, USA) at an excitation wavelength of 420 nm and emission between 440-600 nm. Peak fluorescence intensity at 495 nm was determined in the absence and presence of *TOMM6* co-expression or TOMM6 stabilizer/inducer/activator.

### Example 2: Identification of a plant extract, which inhibits insoluble HBsAg aggregation in yeast

Screening approaches for AD-related protein aggregation processes often apply *in vitro* or cellular systems, which mimic pathological Abeta aggregation, e.g. by spontaneous aggregation of high concentrations of synthetic Abeta peptide(s) or over-expression of Abeta variants in cells (Shariatizi et al., Int. J. Biol. Macromol. 80, 95-106 (2015); Lee et al., Protein Sci. 18, 277-286 (2009)). But specific Abeta-targeting inhibitors were barely successful so far. Because protein aggregation follows overlapping rules and shares common mechanisms between different aggregation-prone proteins (Diaz-Villanueva et al., Int. J. Mol. Sci. 16, 17193-17230 (2015)), a non-Abeta-based protein aggregation assay system was established. The first system used HBsAg (hepatitis B virus major surface antigen), a protein, which forms high-molecular weight protein aggregates when over-expressed and assembled in yeast (Tleugabulova et al., J. Chromatogr. B. Biomed. Sci. Appl. 746, 153-166 (1999)). In this system, different plant extracts were tested, which were applied as media supplements during HBsAg expression in yeast. Among different plant extracts, the extract P1 from *Galium aparine* and P2 from the related *Galium verum,* inhibited HBsAg aggregation and promoted the appearance of monomeric HBsAg, which was demonstrated by immunoblot detection of HBsAg (Figure 1).

### Example 3: Plant extracts from Galium aparine and Galium verum and two active ingredients thereof prevent AT2R aggregation in HEK cells

In the second non-Abeta-based protein aggregation assay, different plant extracts were tested in a mammalian cell system, which analyzed the protein aggregation of the angiotensin II AT2 receptor (AT2R, AGTR2) protein in human embryonic kidney (HEK) cells. The system has physiological relevance because this cellular system is capable to reproduce AT2R protein aggregation, which also occurs in brains of AD patients and AD animal models (AbdAlla et al., J. Biol. Chem. 284, 6554-6565 (2009); AbdAlla et al., J. Biol. Chem. 284, 6566-6574 (2009)). The AT2 receptor in human embryonic kidney (HEK) cells forms stable dimers/oligomers under pro-oxidant conditions, which are resistant to urea-containing reducing SDS-PAGE (Figure 2A, and AbdAlla et al., J. Biol. Chem. 284, 6566-6574 (2009)). Pro-oxidant conditions were induced in cell culture by the co-expression of NADPH oxidase-3, NOX3 (AbdAlla et al., J. Biol. Chem. 284, 6566-6574 (2009). The different plant extracts were added to the culture medium for 72 h, and the AT2 receptor was detected in immunoblot. The experiment revealed that the plant extract P1 from *Galium aparine* had the strongest effect in reducing the formation of stable AT2 receptor dimers in human cells (Figure 2A). As a control, AT2R dimerization/aggregation was prevented with two different P1 extract preparations from *Galium aparine,* and with an extract P2 from the related *Galium verum* (Figure 2B).

Subsequently, the active ingredient of the plant extracts P1 and P2 was investigated. *Galium aparine* and *Galium verum* of the *Rubiaceae* family are characterized specifically by the content of glycosides of colored anthraquinone derivatives, which upon hydrolysis generate alizarin and purpurin-3-carboxylic acid (=pseudopurpurin), and other related hydroxyanthraquinone compounds (Hill and Richter, J. Chem. Soc. 1714-1719 (1936); Hill and Richter, 1936, Proceedings of the Royal Society B, Biological Sciences, 547-560 (1936)). The content of anthraquinone dyes distinguishes the *Galium genus* from all other plant extracts, which were tested and which did not inhibit toxic protein aggregation in yeast and human cells. Therefore, alizarin (Compound-1) and pseudopurpurin (Compound-2) were tested in the cellular AT2R aggregation assay. Cultivation of HEK cells in the presence of Compound-1 (alizarin) and Compound-2 (pseudopurpurin) at a concentration of 10 microM for 72h inhibited the aggregation of the AT2 receptor in human embryonic kidney cells (Figure 2B). Taken together, extracts P1 and P2 from *Galium* species, and compound-1 and compound-2 prevented protein aggregation of HBsAg and AT2R in yeast and human cells.

### Example 4: The P1 extract from Galium aparine and alizarin (Compound-1) retard the accumulation of SDS-insoluble Abeta peptides in the hippocampus of Tg2576 AD mice

The effect of the P1 extract from *Galium aparine* and Compound-1 (alizarin) was analysed *in vivo,* on insoluble hippocampal Abeta peptide accumulation and hippocampal contents of SDS-insoluble Abeta was determined in the Tg2576 AD mouse model (Hsiao et al., Science 274, 99-103 (1996)). Animals were treated for 6 months (age 12 months to 18 months) with extract P1 from *Galium aparine,* and Compound-1 (alizarin, 10 mg/kg/d), given in drinking water. The hippocampus was isolated and hippocampal contents of SDS-insoluble Abetal-40 and Abeta1-42 peptides were quantified (Figure 3A,B). Extract P1 and compound-1 significantly retarded the accumulation of SDS-insoluble Abetal-40 and Abeta1-42 in the hippocampus of Tg2576 mice (Figure 3A,B). These findings showed that anthraquinones such as Compound-1 (alizarin) as a prototypic and representative anthraquinone of plant extract P1 from *Galium aparine* and the plant extract P1 itself inhibited toxic Abeta aggregation *in vivo,* in a genetic model of familial Alzheimer's disease, FAD.

### Example 5: The P1 extract from Galium aparine and alizarin (Compound-1) retard PHF tau hyperphosphorylation and neuronal loss in Tg2576 AD mice subjected to the CUMS (chronic unpredictable mild stress) protocol

The sole reduction of aggregated Abeta is not sufficient to reduce symptoms of AD in patients. Hyperphosphorylated PHF tau is a major factor, which promotes neurodegeneration in concert with aggregated Abeta peptides (Calderon-Garciduenas and Duyckaerts, Handb. Clin. Neurol. 145, 325-337 (2017)). To enhance PHF tau hyperphosphorylation and the process of neurodegeneration in the Tg2576 AD model, Tg2576 AD mice were subjected to the chronic unpredictable mild stress (CUMS) protocol (AbdAlla et al., J. Biol. Chem. 284, 6554-6565 (2009); Briones et al., Br. J. Pharmacol. 165, 897-907 (2012)). Hyperphosphorylated PHF-Tau was detected in the hippocampus of 15 months-old Tg-2576 mice after 3 months of CUMS (Figure 3C). Treatment with P1 extract from *Galium aparine* and alizarin (Compound-1) during the CUMS protocol led to a significantly decreased hippocampal content of hyperphosphorylated PHF-Tau in 15 months-old Tg2576 mice as determined by direct binding assay with [¹²⁵I]-labeled AT8 antibody, which is specific for the PHF (paired helical filament) form of hyperphosphorylated Tau (Figure 3C).

In addition, the overt neuronal loss in the hippocampus of Tg2576 mice subjected to CUMS was also prevented by treatment with P1 extract from *Galium aparine* and alizarin (Compound-1) as determined by binding assay with [¹²⁵I]-labeled anti-NeuN antibody (Figure 3D). Thus, the anthraquinone-containing P1 extract from *Galium aparine* and a prototypical and representative anthraquinone thereof (Compound-1; alizarin) are neuroprotective and prevent neuronal loss in the Tg-2576 genetic model of familial AD, which adopts features of sporadic AD by the CUMS protocol.

### Example 6: Commpound-1 (alizarin) and P3 extract from the alizarin-containing Rubia cordifolia retard Abeta plaque accumulation in Tg2576 mice

Immunohistological analysis with Abeta-specific antibody BAM-10 confirmed that treatment with alizarin (Compound-1) significantly inhibited the accumulation of Abeta plaques in brains of aged 18-month-old Tg2576 mice compared to untreated 18-month-old Tg2576 AD controls (Figure 4A,B).

In addition to the *genus Galium* from the rubiaceae family, the *Rubia genus* from the same family is also characterized by a high content of anthraquinones, notably alizarin and pseudopurpurin (Hill and Richter, 1936, Proceedings of the Royal Society B, Biological Sciences, 547-560 (1936)). Therefore, the treatment effect of extract P3 from *Rubia cordifolia* L was investigated. Treatment of Tg2576 AD mice for 6 months with extract P3 from *Rubia cordifolia* L. led to a highly decreased Abeta plaque load in hippocampal and brain cortical areas of Tg2576 AD mice (Figure 4A,B). The treatment effect of extract P3 was comparable to the treatment effect of alizarin (Figure 4A,B). Thus, alizarin and the alizarin-containing *Rubia cordifolia* L. extract inhibit the formation of senile Abeta plaques in the genetic Tg2576 AD model, which recapitulates the familial form of AD, FAD by neuron-specific expression of human APP695 with the double mutation K670N/M671L, which was isolated from a Swedish family with FAD (Hsiao et al., Science 274, 99-103 (1996)).

### Example 7: Pseudopurpurin (Compound-2) and extract P3 retard Abeta peptide accumulation in Tg2576 mice, and PHF tau hyperphosphorylation and neuronal loss in Tg2576 AD mice subjected to CUMS

The treatment effects of the P3 extract from *Rubia cordifolia* were compared with Compound-2 (pseudopurpurin) in the Tg2576 AD model because pseudopurpurin is another characteristic anthraquinone for the *Galium* and *Rubia* genera (Hill and Richter, 1936, Proceedings of the Royal Society B, Biological Sciences, 547-560 (1936)). Quantitative assessment of hippocampal contents of SDS-insoluble Abetal-40 and Abeta1-42 peptides demonstrated that 6 months of treatment with extract P3 and compound-2 both inhibit the accumulation of SDS-insoluble Abetal-40 and Abeta1-42 in the hippocampus of 18-month-old Tg2576 mice (Figure 5A,B).

In addition, the P3 extract and Compound-2 inhibited hippocampal PHF-Tau hyperphosphorylation in 15 months-old Tg-2576 mice induced by 3 months of CUMS (Figure 5C). In addition, P3 extract from *Rubia cordifolia* L. and Compound-2 prevented the CUMS-induced hippocampal neuronal loss (Figure 5D). Taken together, extract P1 from *Galium aparine,* extract P3 from *Rubia cordifolia,* and two major anthraquinones from the *Galium* and *Rubia* genera, i.e. alizarin (Compound-1) and pseudopurpurin (Compound-2) are neuroprotective and prevent neuronal loss in a genetic model of familial AD with stress-induced symptoms of sporadic AD.

### Example 8: The P3 extract from Rubia cordifolia and Compound-1 retard Tau hyperphosphorylation in the Tg-TauP301L model of tauopathy

The anthraquinone-containing P1 and P3 extracts and two major anthraquinones, Compound-1 and Compound-2, decreased the hippocampal PHF-Tau content of Tg-2576 AD mice. It was investigated whether the P3 extract and Compound-1 also inhibited PHF-tau phosphorylation in a genetic model of tauopathy, the Tg-Tau-P301L mouse model with neuron-specific expression of the most common FTDP-17 (frontotemporal dementia and parkinsonism linked to chromosome 17) mutation (Lewis et al., Nature Genetics 25, 402-405 (2000)). Aged 12-month-old Tg-Tau-P301L mice without treatment showed substantial PHF-tau hyperphosphorylation in axons of the hippocampal CA3 area (Figure 6A). Treatment with P3 extract and Compound-1 for six months led to a significantly decreased hippocampal content of PHF-tau (Figure 6A,B) in 12-month-old Tg-Tau-P301L mice. These findings showed that the P3 extract and its prototypical anthraquinone retard PHF-tau accumulation in different tauopathy models, i.e. Tg-2576 mice subjected to CUMS, and Tg-Tau-P301L mice.

### Example 9: Identification of Tomm6 as an interacting/stabilizing protein of Compound-1, Compound-2, and extract P1 and P3 from Galium aparine and Rubia cordifolia

The major target of the prototypical anthraquinone, Compound-1 (alizarin) was searched for. To this end, a solubilisate of hippocampal proteins from aged Tg2576 mice supplemented with 10 microM Compound-1 was prepared, and the mixture was separated by urea-containing SDS-PAGE under reducing conditions. The Compound-1-interacting proteins were identified by co-migration, cut from the gel, and subjected to protein identification. Nano-LC-ESI-MS/MS analysis identified Tomm6 (Mitochondrial import receptor subunit TOMM6 homolog) as a major Compound-1-interacting protein (Figure 7A).

TOMM6 (Tom6) is an essential component of the TOM machinery, i.e. the multisubunit translocases in the outer mitochondrial membrane, which are required for the import of nucleus-encoded precursor proteins. It was investigated whether treatment with Compound-1, Compound-2, extract P1 and P3 from *Galium aparine* and *Rubia cordifolia* mediated protein stabilization of Tomm6 in the hippocampus of Tg2576 mice. Aged Tg2576 mice were treated for 6 months with Compound-1, Compound-2, P1 and P3 extract. Hippocampal protein contents of Tomm6 were determined in immunoblot. Immunoblot detection showed that the hippocampal protein content of Tomm6 in Tg2576 mice was substantially increased by treatment with Compound-1 (alizarin), Compound-2 (pseudopurpurin), P1 extract from *Galium aparine,* and P3 extract from *Rubia cordifolia* (Figure 7B,C). Thus, two prototypical anthraquinones from the *Galium* and *Rubia* genera, alizarin and pseudopurpurin, and extracts from two different anthraquinone-containing genera, *Galium* and *Rubia,* mediated stabilization/induction of the Tomm6 protein *in vivo,* in the hippocampus of aged Tg2576 AD mice.

### Example 10: TOMM6 enhances AT2R protein folding in human cells

It was found that Compound-1, Compound-2, extract P1 and extract P3 from the *Galium* and *Rubia* genera of the *rubiaceae* family interacted with and stabilized TOMM6. Next, it was analyzed whether stabilization/induction of TOMM6 was sufficient to exert cell/neuro-protection and prevented protein aggregation/misfolding of the AT2R (AGTR2) in HEK cells. Misfolded and aggregated AT2R promotes neurodegeneration whereas the intact AT2R could exert neuroprotection in AD models and patients (AbdAlla et al., J. Biol. Chem. 284, 6554-6565 (2009); AbdAlla et al., J. Biol. Chem. 284, 6566-6574 (2009)).

To analyze the effect of an increased cellular TOMM6 protein, the cDNA of *TOMM6* was inserted into the EcoRI and Xhol sites of the expression plasmid pcDNA3.1, which enables transient and stable expression of a cDNA under control of the ubiquitous CMV immediate-early promoter/enhancer in cells and in vivo (Figure 8A,B). The AT2R-Cerulean (AT2R-Cer) was generated by fusion of the AT2 receptor at the carboxyl-terminus with Cerulean similarly as described for the AT1R-Cer construct (Quitterer et al., Biochem. Biophys. Res. Commun. 409, 544-549 (2011)). The Cerulean variant of the ECFP was used because Cerulean is 2.5-fold brighter than the cognate ECFP (Rizzo et al., Nat. Biotechnol. 22, 445-449 (2004)).

Upon expression in HEK cells, the total amount of cell membrane-localized AT2R-Cer was quantified in a fluorescence spectrophotometer. The co-expression of TOMM6 led to a signifycantly increased AT2R-Cer-specific fluorescence intensity, which is indicative of improved AT2R-Cer protein folding (Figure 9A).

In contrast to AT2R-Cer, the related AT1R-Cer (AGTR1-Cerulean) was not increased by TOMM6 but AT1R-Cer was significantly decreased by TOMM6 (Figure 9B). This finding is significant because the AT1R (AGTR1) protein is enhanced by reactive oxygen species, ROS (Banday et al., Hypertension 57, 452-459 (2011)). Moreover, an increased expression/activation of AT1R contributes to neurodegeneration and increases Alzheimer pathology *in vivo* (AbdAlla et al., Int. J. Mol. Sci. 14, 16917-16942 (2013); AbdAlla et al., Biomed. Res. Int. 2015:917156 (2015)). Taken together, these cellular data are compatible with the notion that an increased TOMM6 level reduces the cellular ROS content by improving the function of mitochondria, which is the major inducer of cellular ROS. This process of "mitochondrial healing" could also be exerted by the stabilization of TOMM6 with the prototypical anthraquinones Compound-1 (alizarin), Compound-2 (pseudopurpurin), and the anthraquhinone-containing plant extracts P1 and P3 from *Galium aparine* and *Rubia cordifolia.* As a consequence of "mitochondrial healing", redox-sensitive folding of AGTR2 is improved whereas folding of AGTR1 is inhibited.

### Example 11: Generation of Tg-TOMM6 mice

The above data showed that increased protein levels of TOMM6 promoted the folding of the AT2R protein in human cells, which is misfolded and aggregated in brains of AD patients with sporadic AD (AbdAlla et al., J. Biol. Chem. 284, 6554-6565 (2009)). In addition, TOMM6 reduced the protein level of neurotoxic AT1R. To further validate the neuroprotective function of TOMM6 *in vivo,* transgenic mice with expression of *TOMM6* under control of the neuron-specific hamster prion protein (HaPrP) promoter were generated. To generate Tg-TOMM6 mice, the cDNA of TOMM6 was inserted into the *SalI* site of the CosSHa.Tet vector (Figure 10A), which directs neuron-specific expression under control of the Syrian hamster prion protein (HaPrP) promoter (Hsiao et al., Science 274, 99-103 (1996); Scott et al., Protein Sci. 1, 986-997 (1992); InPro Biotechnology Inc. San Francisco, CA, USA). Plasmid sequences were removed by *NotI* digestion. The purified linear DNA (2ng/microL) was injected into fertilized oocytes dissected out from super-ovulated FVB or B6 (C57BL/6J) mice. For DNA injection, oocytes were kept in M2 medium. After DNA injection, injected embryos were transferred into M16 medium and incubated overnight in M16 medium in the presence of 10 % CO₂ at 37°C in a humidified incubator. Thereafter, double-cell stage embryos were selected and transferred into the oviduct of pseudo-pregnant CD-1 foster mice. Pseudopregnancy was induced by overnight breeding with an infertile (vasectomized) male and detected by the presence of a vaginal plug. After birth, genomic DNA from F0 mice was isolated from ear punch biopsies taken at 3-4 weeks of age. Mice with stable genomic integration of the *TOMM6* transgene were identified by genotyping PCR and used for further breeding.

To generate double-transgenic Tg-2576-TOMM6 mice, homozygous Tg-TOMM6 mice were crossed with heterozygous Tg2576 AD mice and double-transgenic Tg2576-TOMM6 mice were identified by genotyping PCR (sequences of primers used for genotyping PCR are derived from TOMM6-flanking CosSHa.Tet vector sequences and from the TOMM6 cDNA sequence) (Figure 10B).

### Example 12: Transgenic TOMM6 expression retards major neuropathological features in the hippocampus of Tg2576 AD mice

The goal was to investigate whether an increased TOMM6 level was sufficient to retard neurodegeneration in the Tg-2576 AD model. Aged 18 months-old double-transgenic Tg2576-TOMM6 mice had an increased hippocampal protein level of TOMM6 as determined in immunoblot compared to single-transgenic Tg2576 AD mice (Figure 11A).

Double-transgenic Tg-2576-TOMM6 mice were used and it was analyzed whether an increased hippocampal TOMM6 protein level affected the neuropathological features in double-transgenic Tg2576-TOMM6 mice compared to single-transgenic Tg2576 controls. Hippocampal contents SDS-insoluble Abetal-40 and Abeta 1-42 were significantly decreased in double-transgenic Tg2576-TOMM6 mice compared to single-transgenic Tg2576 controls (Figure 11B,C). Thus, an increased hippocampal TOMM6 protein level is sufficient to retard the development of a major neuropathological feature of AD, i.e. the accumulation of SDS-insoluble Abeta peptides in the hippocampus of Tg2576-TOMM6 mice.

As detailed before, the Tg2586 AD model is largely devoid of major symptoms of clinical AD, i.e. neuronal loss and gross memory impairment (AbdAlla et al., J. Biol. Chem. 284, 6566-6574 (2009); Ashe and Zahs, Neuron 66, 631-645 (2010)). To enhance the process of neurodegeneration in Tg2576 AD mice, we subjected Tg2576 mice to chronic mild environmental stress (AbdAlla et al., J. Biol. Chem. 284, 6554-6565, (2009); Briones et al., Br. J. Pharmacol. 165, 897-907 (2012)), which is known to aggravate symptoms of dementia and neurodegeneration in patients (Peavy et al., Biol. Psychiatry 62, 472-478 (2007); Wilson et al., Neuroepidemiology 27, 143-163 (2006)). Quantitative analysis detected hyperphosphorylated PHF-Tau in the hippocampus of 15 months-old Tg-2576 mice after 3 months of CUMS (Figure 10D). The presence of increased neuronal TOMM6 in double-transgenic Tg2576-TOMM6 mice led to a significantly decreased hippocampal content of hyperphosphorylated PHF-Tau in 15 months-old Tg2576-TOMM6 mice as determined by direct binding assay with [¹²⁵I]-labeled AT8 antibody, which is specific for PHF (paired helical filament) form of hyperphosphorylated Tau (Figure 11D).

Concomitantly, hippocampal neuronal loss was also prevented by transgenic TOMM6 expression, as determined by direct binding assay with [¹²⁵I]-labeled anti-NeuN antibody (Figure 10E). These findings show that an increased hippocampal protein level of TOMM6 is sufficient to promote neuroprotection against major neuropathological features in the hippocampus of Tg2576 AD mice, i.e. accumulation of insoluble Abeta peptides, formation of hyperphosphorylated PHF-Tau and overt neuronal loss.

### Example 13: Identification of TOMM6-interacting (inducing) compounds without antioxidant activity (Figure 12 and Figure 13)

TOMM6-inducing compounds were searched for and a screening system was established in HEK cells. HEK cells were cultured in the absence and presence of a test compound at a final concentration of 20 microM. Cells were harvested, proteins were extracted and TOMM6 protein levels were determined in immunoblot with TOMM6-specific antibodies. This assay identified compound 3 - 10 as TOMM6-interacting compounds and inducers of TOMM6 (Figure 12 and Figure 13). The following TOMM6-interacting (inducing) compounds were identified: Compound-3: N-[(3-chlorophenyl)methyl]-2-(3-piperidyl)oxazole-4-carboxamide; Compound-4: N-[(3,4-dichlorophenyl)methyl]-2-(3-piperidyl)oxazole-4-carboxamide; Compound-5: 2-[(1S)-1-amino-2-methyl-butyl]-N-[(3,4-dichlorophenyl)methyl]oxazole-4-carboxamide; Compound-6: 2-[(1S)-1-amino-2-methyl-propyl]-N-[(3,4-dichlorophenyl)methyl]oxazole-4-carboxamide; Compound-7: 6-(aminomethyl)-4-(4-chlorophenyl)-2-methyl-pyridine-3-carboxamide; Compound-8: 4-(2,4-dichlorophenyl)-6-[(1,3-dioxoisoindolin-2-yl)methyl]-2-methyl-pyridine-3-carboxamide; Compound-9: 4-(4-chlorophenyl)-6-[(1,3-dioxoisoindolin-2-yl)methyl]-2-methyl-pyridine-3-carboxamide; and Compound-10: 4-(4-chlorophenyl)-6-(hydroxymethyl)-2-methyl-pyridine-3-carboxamide).

### Example 14: TOMM6-interacting (inducing) compound-4 and compound-7 inhibit symptoms of AD and neurodegeneration in AD model mice

The TOMM6-interacting (inducing) compound-4 and compound-7 were selected for in vivo testing. Treatment with compound-4 and compound-7 led to a strong increase in hippocampal Tomm6 protein content of Tg2576 AD mice subjected to the CUMS protocol (Figure 14A). In addition, compound-4 and compound-7 inhibited hippocampal accumulation of aggregated Abetal-40 and Abeta1-42 in aged 18-month-old Tg2576 AD mice (Figure 14B). Treatment with compound-4 and compound-7 also decreased hippocampal PHF tau hyperphosphorylation (Figure 14C) and retarded overt neuronal loss in Tg2576 mice subjected to chronic mild stress with the CUMS protocol (Figure 14D). Together these experiments show that treatment with TOMM6-inducing compounds retard major symptoms of AD and neurodegeneration in vivo.

## Claims

1. A TOMM6 (Translocase of Outer Membrane 6kDa subunit homologue)-interacting compound or composition selected from the group consisting of
(i) a plant or fungal extract comprising an anthraquinone or anthraquinone derivative;
(ii) an anthraquinone or anthraquinone derivative; and
(iii) a compound selected from the group consisting of compounds of Formula (I) and (II), 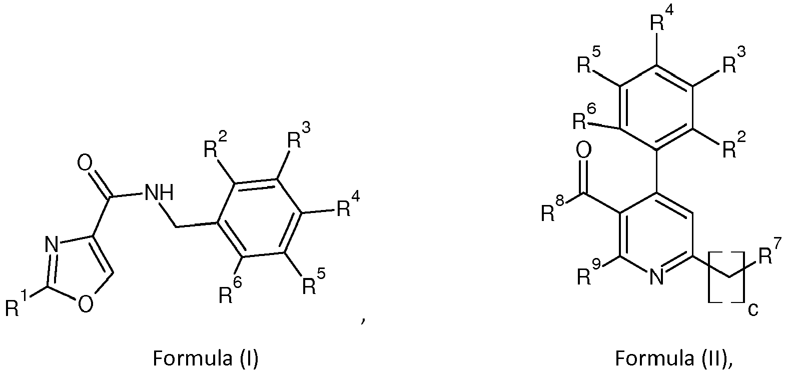 wherein
**R**¹ is selected from the group consisting of
(i) H, F, Cl, Br, or-OH;
(ii) -O(C₁₋₁₀)alkyl, preferably -OMe and -OEt, -O(C₃₋₁₀)cycloalkyl, -O(C=O)(C₁₋₁₀)alkyl, preferably -O(C=O)Me, or -O(C=O)(C₃₋₁₀)cycloalkyl;
(iii) -COOH, -COO(C₁₋₁₀)alkyl, -COO(C₃₋₆)cycloalkyl, -COONH₂, -COON((C₁₋₁₀)alkyl)₂,-COONH(C₁₋₁₀)alkyl, preferably -COOH, -COOMe or COOEt;
(iv) linear or branched, substituted or non-substituted (C₂₋₁₀)alkyl ether, (C₃₋₁₀)alkenyl ether, (C₃₋₁₀)alkynyl ether or (C₄₋₁₀)carbocyclic ether, wherein the ether is bonded to formula (I) via its carbon atom;
(v) linear or branched, substituted or non-substituted (C₁₋₁₀)alkyl, (C₂₋₁₀)alkenyl, (C₂₋₁₀)alkynyl, preferably (C₁₋₁₀)alkyl, more preferably substituted or non-substituted-CHO, butyl or pentyl;
(vi) substituted or non-substituted carbocycle selected from the group consisting of (C₃₋₁₀)carbocycle, preferably (C₃)carbocycle and (C₅₋₆)carbocycle, preferably aromatic (C₆)carbocycle, more preferably a non-substituted phenyl or a phenyl that is substituted by a substituent selected from the group consisting of Cl, F, Br, substituted or non-substituted methyl, preferably -(CF₃), ethyl, propyl and cyclopropyl;
(vii) substituted or non-substituted (C₃₋₆) heterocycle or (C₇₋₁₀)carbo- or hetero-bicycle having 1 to 3 heteroatoms each independently selected from N, O and S, preferably substituted or non-substituted heterocycle having 1 nitrogen atom, preferably substituted or non-substituted piperidine, most preferably piperidine-3-yl,
preferably R¹ is selected from the group consisting of 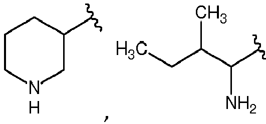 , and 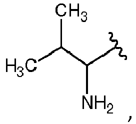 more preferably 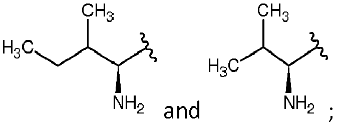
**R¹** to **R⁶** are each independently selected from the group consisting of H, Cl, Br, F , -OMe, - OEt, -O(C=O)Me, or-OH,
preferably for Formula (I), R⁵ is Cl and R² to R⁴ and R⁶ are H, more preferably R³ and R⁴ are CI and R², R⁵ and R⁶ are H,
preferably for Formula (II), R⁴ is Cl and R², R³, R⁵ and R⁶ are H, more preferably R² and R⁴ are Cl and R³, R⁵ and R⁶ are H;
**R⁷** is selected from the group consisting of
(i) NH₂, NH(C₁₋₁₀)alkyl, -OH, or -O(C₁₋₁₀)alkyl, preferably -NH₂ or -OH;
(ii) substituted or non-substituted carbocycle selected from the group consisting of (C₃₋₁₀)carbocycle, preferably (C₃)carbocycle and (C₅₋₆)carbocycle, preferably aromatic (C₆)carbocycle, more preferably a non-substituted phenyl or a phenyl that is substituted by a substituent selected from the group consisting of Cl, F, Br, substituted or non-substituted methyl, preferably -(CF₃), ethyl, propyl and cyclopropyl; and
(iii) substituted or non-substituted (C₃₋₆)heterocycle or (C₇₋₁₀)carbo- or hetero-bicycle having 1 to 3 heteroatoms each independently selected from N, O and S, preferably substituted or non-substituted heterobicycle having 1 nitrogen atom, more preferably isoindoline-1,3-dione, most preferably 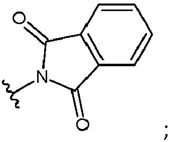
**R⁸** is selected from the group consisting of -NH₂, NH(C₁₋₁₀)alkyl, -OH, or -O(C₁₋₁₀)alkyl, preferably -NH₂;
**R⁹** is selected from the group consisting of H, Me and Et; and/or
**c** is an integer between 1 and 4
and pharmaceutically acceptable salts and solvates thereof;
for use in the treatment or prophylaxis of a nervous system disease or disorder, preferably a human nervous system disease or disorder, preferably a central nervous system (CNS) or peripheral nervous system (PNS) disease or disorder.

2. The extract for use according to claim 1, wherein the extract is prepared from a plant
(i) selected from the group of plant families consisting of *Rubiaceae, Verbenaceae, Bignoniaceae, Rhamnaceae, Polygonaceae, Leguminosae, Scropulariaceae, Fabaceae,* and *Liliaceae;* or
(ii) selected from the group of plant genera consisting of *Rubia; Galium; Rhamnus; Ventilago; Rheum; Rumex; Polygonum; Cassia; Senna; Andira,* and *Aloe.*

3. The extract for use according to claim 1 or 2, wherein the extract is prepared from
(i) a plant selected from the group consisting of *Galium abaujense* Borbás; *Galium abruptorum* Pomel; *Galium absurdum* Krendl; *Galium achurense* Grossh.; *Galium acrophyum* Hochst. ex Chiov.; *Galium acuminatum* Ball; *Galium acutum* Edgew.; *Galium adhaerens* Boiss. & Balansa; *Galium advenum* Krendl; *Galium aegeum* (Stoj. & Kitam.) Ancev; *Galium aetnicum* Biv.; *Galium afropusillum* Ehrend.; *Galium agrophilum* Krendl; *Galium aladaghense* Parolly; *Galium* ×*albertii* Rouy (hybrid from *Galium boreale* × *Galium verum; Galium albescens* Hook. f.; *Galium album* Mill.; *Galium album* Mill. subsp. *album; Galium album* subsp. *amani* Ehrend. & Schönb.-Tem.; *Galium album* subsp. *prusense* (K.Koch) Ehrend. & Krendl; *Galium album* subsp. *pycnotrichum* (Haw. ex Schult. & Schult. f.) Krendl.; *Galium album* subsp. *suberectum* (Klokov) Michalk.; *Galium amatymbicum* Eckl. & Zeyh.; *Galium amblyophyllum* Schrenk; *Galium amorginum* Halácsy; *Galium andrewsii* A.Gray; *Galium andringitrense* Homolle ex Puff; *Galium anfractum* Sommier & Levier; *Galium anguineum* Ehrend. & Schönb.-Tem.; *Galium angulosum* A.Gray; *Galium angustifolium* Nutt.; *Galium angustissimum* (Hausskn. ex Bornm.) Ehrend.; *Galium anisophyllon* Vill.; *Galium ankaratrense* Homolle ex Puff; *Galium antarcticum* Hook. f.; *Galium antitauricum* Ehrend.; *Galium antuneziae* Dempster; *Galium aparine* L.; *Galium aparinoides* Forssk.; *Galium aragonesii* Sennen; *Galium araucanum* Phil.; *Galium arenarium* Loisel.; *Galium arequipicum* Dempster; *Galium aretioides* Boiss.; *Galium argense* Dempster & Ehrend.; *Galium aristatum* L.; *Galium arkansanum* A.Gray; *Galium armenum* Schanzer; *Galium ascendens* Willd. ex Spreng.; *Galium aschenbornii* Schauer; *Galium asparagifolium* Boiss. & Heldr.; *Galium asperifolium* Wall.; *Galium asperuloides* Edgew.; *Galium asprellum* Michx.; *Galium atherodes* Spreng.; *Galium atlanticum* Pomel; *Galium aucheri* Boiss.; *Galium auratum* Klokov; *Galium australe* DC.; *Galium austriacum* Jacq.; *Galium avascense* Krendl; *Galium azerbayjanicum* Ehrend. & Schönb.-Tem.; *Galium azuayicum* Dempster; *Galium babadaghense* Yιld.; *Galium baeticum* (Rouy) Ehrend. & Krendl; *Galium baghlanense* Ehrend. & Schönb.-Tem.; *Galium baillonii* Brandza; *Galium baldense* Spreng.; *Galium baldensiforme* Hand.-Mazz.; *Galium balearicum* Briq.; *Galium* ×*barcinonense* Sennen (*Galium lucidum* × *Galium maritimum*); *Galium basalticum* Ehrend. & Schönb.-Tem.; *Galium baytopianum* Ehrend. & Schönb.-Tem.; *Galium beckhausianum* G.H.Loos; *Galium belizianum* Ortega Oliv., Devesa & Rodr.Riaño; *Galium bellatulum* Klokov; *Galium bermudense* L.; *Galium bifolium* S.Watson; *Galium bigeminum* Griseb.; *Galium binifolium* N.A.Wakef.; *Galium blinii* H.Lév.; *Galium boissierianum* (Steud.) Ehrend. & Krendl; *Galium bolanderi* A.Gray; *Galium boreale* L.; *Galium boreoaethiopicum* Puff; *Galium bornmuelleri* Hausskn. ex Bornm.; *Galium bourgaeanum* Coss. ex Batt.; *Galium boyacanum* Dempster; *Galium brachyphyllum* Schult.; *Galium bracteatum* Boiss.; *Galium bredasdorpense* Puff; *Galium brenanii* Ehrend. & Verdc.; *Galium brevifolium* Sm.; *Galium breviramosum* Krendl; *Galium brockmannii* Briq.; *Galium broterianum* Boiss. & Reut.; *Galium brunneum* Munby; *Galium bryoides* Merr. & L.M.Perry; *Galium buchtienii* Dempster; *Galium* ×*buekkense* Hulják (*Galium abaujense* × *Galium vernum*); *Galium bullatum* Lipsky; *Galium bulliforme* I.Thomps.; *Galium bungei Steud.; Galium bungoniense* I.Thomps.; *Galium buschiorum* Mikheev; *Galium bussei* K.Schum. & K.Krause; *Galium buxifolium* Greene; *Galium cajamarcense* Dempster; *Galium californicum* Hook. & Arn.; *Galium caminianum* Schult.; *Galium campanelliferum* Ehrend. & Schönb.-Tem.; *Galium campylotrichum* Nazim. & Ehrend.; *Galium conescens* Kunth; *Galium cankiriense* Yild.; *Galium canum* Req. ex DC.; *Galium canum* subsp. *antalyense* Ehrend.; *Galium canum* Req. ex DC. subsp. *canum; Galium canum* subsp. *ovatum* Ehrend.; *Galium canum* subsp. *ulukislaense* Yild.; *Galium capense* Thunb.; *Galium capitatum* Bory & Chaub.; *Galium cappadocicum* Boiss.; *Galium caprarium* Natali; *Galium capreum* Krendl; *Galium carmenicola* Dempster; *Galium* ×*carmineum* Beauverd (*Galium anisophyllon* × *Galium pumilum* × *Galium rubrum*).; *Galium carterae* Dempster; *Galium caspicum* Steven; *Galium cassium* Boiss.; *Galium catalinense* A.Gray; *Galium* ×*centroniae* Cariot (*Galium pumilum* × *Galium rubrum*); *Galium ceratoamanianum* Ehrend.; *Galium ceratocarpon* Boiss.; *Galium ceratophylloides* Hook. f.; *Galium ceratopodum* Boiss.; *Galium cespitosum* Lam.; *Galium chaetopodum* Rech. f.; *Galium chekiangense* Ehrend.; *Galium chloroionanthum* K.Schum.; *Galium chloroleucum* Fisch. & C.A.Mey.; *Galium ciliare* Hook. f.; *Galium cilicicum* Boiss.; *Galium cinereum* All.; *Galium circae* Krendl; *Galium circaezans* Michx.; *Galium clausonis* Pomel; *Galium clementis* Eastw.; *Galium cliftonsmithii* (Dempster) Dempster & Stebbins; *Galium collomiae* J.T.Howell; *Galium coloradoense* W.Wight; *Galium comberi* Dempster; *Galium cometerhizon* Lapeyr.; *Galium compactum* Ehrend. & McGill.; *Galium concatenatum* Coss.; *Galium concinnum* Torr. & A.Gray; *Galium confertum* Royle ex Hook. f.; *Galium conforme* Krendl; *Galium consanguineum* Boiss.; *Galium coriaceum* Bunge; *Galium cornigerum* Boiss. & Hausskn.; *Galium coronadoense* Dempster; *Galium correllii* Dempster; *Galium corsicum* Spreng.; *Galium corymbosum* Ruiz & Pav.; *Galium cossonianum* Jafri; *Galium cotinoides* Cham. & Schltdl.; *Galium cracoviense* Ehrend.; *Galium crassifolium* W.C.Chen; *Galium craticulatum* R.R.Mill; *Galium crespianum* Rodr.; *Galium cryptanthum* Hemsl.; *Galium curvihirtum* Ehrend. & McGill.; *Galium cuspidulatum* Miq.; *Galium cyllenium* Boiss. & Heldr.; *Galium czerepanovii* Pobed.; *Galium dahuricum* Turcz. ex Ledeb.; *Galium davisii* Ehrend.; *Galium debile* Desv.; *Galium decorum* Krendl; *Galium decumbens* (Ehrend.) Ehrend. & Schonb.-Tem.; *Galium degenii* Bald. ex Degen; *Galium deistelii* K.Krause; *Galium delicatulum* Boiss. & Hohen.; *Galium demissum* Boiss.; *Galium dempsterae* B.L.Turner; *Galium densum* Hook. f.; *Galium denticulatum* Bartl. ex DC.; *Galium desereticum* Dempster & Ehrend.; *Galium diabolense* Dempster; *Galium dieckii* Bornm.; *Galium diffusoramosum* Dempster & Ehrend.; *Galium xdigeneum* A.Kern. (*Galium sylvaticum* × *Galium verum); Galium diphyllum* (K.Schum.) Dempster; *Galium diploprion* Boiss. & Hohen.; *Galium divaricatum* Pourr. ex Lam.; *Galium domingense* litis; *Galium dumosum* Boiss.; *Galium duthiei* R.Bhattacharjee; *Galium echinocarpum* Hayata; *Galium ecuadoricum* Dempster; *Galium* ×*effulgens* Beck (*Galium lucidum* × *Galium verum*); *Galium ehrenbergii* Boiss.; *Galium elbursense* Bornm. & Gauba; *Galium elegans* Wall. ex Roxb.; *Galium elongatum* C.Presl; *Galium emeryense* Dempster & Ehrend.; *Galium ephedroides* Willk.; *Galium equisetoides* (Cham. & Schltdl.) Standl.; *Galium ericoides* Lam.; *Galium eriocarpum* Bartl. ex DC.; *Galium eruptivum* Krendl; *Galium erythrorrhizon* Boiss. & Reut.; *Galium espiniacicum* Dempster; *Galium estebanii* Sennen; *Galium exaltatum* Krendl; *Galium exile* Hook. f.; *Galium exstipulatum* P.H.Davis; *Galium exsurgens* Ehrend. & Schonb.-Tem.; *Galium extensum* Krendl; *Galium falconeri* R.Bhattacharjee; *Galium fendleri* A.Gray; *Galium ferrugineum* K.Krause; *Galium festivum* Krendl; *Galium* ×*fictum* E.G.Camus (= *Galium glaucum* × *Galium mollugo*); *Galium filipes* Rydb.; *Galium firmum* Tausch; *Galium fissurense* Ehrend. & Schonb.-Tem.; *Galium fistulosum* Sommier & Levier; *Galium flavescens* Borbás ex Simonk.; *Galium flaviflorum* (Trautv.) Mikheev; *Galium floribundum* Sm.; *Galium foliosum* Munby ex Burnat & Barbey; *Galium fontanesianum* Pomel; *Galium formosense* Ohwi; *Galium forrestii* Diels; *Galium fosbergii* Dempster; *Galium friedrichii* N.Torres & al.; *Galium fruticosum* Willd.; *Galium fuegianum* Hook. f.; *Galium fuscum* M.Martens & Galeotti; *Galium galapagoense* Wiggins; *Galium galiopsis* (Hand.-Mazz.) Ehrend.; *Galium gaudichaudii* DC.; *Galium geminiflorum* Lowe; *Galium ghilanicum* Stapf; *Galium gilliesii* Hook. & Arn.; *Galium glaberrimum* Hemsl.; *Galium glabrescens* (Ehrend.) Dempster & Ehrend.; *Galium glabriusculum* Ehrend.; *Galium glaciale* K.Krause; *Galium glandulosum* Hand.-Mazz.; *Galium glaucophyllum* Em.Schmid; *Galium glaucum* L.; *Galium globuliferum* Hub.-Mor. & Reese; *Galium gracilicaule* Bacigalupo & Ehrend.; *Galium graecum* L.; *Galium grande* McClatchie; *Galium grayanum* Ehrend.; *Galium gymnopetalum* Ehrend. & Schonb.-Tem.; *Galium hainesii* Schonb.-Tem.; *Galium hallii* Munz & I.M.Johnst.; *Galium hardhamae* Dempster; *Galium hatschbachii* Dempster; *Galium haussknechtii* Ehrend.; *Galium heldreichii* Halácsy; *Galium hellenicum* Krendl; *Galium hexanarium* Knjaz.; *Galium hierochuntinum* Bornm.; *Galium hierosolymitanum* L.; *Galium hilendiae* Dempster & Ehrend.; *Galium* ×*himmelbourianum* (Ronniger) Soó (*Galium humifusum* × *Galium verum*); *Galium hintoniorum* B.L.Turner; *Galium hirtiflorum* Req. ex DC.; *Galium hirtum* Lam.; *Galium hoffmeisteri* (Klotzsch) Ehrend. & Schönb.-Tem. ex R.R.Mill; *Galium homblei* De Wild.; *Galium huancavelicum* Dempster; *Galium huber-morathii* Ehrend. & Schönb.-Tem.; *Galium humifusum* M.Bieb.; *Galium humile* Cham. & Schltdl.; *Galium* ×*hungaricum* A.Kern. (*Galium mollugo* × *Galium schultesii*); *Galium hupehense* Pamp.; *Galium* ×*huteri* A.Kern. (*Galium laevigatum* × *Galium lucidum*); *Galium hypocarpium* (L.) Endl. ex Griseb.; *Galium hypotrichium* A.Gray; *Galium hypoxylon* Ehrend. & Schonb.-Tem.; *Galium hyrcanicum* C.A.Mey; *Galium hystricocarpum* Greenm.; *Galium idubedae* (Pau & Debeaux) Pau ex Ehrend.; *Galium iltisii* Dempster; *Galium incanum* Sm.; *Galium incanum* subsp. *centrale* Ehrend.; *Galium incanum* subsp. *creticum* Ehrend.; *Galium incanum* subsp. *elatius* (Boiss.) Ehrend.; *Galium incanum* Sm. subsp. *Incanum; Galium incanum* subsp. *libanoticum* Ehrend.; *Galium incanum* subsp. *pseudocornigerum* Ehrend.; *Galium inconspicuum* Phil.; *Galium incrassatum* Halácsy; *Galium incurvum* Sibth. & Sm.; *Galium innocuum* Miq.; *Galium insulare* Krendl; *Galium intermedium* Schult., Syn.; *Galium schultesii* Vest; *Galium aristatum* subsp. *schultesii* (Vest) Nyman); *Galium intricatum* Margot & Reut.; *Galium ionicum* Krendl; *Galium iranicum* Hausskn. ex Bornm.; *Galium irinae* Pachom.; *Galium isauricum* Ehrend. & Schonb.-Tem.; *Galium* ×*jansenii* Kloos (*Galium sylvaticum* × *Galium mollugo*); *Galium japonicum* Makino; *Galium* ×*jorynae* Wol. (*Galium aristatum* × *Galium mollugo*); *Galium javalambrense* López Udias, Mateo & M.B.Crespo; *Galium javanicum* Blume; *Galium jemense* Kotschy; *Galium jepsonii* Hilend & J.T.Howell; *Galium johnstonii* Dempster & Stebbins; *Galium jolyi* Batt.; *Galium judaicum* Boiss.; *Galium jungermannioides* Boiss.; *Galium junghuhnianum* Miq.; *Galium juniperinum* Standl.; *Galium kaganense* R.Bhattacharjee; *Galium kahelianum* Deflers; *Galium kamtschaticum* Steller ex Schult.; *Galium karakulense* Pobed.; *Galium karataviense* (Pavlov) Pobed.; *Galium kasachstanicum* Pachom.; *Galium kenyanum* Verdc.; *Galium kerneri* Degen & Dörfl.; *Galium khorasanense* Griff.; *Galium kikumuyura* Ohwi; *Galium killipii* Dempster & Ehrend.; *Galium kinuta* Nakai & H.Hara.; *Galium kitaibelianum* Schult.; *Galium* ×*kondratjukii* Ostapko (*Galium cincinnatum* × *Galium tomentosum*); *Galium kuetzingii* Boiss. & Buhse; *Galium kunmingense* Ehrend.; *Galium kurdicum* Boiss. & Hohen.; *Galium labradoricum* (Wiegand) Wiegand; *Galium laconicum* Boiss. & Heldr.; *Galium lacrimiforme* Dempster; *Galium laevigatum* L.; *Galium lahulense* Ehrend. & Schonb.-Tem.; *Galium lanceolatum* (Torr. & A.Gray) Torr.; *Galium lanuginosum* Lam.; *Galium* ×*lanulosum* Ostapko (*Galium humifusum* × *Galium tomentosum*); *Galium lasiocarpum* Boiss.; *Galium latifolium* Michx.; *Galium latoramosum* Clos; *Galium leiocarpum* I.Thomps.; *Galium leptogonium* I.Thomps.; *Galium leptum* Phil.; *Galium libanoticum* Ehrend.; *Galium lilloi* Hicken; *Galium* ×*lindbergii* Giraudias; *Galium linearifolium* Turcz.; *Galium liratum* N.A.Wakef.; *Galium litorale* Guss.; *Galium lovcense* Urum.; *Galium lucidum* All.; *Galium macedonicum* Krendl; *Galium magellanicum* Hook. f.; *Galium magellense* Ten.; *Galium magnifolium* (Dempster) Dempster; *Galium mahadivense* G.Singh; *Galium malickyi* Krendl.; *Galium mandonii* Britton; *Galium maneauense* P.Royen; *Galium marchandii* Roem. & Schult.; *Galium margaceum* Ehrend. & Schonb.-Tem.; *Galium margaritaceum* A.Kern.; *Galium maritimum* L.; *Galium martirense* Dempster & Stebbins; *Galium masafueranum* Skottsb.; *Galium matthewsii* A.Gray; *Galium maximowiczii* (Kom.) Pobed.; *Galium mechudoense* Dempster; *Galium megacyttarion* R.R.Mill; *Galium megalanthum* Boiss.; Schweizer Labkraut (*Galium megalospermum* All.); *Galium megapotamicum* Spreng.; *Galium melanantherum* Boiss.; *Galium meliodorum* (Beck) Fritsch; *Galium membranaceum* Ehrend.; *Galium mexicanum* Kunth; *Galium microchiasma* Gilli; *Galium microlobum* I.Thomps.; *Galium microphyllum* A.Gray; *Galium migrans* Ehrend. & McGill.; *Galium minutissimum* T.Shimizu; *Galium minutulum* Jord.; *Galium mirum* Rech. f.; *Galium mite* Boiss. & Hohen.; *Galium moldavicum* (Dobrocz.) Franco; *Galium mollugo* L.; *Galium monachinii* Boiss. & Heldr.; *Galium monasterium* Krendl; *Galium monticolum* Sond.; *Galium montis-arerae* Merxm. & Ehrend.; *Galium moralesianum* Ortega Oliv. & Devesa; *Galium moranii* Dempster; *Galium morii* Hayata; *Galium mucroniferum* Sond.; *Galium muelleri* (K.Schum.) Dempster; *Galium multiflorum* Kellogg; *Galium munzii* Hilend & J.T.Howell; *Galium murale* (L.) All.; *Galium murbeckii* Maire; *Galium muricatum* W.Wight; *Galium* ×*mutabile* Besser (*Galium mollugo* × *Galium verum); Galium nabelekii* Ehrend. & Schonb.-Tem.; *Galium nakaii* Kudô; *Galium nankotaizanum* Ohwi; *Galium* ×*neglectum* Le Gall ex Gren. & Godr. (*Galium album* × *Galium arenarium*); *Galium nepalense* Ehrend. & Schonb.-Tem.; *Galium nevadense* Boiss. & Reut.; *Galium nigdeense* Yild.; *Galium nigricans* Boiss.; *Galium nigroramosum* (Ehrend.) Dempster; *Galium nolitangere* Ball; *Galium noricum* Ehrend.; *Galium normanii* Dahl; *Galium novoguineense* Diels; *Galium noxium* (A.St.-Hil.) Dempster; *Galium numidicum* Pomel; *Galium nupercreatum* Popov; *Galium nuttallii* A.Gray; *Galium obliquum* Vill.; *Galium obovatum* Kunth; *Galium obtusum* Bigelow; *Galium octonarium* (Klokov) Pobed.; *Galium odoratum* (L.) Scop.; *Galium oelandicum* Ehrend.; *Galium olgae* Klokov; *Galium olivetorum* Le Houér; *Galium olympicum* Boiss.; *Galium ophiolithicum* Krendl; *Galium oreganum* Britton; *Galium oreophilum* Krendl; *Galium oresbium* Greenm.; *Galium orizabense* Hemsl.; *Galium oshtenicum* Ehrend. & Schanzer ex Mikheev; *Galium ossirwaense* K.Krause; *Galium ostenianum* (Standl.) Dempster; *Galium ovalleanum* Phil.; *Galium pabulosum* Sommier & Levier; *Galium palaeoitalicum* Ehrend.; *Galium palustre* L.; *Galium pamiroalaicum* Pobed.; *Galium pamphylicum* Boiss. & Heldr.; *Galium paniculatum* (Bunge) Pobed.; *Galium papilliferum* Ehrend. & Schonb.-Tem.; *Galium papillosum* Lapeyr.; *Galium papuanum* Wernham; *Galium paradoxum* Maxim.; *Galium parishii* Hilend & J.T.Howell; *Galium parisiense* L.; *Galium parvulum* Hub.-Mor. ex Ehrend. & Schonb.-Tem.; *Galium paschale* Forssk.; *Galium pastorale* Krendl; *Galium patzkeanum* G.H.Loos; *Galium peloponnesiacum* Ehrend. & Krendl; *Galium penduliflorum* Boiss.; *Galium pendulum* Greenm.; *Galium penicillatum* Boiss.; *Galium pennellii* Dempster; *Galium peplidifolium* Boiss.; *Galium perralderi* Coss.; *Galium peruvianum* Dempster & Ehrend.; *Galium pestalozzae* Boiss.; *Galium petrae* Oliv. ex Hart; *Galium philippianum* Dempster; *Galium philippinense* Merr.; *Galium philistaeum* Boiss.; *Galium pilosum* Aiton; *Galium pisiferum* Boiss.; *Galium pisoderium* Krendl; *Galium platygalium* (Maxim.) Pobed.; *Galium plumosum* Rusby; *Galium poiretianum* Ball; *Galium pojarkovae* Pobed.; *Galium polyacanthum* (Baker) Puff; *Galium polyanthum* I.Thomps.; *Galium xpomeranicum* Retz. (*Galium album* × *Galium verum); Galium porrigens* Dempster; *Galium praemontanum* Mardal.; *Galium praetermissum* Greenm.; *Galium* × *pralognense* Beauverd (*Galium pumilum* × *Galium verum*); *Galium prattii* Cufod.; *Galium pringlei* Greenm.; *Galium problematicum* (Ehrend.) Ehrend. & Schonb.-Tem.; *Galium procurrens* Ehrend.; *Galium productum* Lowe; *Galium* ×*prolazense* Nyár. (*Galium album* × *Galium flavescens*); *Galium proliferum* A.Gray; *Galium propinquum* A.Cunn.; *Galium pruinosum* Boiss.; *Galium pseudoaristatum* Schur; *Galium* ×*pseudoboreale* Klokov (*Galium boreale* × *Galium rubioides*); *Galium pseudocapitatum* Hub.-Mor. ex Ehrend. & Schonb.-Tem.; *Galium pseudohelveticum* Ehrend.; *Galium pseudokurdicum* (Ehrend.) Schonb.-Tem.; *Galium pseudorivale* Tzvelev; *Galium pseudotriflorum* Dempster & Ehrend.; *Galium psilocladum* Ehrend.; *Galium pterocarpum* Ehrend.; *Galium pulvinatum* Boiss.; *Galium pumilio* Standl.; *Galium pumilum* Murray; *Galium pusillosetosum* H.Hara; *Galium pusillum* L.; *Galium pyrenaicum* Gouan; *Galium qaradaghense* Schonb.-Tem.; *Galium* ×*querceticola* Wol. (*Galium abaujense* subsp. *polonicum* × *Galium intermedium*); *Galium quichense* Dempster; *Galium radulifolium* Ehrend. & Schonb.-Tem.; *Galium ramboi* Dempster; *Galium rebae* R.R.Mill; *Galium reiseri* Halácsy; *Galium* ×*retzii* Bouchard (*Galium papillosum* × *Galium verum*); *Galium rhodopeum* Velen.; *Galium richardianum* (Gillies ex Hook. & Arn.) Endl. ex Walp.; *Galium rigidifolium* Krendl; *Galium rivale* (Sibth. & Sm.) Griseb.; *Galium roddii* Ehrend. & McGill.; *Galium rosellum* (Boiss.) Boiss. & Reut.; *Galium rotundifolium* L.; *Galium rourkei* Puff; *Galium rubidiflorum* Dempster; *Galium rubioides* L.; *Galium rubrum* L.; *Galium runcinatum* Ehrend. & Schonb.-Tem.; *Galium rupifragum* Ehrend.; *Galium ruwenzoriense* (Cortesi) Ehrend.; *Galium rzedowskii* Dempster; *Galium sacrorum* Krendl; *Galium saipalense* Ehrend. & Schonb.-Tem.; *Galium salsugineum* Krylov & Serg.; *Galium salwinense* Hand.-Mazz.; *Galium samium* Krendl; *Galium samuelssonii* Ehrend.; *Galium saturejifolium* Trevir.; *Galium saurense* Litv.; *Galium saxatile* L., Syn.: *Galium harcynicum* Weigel, *Galium pawlowskii* Kucowa, *Galium pumilum* subsp. *saxatile* (L.) Dostál; *Galium saxosum* (Chaix) Breistr.; *Galium scabrelloides* Puff; *Galium scabrellum* K.Schum.; *Galium scabrifolium* (Boiss.) Hausskn.; *Galium scabrum* L.; *Galium schlumbergeri* Boiss.; *Galium* ×*schmidelyi* Chenevard & W.Wolf (*Galium mollugo* × *Galium rubrum*); *Galium schmidii* Arrigoni; *Galium* ×*schneebergense* Ronniger (*Galium anisophyllon* × *Galium meliodorum*); *Galium schoenbeck-temesyae* Ehrend.; *Galium scioanum* Chiov.; *Galium scopulorum* Schonb.-Tem.; *Galium seatonii* Greenm.; *Galium sellowianum* (Cham.) Walp.; *Galium semiamictum* Klokov; *Galium serpenticum* Dempster; *Galium serpylloides* Royle ex Hook. f.; *Galium setaceum* Lam.; *Galium setuliferum* Ehrend. & Schonb.-Tem.; *Galium shanense* R.Bhattacharjee; *Galium shepardii* Post; *Galium shepherdii* Jung-Mend.; *Galium sichuanense* Ehrend.; *Galium sidamense* Chiov. ex Chiarugi; *Galium sieheanum* Ehrend.; *Galium simense* Fresen.; *Galium similii* Pavlov; *Galium sinaicum* (Delile ex Decne.) Boiss.; *Galium smithreitzii* Dempster; *Galium sojakii* Ehrend. & Schonb.-Tem.; *Galium songaricum* Schrenk; *Galium sorgerae* Ehrend. & Schonb.-Tem.; *Galium sparsiflorum* W.Wight; *Galium spathulatum* I.Thomps.; *Galium speciosum* Krendl; *Galium sphagnophilum* (Greenm.) Dempster; *Galium spurium* L.; *Galium stellatum* Kellogg; *Galium stenophyllum* Baker; *Galium stepparum* Ehrend. & Schonb.-Tem.; *Galium sterneri* Ehrend.; *Galium subfalcatum* Nazim. & Ehrend.; *Galium subnemorale* Klokov & Zaver.; *Galium subtrifidum* Reinw. ex Blume; *Galium subtrinervium* Ehrend. & Schonb.-Tem.; *Galium subuliferum* Sommier & Levier; *Galium subvelutinum* (DC.) K.Koch; *Galium subvillosum* Sond.; *Galium sudeticum* Tausch; *Galium suecicum* (Sterner) Ehrend.; *Galium suffruticosum* Hook. & Arn. (*Rubia margaritifera* Reiche); *Galium sungpanense* Cufod.; *Galium surinamense* Dempster; *Galium sylvaticum* L.; *Galium taiwanense* Masam.; *Galium takasagomontanum* Masam.; *Galium talaveranum* Ortega Oliv. & Devesa; *Galium tanganyikense* Ehrend. & Verdc.; *Galium tarokoense* Hayata; *Galium taygeteum* Krendl; *Galium tendae* Rchb. f.; *Galium tenuissimum* M.Bieb.; *Galium terrae-reginae* Ehrend. & McGill.; *Galium tetraphyllum* Nazim. & Ehrend.; *Galium texense* A.Gray; *Galium thasium* Stoj. & Kitanov; *Galium thiebautii* Ehrend.; *Galium thracicum* Krendl; *Galium thunbergianum* Eckl. & Zeyh.; *Galium thymifolium* Boiss. & Heldr.; *Galium tianschanicum* Popov; *Galium timeroyi* Jord.; *Galium tinctorium* L.; *Galium tmoleum* Boiss.; *Galium tokyoense* Makino; *Galium tolosianum* Boiss. & Kotschy; *Galium tomentosum* Thunb.; *Galium tortumense* Ehrend. & Schonb.-Tem.; *Galium transcarpaticum* Stojko & Tasenk.; *Galium trichocarpum* DC.; *Galium tricornutum* Dandy; *Galium trifidum* L.; *Galium trifloriforme* Kom.; *Galium triflorum* Michx.; *Galium trilobum* Colenso; *Galium trinioides* Pomel; *Galium trojanum* Ehrend.; *Galium truniacum* (Ronniger); *Galium tubiflorum* Ehrend.; *Galium tuncelianum* Yild.; *Galium tunetanum* Lam.; *Galium turgaicum* Knjaz.; *Galium turkestanicum* Pobed.; *Galium tyraicum* Klokov; *Galium uliginosum* L.; *Galium uncinulatum* DC.; *Galium undulatum* Puff; *Galium uniflorum* Michx.; *Galium uruguayense* Bacigalupo; *Galium valantioides* M.Bieb.; *Galium valdepilosum* Heinr.Braun; *Galium valentinum* Lange; *Galium vartanii* Grossh.; *Galium vassilczenkoi* Pobed.; *Galium velenovskyi* Ancev; *Galium verrucosum* Huds.; *Galium verticillatum* Danthoine ex Lam.; *Galium verum* L.; *Galium verum* subsp. *asiaticum* (Nakai) T.Yamaz.; *Galium verum* subsp. *glabrescens* Ehrend.; *Galium verum* L. subsp. *verum; Galium verum* subsp. *wirtgenii* (F.W.Schultz) Oborny (Syn. *Galium wirtgenii* F.W.Schultz); *Galium* ×*viciosorum* Sennen & Pau (*Galium maritimum* × *Galium verum*); *Galium vile* (Cham. & Schltdl.) Dempster; *Galium violaceum* Krendl; *Galium virgatum* Nutt.; *Galium viridiflorum* Boiss. & Reut.; *Galium viscosum* Vahl; *Galium volcanense* Dempster; *Galium volhynicum* Pobed.; *Galium watsonii* (A.Gray) A.Heller; *Galium weberbaueri* K.Krause; *Galium wendelboi* Ehrend. & Schonb.-Tem.; *Galium werdermannii* Standl.; *Galium wigginsii* Dempster; *Galium wrightii* A.Gray; *Galium xeroticum* (Klokov) Pobed.; *Galium xylorrhizum* Boiss. & A.Huet; *Galium yunnanense* H.Hara & C.Y.Wu; *Galium zabense* Ehrend.; *Rubia agostinhoi* Dans. & P.Silva; *Rubia aitchisonii* Deb & Malick; *Rubia alaica* Pachom.; *Rubia alata* Wall.; *Rubia albicaulis* Boiss.; *Rubia angustisissima* Wall. ex G.Don; *Rubia argyi* (H.Lév. & Vaniot) Hara ex Lauener, Syn.; *Rubia akane* Nakai, *Rubia chekiangensis* Deb, *Rubia nankotaizana* (Masam); *Rubia atropurpurea* Decne.; *Rubia austrozhejiangensis* Z.P.Lei, Y.Y.Zhou & R.W.Wang; *Rubia balearica* (Willk.) Porta; *Rubia balearica* (Willk.) Porta subsp. *Balearica; Rubia balearica* subsp. *caespitosa* (Font Quer & Marcos) Rosselló, L.Sáez & Mus (*Rubia angustifolia* var. *caespitosa* Font Quer & Marcos, *Rubia angustifolia* subsp. *caespitosa* (Font Quer & Marcos) Rosselló, *Rubia caespitosa* (Font Quer & Marcos) Rosselló); *Rubia caramanica* Bornm.; *Rubia charifolia* Wall. ex G.Don.; *Rubia chinensis* Regel & Maack; *Rubia chinensis* f. *chinensis* (*Rubia chinensis* f. *mitis* (Miq.) Kitag.); *Rubia chinensis* f. *glabrescens* (Nakai) Kitag.; *Rubia chitralensis* Ehrend.; *Rubia clematidifolia* Blume ex Decne.; *Rubia cordifolia* L.; *Rubia cordifolia* subsp. *conotricha* (Gand.) Verdc.; *Rubia cordifolia* subsp. *Cordifolia; Rubia crassipes* Collett & Hemsl.; *Rubia cretacea* Pojark.; *Rubia danaensis* Danin; *Rubia davisiana* Ehrend.; *Rubia deserticola* Pojark.; *Rubia discolor* Turcz.; *Rubia dolichophylla* Schrenk; *Rubia edgeworthii* Hook. f.; *Rubia falciformis* H.S.Lo; *Rubia filiformis* F.C.How ex H.S.Lo; *Rubia florida* Boiss; *Rubia garrettii* Craib; *Rubia gedrosiaca* Bornm.; *Rubia haematantha* Airy Shaw; *Rubia hexaphylla* (Makino) Makino; *Rubia hispidicaulis* D.G.Long; *Rubia infundibularis* Hemsl. & Lace; *Rubia jesoensis* (Miq.) Miyabe & Kudo; *Rubia komarovii* Pojark; *Rubia krascheninnikovii* Pojark; *Rubia laevissima* Tsckern.; *Rubia latipetala* H.S.Lo; *Rubia laurae* (Holmboe) Airy Shaw; *Rubia laxiflora* Gontsch.; *Rubia linii* J.M.Chao; *Rubia magna* P.G.Xiao; *Rubia mandersii* Collett & Hemsl.;
*Rubia manjith* Roxb. ex Fleming; *Rubia maymanensis* Ehrend. & Schönb.-Tem; *Rubia membranacea* Diels; *Rubia oncotricha* Hand.-Mazz.; *Rubia oppositifolia* Griff.; *Rubia ovatifolia* Z.Ying Zhang ex Q.Lin; *Rubia pallida* Diels; *Rubia pauciflora* Boiss.; *Rubia pavlovii* Bajtenov & Myrz.; *Rubia peregrina* L.; *Rubia petiolaris* DC.; *Rubia philippinensis* Elmer; *Rubia pianmaensis* R.Li & H.Li; *Rubia podantha* Diels; *Rubia polyphlebia* H.S.Lo; *Rubia pseudogalium* Ehrend.; *Rubia pterygocaulis* H.S.Lo; *Rubia rechingeri* Ehrend.; *Rubia rezniczenkoana* Litv.; *Rubia rigidifolia* Pojark.; *Rubia rotundifolia* Banks & Sol.; *Rubia salicifolia* H.S.Lo; *Rubia schugnanica* B.Fedtsch. ex Pojark.; *Rubia schumanniana* E.Pritz.; *Rubia siamensis* Craib; *Rubia sikkimensis* Kurz; *Rubia sylvatica* (Maxim.) Nakai; *Rubia tatarica* (Trevir.) F.Schmidt; *Rubia tenuifolia* d'Urv.; *Rubia tenuifolia* subsp. *brachypoda* (Boiss.) Ehrend. & Schönb.-Tem.; *Rubia tenuifolia* subsp. *doniettii* (Griseb.) Ehrend. & Schönb.-Tem.; *Rubia tenuifolia* subsp. *Tenuifolia; Rubia tenuissima* ined. (Syn.: *Rubia tenuis* H.S.Lonom. illeg.); *Rubia thunbergii* DC.; *Rubia tibetica* Hook. f.; *Rubia tinctorum* L.; *Rubia transcaucasica* Grossh.; *Rubia trichocarpa* H.S.Lo; *Rubia truppeliana* Loes.; *Rubia wallichiana* Decne.; and *Rubia yunnanensis* Diels; preferably a plant selected from *Galium aparine, Galium verum* and *Rubia cardifolia L.;*
(ii) from a fungus of a fungal genus selected from the group consisting of: *Alternaria genus; Aspergillus genus,* preferably *Aspergillus chevalieri, Aspergillus glaucus* and *Aspergillus fumigatus;* Boletus genus, preferably comprising Boletol and Carminic acid; *Cortinarius genus; Curvularia genus; Eurotium genus; Fusarium genus; Haloresellinia genus; Helminthosporium genus,* preferably *H. cynodontis, H. catenarium* and *H. triticivulgaris; Microshaeropsis genus; Monodictys genus; Nigrospora genus; Paecilomyces genus; Penicillium genus,* preferably *P. cyclopium, P. citreo-roseum, P. carmine-violaceum, P. roseo-purpureum* and *P. cyclopium; Phomopsis; Preussia genus,* preferably *Pressuia multispora (Saito et Minoura) Cain; Valsaria genus,* preferably *Valsaria rubricosa,* preferably comprising Papulosin, valsarin; *Talaromyces genus,* preferably *Talaromyces avellaneus (Thom et Turreson) C.R. Benjamin;* or
(iii) from a fungus of a *Lichen* genus, preferably a *Lichen* genus selected from the group consisting of *Xanthoria genus,* preferably *Xanthoria parietina; Lecidella genus,* preferably *Lecidella asema; Lasallia genus,* preferably *Lasallia papulose var. rubiginosa; Trypethelium genus,* preferably *Trypethelium cruentum* (*Pyrenula cruenta).*

4. The anthraquinone or anthraquinone derivative for use according to claim 1, wherein the anthraquinone or anthraquinone derivative is selected from the group consisting of compounds of Formula (III), (IV) and (V) or a pharmaceutically acceptable salt or solvate thereof, 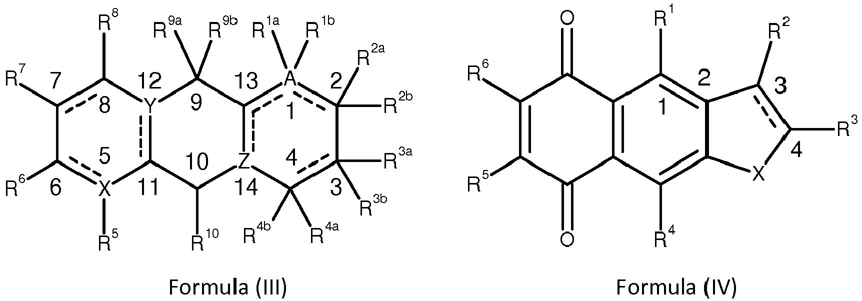 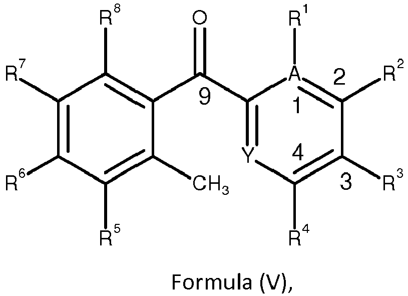 wherein
the bonds between positions 1 and 2, 1 and 13, 3 and 4, 5 and 6, 7 and 8, 11 and 12, and 13 and 14 are independently selected from single bonds or double bonds, preferably double bonds;
**A, X, Y** and **Z** are each independently selected from the group consisting of C, N and O, preferably X is C or O, and A, Y and Z are C or N, more preferably A, X, Y and Z are C, preferably for Formula (IV) **X** is O and/or the bond between position 3 and 4 is a double bond;
**R¹, R², R³, R⁴, R⁵, R⁶, R⁷, R⁸, R^{1a}** and **R^{1b}, R^{2a}** and **R^{2b}, R^{3a}** and **R^{3b},** and **R^{4a}** and **R^{4b},** are each independently selected from the group consisting of:
(i) H, F, Cl, Br, -OH, -OSO₃H and -SO₃H;
(ii) -O(C₁₋₁₀)alkyl, preferably -OMe and -OEt, -O(C₃₋₁₀)cycloalkyl, -O(C=O)(C₁₋₁₀)alkyl, preferably -O(C=O)Me, or -O(C=O)(C₃₋₁₀)cycloalkyl;
(iii) -COOH, -COO(C₁₋₁₀)alkyl, -COO(C₃₋₆)cycloalkyl, -COONH₂, -COON((C₁₋₁₀)alkyl)₂,-COONH(C₁₋₁₀)alkyl, preferably -COOH, -COOMe or COOEt;
(iv) linear or branched, substituted or non-substituted (C₂₋₁₀)alkyl ether, (C₃₋₁₀)alkenyl ether, (C₃₋₁₀)alkynyl ether or (C₄₋₁₀)carbocyclic ether, wherein the ether is bonded to formula (III), (IV) or (V) via its carbon atom;
(v) linear or branched, substituted or non-substituted (C₁₋₁₀)alkyl, (C₂₋₁₀)alkenyl, (C₂-₁₀)alkynyl, preferably (C₁₋₁₀)alkyl, more preferably substituted or non-substituted-CHO, methyl, ethyl or propyl, most preferably substituted or non-substituted methyl;
(vi) substituted or non-substituted carbocycle selected from the group consisting of (C₃-₁₀)carbocycle, preferably (C₃)carbocycle and (C₅₋₆)carbocycle, preferably aromatic (C₆)carbocycle, more preferably a non-substituted phenyl or a phenyl that is substituted by a substituent selected from the group consisting of Cl, F, Br, substituted or non-substituted methyl, preferably-(CF₃), ethyl, propyl and cyclopropyl;
(vii) substituted or non-substituted (C₃₋₆)heterocycle or (C₇₋₁₀)carbo- or hetero-bicycle having 1 to 3 heteroatoms each independently selected from N, O and S, preferably substituted or non-substituted heterobicycle having 2 oxygen atoms, preferably substituted or non-substituted benzodioxolyl connected via position (5) or (6) of the benzodioxolyl;
(viii) a mono- or polysaccharide, preferably a mono-, di- or trisaccharide, more preferably a mono- or disaccharide, wherein the mono- or polysaccharide ring carbon is directly attached or attached via an oxygen atom;
preferably for Formula (V) **R²** is 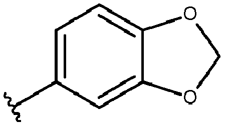 and/or **A** and/or **Y** are C or N;
**R², R ^{2a}** or **R^{2b}** and **R³, R^{3a}** or **R^{3b}** optionally together form a ring selected from the group consisting of
(i) substituted or non-substituted carbocycle selected from the group consisting of (C₃-₁₀)carbocycle, preferably (C₃)carbocycle, and (C₅₋₆)carbocycle, preferably aromatic (C₆)carbocycle, more preferably a non-substituted phenyl or a phenyl that is substituted by a substituent selected from the group consisting of Cl, F, Br, substituted or non-substituted methyl, preferably -(CF₃), ethyl, propyl and cyclopropyl; and
(ii) substituted or non-substituted (C₃-₆)heterocycle or (C₇-₁₀)carbo- or hetero-bicycle having 1 to 3 heteroatoms each independently selected from N, O and S, preferably substituted or non-substituted heterobicycle having 2 oxygen atoms;
**R^{9a}** and **R^{9b}** are independently selected from the group consisting of
(i) H, Me, OH, OMe, and =O;
(ii) a mono- or polysaccharide, preferably a mono-, di- or trisaccharide, more preferably a mono- or disaccharide, wherein the mono- or polysaccharide ring carbon is directly attached or attached via an oxygen atom;
preferably R^{9a} is H and R^{9b} is OH, more preferably R^{9a} is =O and R^{9b} is absent; and/or
**R¹⁰** is H, -OH, =O, or a mono- or polysaccharide, preferably a mono-, di- or trisaccharide, more preferably a mono- or disaccharide, wherein the mono- or polysaccharide ring carbon is directly attached or attached via an oxygen atom.

5. The anthraquinone or anthraquinone derivative for use according to claim 4, wherein the anthraquinone or anthraquinone derivative is an anthraquinone or anthraquinone derivative according to Formula (IIIa), 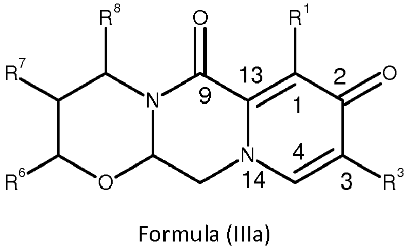 and R¹, R³ and R⁶ to R⁸ are as defined in claim 4, preferably:
R⁶ and R⁷ are hydrogen;
R⁸ is a linear or branched, substituted or non-substituted (C₁₋₁₀)alkyl, (C₂₋₁₀)alkenyl, (C₂-₁₀)alkynyl, preferably (C₁₋₁₀)alkyl, more preferably substituted or non-substituted methyl, ethyl or propyl, most preferably substituted or non-substituted methyl; and/or
R¹ is OH or -O(C₁₋₁₀)alkyl, preferably -OMe, -OEt, or -O(C=O)(C₁₋₁₀)alkyl, more preferably -O(C=O)Me or OH; and/or
R³ is OH, a linear or branched, substituted or non-substituted (C₁₋₁₀)alkyl, (C₂₋₁₀)alkenyl, (C₂₋₁₀)alkynyl, preferably (C₁₋₁₀)alkyl, more preferably substituted or non-substituted methyl, ethyl or propyl, most preferably substituted or non-substituted methyl, -O(C₁-₁₀)alkyl, preferably -OMe or -OEt, -O(C₃₋₁₀)cycloalkyl, preferably -O-cyclopropyl,-O(C=O)(C₁₋₁₀)alkyl, preferably -O(C=O)Me, -O(C=O)(C₃₋₁₀)cycloalkyl, preferably -O(C=O)-cyclopropyl, COOH, COOMe, COOEt, or C(C=O)NH₂.

6. The anthraquinone or anthraquinone derivative for use according to claim 4, wherein the anthraquinone or anthraquinone derivative is an anthraquinone or anthraquinone derivative according to Formula (IIIb), 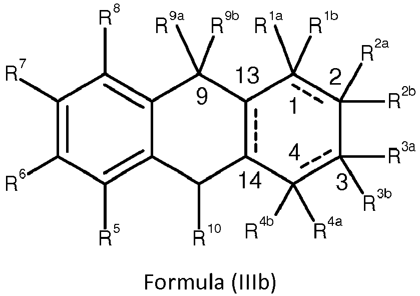 wherein R¹ to R⁹ are as defined in claim 4 and R¹⁰ is -OH or =O.

7. The anthraquinone or anthraquinone derivative for use according to claim 4, wherein the anthraquinone or anthraquinone derivative is an anthraquinone or anthraquinone derivative according to Formula (IIIc), 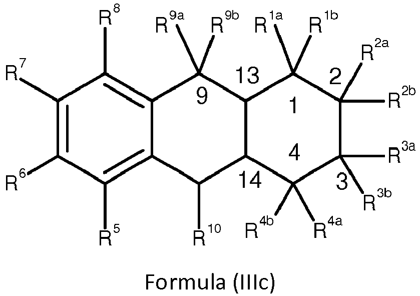 wherein
**R^{1a}** and **R^{1b}, R^{2a}** and **R^{2b}, R^{3a}** and **R^{3b}, R^{4a}** and **R^{4b},** and **R⁵** to **R⁸** are each independently selected from the group consisting of:
(i) H, or -OH;
(ii) -O(C₁₋₆)alkyl, preferably -OMe or -OEt, -O(C₃₋₆)cycloalkyl, preferably -O-cyclopropyl,-O(C=O)(C₁₋₃)alkyl, preferably -O(C=O)Me, or -O(C=O)(C₃₋₆)cycloalkyl, preferably-O(C=O)cyclopropyl;
(iii) linear or branched, substituted or non-substituted (C₂₋₁₀)alkyl ether, (C₃₋₁₀)alkenyl ether, (C₃₋₁₀)alkynyl ether or (C₄₋₁₀)carbocyclic ether, wherein the ether is bonded to formula (IIIc) via its carbon atom;
(iv) linear or branched, substituted or non-substituted (C₁₋₆)alkyl, (C₂₋₆)alkenyl, (C₂-₆)alkynyl, preferably (C₁₋₁₆)alkyl, more preferably substituted or non-substituted methyl, ethyl or propyl, most preferably substituted or non-substituted methyl;
(v) substituted or non-substituted carbocycle selected from the group consisting of (C₃-₁₀)carbocycle, preferably (C₃)carbocycle and (C₅₋₆)carbocycle, preferably aromatic (C₆)carbocycle, more preferably a non-substituted phenyl or a phenyl that is substituted by a substituent selected from the group consisting of Cl, F, Br, substituted or non-substituted methyl, preferably-(CF₃), ethyl, propyl and cyclopropyl;
(vi) substituted or non-substituted (C₃₋₆)heterocycle or (C₇₋₁₀)carbo- or hetero-bicycle having 1 to 3 heteroatoms each independently selected from N, O and S, preferably substituted or non-substituted heterobicycle having 2 oxygen atoms, preferably substituted or non-substituted benzodioxolyl connected via position (5) or (6) of the benzodioxolyl;
**R^{9a}** and **R^{9b}** are independently selected from the group consisting of H, Me, -OH, -OMe, and =O, preferably R^{9a} is H and R^{9b} is -OH, more preferably R^{9a} is OH and R^{9b} is Me; and/or **R¹⁰** is -OH or =O.

8. The anthraquinone or anthraquinone derivative for use according to claim 4 or 6, wherein the anthraquinone or anthraquinone derivative is an anthraquinone or anthraquinone derivative according to Formula (IIId), 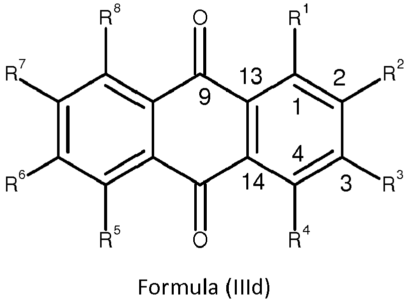 wherein
**R¹** to **R⁸** are each independently selected from the group consisting of:
(i) H, F, Cl, Br, -OH, -OSO₃H and -SO₃H;
(ii) -O(C₁₋₆)alkyl, preferably -OMe or -OEt, -O(C₃₋₆)cycloalkyl, preferably -O-cyclopropyl,-O(C=O)(C₃₋₆)cycloalkyl, preferably -O(C=O)cyclopropyl or -O(C=O)(C₁₋₃)alkyl, preferably -O(C=O)Me;
(iii) -COOH, -COO(C₁₋₁₀)alkyl, -COO(C₃₋₆)cycloalkyl, -COONH₂, -COON((C₁₋₁₀)alkyl)₂, or-COONH(C₁₋₁₀)alkyl, preferably -COOH, -COOMe or COOEt;
(iv) linear or branched, substituted or non-substituted (C₂₋₁₀)alkyl ether, (C₃₋₁₀)alkenyl ether, (C₃₋₁₀)alkynyl ether or (C₄₋₁₀)carbocyclic ether, wherein the ether is bonded to formula (IIId) via its carbon atom;
(v) linear or branched, substituted or non-substituted (C₁₋₁₀)alkyl, (C₂₋₁₀)alkenyl, or (C₂-₁₀)alkynyl, preferably (C₁₋₁₀)alkyl, more preferably substituted or non-substituted-CHO, methyl, ethyl or propyl, most preferably substituted or non-substituted methyl;
(vi) substituted or non-substituted carbocycle selected from the group consisting of (C₃₋₁₀)carbocycle, preferably (C₃)carbocycle and (C₅-₆)carbocycle, preferably aromatic (C₆)carbocycle, more preferably a non-substituted phenyl or a phenyl that is substituted by a substituent selected from the group consisting of Cl, F, Br, substituted or non-substituted methyl, preferably -(CF₃), ethyl, propyl and cyclopropyl;
(vii) substituted or non-substituted (C₃₋₆)heterocycle or (C₇₋₁₀)carbo- or hetero-bicycle having 1 to 3 heteroatoms each independently selected from N, O and S, preferably substituted or non-substituted heterobicycle having 2 oxygen atoms, preferably substituted or non-substituted benzodioxolyl connected via position (5) or (6) of the benzodioxolyl; and/or
**R²** and **R³** together form a ring selected from the group consisting of
(i) substituted or non-substituted (C₃₋₁₀)carbocycle, preferably (C₃)carbocycle or (C₅₋₆)carbocycle, preferably aromatic (C₆)carbocycle, more preferably a non-substituted phenyl or a phenyl that is substituted by a substituent selected from the group consisting of Cl, F, Br, substituted or non-substituted methyl, preferably -(CF₃), ethyl, propyl and cyclopropyl; and
(ii) substituted or non-substituted (C₃₋₆)heterocycle or (C₇₋₁₀)carbo- or hetero-bicycle having 1 to 3 heteroatoms each independently selected from N, O and S, preferably substituted or non-substituted heterobicycle having 2 oxygen atoms.

9. The anthraquinone or anthraquinone derivative for use according to any of claims 4, 6 or 8, wherein the anthraquinone or anthraquinone derivative is an anthraquinone or anthraquinone derivative according to Formula (IIId), 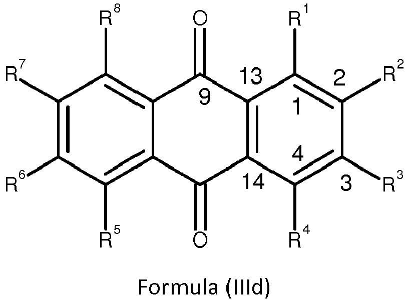 wherein
**R¹** to **R⁸** are each independently selected from the group consisting of:
(i) H, F, Cl, -OH, and -OSO₃H;
(ii) -OMe, -OEt, -O-cyclopropyl, -O(C=O)cyclopropyl, -O(C=O)Me or -O(C=O)Et;
(iii) -COOH, -COONH₂, -COOMe or -COOEt;
(iv) linear or branched, substituted or non-substituted (C₂₋₁₀)alkyl ether wherein the ether is bonded to formula (IIId) via its carbon atom, preferably an ether selected from the group consisting of 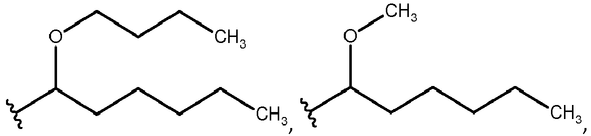 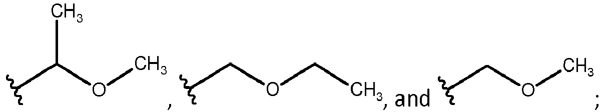
(v) linear or branched, substituted or non-substituted (C₁₋₁₀)alkyl or (C₂₋₁₀)alkenyl, preferably substituted or non-substituted methyl, ethyl and propyl, most preferably selected from the group consisting of methyl, ethyl, -MeOH, -EtOH, -CHO, 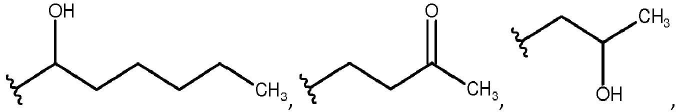 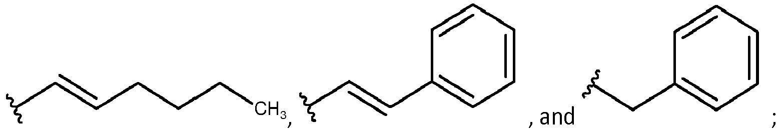
(vi) substituted or non-substituted carbocycle (C₃₋₆)carbocycle, preferably (C₃)carbocycle or (C₅₋₆)carbocycle, preferably aromatic (C₆)carbocycle, more preferably a substituted or non-substituted carbocycle selected from the group consisting of phenyl, benzenediol, preferably p,m-benzenediol, cyclohexyl, cyclopentyl, and cyclopenta-1,3-dienyl;
(vii) substituted or non-substituted (C₃₋₆)heterocycle or (C₇₋₁₀)carbo- or hetero-bicycle having 1 to 3 heteroatoms each independently selected from N, O and S, preferably substituted or non-substituted benzodioxolyl connected via position (5) or (6) of the benzodioxolyl; and/or
**R²** and **R³** together form a ring selected from the group consisting of
(i) substituted or non-substituted (C₃₋₁₀)carbocycle; and
(ii) substituted or non-substituted (C₃₋₆)heterocycle or (C₇₋₁₀)carbo- or hetero-bicycle having 1 to 3 heteroatoms each independently selected from N, O and S, preferably substituted or non-substituted heterobicycle having 2 oxygen atoms,
preferably R² and R³ together form a ring selected from the group consisting of

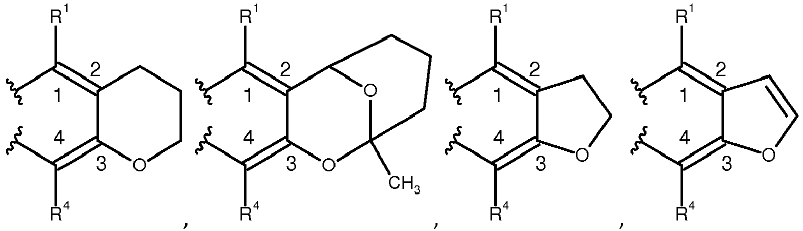

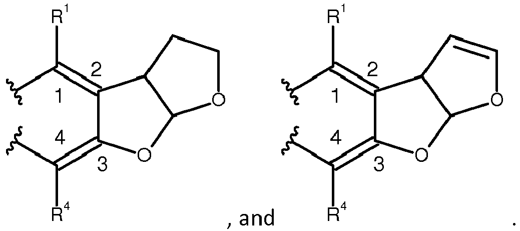

10. The anthraquinone or anthraquinone derivative for use according to any of claims 4 to 9, wherein the anthraquinone or anthraquinone derivative is selected from the group consisting of: 1,2-dihydroxyanthracene-9,10-dione (Alizarin); 1,3,4-trihydroxy-9,10-dioxo-anthracene-2-carboxylic acid (Pseudopurpurin); 1,2,4-trihydroxyanthracene-9,10-dione (Purpurin); 1,3-dihydroxy-2-methyl-anthracene-9,10-dione (Rubiadin); 1,3,-dihydroxy-2(hydroxymethyl)anthracene-9,10-dione (Lucidin); 2-methylanthracene-9,10-dione (Tectochinone); 2-(hydroxymethyl)anthracene-9,10-dione; 2-methoxyanthracene-9,10-dione; 2-hydroxyanthracene-9,10-dione; 1-hydroxyanthracene-9,10-dione; 1-hydroxy-2-methyl-anthracene-9,10-dione; 1-hydroxy-2-(hydroxymethyl)anthracene-9,10-dione; ethyl 1-hydroxy-9,10-dioxo-anthracene-2-carboxylate; 1-methoxy-2-methyl-anthracene-9,10-dione; 1-hydroxy-2-methoxy-anthracene-9,10-dione; 2-hydroxy-1-methoxy-anthracene-9,10-dione; 1,2,-dimethoxyanthracene-9,10-dione; 1,3-dihydroxy-9,10-dioxo-anthracene-2-carbaldehyde; 1,3-dihydroxy-9,10-dioxo-anthracene-2-carboxylic acid; methyl-1,3-dihydroxy-9,10-dioxo-anthracene-2-carboxylate; 2-(ethoxymethyl)-1,3,-dihydroxy-anthracene-9,10-dione; 1,3-dihydroxy-2-(methoxymethyl)anthracene-9,10-dione; 2-(hydroxymethyl)-1,3-dimethoxy-anthracene-9,10-dione; 1,3-dihydroxy-9,10-dioxo-anthracene-2-carboxylic acid; 1,3-dimethoxy-9,10-dioxo-anthracene-2-carboxylic acid; 1,2,3-trihydroxyanthracene-9,10-dione; 1,3-dihydroxyanthracene-9,10-dione; 1,3-dihydroxy-2-phenyl-anthracene-9,10-dione; 2-benzyl-1,3-dihydroxy-anthracene-9,10-dione; 1,3-dihydroxy-2-methoxy-anthracene-9,10-dione; 1,2-dihydroxy-3-methoxy-anthracene-9,10-dione; 1-hydroxy-2,3-dimethoxy-anthracene-9,10-dione; 1-hydroxy-3-methoxy-9,10-dioxo-anthracene-2-carboxylic acid; 3-hydroxy-1-methoxy-2-(methoxy-methyl)anthracene-9,10-dione; 1,4-dihydroxyanthracene-9,10-dione (Quinizarin); 1,4-dihydroxy-2-(hydroxymethyl)anthracene-9,1-dione; ethyl-1,4-dihydroxy-9,10-dioxo-anthracene-2-carboxylate; 2-(ethoxymethyl)-1,4-dihydroxy-anthracene-9,10-dione (Christofin); 1,4-dihydroxy-2-methyl-anthracene-9,10-dione; 1-hydroxy-3-methoxy-anthracene-9,10-dione; 1,3-dimethoxyanthracene-9,10-dione; methyl-4-hydroxy-9,10-dioxo-anthracene-2-carboxylate; 1,4-dihydroxy-5-methoxy-2-methyl-anthracene-9,10-dione; 1,4-dihydroxy-8-methoxy-2-methyl-anthracene-9,10-dione; 1,4-diyhdroxy-6-methyl-anthracene-9,10-dione; 1,5-dihydroxy-2-methyl-anthracene-9,10-dione; 1,8-dihydroxy-3-methoxy-6-methyl-anthracene-9,10-dione (Physcion, Parietin); 1,3,6-tri-hydroxy-2-methyl-anthracene-9,10-dione; 1,8-dihydroxy-3-methoxy-6-methyl-anthracene-9,10-dione; 3,6-dihydroxy-1-methoxy-2-methyl-anthracene-9,10-dione; 2-hydroxy-7-methyl-anthracene-9,10-dione; 4-hydroxy-9,10-dioxo-anthracene-9,10-dione; 1,6-dihydroxy-2-methyl-anthracene-9,10-dione; 1,3,8-trihydroxy-6-(hydroxymethyl)anthracene-9,10-dione (Citreorosein); 1,8-dihydroxy-3-(hydroxymethyl)-6-methoxy-anthracene-9,10-dione (Telochistin); 3,4,6-trihydroxy-2,7-dimethoxy-benzo[f]benzofuran-5,8-dione (Rhodocladonic acid); 1,8-dihydroxy-3,6-dimethoxy-anthracene-9,10-dione (Fallacinol); 1,3,6,8-tetrahydroxy-2-[E-styryl]anthracene-9,10-dione (Averythrin); 1,6,8-trihydroxy-3-methyl-9,10-dioxo-anthracene-2-carboxylic acid (Endocrocin); 1,6,8-trihydroxy-3-methyl-9-oxo-10H-anthracene-2-carboxylic acid; 1,4,5-trihydroxy-7-methoxy-2-methyl-anthracene-9,10-dione; 1,4,5,7-tetrahydroxy-2-(hydroxymethyl)anthracene-9,10-dione (Tritisporin); 1,5,8-trihydroxy-3-methyl-anthracene-9,10-dione (Helminthosporin); 1,4,5,8-tetrahydroxy-2-methyl-anthracene-9,10-dione (Cynodontin); 7-alpha-D-Glucopyranosyl-9,10-dihydro-3,5,6,8-tetrahydroxy-1-methyl-9,10-dioxoanthracenecarboxylic acid (Carminic acid); 1,5,-dihydroxy-8-methoxy-3-methyl-anthracene-9,10-dione (Questin); 4,5,7-trihydroxy-9,10-dioxo-anthracene-2-carboxylic acid (Emodic acid); 1,6-dihydroxy-3-(hydroxymethyl)-8-methoxy-anthracene-9,10-dione (Questinol); 1,3-dihydroxy-6-(hydroxymethyl)-8-methoxy-anthracene-9,10-dione (Carviolin); 1,3,8-trihydroxy-6-(hydroxymethyl)anthracene-9,10-dione (Citreorosein); 2,8-dihydroxy-1-methoxy-3-methyl-anthracene-9,10-dione; 3-acetyl-1,2,4,5,7-pentahydroxy-anthracene-9,10-dione; 1,3,8-trihydroxy-6-methyl-anthracene-9,10-dione (Emodin); Emodin-6-O-rhamnoside (Cascarin); 4,5-dihydroxy-9,10-dioxo-anthracene-2-carboxylic acid (Rhein); 1,4,5,7-tetrahydroxy-2-methyl-anthracene-9,10-dione (Catenarin); 1,8-dihydroxy-3-(hydroxymethyl)anthracene-9,10-dione (Aloe-emodin); 1,8-dihydroxy-3-methyl-anthracene-9,10-dione (Chrysophanol); Rhein-8-glucoside; 1,3,8-trihydroxy-6-methyl-anthracene-9,10-dione (Alatinone); 4,5-diacetoxy-7-hydroxy-9,10-dioxo-anthracene-2-carboxylic acid (Diacerhein); 1,3,5,8-tetrahydroxy-6-methyl-anthracene-9,10-dione; 1,4-dihydroxy-6,7-dimethoxy-3-methyl-9,10-dioxo-anthracene-2-carboxylic acid; 1,3-dihydroxy-6,8-dimethoxy-anthracene-9,10-dione; 1,3,5,8-tetrahydroxy-2-methyl-anthracene-9,10-dione; 1,3,8-trihydroxy-2-methyl-1,2-dihydroanthracene-9,10-dione; 1-methoxy-anthracene-9,10-dione; 1,8-dihydroxy-6-methoxy-2-methyl-anthracene-9,10-dione; 1,8-dihydroxy-3-methoxy-6-methyl-anthracene-9,10-dione; 2,3,4,8-tetra-hydroxy-6-methoxy-3-methyl-2,4-dihydro-1H-anthracene-9,10-dione; 1,3,6,8-tetrahydroxyanthracene-9,10-dione; 2,3,5-trihydroxy-7-methyl-1,2,3,4-tetrahydroanthracene-9,10-dione; 1,3-dihydroxy-8-methoxy-6-methyl-anthracene-9,10-dione; 2,3,4,8,10-pentahydroxy-6-methoxy-3-methyl-1,2,4,10-tetrahydroanthracen-9-one; 1,3,7-trihydroxy-6-(2-hydroxypropyl)anthracene-9,10-dione; 1,3,6,8-tetrahydroxy-2-(1-hydroxyethyl)anthracene-9,10-dione; 5,7,9-trihydroxy-3,4-dihydro-2H-naptho[2,3-g]chromene-6,11-dione; 2-chloro-1,6-dihydroxy-8-methoxy-3-methyl-anthracene-9,10-dione; 1,3,6,8-tetrahydroxy-2-(1-methoxyethyl)anthracene-9,10-dione; 1,3,8-trihydroxy-9,10-dioxo-anthracene-2-carbaldehyde; 2,3,5,8,10-pentahydroxy-6-methoxy-3-methyl-1,2,4a,9a,10-hexahydroanthracen-9-one; (3,5,8,10-tetrahydroxy-6-methoxy-3-methyl-9-oxo-1,2,4,4a,9a,10-hexahydroanthracen-2-yl)acetate; 2,16,18-trihydroxy-7,9-dioxapentacyclo[10.8.0.03,10.04,8.014,19]-icosa-1(12),2,10,14(19),15,17-hexaene-13,20-dione; 2,16,18-trihydroxy-7,9-dioxapentacyclo[10.8.0.03,10.04,8.014,19]icosa-1(12),2,5,10,14(19),15,17-heptaene-13,20-dione; 4-hydroxy-5-methoxy-9,10-dioxo-anthracene-2-carbaldehyde; 6,8-dimethoxy-1-methyl-2-(3-oxobutyl)anthracene-9,10-dione; 2-[(E)-hex-1-enyl]-1,3,6,8-tetrahydroxy-anthracene-9,10-dione; 3,7-dihydroxy-9-methoxy-17-methyl-16,21-dioxapentacyclo[15.3.1.02,15.04,-13.06,11]henicosa-2,4(13),6(11),7,9,14-hexaene-5,12-dione;1,3,6,8-tetrahydroxy-2-(1-methoxyhexyl)anthracene-9,10-dione; 1,3,6,8-tetrahydroxy-2-(1-hydroxyhexyl)anthracene-9,10-dione; 2-chloro-1,3,6,8-tetrahydroxy-7-(1-hydroxyhexyl)anthracene-9,10-dione; 2-(1-butoxyhexyl)-7-chloro-1,3,6,8-tetrahydroxy-anthracene-9,10-dione; (3,5,8,10-tetrahydroxy-6-methoxy-3-methyl-9-oxo-1,2,4,4a,9a,10-hexahydroanthracen-2-yl) acetate; 11-hydroxy-7-methyl-9,12-dioxo-4-oxa-1,8-diazatricyclo[8.4.0.03,8]tetradeca-10,13-diene-13-carboxylic acid; 11-hydroxy-7,13-dimethyl-4-oxa-1,8-diazatricyclo[8.4.0.03,8]tetradeca-10,13-diene-9,12-dione; 11-hydroxy-7-methyl-9,12-dioxo-4-oxa-1,8-diazatricyclo-[8.4.0.03,8]tetradeca-10,13-diene-13-carboxylic acid; 1,3-dihydroxy-2-phenyl-anthracene-9,10-dione; 2-(3,4-dihydroxyphenyl)-1,3-dihydroxy-anthracene-9,10-dione; [3-(1,3-benzodioxol-5-yl)phenyl]-(o-tolyl)methanone; [4-(1,3-benzodioxol-5-yl)pyrimidin-2-yl]-(o-tolyl)methanone; 2-(1,3-benzodioxol-5-yl)-3-hydroxy-benzo[g]quinoline-5,10-dione; 2-(1,3-benzodioxol-5-yl)-1,3-dihydroxy-anthracene-9,10-dione; 1-hydroxy-3-methylanthraquinone; 1,3,6,8-Tetrahydroxyanthraquinone; Coniothranthraquinone 1 ((2*S*,3*R*)-2,3,5-trihydroxy-7-methyl-1,2,3,4-tetrahydroanthraquinone); Macrosporin (1,7-dihydroxy-3-methoxy-6-methylanthraquinone); Austrocortinin (1,4-dihydroxy-2-methoxy-7-methylanthraquinone); 1-Methylemodin (1,3-dihydroxy-8-methoxy-6-methylanthraquinone); Macrospin (1,6-dihydroxy-3-methoxy-7-methylanthraquinone); Monodictyquinone (1,8-dihydroxy-2-methoxy-6-methylanthraquinone); 2,3,5,8-Tetrahydroxy-7-methoxy-2-methyl-1,2,3,4-tetrahydroanthraquinone; 10-Deoxybostrycin; Dihydroaltersolanol C ((1*R,*2*R,*3*R*)-1,2,3,5-tetra-hydroxy-7-methoxy-2-methyl-1,2,3,4,4a,9a-hexahydroanthraquinone); Lunatin (1,3,8-trihydroxy-6-methoxyanthraquinone); Trichodermaquinone ((2*S,*3*R*)-2,3,5-trihydroxy-7-(hydroxylmethyl)-1,2,3,4-tetrahydroanthraquinone); 2,8-dihydroxy-1,3-dimethoxy-6-methyl anthraquinone; Erythroglaucin (1,4,5-trihydroxy-7-methoxy-2-methylanthraquinone); Fusaquinon C ((2*S,*3*R,*4a*R,*9a*R,*10*S*)-2,3,5,8,10-pentahydroxy-6-methoxy-3-methyl-1,3,4,4a,9a,10-hexahydroanthracen-9(2*H*)-one); Rubocristin (1,4,7-trihydroxy-5-methoxy-2-methylanthraquinone); 6-*O*-methylalaternin (1,2,8-trihydroxy-6-methoxy-3-methylanthraquinone); 1,4,6-Trihydroxy-2-methoxy-7-methyl-anthraquinone; 7-Chloro-emodin; Altersolanol B ((2*S,*3*R*)-2,3,5-trihydroxy-7-methoxy-2-methyl-1,2,3,4-tetrahydro-anthraquinone); Fusaquinon A ((2*R,*3*S,*4a*R,*9*S,*9a*S*)-3,5,8-trihydroxy-7-methoxy-2-methyl-2,3,4,4a,9,9a-hexahydro-2,9-epoxyanthracen-10(1*H*)-one); Dihydroaltersolanol B ((2*S,*3*R*)-2,3,5-trihydroxy-7-methoxy-2-methyl-1,2,3,4,4a,9a-hexahydroanthraquinone); Xylanthraquinone; Isorhodoptilometrin ((*R*)-1,3,8-trihydroxy-6-(2-hydroxypropyl)anthraquinone); 1,3,6,8-Tetrahydroxy-2-(1-hydroxyethyl) anthraquinone; 1-*O*-Methyl-7-chloroemodin (2-chloro-1,6-dihydroxy-8-methoxy-3-methylanthraquinone); 7-Chlorocitreorosein; Austrocortirubin (1,4-dihydroxy-2-methoxy-7-methylanthraquinone); 1-Deoxytetrahydrobostrycin ((2*R,*3*S*)-2,3,5,8,10-pentahydroxy-6-methoxy-3-methyl-1,3,4,4a,9a,10-hexa-hydroanthracen-9(2*H*)-one); Fragilin (2-chloro-1,8-dihydroxy-3-methoxy-6-methylanthraquinone); 5-Acetyl-2-methoxy-1,4,6-trihydroxyanthraquinone; Isorhodoptilometrin-1-methylether (1,3-dihydroxy-6-2-hydroxypropyl-8-methoxyanthraquinone); 1,3,6,8-Tetrahydroxy-2-(1-methoxyethyl)anthraquinone; Phomopsanthraquinone ((2*R,*3*S*)-7-ethyl-1,2,3,4-tetrahydro-2,3,8-trihydroxy-6-methoxy-3-methylanthraquinone); Altersolanol A ((1*R,*2*S,*3*R,*4*S*)-1,2,3,4,5-pentahydroxy-7-methoxy-2-methyl-1,2,3,4-tetrahyd roa nth raquinone); Bostrycin ((5*S,*6*R,*7*S*)-5,6,7,9,10-pentahydroxy-2-methoxy-7-methyl-5,6,7,8-tetrahydroanthracene-1,4-dione); SZ-685C ((1,2,3,5,8-pentahydroxy-6-methoxy-3-methyl-1,2,3,4-tetrahydroanthraquinone); Fusaquinon B ((1*R,*2*S,*3*R,*4a*R*,9a*S,*10*S*)-1,2,3,5,8,10-hexa hyd roxy-6-methoxy-3-methyl-1,3,4,4a,9a,10-hexahyd roa nth racen-9(2*H*)-one); Tetrahydroxybostrycin ((1,2,3,5,8,10-hexahydroxy-6-methoxy-3-methyl-1,3,4,4a,9a,10-hexahydroanthracen-9(2*H*)-one); Versicolorin C ((4,6,8-trihydroxy-3,3a-dihydroanthra[2,3-b]furo[3,2-d]furan-5,10(2*H,*12a*H*)-dione); 2-*O*-Acetylaltersolanol B ((2*R,*3*S*)-3,8-dihydroxy-6-methoxy-3-methyl-9,10-dioxo-1,2,3,4,9,10-hexahydroanthracen-2-yl acetate); 1,2,3,6,8-Pentahydroxy-7-(1-methoxyethyl)anthraquinone; Emodin-3-*O* Sulfate ((4,5-dihydroxy-7-methyl-9,10-dioxo-9,10-dihydroanthracen-2-yl hydrogen sulfate); Auxarthrol C ((1*S,*2*R,*3*R,-*4*R,*4a*R,*9a*S*)-1,2,3,4,5-pentahydroxy-7-methoxy-2-methyl-1,2,3,4-tetrahydro-4a,9a-epoxy-anthraquinone); 8-*O*-MethylversicolorinB ((4,8-dihydroxy-6-methoxy-3,3a-dihydroanthra-[2,3-b]furo[3,2-d]furan-5,10(2*H,*12a*H*)-dione); 6,8-Dimethoxy-1-methyl-2-(3-oxobutyl)-anthraquinone; 8-*O*-Methylversicolorin A (4,8-dihydroxy-6-methoxyanthra[2,3-b]furo[3,2-d]furan-5,10(3a*H,*12a*H*)-dione); Averythrin ((*E*)-2-(hex-1-en-1-yl)-1,3,6,8-tetrahydroxyanthraquinone); Skyrin (2,2',4,4',5,5'-hexahydroxy-7,7'-dimethyl-[1,1'-bianthracene]-9,9',10,10'-tetraone); Macrosporin-7-*O*-sulfate (Sodium 8-hydroxy-6-methoxy-3-methyl-9,10-dioxo-9,10-dihydroanthracen-2-ylsulfate); Citreorosein-3-*O*-sulfate (4,5-dihydroxy-7-(hydroxymethyl)-9,10-dioxo-9,10-dihydroanthracen-2-ylhydrogen sulfate); 6,8-di-*O-*methylversicolorinA (4-hydroxy-6,8-dimethoxyanthra[2,3-b]furo[3,2-d]furan-5,10(3a*H,-*12a*H*)-dione); 8-*O*-Methylaverythrin ((*E*)-2-(hex-1-en-1-yl)-1,3,6-trihydroxy-8-methoxy-anthraquinone); Aversin (4-hydroxy-6,8-dimethoxy-3,3a-dihydroanthra[2,3-b]furo[3,2-d]furan-5,10(2*H,*12a*H*)-dione); Aversin ((-)-isomer of (4-hydroxy-6,8-dimethoxy-3,3a-dihydroanthra[2,3-b]furo[3,2-d]furan-5,10(2*H,*12a*H*)-dione); Averantin ((*S*)-1,3,6,8-tetrahydroxy-2-(1-hydroxyhexyl)anthraquinone); 6-*O*-Methylaverufin (7,9-dihydroxy-11-methoxy-2-methyl-3,4,5,6-tetrahydro-2*H*-2,6-epoxyanth ra[2,3-b]oxocine-8,13-dione); Nidurufin (5,7,9,11-tetrahydroxy-2-methyl-3,4,5,6-tetrahydro-2*H*-2,6-epoxyanthra[2,3-b]oxocine-8,13-dione); Averufin (7,9,11-trihydroxy-2-methyl-3,4,5,6-tetrahydro-2*H*-2,6-epoxyanthra[2,3-b]oxocine-8,13-dione); 1*'-O*-Methylaverantin (1,3,6,8-tetrahydroxy-2-(1-methoxyhexyl)anthraquinone); (2*S*)-2,3-Dihydroxy-propyl-1,6,8-trihydroxy-3-methyl-9,10-dioxoanthracene-2-carboxylate; 7-Chloroaverythrin ((*E*)-2-chloro-7-(hex-1-en-1-yl)-1,3,6,8-tetrahydroxyanthraquinone); 6,8-Di-*O*-methylversiconol (2-(1,4-dihydroxybutan-2-yl)-1,3-dihydroxy-6,8-dimethoxyanthraquinone); 6,8-Di-*O*-methylaverufin (7-hydroxy-9,11-di-metho-xy-2-methyl-3,4,5,6-tetra-hydro-2*H*-2,6-epoxyanthra[2,3-b]oxocine-8,13-dione); 6,8-Di-*O*-methylnidurufin (5,7-dihydroxy-9,11-dime-thoxy-2-methyl-3,4,5,6-tetrahydro-2*H-*2,6-epoxyanthra[2,3-b]oxocine-8,13-dione); 1,3-dihydroxy-6,8-dimethoxy-2-(6-methyl-tetra-hydro-2*H*-pyran-2-yl)anthracene-9,10-dione; 6,1*'-O,O*-Dimethylaverantin ((*S*)-1,3,8-trihydroxy-6-methoxy-2-(1-methoxyhexyl)anthraquinone); Variecolorquinone A ((*S*)-2,3-dihydroxypropyl 1,6-dihydroxy-8-methoxy-3-methyl-9,10-dioxo-9,10-dihydroanthracene-2-carboxylate); 6,8-Di-*O*-methylaverantin ((*S*)-1,3-dihydroxy-2-(1-hydroxyhexyl)-6,8-dimetho-xyanthraquinone); 6-*O*-Methyl-7-chloroaverythrin ((*E*)-2-chloro-7-(hex-1-en-1-yl)-1,6,8-trihydroxy-3-methoxyanthraquinone); (1*'*S)-7-Chloroaverantin ((*S*)-2-chloro-1,3,6,8-tetrahydroxy-7-(1-hydroxyhexyl)anthraquinone); 6,1*'-O,O*-Dimethyl-7-chloroaverantin ((*S*)-2-chloro-1,6,8-trihydroxy-3-methoxy-7-(1-methoxyhexyl)anthraquinone); 6,8,1*'*-Tri-*O-*methyl-averantin (1,3-dihydroxy-6,8-dimethoxy-2-(1-methoxyhexyl)anthraquinone); 6-3-*O*-(Ribofuranosyl)questin (1,6-dihydroxy-6-*O*-(ribofuranosyl)-8-methoxy-3-methylanthraquinone); 1*'-O*-methyl-7-chloro averantin ((*S*)-2-chloro-1,3,6,8-tetrahydroxy-7-(1-methoxy-hexyl)anthraquinone); 6-*O*-methyl-7-chloro-averantin ((*S*)-2-chloro-1,6,8-trihydroxy-7-(1-hydroxyhexyl)-3-methoxyanthraquinone); Averantin-1*'*-butyl ether ((*S*)-2-(1-butoxyhexyl)-1,3,6,8-tetrahydroxy-anthraquinone); 7-Chloroaverantin-1*'*-butyl ether ((*S*)-2-(1-butoxyhexyl)-7-chloro-1,3,6,8-tetrahy-droxyanthraquinone); 6-*O*-Methyl-7-bromoaverantin ((*S*)-2-bromo-1,6,8-trihydroxy-7-(1-hydroxyhexyl)-3-methoxyanthraquinone); 6,1*'-O,O*-Dimethyl-7-bromoaverantin ((*S*)-2-bromo-1,6,8-trihydroxy-3-methoxy-7-(1-methoxyhexyl)anthraquinone); Macrosporin2-*O*-(6'-acetyl)-a-d-glucopyranoside ((2*R,*3*S,*4*S,*5*R,*6*R*)-3,4,5-trihydroxy-6-((8-hydroxy-6-methoxy-3-methyl-9,10-dioxo-9,10-dihydroanthracen-2-yl)oxy)tetrahydro-2H-pyran-2-yl)methyl acetate); Penicillanthranin A ((1*S,*3*P,*4*S*)-1-(6-ethyl-1,3,8-trihyd roxy-9,10-dioxo-9,10-dihyd roa nth racen-2-yl)-6,8-dihydroxy-3,4,5-trimethyliso-chroman-7-carboxylic acid); Penicillanthranin B ((1*S,*3*R,*4*S*)-6,8-dihydroxy-3,4,5-trimethyl-1-(1,3,8-trihydroxy-6-(hydroxy-methyl)-9,10-dioxo-9,10-dihydroanthracen-2-yl)isochroman-7-carboxylic acid); (*trans*)*-R* (*cis*)-Emodin-Physcion bianthrone (2,4,4',5,5'-pentahydroxy-2'-methoxy-7,7'-dimethyl-[9,9'-bianthracene]-10,10'(9*H,*9'*H*)-dione); Alterporriol Q (1',2,7',8-tetrahydroxy-3',6-dimethoxy-3,6'-dimethyl-[1,2'-bianthracene]-9,9',10,10'-tetraone); Alterporriol R (2,4',6',8-tetrahydroxy-2',6-dimethoxy-3,7'-dimethyl-[1,1*'*-bianthracene]-9,9',10,10'-tetraone); Alterporriol V (2,2',8,8'-tetrahydroxy-6,6*'*-dimethoxy-3,3*'*-dimethyl-[1,1*'*-bianthracene]-9,9*'*,10,10*'*-tetraone);Cytoskyrin A ((6*R,*14*R,*17*S,*18*R,*19*R,*20*S*)-1,7,9,15,17,20-hexahydroxy-3,11-dimethoxy-6,13a,5a,14-(epibutane[1,2,3,4]tetrayl)cycloocta[1,2-b:5,6-b']dinaphtha-lene-5,8,13,16(6*H*,14*H*)-tetraone); Alterporriol K ((5*S,*8*R*)-4,4',5,7',8-penta-hydroxy-1,1'-dimethoxy-6',8-dimethyl-5,6,7,8-tetrahydro-[2,2'-bianthracene]-9,9',10,10'-tetraone); Alterporriol T ((6*R*,6'*S*,7*R*,-7*'R,*8*R*)-1*'*,4,6,6*'*,7,7*'*,8-heptahydroxy-2,3*'*-dimethoxy-6*'*,7-dime-thyl-5,5*'*,6,6*'*,7,7*'*,8,8*'-*octahydro-[1,2*'*-bianthracene]-9,9*'*,10,10*'*-tetraone); Alterporriol L ((6*S,*7*R,*8*R*)-4,4*'*,6,7,7*'*,8-hexahydroxy-1,1*'*-dimethoxy-6*'*,7-dimethyl-5,6,7,8-tetrahydro-[2,2*'*-bianthracene]-9,9',10,10'-tetraone); Alterporriol M ((6*S,*7*S,*8*R*)-4,4*'*,6,7,7*'*,8-hexahydroxy-1,1*'*-dimethoxy-6*'*,7-dimethyl-5,6,7,8-tetrahydro-[2,2*'*-bianthracene]-9,9*'*,10,10'-tetraone); Alterporriol P ((5*'R,*6*'R,*7*'R*)-1*'*,2,5*'*,6*'*,7*'*,8-hexahydroxy-3*'*,6-dimethoxy-3,6*'*-dimethyl-5*'*,6*'*,7*'*,8*'*,8*'*a,10*'*a-hexahydro-[1,2*'*-bianthracene]-9,9*'*,10,10*'*-tetraone); Alterporriol W ((1*'R,6*R,*7*R,*8R)-*2*'*,4,6,7,8,8*'*-hexahydroxy-2,6*'*-dimethoxy-3*'*,7-dimethyl-5,6,7,8-tetrahydro-[1,1*'*-bianthracene]-9,9',10,10'-tetraone); Alterporriol U ((6*R,*6*'S,*7*S,*7*'R*)-1*'*,4,6,6*'*,7,7*'*-hexahydroxy-2,3*'-*di-methoxy-6*'*,7-dimethyl-5,5*',*6,6*'*,7,7*'*,8,8*'*-octahy-dro-[1,2*'*-bianthracene]-9,9*'*,10,10*'-*tetraone); Alterporriol S ((2*'S,*3*'P,*4*'S,*6*R,*7*S,*9*'R*)-1,2*'*,3*'*,4,5*'*,6,7,8*'*-octahy-droxy-7*'*-methoxy-2*'*,4*'*,7-trimethyl-2*'*,3*'*,4*'*,4*'*a,5,6,7,8,9*'*,9*'*a-decahydro-[2,9*'*-bianthracene]-9,10,10*'*(1*'H*)-trione); (+)-aS-alterporriol C ((1*S,*5*'S,*6*'R,*7*'S,*8*'R*)-1*'*,2,5*'*,6*'*,7*'*,8,8*'*-heptahydroxy-3*'*,6-dimethoxy-3,6*'*-dime-thyl-5*'*,6*'*,7*'*,8*'*,8*'*a,10*'*a-hexahydro-[1,2*'*-bianthracene]-9,9*'*,10,10*'-*tetraone); Alterporriol C ((1*S,*5*'R,*6*'S,*7*'R,*8*'S*)-1*'*,2,5*'*,6*'*,7*'*,8,8*'*-heptahydroxy-3*'*,6-dimethoxy-3,6*'*-dime-thyl-5*'*,6*'*,7*'*,8*'*,8*'*a,10*'*a-hexahydro-[1,2*'*-bianthracene]-9,9*'*,10,10*'*-tetraone); Alterporriol N ((6*R,*6*'R,*7*R,*7*'R,*8*R,*8*'R*)-4,4*'*,6,6*'*,7,7*'*,8,8*'*-octahy-droxy-2,2*'*-dimethoxy-7,7*'-*dimethyl-5,5*'*,6,6*'*,7,7*'*,8,8*'*-octahydro-[1,1*'*-bianthracene]-9,9*'*,10,10*'*-tetraone); Alterporriol O ((2*R,*2*'R,*3*S,*3*'S*)-2,2*'*,3,3*'*,8,8*'*-hexahydroxy-6,6*'*-dimethoxy-3,3*'*-dimethyl-1,1*'*,2,2',3,3',4,4'-octahy-dro-[1,1*'*-bianthracene]-9,9',10,10'-tetraone); Acetylalterporriol D ((1*'S,*5*S,*5*'S,*6*R,*6*'R,*7*S,*7*'S,*8*R,*8*'R*)-4,4*'*,5,5*'*,6*'*,7,7*'*,8,8*'*-nonahydroxy-2,2*'*-dimethoxy-7,7*'-*dimethyl-9,9*'*,10,10*'*-tetraoxo-5,5*'*,6,6*'*,7,7*'*,8,8*'*,9,9*'*,10,10*'*-dodecahydro-[1,1*'*-bianthracen]-6-yl acetate); Acetylalterporriol E ((1*'R,*5*S,*5*'S,*6*R,*6*'R,*7*S,*7*'S,*8*R,*8*'R*)-4,4*'*,5,5*'*,6*'*,7,7*'*,8,8*'-*nonahydroxy-2,2*'*-dimethoxy-7,7*'*-dimethyl-9,9*'*,10,10*'*-tetraoxo-5,5*'*,6,6*'*,7,7*'*,8,8*'*,9,9*'*,-10,10*'*-dodecahydro-[1,1*'*-bianthracen]-6-yl acetate); Alterporriol D ((1*S,*5*S,*5*'S*,6*R,*6*'R,*7*S,-*7*'S,*8*R*,8*'R*)-4,4*'*,5,5*'*,6,6*'*,7,7*'*,8,8*'*-decahydroxy-2,2*'*-dimethoxy-7,7*'*-dimethyl-5,5*'*,6,6*'*,7,-7*'*,8,8*'*-octahy-dro-[1,1*'*-bianthracene]-9,9*'*,10,10*'*-tetraone); Alterporriol E ((1*R,*5*S,*5*'S*,6*R,-*6*'R,*7*S,*7*'S,*8*R,*8*'R*)-4,4*',*5,5*'*,6,6*'*,7,7*'*,8,8*'*-decahydroxy-2,2*'*-dimethoxy-7,7*'*-dimethyl-5,5*'*,6,6*'*,7,7*'*,8,8*'*-octahy-dro-[1,1*'*-bianthracene]-9,9*'*,10,10*'*-tetraone); Stemphylanthranol A((5*S,*5*"S,*6*R,*6*"R,*7*S,*7*"S,*8*R,*8*"R*)-2*'*,4,4*"*,5,5*"*,6,6*"*,7,7*"*,8,8*'*,8*"*-dodecahydroxy-2,2*"*,6*'-*trimethoxy-3*'*,7,7*"*-trimethyl-5,5*"*,6,6*"*,7,7*"*,8,8*"*-octahydro-[1,1*'*:5*'*,1*"*-teranthracene]-9,9*'*,9",10,10',10"-hexaone); Stemphylanthranol B ((5*S,*5*"R,*6*R,*6*"R,*7*S,*7*"R,*8*R,*8*"R*)-2',4,4",5,5",6,7,7",8,8',8"-undecahydroxy-2,2",6'-trimethoxy-3',6",7,7"-tetramethyl-5,5",6,6",7,7",8,8"-octa hyd ro-[1, 1':7', 1"-tetra nth racene]-9,9',9", 10, 10',10"-hexaone); 7-Chloroemodic acid; 7-Chloroemodinal; 7-Chloro-1,6,8-trihydroxy-3-methyl-10-anthrone; Damnacanthal (3-hydroxy-1-methoxy-anthraquinone-2-carboxaldehyde); 2,6-dihydroxy-anthraquinone; Austrocortinin (2-methoxy-7-methyl-1,4-dihydroxy-anthraquinone); Danthron (1,8-dihydroxy-anthraquinone); Dermolutein (8-methoxy-3-methyl-1,6-dihydroxy- anthraquinone-2-carboxylic acid); Fallacinal 3-formyl-6-methoxy-1,8-dihydroxy-anthraquinone); Phomarin (3-methyl-1,6-dihydroxy-anthraquinone); Anthragallol (alizarin brown; 1,2,3-trihydroxy-anthraquinone); Citreorosein (6-hydroxymethyl- 1,3,8-trihydroxy-anthraquinone); Dermoglaucin (3-methyl-1,7,8-trihydroxy-6-methoxy-anthraquinone); Dermorubin (3-methyl-1,4,6-trihydroxy-8-methoxy-anthraquinone-2-carboxylic acid); Endocrocin (3-methyl-1,6,8-trihydroxy-anthraquinone-2-carboxylic acid); Erythroglaucin (3-methyl-1,4,8-trihydroxy-6-methoxy-anthraquinone); Flavokermesic acid (Laccaic acid D; 1-methyl-3,6,8-trihydroxy-anthraquinone-2-carboxylic acid); Flavopurpurin (alizarin Y; 1,2,6-trihydroxy-anthraquinone); Islandicin (2-methyl-1,4,5-trihydroxy-anthraquinone); Morindone (6-methyl-1,2,5-trihydroxy-anthraquinone); Rubrocristin (2-methyl-1,4,7-trihydroxy- 5-methoxy-anthraquinone); Dermocybin (3-methyl-1,5,7,8-tetrahydroxy-6-methoxy-anthraquinone); and Kermesic acid (8-methyl-1,3,4,6-tetrahydroxy-anthraquinone-7-carboxylic acid); 3-(beta-D-Glucopyranosyloxy)-1,6-dihydroxy-2-methyl-anthraquinone; 3-(6-O-Acetyl-beta-D-glucopyranosyloxy)-1,6-dihydroxy-2-methyl-anthraquinone; 3-[(2-O-6-Deoxy-alpha-L-mannopyranosyl-beta-D-glucopyranosyl)oxy]-1,6-dihydroxy-2-methyl-anthraquinone; 3-[(3-O-Acetyl-2-O-6-deoxy-beta-D-mannopyranosyl-beta-D-glucopyranosyl)oxy]-1,6-dihydroxy-2-methyl-anthraquinone; 3-[(6-0-Acetyl-2-0-6-deoxy-beta-D-ma nnopyranosyl-beta-D-glucopyranosyl)oxy]-1,6-dihydroxy-2-methyl-anthraquinone; 3-[(3,6-O-Diacetyl-2-O-6-deoxy-beta-D-mannopyranosyl-beta-D-glucopyranosyl)-oxy]-1,6-dihydroxy-2-methyl-anthraquinone; 3-[(4,6-O-Diacetyl-2-O-6-deoxy-beta-D-mannopyranosyl-beta-D-glucopyranosyl)oxy]-1,6-dihydroxy-2-methyl-anthraquinone; 3-[(4-O-Acetyl-2-O-6-deoxy-beta-D-mannopyranosyl-beta-D-glucopyranosyl)oxy]-1,6-dihydroxy-2-methyl-anthraquinone; 3-[(6-O-Acetyl-2-O-beta-D-xylopyranosyl-beta-D-glucopyranosyl)oxy]-1,6-dihydroxy-2-methyl-anthraquinone; Ruberythric acid (1-Hydroxy-2-[(6-O-beta-D-xylopyranosyl-beta-D-glucopyranosyl)oxy]-anthraquinone); Lucid in primeveroside (1-Hydroxy-2-(hydroxymethyl)-3-[(6-O-beta-D-xylopyranosyl-beta-D-glucopyranosyl)-oxy]-anthraquinone); 1-Acetyl-3-[(4-O-6-deoxy-beta-D-mannopyranosyl-beta-D-glucopyranosyl)oxy]-6-hydroxy-2-methyl-anthraquinone; 2-{[(6-O-beta-D-Glucopyranosyl-beta-D-glucopyranosyl)oxy]methyl}-11-hydroxy-anthraquinone; 3-[(2-O-6-Deoxy-beta-D-mannopyranosyl-beta-D-glucopyranosyl)oxy]-1-hydroxy-2-(methoxycarbonyl)-anthraquinone; 3-(beta-D-Glucopyranosyloxy)-2-(hydroxymethyl) anthraquinone; 3-(beta-D-Glucopyranosyloxy)-8-hydroxy-2-(hydroxymethyl)-anthraquinone; 2-(beta-D-Glucopyranosyloxy)-1,3-dihydroxy-anthraquinone; and 3-(beta-D-Glucopyranosyloxy)-1-hydroxy-2-(hydroxylmethyl)- anthraquinone.

11. The compound selected from Formula (I) or (II) for use according to claim 1, wherein
**R¹** is selected from the group consisting of
(i) linear or branched, substituted or non-substituted (C₂₋₆)alkyl, preferably substituted or non-substituted butyl or pentyl, more preferably, 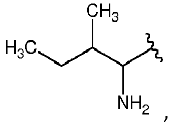 and 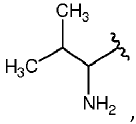 most preferably 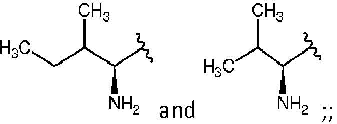
(ii) substituted or non-substituted (C₃₋₆)heterocycle having 1 to 3 heteroatoms each independently selected from N, O and S, preferably substituted or non-substituted heterobicycle having 1 nitrogen atom, preferably substituted or non-substituted piperidine, most preferably 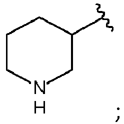
**R¹** to **R⁶** are each independently selected from the group consisting of H, Cl, Br, or F, preferably for Formula (I), R⁵ is Cl and R² to R⁴ and R⁶ are H, more preferably and R⁴ are CI and R², R⁵ and R⁶ are H,
preferably for Formula (II), R⁴ is Cl and R², R³, R⁵ and R⁶ are H, more preferably R² and R⁴ are Cl and R³, R⁵ and R⁶ are H;
**R⁷** is selected from the group consisting of
(i) -NH₂, or -OH;
(ii) substituted or non-substituted (C₃₋₆)heterocycle or (C₇₋₁₀)carbo- or hetero-bicycle having 1 to 3 heteroatoms each independently selected from N, O and S, preferably substituted or non-substituted heterobicycle having 1 nitrogen atom, more preferably isoindoline-1,3-dione, most preferably 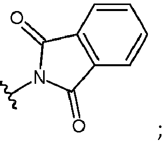
**R⁸** is -NH₂;
**R⁹** is selected from the group consisting of H and Me; and/or
**c** is 1.

12. The compound selected from Formula (I) or (II) for use according to claim 1 or 11, wherein the compound is selected from the group consisting of 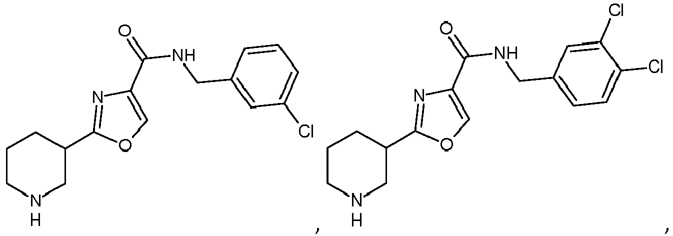 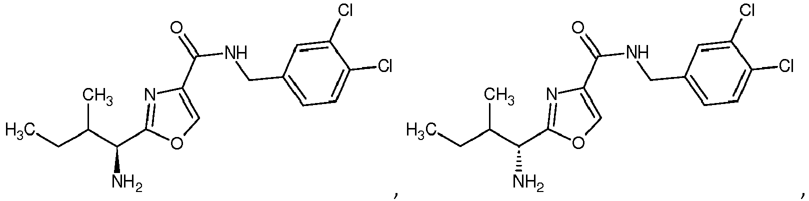 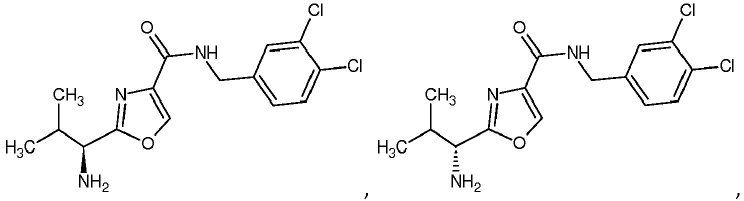 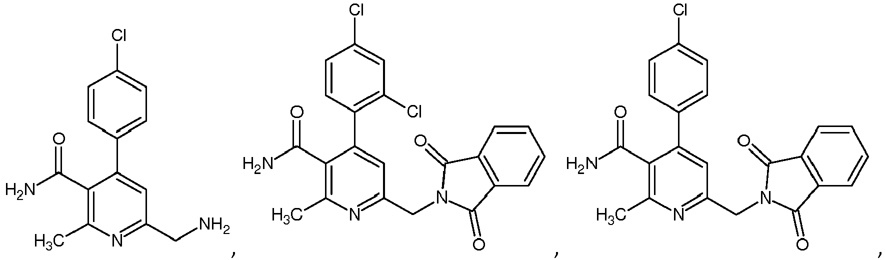 and

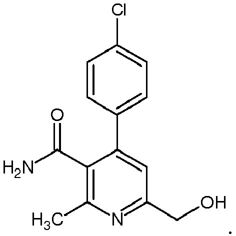

13. The TOMM6 (Translocase of Outer Membrane 6kDa subunit homologue)-interacting compound or composition for use according to any of claims 1 to 12, wherein the nervous system disease or disorder is selected from the group consisting of:
brain injuries; cerebrovascular diseases; consequences of cerebrovascular diseases; motor neuron disease; dementias; ALS; multiple sclerosis; traumatic brain injury; small-vessel cerebrovascular disease; familial forms of Alzheimer's Disease; sporadic forms of Alzheimer's Disease; vascular dementia; M. Parkinson; frontotemporal dementia; Parkinson linked to chromosome-17; diabetic neuropathy; symptoms of depression and depression-related symptoms, preferably anhedonia and anorexia; schizophrenia with dementia; Korsakoff's psychosis; Lewy Body diseases; progressive supranuclear palsy; corticobasal degeneration; Pick's disease; Huntington's disease; thalamic degeneration; Creutzfeld-Jacob disease; HIV Dementia; disorders with mitochondrial dysfunction, preferably neurodegenerative diseases of ageing; cognitive-related disorder; mild cognitive impairment; age-associated memory impairment; age-associated cognitive decline; vascular cognitive impairment; central and peripheral symptoms of atherosclerosis and ischemia; perivascular disease; renal dysfunction and renal failure; stress-related disorders; attention deficit disorders; attention deficit hyperactivity disorders; and memory disturbances in children.

14. A method for the identification of a TOMM6-interacting compound or composition, preferably a compound or composition for use in the treatment or prophylaxis of a nervous system disease or disorder, which modifies TOMM6 function and/or activity, the method comprising the steps of:
(i) providing a TOMM6 or TOMM6 homologue protein, preferably in the form of a homogenized cell solubilisate, preferably prepared from the frontal cortex or hippocampus of a mammalian animal, more preferably prepared from AD (Alzheimer's disease) model Tg2576 mice;
(ii) addition of a compound or composition of interest to the TOMM6 or TOMM6 homologue under physiological conditions that allow for the interaction of the compound of interest with the TOMM6 or TOMM6 homologue; and
(iii) identifying the absence or presence of an interaction of the compound of interest with the TOMM6 or TOMM6 homologue.

15. A method for the identification of a TOMM6-interacting compound or composition, preferably a compound or composition for use in the treatment or prophylaxis of a nervous system disease or disorder, which modifies TOMM6 and TOMM6 homolog protein amounts, the method comprising the steps of:
(i) providing isolated cells or isolated tissue, preferably isolated cells or tissue derived from the group of cells and tissues consisting of mammalian frontal cortex or hippocampus, mammalian brain, peripheral or circulating mammalian blood cells, human HEK cells, and AD (Alzheimer's disease) model Tg2576 mice, a non-human mammal, or a transgenic disease animal model, preferably Tg2576 mice;
(ii) optionally providing a control group from step (i) not to be contacted with a TOMM6- and/or TOMM6 homolog-interacting compound or composition;
(iii) contacting the isolated cells, isolated tissue, non-human mammal, or a transgenic disease animal model of step (i) with a TOMM6- and/or TOMM6 homolog-interacting compound or composition, preferably a TOMM6- and/or TOMM6 homolog-interacting compound or composition for use according to any of claims 1 to 12 in a physiologically effective concentration under physiological conditions suitable for the interaction of the interacting compound or composition;
(iv) contacting the mixture of isolated cells, isolated tissue, non-human mammal, or a transgenic disease animal model and the TOMM6- and/or TOMM6 homolog-interacting compound or composition of step (iii) with a quantifying agent specifically binding to TOMM6 protein and TOMM6 homologue protein under physiological conditions suitable for the specific binding of TOMM6 protein and TOMM6 homologue protein to the quantifying agent, preferably a quantifying agent selected from the group consisting of an antibody, an antibody fragment, antibody derivative or analogue, and a TOMM6- or TOMM6 homolog-binding compound according to any of claims 1 to 12, more preferably a labelled compound;
(v) optionally also contacting the control group of step (ii) with the quantifying agent of step (iv) under the same physiological conditions; and
(vi) determining the amount of quantifying agent bound specifically to the TOMM6 protein and TOMM6 homologue protein, optionally versus the control.

16. A method for the identification of a physiological effect of a TOMM6- and TOMM6 homologue-interacting compound or composition comprising the steps of:
(i) contacting isolated cells, isolated tissue, a non-human mammal, a transgenic disease animal model with a TOMM6- and/or TOMM6 homolog-interacting compound or composition, preferably a TOMM6- and/or TOMM6 homolog-interacting compound or composition for use according to any of claims 1 to 12 in a physiologically effective concentration under physiological conditions suitable for the interaction with the interacting compound or composition; and
(ii) optionally providing a control group not to be contacted with a TOMM6- and/or TOMM6 homolog-interacting compound or composition;
(iii) quantifying dysfunctional AGTR2 protein aggregates in the isolated cells, isolated tissue, non-human mammal, or transgenic disease animal model of step (i), and optionally in the control group of step (ii), preferably quantifying dysfunctional AGTR2 protein aggregates by an antibody, an antibody fragment, antibody derivative or antibody analogue.

17. A non-human transgenic animal expressing a transgene for TOMM6 or TOMM6 homolog, preferably from mouse, rat, bovine, zebrafish, chimpanzee, canis familiaris, or horse, preferably expressing a transgene for TOMM6 or a TOMM6 homolog having an amino acid sequence with at least 85 % sequence identity to one of SEQ ID NOs: 1 to 3, preferably under control of a neuron-specific promoter, preferably selected from the group consisting of the synapsin I (SYN) promoter, the calcium/calmodulin-dependent protein kinase II promoter, the tubulin alpha I promoter, the neuron-specific enolase promoter, the platelet-derived growth factor beta chain promoter, the tyrosine hydroxylase promoter (Thy1.2 regulatory element), a prion protein promoter, the cytomegalovirus immediate early promoter (CMV promoter) and hybrid promoters comprising the cytomegalovirus promoter fused to a neuron-specific promoter, more preferably the SYN promoter, the Thy1.1 promoter, the mouse prion protein promoter and the hamster prion protein promoter.

18. Use of a transgenic animal according to claim 17 for identifying TOMM6-interacting compounds or compositions for use in the treatment or prophylaxis of a disease or disorder selected from the group consisting of a nervous system disease or disorder from the group consisting of:
brain injuries; cerebrovascular diseases; consequences of cerebrovascular diseases; motor neuron disease; dementias; ALS; multiple sclerosis; traumatic brain injury; small-vessel cerebrovascular disease; familial forms of Alzheimer's Disease; sporadic forms of Alzheimer's Disease; vascular dementia; M. Parkinson; frontotemporal dementia; Parkinson linked to chromosome-17; diabetic neuropathy; symptoms of depression and depression-related symptoms, preferably anhedonia and anorexia; schizophrenia with dementia; Korsakoff's psychosis; Lewy Body diseases; progressive supranuclear palsy; corticobasal degeneration; Pick's disease; Huntington's disease; thalamic degeneration; Creutzfeld-Jacob disease; HIV Dementia; disorders with mitochondrial dysfunction, preferably neurodegenerative diseases of ageing; cognitive-related disorder; mild cognitive impairment; age-associated memory impairment; age-associated cognitive decline; vascular cognitive impairment; central and peripheral symptoms of atherosclerosis and ischemia; perivascular disease; renal dysfunction and renal failure; stress-related disorders; attention deficit disorders; attention deficit hyperactivity disorders; and memory disturbances in children.

19. Method for identifying TOMM6-interacting compounds or compositions for use in the treatment or prophylaxis of a nervous system disease or disorder comprising the steps of:
(i) administering a compound of interest to a transgenic animal according to claim 17 in a physiologically effective amount;
(ii) identifying a change in TOMM6 and TOMM6 homolog-related physiological parameter(s) in the transgenic animal; and
(iii) correlating the change in TOMM6 and TOMM6 homolog-related physiological parameter(s) in the transgenic animal with TOMM6- and TOMM6 homolog-interacting activity of the compound of interest.
